# EUROPÄISCHE PATENTSCHRIFT

(11) **EP 0 604 368 B1**
(45) Veröffentlichungstag und Bekanntmachung des Hinweises auf die Patenterteilung: **18.09.1996**
(21) Anmeldenummer: 93810879.2
(22) Anmeldetag: 14.12.1993
(51) Int. Cl.: C07C 281/02, C07C 281/06, C07C 243/14, C07C 243/18, C07D 215/48, C07D 213/79, C07C 271/22, C07D 295/12

(54) **Antiretrovirale hydrazinderivate**
Anti retroviral hydrazine derivatives
Dérivés antiviraux de hydrazine

(30) Priorität: 23.12.1992 CH 3942/92
(43) Veröffentlichungstag der Anmeldung: 29.06.1994
(73) Patentinhaber: CIBA-GEIGY AG, 4002 Basel (CH)
(72) Erfinder: Fässler, Alexander, Dr., CH-4104 Oberwil (CH); Bold, Guido, Dr., CH-5264 Gipf-Oberfrick (CH); Lang, Marc, Dr., F-68200 Mulhouse (FR); Bhagwat, Shripad, Dr., Scotch Plains, NJ 07076 (US)

(56) Entgegenhaltungen:
- EP-A- 0 491 538
- EP-A- 0 521 827
- WO-A-92/08698
- WO-A-93/18006
- DE-B- 1 254 461
- CHEMISCHE BERICHTE Bd. 108 , März 1975 , WEINHEIM DE Seiten 813 - 9 W. STREICHER ET. AL. 'Synthese eines Azaanalogen des Antibiotikums Negamycin'
- W. Streicher und H. Reinshagen, Chemische Berichte 108; 813-819 (1975)

## Beschreibung

Die Erfindung betrifft neue antiretroviral wirksame Verbindungen, Verfahren zur Herstellung dieser Verbindungen, neue Zwischenverbindungen zur Herstellung dieser Verbindungen, insbesondere solche mit antiretroviraler Wirksamkeit, pharmazeutische Präparate mit diesen Verbindungen, diese Verbindungen zur Anwendung in einem therapeutischen Verfahren zur Behandlung des menschlichen oder tierischen Körpers und die Verwendung dieser Verbindungen zur therapeutischen Behandlung des menschlichen oder tierischen Körpers oder zur Herstellung pharmazeutischer Präparate.

Eine ganze Reihe von Erkrankungen wird durch Retroviren hervorgerufen.

AIDS ist nach bisherigem Wissen eine durch das Retrovirus HIV ("Human Immunodeficiency Virus") hervorgerufene Erkrankung. Bereits heute gibt es Millionen von Infizierten. Die Erkrankung breitet sich stetig aus und führt praktisch stets zum Tod des Patienten.

Bisher wurden die Retroviren HIV-1 und HIV-2 (HIV steht für "Human Immunodeficiency Virus") als Ursache für die Erkrankung identifiziert. Für die Therapie ist die Suche nach Präparaten interessant, welche die Vermehrung des Virus selbst beeinträchtigen, ohne die intakten Zellen und Gewebe der Patienten zu schädigen.

Die retrovirale Protease ist ein proteolytisches Enzym, das wegen eines im aktiven Zentrum vorliegenden Aspartatrestes zu den Aspartat-Proteasen gezählt wird. Sie wirkt im Vermehrungscyclus einer Reihe von Retroviren an der Reifung neuer infektiöser Virionen innerhalb von infizierten Zellen mit.

HIV-1 und HIV-2 haben in ihrem Genom beispielsweise je einen Bereich, der für eine "gag-Protease" kodiert. Dieses Enyzm ist für die korrekte proteolytische Spaltung der Vorläuferproteine verantwortlich, die aus den für die "Group Specific Antigens" (gag) kodierenden Genomabschnitten hervorgehen. Hierbei werden die Strukturproteine des Viruskerns, englisch "Core", freigesetzt. Die "gag-Protease" selbst ist Bestandteil eines durch den pol-Genomabschnitt von HIV-1 und HIV-2 kodierten Vorläuferproteines, das auch die Abschnitte für die "Reverse Transcriptase" und die "Integrase" enthält und vermutlich autoproteolytisch gespalten wird.

Die "gag-Protease" spaltet das Hauptkernprotein ("Major Core Protein") p24 von HIV-1 und HIV-2 bevorzugt N-terminal von Prolinresten, z. B. in den bivalenten Radikalen Phe-Pro, Leu-Pro oder Tyr-Pro.

Aufgrund der zentralen Rolle der "gag-Protease" bei der Prozessierung der genannten "Core-Proteine" wird davon ausgegangen, dass eine wirksame Inhibierung dieses Enzyms in vivo den Zusammenbau reifer Virionen unterbindet, so dass entsprechende Inhibitoren therapeutisch eingesetzt werden können.

Generell gibt es zur Bekämpfung retroviraler Erkrankungen, wie AIDS, seit einiger Zeit Bemühungen, Verbindungen zur Verfügung zu stellen, die in vivo als Hemmstoffe gegen die genannten retroviralen gag-Proteasen, insbesondere die gag-Protease von HIV-1 (HIV-1-Protease), aber auch gegen die von HIV-2 oder weitere AIDS-Viren, wirksam sind.

Hauptziel ist derzeit, solche Verbindungen zur Verfügung zu stellen, die bestmögliche pharmakokinetische Eigenschaften aufweisen.

Voraussetzung für die therapeutische Wirksamkeit in vivo ist nämlich das Erreichen guter Bioverfügbarkeit, z. B. einer guten Resorbierbarkeit und/oder eines hohen Blutspiegels, auch bei enteraler, wie oraler Verabreichung, um so an den infizierten Zellen ausreichend hohe Konzentrationen zu erreichen.

Die Aufgabe der vorliegenden Erfindung besteht in der Bereitstellung von Verbindungen mit ausgezeichneter antiretroviraler Wirksamkeit, insbesondere sehr guter Bioverfügbarkeit.

Bei den erfindungsgemässen Verbindungen handelt es sich um Verbindungen der Formel 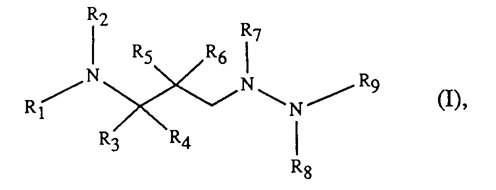 worin R₁ und R₉ unabhängig voneinander Wasserstoff, Acyl, unsubstituiertes oder substituiertes Alkyl; Sulfo; oder durch unsubstituiertes oder substituiertes Alkyl, Aryl oder Heterocyclyl substituiertes Sulfonyl bedeuten, mit der Massgabe, dass höchstens einer der Reste R₁ und R₉ Wasserstoff bedeutet; und R₂ und R₈ jeweils unabhängig voneinander Wasserstoff oder unsubstituiertes oder substituiertes Alkyl bedeuten;
R₃ und R₄ unabhängig voneinander Wasserstoff, unsubstituiertes oder substituiertes Alkyl, unsubstituiertes oder substituiertes Cycloalkyl oder Aryl bedeuten;
R₅ Acyloxy bedeutet;
R₆ Wasserstoff bedeutet;
und R₇ unsubstituiertes oder substituiertes Alkyl, unsubstituiertes oder substituiertes Cycloalkyl oder Aryl bedeutet;
sowie Salze der genannten Verbindungen, sofern salzbildende Gruppen vorliegen; wobei die Verbindung ausgeschlossen ist, worin R₁ und R₉ jeweils Acetyl bedeuten, R₂, R₃, R₄, R₆ und R₈ jeweils Wasserstoff bedeutet, R₅ Acetyloxy bedeutet und R₇ 2,2-[N-Ethoxycarbonylmethyl)-N-methyl]hydrazin-1-ylcarbonylmethyl bedeutet.

In der Beschreibung der vorliegenden Erfindung bedeutet der bei der Definition von Gruppen oder Resten, z. B. Niederalkyl, Niederalkoxy, Niederalkanoyl etc., verwendete Ausdruck "Nieder", dass die so definierten Gruppen oder Reste, falls nicht ausdrücklich anders definiert, bis maximal einschliesslich 7 und bevorzugt bis einschliesslich 4 C-Atome enthalten. Im Falle von Niederalkenyl oder Niederalkinyl liegen 2 bis 7, vorzugsweise 3 bis 7, insbesondere 3 oder 4 Kohlenstoffatome vor.

Sofern nichts anderes angegeben ist, liegen Substituenten innerhalb der Reste R₁, R₂, R₃, R₄, R₇, R₈ und/oder R₉ unabhängig voneinander ein- oder mehrfach, insbesondere ein- bis dreifach, z. B. einfach, vor.

Wird ein Rest, dessen Definition durch Rückbezug auf einen anderen Substituenten erfolgt, "unabhängig" von dem jeweils zur Definition herangezogenen Rest definiert, heisst das, dass bei Vorliegen beider Reste in einer Verbindung diese nicht identisch sein müssen; sie können jedoch auch identisch sein.

Die durch R₃ und R₄ beziehungsweise durch R₅ und R₆ substituierten Kohlenstoffatome in Verbindungen der Formel I können, sofern sie asymmetrisch sind, ebenso wie gegebenenfalls vorhandene weitere asymmetrische Kohlenstoffatome in der (R)-, (S)- oder (R,S)-Konfiguration vorliegen. Somit können die vorliegenden Verbindungen als Isomerengemische oder als reine Isomeren, insbesondere als Diastereomerengemische, Enantiomerenpaare oder reine Enantiomere vorliegen. Bevorzugt sind Verbindungen der Formel I, worin die durch R₃ und durch R₅ substituierten Kohlenstoffatome jeweils die (S)-Konfiguration aufweisen und weitere asymmetrische Kohlenstoffatome, falls vorhanden, unabhängig voneinander in der (R)-, (S)- oder (R,S)-Konfiguration vorliegen.

Die in der Beschreibung der vorliegenden Erfindung verwendeten allgemeinen Ausdrücke und Bezeichnungen haben vorzugsweise die folgenden Bedeutungen, sofern nichts anderes angegeben ist:

Acyl R₁ oder R₉ hat z. B. bis zu 25, vorzugsweise bis zu 19 C-Atome und ist in erster Linie die Acylgruppe einer Carbonsäure, eines Halbesters der Kohlensäure, einer unsubstituierten oder N-substituierten Carbaminsäure oder einer unsubstituierten oder substituierten Aminosäure.

Bevorzugte Acylgruppen R₁ oder R₉ einer Carbonsäure sind unsubstituiertes oder substituiertes Alkanoyl, Alkenoyl oder Alkinoyl mit bis zu 19 Kohlenstoffatomen, z. B. n-Decanoyl, oder vorzugsweise,
Niederalkanoyl, wie Formyl, Acetyl, Propionyl, Butyryl oder Pivaloyl; oder substituiertes Niederalkanoyl, worin vorzugsweise bis zu vier, insbesondere (ausser bei Halogen, welches bis zu dreimal als Substituent vorliegen kann) bis zu zwei Substituenten vorliegen können, insbesondere ein Substituent (ausser bei Halogen, welches bis zu dreimal als Substituent vorliegen kann) vorliegen kann, wobei die Substituenten in erster Linie aus
Cycloalkyl, welches vorzugsweise 3 - 7 Kohlenstoffatome hat, insbesondere in Cycloalkylniederalkanoyl, worin Niederalkanoyl wie oben definiert ist, z. B. Cycloalkylcarbonyl, z.B. mit insgesamt 4 - 8 Kohlenstoffatomen, wie Cyclopropyl-, Cyclobutyl-, Cyclopentyl- oder Cyclohexylcarbonyl, oder 2-Cyclohexyl- oder 2-Cyclopentylacetyl,
Cycloalkenyl, welches vorzugsweise 3 - 7 Kohlenstoffatome hat, insbesondere in Cycloalkenylniederalkanoyl, wie Cycloalkenylcarbonyl, z.B. mit 4 - 8 Kohlenstoffatomen, wie 1-Cyclohexenylcarbonyl, 1,4-Cyclohexadienylcarbonyl oder 1-Cyclohexenylacetyl oder 1,4-Cyclohexadienylacetyl,
Bicycloalkyl, welches vorzugsweise 5 - 10 Kohlenstoffatome enthält, insbesondere in Bicycloalkylniederalkanoyl, beispielsweise Bicycloalkylcarbonyl, z.B. mit 8 - 11 Kohlenstoffatomen, wie Decahydronaphthyl-2-carbonyl, Bicyclohexyl-, Bicycloheptyl-, Bicyclooctyl-, Bicyclononyl- oder Bicyclodecyl-acetyl oder -3-propionyl,
Bicycloalkenyl, vorzugsweise mit 8 - 12 Kohlenstoffatomen, insbesondere in Bicycloalkenylcarbonyl, wie 5-Norbornen-2-ylcarbonyl oder Bicyclo[2.2.2]octen-2-ylcarbonyl,
Tricycloalkyl, worin Tricycloalkyl z. B. 8 - 10 Kohlenstoffatome enthält, insbesondere in Tricycloalkylniederalkanoyl, beispielsweise Tricycloalkylcarbonyl, z.B. mit 8 - 11 Kohlenstoffatomen, wie 1- oder 2-Adamantylcarbonyl,
Aryl, welches vorzugsweise 6 - 14 Ringkohlenstoffatome hat, wie in Phenyl, Indenyl, Indanyl, Naphthyl, Anthryl, Phenanthryl, Acenaphthyl oder Fluorenyl, und unsubstituiert oder insbesondere durch Niederalkyl, z. B. Methyl, Ethyl oder Propyl, Halogenniederalkyl, z. B. Trifluormethyl, Phenyl, 1- oder 2-Naphthyl, Hydroxy, Niederalkoxy, z. B. Methoxy, Carbamoylniederalkoxy, N-Niederalkylcarbamoylniederalkoxy oder N,N-Diniederalkylcarbamoylniederalkoxy, Amino, Mono- oder Diniederalkylamino, Niederalkanoylamino, z. B. Pivaloylamino, Halogen, z. B. Fluor, Chlor oder Brom, Carboxy, Niederalkoxycarbonyl, wie tert-Butoxycarbonyl, Phenyl-, Naphthyl- oder Fluorenylniederalkoxycarbonyl, wie Benzyloxycarbonyl, Niederalkanoyl, Sulfo, Niederalkylsulfonyl, z. B. Methylsulfonyl, Phosphono, Hydroxyniederalkoxy-phosphoryl oder Diniederalkoxyphosphoryl, Carbamoyl, Mono- oder Diniederalkylcarbamoyl, Sulfamoyl, Mono- oder Diniederalkylaminosulfonyl, Nitro und/oder Cyano ein- oder bis zu dreifach substituiert sein kann, insbesondere in Arylniederalkanoyl, worin Phenyl einfach oder auch bis zu dreimal vorliegen kann, z. B. in Diphenyl-, Dibenzyl- oder Triphenylniederalkanoyl, wie Diphenyl-, Dibenzyl- oder Triphenylacetyl, und worin Niederalkanoyl unsubstituiert oder ferner substituiert z. B. durch Carboxy, Niederalkoxycarbonyl, z. B. Methoxy-, Ethoxy-, n-Propoxy-, Isopropoxy-, n-Butoxy-, Isobutoxy-, sec-Butoxy-, tert-Butoxy-, n-Pentoxy-, Isopentoxy-, Neopentoxy-, tert-Pentoxy-, n-Hexoxy-, Isohexoxy- oder n-Heptoxycarbonyl, Arylniederalkoxycarbonyl, worin Aryl 6 - 12 Kohlenstoffatome hat, z. B. Benzyloxycarbonyl, Carbamoyl, durch einen oder zwei aus Niederalkyl, wie Methyl, Ethyl, Propyl, Isopropyl, n-Butyl, Isobutyl, sec-Butyl, oder tert-Butyl, z. B. in N-Methylcarbamoyl, N-n-Butylcarbamoyl oder N,N-Dimethylcarbamoyl, aus Carboxyniederalkyl oder Niederalkoxycarbonyl-niederalkyl, z. B. als Carboxymethylcarbamoyl (Glycinylcarbonyl) oder als tert-Butoxycarbonylmethylcarbamoyl, aus Diniederalkylaminoniederalkyl, z. B. 2-Dimethylaminoethyl, aus Hydroxyniederalkyl, z. B. Hydroxymethyl oder Hydroxyethyl, und aus Diniederalkoxyniederalkyl, z. B. 2-(2,2-Dimethoxyethyl), ausgewählte Reste am Stickstoff substituiertes Carbamoyl und/oder Cyano sein kann und unverzweigt oder verzweigt ist, in erster Linie ausgewählt aus Phenylniederalkanoyl, wie Benzoyl, Phenylacetyl oder 3-Phenylpropionyl, welche am Phenylring unsubstituiert, mono- oder mehrfachsubstituiert vorliegen können, beispielsweise durch Niederalkyl, z. B. Methyl, Phenyl, Halogen, z. B. Fluor oder Chlor, Hydroxy, Niederalkoxy, z. B. Methoxy, und/oder Nitro, wie 4-Chlor-, 4-Methoxy- oder 4-Nitrobenzoyl, Naphthylcarbonyl, wie α- oder β-Naphthylcarbonyl oder über beide Carbonylgruppen an die Aminogruppe gebundenem 1,8-Naphthalindicarbonyl, Indenylcarbonyl, wie 1-, 2- oder 3-Indenylcarbonyl, Indanylcarbonyl, wie 1- oder 2-Indanylcarbonyl, Phenanthrenylcarbonyl, wie 9-Phenanthrenylcarbonyl, α-Naphthylacetyl, β-Naphthylacetyl, Niederalkylphenyl-acetyl, wie 4-Methylphenylacetyl, Niederalkoxyphenylacetyl, wie 4-Methoxyphenylacetyl, 3-(p-Hydroxyphenyl)-propionyl, Diphenylacetyl, Di-(4-methoxyphenyl)-acetyl, Triphenylacetyl, 2,2-Dibenzylacetyl, 3-α- oder 3-β-Naphthylpropionyl, Phenylniederalkanoyl, worin der Niederalkanoylrest durch Carbamoyl substituiert ist, z.B. 2-Carbamoyl-3-phenylpropionyl, wie 2(R,S)-Carbamoyl-3-phenylpropionyl, 3-α-Naphthyl-2-carbamoylpropionyl, 3-Phenyl- oder 3-α-Naphthyl-2-tert-butylcarbamoylpropionyl, 3-Phenyl- oder 3-α-Naphthyl-2-(2-dimethylaminoethyl)carbamoyl-propionyl, 3-α-Naphthyl-2-(carboxy- oder tert-butoxycarbonyl)methylcarbamoyl-propionyl, 3-Phenyl- oder 3-α-Naphthyl-2-(3-hydroxy-2-propyl)carbamoyl-propionyl, 3-Phenyl- oder 3-α-Naphthyl-2-(2,2-dimethoxyethyl)-carbamoylpropionyl, und 3-Phenyl- oder 3-α-Naphthyl-2(5-amino-5-carboxypentyl)-carbamoylpropionyl, in erster Linie Phenylniederalkanyol, wie Phenylacetyl, oder Phenyl-niederalkanoyl, worin der Niederalkanoylrest durch Carbamoyl substituiert ist, wie 2(R,S)-Carbamoyl-3-phenylpropionyl,
Heterocyclyl, welches vorzugsweise ein einfaches oder doppeltes Ringsystem mit 3 - 10 Ringatomen ist, über ein Kohlenstoff- oder insbesondere Stickstoffatom gebunden ist und bis zu 3 weitere Heteroatome ausgewählt aus Sauerstoff, Stickstoff, Schwefel und mit 1 oder 2 Sauerstoffatomen verknüpftem Schwefel enthält, wobei das genannte Ringsystem zusätzlich mit 1 oder 2 Phenyl- oder Naphthylresten anneliert sein kann, wobei Naphthyl auch über zwei Seiten ankondensiert sein kann, oder mit 1 oder 2 Cycloalkylresten anneliert sein kann, wobei Cycloalkyl vorzugsweise 5 - 7 Ringatome hat; und ungesättigt oder auch teilweise oder ganz gesättigt sein kann, z. B. Thienyl, Furyl, Pyrrolyl, Imidazolyl, Pyrazolyl, Oxazolyl, Thiazolyl, Tetrazolyl, Pyridyl, Pyrazinyl, Pyrimidinyl, Pyridazinyl, Indolyl, Benzimidazolyl, Chinolyl, Isochinolyl, 3,1-Benzfuranyl, Cyclohexa[b]pyrrolyl, Cyclohexa[b]pyridyl, Cyclohexa[b]pyrazinyl, Cyclohexa[b]pyrimidinyl, Pyrrolidinyl, Pyrrolinyl, Imidazolidyl, Piperidyl, Piperazinyl, Morpholinyl, Thiomorpholinyl, S,S-Dioxo-thiomorpholinyl, Indolinyl, Isoindolinyl, 4,5,6,7-Tetrahydroindolyl, 1,2,3,4-Tetrahydrochinolyl oder 1,2,3,4-Tetrahydroisochinolyl, wobei Heterocyclyl, z.B. einer der zuletzt genannten Reste, unsubstituiert oder durch Niederalkyl, z. B. Methyl, Phenyl, 1- oder 2-Naphthyl, Phenylniederalkyl, z. B. Benzyl, Hydroxyniederalkyl, z. B. Hydroxymethyl oder 2-Hydroxyethyl, Niederalkoxyniederalkyl, z. B. Methoxymethyl oder 2-Methoxyethyl, Niederalkanoyloxy-niederalkyl, z. B. Acetyloxymethyl, Phenyl- oder Napthyl-niederalkanoyloxy-niederalkyl, z. B. Benzoyloxy-, Phenylacetyloxy-oder 1- oder 2-Naphthoyloxy-methyl, -2-ethyl oder -2-(2,2-dimethylethyl), Niederalkoxycarbonyloxy-niederalkyl, z. B. tert-Butyloxycarbonyloxyniederalkyl, Phenylniederalkoxycarbonyloxy-niederalkyl, z. B. 2-Benzyloxycarbonyloxyethyl, Aminoniederalkyl, z. B. Aminomethyl, Hydroxy, Niederalkoxy, z. B. Methoxy oder Ethoxy, Amino, Niederalkylamino, z. B. Methyl-, Ethyl- oder tert-Butylamino, Diniederalkylamino, z. B. Dimethyl- oder Diethylamino, Carboxy, Niederalkoxycarbonyl, z. B. Methoxy-, Isopropoxy-, sec-Butoxy- oder tert-Butoxy-carbonyl, Phenyl- oder Napthylniederalkoxycarbonyl, z. B. Benzyloxycarbonyl, Halogen, z. B. Fluor, Chlor, Brom oder Jod, insbesondere Chlor oder Brom, Niederalkanoyl, z. B. Acetyl oder Pivaloyl, Carbamoyl, Mono- oder Diniederalkylcarbamoyl, z. B. N-Methylcarbamoyl, N-n-Butylcarbamoyl oder N,N-Dimethylcarbamoyl, Hydroxy- oder Carboxyniederalkylcarbamoyl, z. B. Hydroxy- oder Carboxymethylcarbamoyl oder Hydroxy- oder Carboxyethylcarbamoyl, durch Nitro, Oxo und/oder durch Cyano substituiert sind; insbesondere in Heterocyclylniederalkanoyl, worin Niederalkanoyl unsubstituiert oder unabhängig durch einen der oben unter Arylniederalkanoyl R₁ oder R₉ definierten Substituenten substituiert ist; wobei Heterocyclylniederalkanoyl insbesondere ausgewählt ist aus Pyrrolylcarbonyl, z. B. 2- oder 3-Pyrrolylcarbonyl, Thienylcarbonyl, wie 2-Thienylcarbonyl, Furylcarbonyl, wie 2-Furylcarbonyl, Indolylcarbonyl, wie 2-, 3- oder 5-Indolylcarbonyl, 4,5,6,7-Tetrahydroindolyl-2-carbonyl, Chinolylniederalkanoyl, z. B. Chinolylcarbonyl, wie 2-, 3- oder 4-Chinolylcarbonyl, Isochinolylcarbonyl, wie 1-, 3- oder 4-Isochinolylcarbonyl, Piperidylcarbonyl, wie Piperidinocarbonyl oder 2-, 3- oder 4-Piperidylcarbonyl, Piperazinylcarbonyl, wie Piperazin-1-yl-carbonyl, Morpholinylniederalkanoyl, z. B. Morpholinoniederalkanoyl, wie Morpholinocarbonyl, Thiomorpholinyl-niederalkanoyl, z. B. Thiomorpholinoniederalkanoyl, wie Thiomorpholinocarbonyl, S,S-Dioxothiomorpholinylcarbonyl, wie S,S-Dioxothiomorpholinocarbonyl, 1,2,3,4-Tetrahydrochinolylcarbonyl, wie 1,2,3,4-Tetrahydrochinolyl-2-, - 3- oder -4-carbonyl, 1,2,3,4-Tetrahydroisochinolylcarbonyl, wie 1,2,3,4-Tetrahydroisochinolyl-1-, -3- oder 4-carbonyl, Tetrazolylniederalkanoyl, wie 3-(Tetrazol-1-yl)-propionyl, und Pyridylniederalkanoyl, z. B. Pyridylcarbonyl, wie 2-, 3- oder 4-Pyridylcarbonyl, oder Pyridylacetyl, wie 2-, 3- oder 4-Pyridylacetyl, in allererster Linie ausgewählt aus Morpholinocarbonyl, Thiomorpholinocarbonyl, Chinolin-2-ylcarbonyl, 3-(Tetrazol-1-yl)-propionyl, 2-Pyridylcarbonyl oder 2- oder 3-Pyridylacetyl,
Hydroxy, insbesondere in Hydroxyniederalkanoyl, wie 3-Hydroxypropionyl oder 2-Hydroxy-3-methylpentanoyl,
Hydroxyniederalkoxy, insbesondere in Hydroxyniederalkoxy-niederalkanoyl, wie 3-Hydroxy-n-propoxycarbonyl,
Niederalkoxy, insbesondere in Niederalkoxyniederalkanoyl, z. B. Niederalkoxyacetyl oder Niederalkoxypropionyl, wie Methoxyacetyl, Ethoxyacetyl oder 3-Methoxypropionyl,
Niederalkoxyniederalkoxy, insbesondere in Niederalkoxyniederalkoxyniederalkanoyl, wie 2-Methoxymethoxy-3-methyl-pentanoyl,

Niederalkanoyloxy, insbesondere in Niederalkanoyloxyniederalkanoyl, worin Niederalkanoyloxy z. B. Acetoxy, Propionyloxy, Butyroxy, Isobutyroxy oder Pivaloyloxy bedeutet, wie Acetoxyacetyl oder 3-Acetoxypropionyl,
Amino, welches nicht in α- oder β-Stellung zur bindenden Carboxygruppe des Acylrestes vorliegt, z.B. in Aminoniederalkanoyl, worin die Aminogruppe nicht in α- oder β-Stellung vorliegt, wie 5-Aminopentanoyl,
Niederalkanoylamino, welches nicht in α- oder β-Stellung zur bindenden Carboxygruppe des Acylrestes vorliegt, insbesondere in Niederalkanoylamino-niederalkanoyl, worin die Aminogruppe nicht in α- oder β-Stellung des Niederalkanoylrestes steht, wie 5-Pivaloylamino-pentanoyl,
Niederalkoxycarbonylamino, welches nicht in α- oder β-Stellung zur bindenden Carboxygruppe des Acylrestes vorliegt, insbesondere in Niederalkoxycarbonylamino-niederalkanoyl, worin die Aminogruppe nicht in α- oder β-Stellung des Niederalkanoylrestes steht, wie 5-(tert-Butoxycarbonylamino)-pentanoyl,
Phenylniederalkoxycarbonylamino, welches nicht in α- oder β-Stellung zur bindenden Carboxygruppe des Acylrestes vorliegt, insbesondere in Phenylniederalkoxycarbonylamino-niederalkanoyl, worin die Aminogruppe nicht in α- oder β-Stellung des Niederalkanoylrestes steht, wie 5-Benzyloxycarbonylaminopentanoyl oder 6-Benzyloxycarbonylaminohexanoyl,
durch Heterocyclylniederalkanoyl, worin Heterocyclyl unabhängig wie oben als Substituent von Niederalkanoyl R₁ oder R₉ definiert ist, insbesondere durch N-Morpholino- oder N-Thiomorpholinocarbonyl, am Aminostickstoff substituiertes Amino, insbesondere in Heterocyclylniederalkanoylaminoniederalkanoyl, z. B. N-Morpholino- oder N-Thiomorpholino-carbonylamino-niederalkanoyl, wie N-Morpholino- oder N-Thiomorpholino-carbonylamino-acetyl,
vorzugsweise bis zu drei Halogenatome, insbesondere in Halogenniederalkanoyl, welches bis zu 3 Halogenatome enthält, z. B. α-Halogenacetyl, wie α-Fluor-, α-Chlor-, α-Brom-, α-Iod-, α,α,α Trifluor- oder α,α,α-Trichloracetyl, oder Halogenpropionyl, wie β-Chlor- oder β-Brompropionyl,
Carboxy, insbesondere in Carboxyniederalkanoyl, z. B. Carboxyacetyl oder β-Carboxypropionyl,
Niederalkoxycarbonyl, insbesondere in Niederalkoxycarbonylniederalkanoyl, z. B. Niederalkoxycarbonylacetyl oder Niederalkoxycarbonylpropionyl, wie Methoxycarbonylacetyl, 3-Methoxycarbonylpropionyl, Ethoxycarbonylacetyl, 3-Ethoxycarbonylpropionyl oder 3-tert-Butoxycarbonylpropionyl,
Sulfonyl, insbesondere in Sulfonylniederalkanoyl, wie 3-Sulfonylpropionyl,
Carbamoyl, insbesondere in Carbamoylniederalkanoyl, wie Carbamoylacetyl oder 3-Carbamoylpropionyl,
Niederalkylcarbamoyl, insbesondere in Niederalkylcarbamoylniederalkanoyl, z. B. Niederalkylcarbamoylacetyl oder Methylcarbamoylniederalkanoyl, wie Methylcarbamoylacetyl,
Diniederalkylcarbamoyl, insbesondere in Diniederalkylcarbamoylniederalkanoyl, z. B. Diniederalkylcarbamoylacetyl oder Dimethylcarbamoylniederalkanoyl, wie Dimethylcarbamoylacetyl,
durch einen aus Ethylen, Trimethylen, Tetramethylen und Pentamethylen ausgewählten Rest, worin ein Kohlenstoffatom durch Stickstoff, Sauerstoff, Schwefel oder mit Sauerstoff ein- oder zweifach substituiertem Schwefel ersetzt sein kann, am Stickstoff substituiertes Carbamoyl, insbesondere in entsprechend N-substituiertem Carbamoylniederalkanoyl, wobei der so gebildete Rest auch ganz oder teilweise ungesättigt sein kann, z. B. als Piperidino-, Pyrazin-1-yl-, Piperazin-1-yl-, Pyrimidin-1-yl-, Pyridazin-1-yl-, Morpholino-, Thiomorpholino- oder S,S-Dioxothiomorpholinocarbonylniederalkanoyl, wie in Morpholinocarbonyl-acetyl, 3-(Morpholinocarbonyl)-propionyl oder 3-(Morpholinocarbonyl)-2-isobutyl-propionyl,
N-Heterocyclylniederalkyl-carbamoyl oder N-Niederalkyl-N-heterocyclylniederalkylcarbamoyl, insbesondere in N-Heterocyclylniederalkyl-carbamoyl-niederalkanoyl oder N-Niederalkyl-N-heterocyclylniederalkylcarbamoylniederalkanoyl, worin Heterocyclyl vorzugsweise aus Pyrrolyl, Furyl, Thienyl, Imidazolyl, Pyrazolyl, Oxazolyl, Thiazolyl, Pyridyl, Pyrazinyl, Pyrimidinyl, Indolyl, Chinolyl und Isochinolyl, die auch ganz oder teilweise gesättigt sein können, aus Morpholinyl und Thiomorpholinyl ausgewählt ist, wie N-Methyl-2-(N-2-pyridylmethyl)-carbamoylacetyl, 2-(N-Morpholinoniederalkylcarbamoyl)-niederalkanoyl, wie 2(R,S)-(N-(2-Morpholinoethyl)-carbamoyl-3-methylbutyryl, oder 2-(N-(Pyridylniederalkyl)-carbamoyl)-niederalkanoyl, wie (2(R,S)-(N-(2-Pyridylmethyl)-carbamoyl)-3-methyl)-butyryl,
Oxo, insbesondere in Oxoniederalkanoyl, wie Acetoacetyl oder Propionylacetyl,
und Cyano, insbesondere in Cyanoniederalkanoyl, wie Cyanoacetyl, 2- oder 3-Cyanopropionyl oder 2-, 3- oder 4-Cyanobutyryl, ausgewählt sind;

Niederalkenoyl mit 3 - 7 Kohlenstoffatomen, bevorzugt mit 3 - 4 Kohlenstoffatomen, wie Acryloyl, Vinylacetyl, Crotonoyl oder 3- oder 4-Pentenoyl, oder

Niederalkinoyl mit 3 - 7, vorzugsweise 3 oder 4 Kohlenstoffatomen, z. B. Propioloyl oder 2- oder 3-Butinoyl.

Bevorzugte Acylgruppen R₁ oder R₉ eines Halbesters der Kohlensäure sind
Niederalkoxycarbonyl, z. B. Methoxy-, Ethoxy-, Isopropoxy-, Isobutoxy- oder tert-Niederalkoxycarbonyl, wie tert-Butoxycarbonyl oder Isobutoxycarbonyl,
2-Halogenniederalkoxycarbonyl, wie 2-Chlor-, 2-Brom-, 2-Iod- oder 2,2,2-Trichlorethoxycarbonyl,
Arylniederalkoxycarbonyl, z. B. Arylmethoxy-carbonyl, worin Aryl 6 bis 14 Kohlenstoffatome hat und beispielsweise Phenyl, Biphenylyl, 1- oder 2-Naphthyl, Fluorenyl oder durch Niederalkyl, z. B. Methyl oder tert-Butyl, Hydroxy, Niederalkoxy, z. B. Methoxy, Ethoxy oder tert-Butoxy, Halogen, z. B. Chlor oder Brom, und/oder Nitro mono- oder mehrfach substituiertes Phenyl ist, z. B. Phenylniederalkoxycarbonyl, wie Benzyloxycarbonyl oder 4-Methoxybenzyloxycarbonyl,
Heterocyclylniederalkoxycarbonyl, worin Heterocyclyl vorzugsweise aus Pyrrolyl, Furyl, Thienyl, Imidazolyl, Pyrazolyl, Oxazolyl, Thiazolyl, Pyridyl, Pyrazinyl, Pyrimidinyl, Indolyl, Chinolyl und Isochinolyl, die auch ganz oder teilweise gesättigt vorliegen können, aus Morpholinyl und aus Thiomorpholinyl ausgewählt ist und unsubstituiert oder substituiert, insbesondere durch Niederalkyl, wie Methyl, sein kann, wie Furylmethoxycarbonyl, Tetrahydrofurylniederalkoxycarbonyl, wie 2-Tetrahydrofuryl-methoxycarbonyl, 2-Morpholino-ethoxycarbonyl, oder 2-, 3- oder 4-Pyridylmethoxycarbonyl,
Niederalkenyloxycarbonyl, worin vorzugsweise der Niederalkenylrest an den bindenden Sauerstoff über ein gesättigtes Kohlenstoffatom gebunden ist, wie Allyloxycarbonyl,
Niederalkoxyniederylkoxycarbonyl, wie 2-Methoxyethoxycarbonyl, oder
(Niederalkoxyniederalkoxy)niederalkoxycarbonyl, wie 2-(2-Methoxyethoxy)ethoxycarbonyl.

Bevorzugte Acylgruppen R₁ oder R₉ einer unsubstituierten oder substituierten Carbaminsäure sind
Carbamoyl oder
unsubstituiertes oder substituiertes N-Alkyl- oder N,N-Dialkylcarbamoyl, worin der Alkylrest bis zu 12 Kohlenstoffatome aufweist, vorzugsweise unsubstituiertes oder substituiertes Niederalkyl- oder Diniederalkylcarbamoyl, wie Methyl-, Ethyl-, Propyl-, tert-Butyl-, Dimethyl-, Diethyl- oder Di-n-propyl-carbamoyl, wobei die Substituenten aus Phenyl, z. B. in Benzylcarbamoyl, N-Phenylniederalkyl-N-niederalkylcarbamoyl, wie N-Benzyl-N-methylcarbamoyl, oder Dibenzylcarbamoyl, Heterocyclyl, welches unabhängig wie als Substituent von Niederalkanyol R₁ und R₉ definiert ist, vorzugsweise Pyridyl, wie 2-, 3- oder 4-Pyridyl, in erster Linie in N-Heterocyclylniederalkyl-N-niederalkylcarbamoyl, z. B. N-Pyridylniederalkyl-N-niederalkyl-carbamoyl, wie N-(2-, 3- oder 4-Pyridylmethyl)-N-methyl-carbamoyl, oder in N-Heterocylclyl-niederalkyl-carbamoyl, z. B. 2- oder 3-Pyridylniederalkylaminocarbonyl, wie 2- oder 3-Pyridylmethylaminocarbonyl, Hydroxy, z. B. in Hydroxymethyl, 2-Hydroxyethyl, 3-Hydroxypropyl, 2-Hydroxypropyl, und Niederalkoxy, bevorzugt in Niederalkoxyniederalkyl, z. B. Methoxymethyl oder 2-Methoxyethyl, ausgewählt sind.

Bevorzugte Acylgruppen R₁ oder R₉ einer unsubstituierten oder substituierten Aminosäure werden gebildet durch die über das Carbonyl ihrer Carboxygruppe gebundenen Aminosäurereste einer α- oder β-Aminosäure, insbesondere
einer natürlichen α-Aminosäure mit der L-Konfiguration, wie sie normalerweise in Proteinen vorkommen, oder eines Epimeren einer solchen Aminosäure, d. h. mit der unnatürlichen D-Konfiguration, oder deren D,L-Isomerengemisch, eines Homologen einer solchen Aminosäure, z. B. worin die Aminosäurenseitenkette um eine oder zwei Methylengruppen verlängert oder verkürzt ist, worin die Aminogruppe in β-Stellung vorliegt und/oder worin eine Methylgruppe durch Wasserstoff ersetzt ist, einer substituierten aromatischen Aminosäure, worin der aromatische Rest 6 - 14 Kohlenstoffatome hat, z. B. eines substituierten Phenylalanins oder Phenylglycins, worin der Phenyl-Substituent Niederalkyl, z. B. Methyl, Hydroxy, Niederalkoxy, z. B. Methoxy, Niederalkanoyloxy, z. B. Acetoxy, Amino, Niederalkylamino, z. B. Methylamino, Diniederalkylamino, z. B. Dimethylamino, Niederalkanoylamino, z. B. Acetylamino oder Pivaloylamino, Niederalkoxycarbonylamino, z. B. tert-Butoxycarbonylamino, Arylmethoxycarbonylamino, worin Aryl vorzugsweise 6 - 14 Kohlenstoffatome hat, z. B. Benzyloxycarbonylamino oder 9-Fluorenylmethoxycarbonylamino, Halogen, z. B. Fluor, Chlor, Brom oder Jod, Carboxy und/oder Nitro sein kann und ein- oder mehrfach vorkommt, eines benzannelierten Phenylalanins oder Phenylglycins, wie α-Naphthylalanins, oder eines hydrierten Phenylalanins oder Phenylglycins, wie Cyclohexylalanins oder Cyclohexylglycins.

Diese Aminosäuren können an freien Amino- oder Hydroxyfunktionen, vorzugsweise an einer freien Aminofunktion, durch einen der oben unter Acyl R₁ oder R₉ als Acylgruppe einer Carbonsäure, eines Halbesters der Kohlensäure oder einer unsubstituierten oder N-substituierten Carbaminsäure genannten Reste oder einen der unten unter unsubstituiertes oder substituiertes Alkyl R₁, R₂, R₈ oder R₉ genannten Reste substituiert sein.

Besonders bevorzugt ist das über seine Carbonylgruppe gebundene Radikal einer Aminosäure ausgewählt aus Glycin (H-Gly-OH), Alanin (H-Ala-OH), Valin (H-Val-OH), Norvalin (α-Aminovaleriansäure), Leucin, (H-Leu-OH), Isoleucin (H-Ile-OH), Norleucin (α-Aminohexansäure, H-Nle-OH), Serin (H-Ser-OH), Homoserin (α-Amino-γ-hydroxybuttersäure), Threonin (H-Thr-OH), Methionin (H-Met-OH), Cystein (H-Cys-OH), Prolin (H-Pro-OH), trans-3- und trans-4-Hydroxyprolin, Phenylalanin (H-Phe-OH), Tyrosin (H-Tyr-OH), 4-Aminophenylalanin, 4-Chlorphenylalanin, 4-Carboxyphenylalanin, β-Phenylserin (β-Hydroxyphenylalanin), Phenylglycin, α-Naphthylalanin (H-Nal-OH), Cyclohexylalanin (H-Cha-OH), Cyclohexylglycin, Tryptophan (H-Trp-OH), Indolin-2-carbonsäure, 1,2,3,4-Tetrahydroisochinolin-3-carbonsäure, Asparaginsäure (H-Asp-OH), Asparagin (H-Asn-OH), Aminomalonsäure, Aminomalonsäure-monoamid, Glutaminsäure (H-Glu-OH), Glutamin (H-Gln-OH), Histidin (H-His-OH), Arginin (H-Arg-OH), Lysin (H-Lys-OH), δ-Hydroxylysin, Ornithin (α,δ-Diaminovaleriansäure), 3-Aminopropansäure, α,γ-Diaminobuttersäure und α,β-Diaminopropionsäure, besonders bevorzugt der Rest einer aliphatischen Aminosäure ausgewählt aus Valin, Alanin, Leucin und Isoleucin, oder einer Aminosäure ausgewählt aus Glycin, Glutaminsäure und Asparagin, wobei (ausser Glycin) jede der genannten Aminosäuren in der D-, L- oder (D,L)-, vorzugsweise (ausser Val, das auch in der (D)- oder (D,L)-Form vorliegen kann) in der L-Form vorliegen kann,
die α-Aminogruppe unsubstituiert oder ein- oder zweifach N-alkyliert, z. B. durch Niederalkyl, wie Methyl oder n-Propyl, oder durch Aminoniederalkyl, wie 3-Aminopropyl, oder N-acyliert durch einen der oben unter Acyl R₁ als Rest einer Carbonsäure, eines Halbesters der Kohlensäure oder einer unsubstituierten oder N-substituierten Carbaminsäure genannten Acylreste, vorzugsweise durch Niederalkanoyl, wie Acetyl, durch Arylniederalkanoyl, worin Aryl ausgewählt ist aus Phenyl, Indenyl, Indanyl, Naphthyl, Anthryl, Phenanthryl, Acenaphthyl und Fluorenyl, und unsubstituiert oder insbesondere durch Niederalkyl, z. B. Methyl, Ethyl oder Propyl, Halogenniederalkyl, z. B. Trifluormethyl, Phenyl, 1- oder 2-Naphthyl, Hydroxy, Niederalkoxy, z. B. Methoxy, Carbamoylniederalkoxy, N-Niederalkylcarbamoylniederalkoxy oder N,N-Diniederalkylcarbamoylniederalkoxy, Amino, Mono- oder Diniederalkylamino, Niederalkanoylamino, z. B. Pivaloylamino, Halogen, z. B. Fluor, Chlor oder Brom, Carboxy, Niederalkoxycarbonyl, wie tert-Butoxycarbonyl, Phenyl-, Naphthyl- oder Fluorenylniederalkoxycarbonyl, wie Benzyloxycarbonyl, Niederalkanoyl, Sulfo, Niederalkylsulfonyl, z. B. Methylsulfonyl, Phosphono, Hydroxy-niederalkoxy-phosphoryl oder Diniederalkoxyphosphoryl, Carbamoyl, Mono- oder Diniederalkylcarbamoyl, Sulfamoyl, Mono- oder Diniederalkylaminosulfonyl, Nitro und/oder Cyano ein- oder bis zu dreifach substituiert sein kann, und Niederalkanoyl unsubstituiert oder substituiert insbesondere durch Carboxy, Niederalkoxycarbonyl, z. B. Methoxy-, Ethoxy-, n-Propoxy-, Isopropoxy-, n-Butoxy-, Isobutoxy-, sec-Butoxy-, tert-Butoxy-, n-Pentoxy-, Isopentoxy-, Neopentoxy-, tert-Pentoxy-, n-Hexoxy-, Isohexoxy- oder n- Heptoxycarbonyl, Arylniederalkoxycarbonyl, worin Aryl 6 - 12 Kohlenstoffatome hat, z. B. Benzyloxycarbonyl, Carbamoyl, durch einen oder zwei aus Niederalkyl, wie Methyl, Ethyl, Propyl, Isopropyl, n-Butyl, Isobutyl, sec-Butyl, tert-Butyl, n-Pentyl, Isopentyl, Neopentyl, tert-Pentyl, n-Hexyl, Isohexyl oder n-Heptyl, z. B. in N-Methylcarbamoyl, N-n-Butylcarbamoyl oder N,N-Dimethylcarbamoyl, aus Carboxyniederalkyl oder Niederalkoxycarbonyl-niederalkyl, z. B. als Carboxymethylcarbamoyl (Glycinylcarbonyl) oder als tert-Butoxycarbonylmethylcarbamoyl, aus Diniederalkylaminoniederalkyl, z. B. 2-Dimethylaminoethyl, aus Hydroxyniederalkyl, z. B. Hydroxymethyl oder Hydroxyethyl, und aus Diniederalkoxyniederalkyl, z. B. 2-(2,2-Dimethoxyethyl), ausgewählte Reste, substituiertes Carbamoyl und/oder Cyano sein kann und unverzweigt oder verzweigt ist, wobei Phenylniederalkanoyl, wie Phenylacetyl, oder Phenylniederalkanoyl, worin der Niederalkanoylrest durch Carbamoyl substituiert ist, wie 2-Carbamoyl-3-phenyl-propionyl, besonders bevorzugt sind, durch Heterocyclylniederalkanoyl, worin Heterocyclyl unabhängig wie als Substituent von Niederalkanoyl R₁ definiert ist und insbesondere Morpholino, Thiomorpholino, Pyridyl, Chinolyl oder Tetrazolyl bedeutet, vor allem Pyridylcarbonyl, wie 2-, 3- oder 4-Pyridylcarbonyl, Morpholinocarbonyl, Thiomorpholinocarbonyl, S,S-Dioxothiomorpholinocarbonyl, Indol-2-ylcarbonyl, Chinolin-2-ylcarbonyl, Pyridylacetyl, wie 2- oder 3-Pyridylacetyl, Imidazolylacetyl, wie Imidazol-1-ylacetyl, Morpholinylacetyl, wie Morpholinoacetyl, Pyridylpropionyl, wie 3-(2- oder 3-Pyridyl)propionyl, Pyrrolidinylpropionyl, wie 3-(4-Pyrrolidinyl)propionyl, Morpholinylpropionyl, wie 3-Morpholinopropionyl, oder Tetrazolylpropionyl, wie 3-(Tetrazol-1-yl)-propionyl, durch Halogenniederalkanoyl, welches bis zu 3 Halogenatome enthält, z. B. α-Halogenacetyl, wie α-Fluor-, α-Chlor-, α-Brom-, α-Iod-, α,α,α-Trifluor- oder α,α,α-Trichloracetyl, oder Halogenpropionyl, wie β-Chlor- oder β-Brompropionyl, durch Niederalkoxyniederalkoxyniederalkanoyl, durch Niederalkoxycarbonyl, wie tert-Butoxycarbonyl, durch Arylniederalkoxycarbonyl, worin Aryl 6 - 14 Kohlenstoffatome hat und z. B. ausgewählt ist aus Phenyl, Naphthyl und Fluorenyl, z. B. Phenylniederalkoxycarbonyl, wie Benzyloxycarbonyl, oder 9-Fluorenylmethoxycarbonyl, durch Heterocyclylniederalkoxycarbonyl, worin Heterocyclyl insbesondere ausgewählt ist aus Pyrrolyl, Furyl, Thienyl, Imidazolyl, Pyrazolyl, Oxazolyl, Thiazolyl, Pyridyl, Pyrazinyl, Pyrimidinyl, Indolyl, Chinolyl und Isochinolyl, die auch ganz oder teilweise gesättigt und unsubstituiert oder substituiert insbesondere durch Niederalkyl, wie Methyl, sein können, beispielsweise 2-Furylmethoxycarbonyl, Tetrahydrofuryl-niederalkoxycarbonyl, wie 2-Tetrahydrofuryl-methoxycarbonyl, oder 2-Morpholino-ethoxycarbonyl, durch Niederalkenyloxycarbonyl (vorzugsweise mit einem gesättigtem Kohlenstoffatom am bindenden Sauerstoff), wie Allyloxycarbonyl, durch Niederalkoxyniederalkoxycarbonyl, wie 2-Methoxyethoxycarbonyl, durch (Niederalkoxyniederalkoxy)niederalkoxycarbonyl, wie 2-(2-Methoxyethoxy)ethoxycarbonyl, durch Carboxyniederalkanoyl, wie 3-Carboxypropionyl, durch Niederalkoxycarbonylniederalkanoyl, durch am Aminostickstoff durch Heterocyclylniederalkanoyl, worin Heterocyclyl vorzugsweise unabhängig wie oben als Substituent von Niederalkanoyl R₁ oder R₉ definiert ist, insbesondere durch N-Morpholino- oder N-Thiomorpholinocarbonyl, substituiertes Aminoniederalkanoyl, z. B. N-Morpholino- oder N-Thiomorpholino-carbonylamino-niederalkanoyl, wie N-Morpholino- oder N-Thiomorpholino-carbonylamino-acetyl, durch Carbamoyl, durch Phenylniederalkylaminocarbonyl, wie Benzylaminocarbonyl, durch N-Heterocyclylniederalkyl-N-niederalkylcarbamoyl oder N-Heterocyclylniederalkyl-carbamoyl, worin Heterocyclyl unabhängig wie oben als Substituent von Niederalkanoyl R₁ oder R₉ beschrieben definiert ist, insbesondere als Pyridyl, wie 2-, 3- oder 4-Pyridyl, in erster Linie 2- oder 3-Pyridylniederalkylaminocarbonyl, wie 2- oder 3-Pyridylmethylaminocarbonyl, oder N-2-, N-3- oder N-4-Pyridylniederalkyl-N-niederalkylaminocarbonyl, wie N-2-, N-3- oder N-4-Pyridylmethyl-N-methylaminocarbonyl, durch Heterocyclylniederalkyl-carbamoyl-niederalkanoyl, worin Heterocyclyl unabhängig wie bei der Definition als Substituent von Niederalkyl R₁, R₂, R₈ oder R₉ beschrieben definiert ist, z. B. 2-(N-Morpholinoniederalkyl-carbamoyl)-niederalkanoyl, wie 2(R,S)-(N-(2-Morpholinoethyl)-carbamoyl-3-methyl-butyryl, oder 2-(N-(Pyridylniederalkyl)-carbamoyl)-niederalkanoyl, wie (2(R,S)-(N-(2-Pyridylmethyl)-carbamoyl)-3-methyl)-butyryl, durch Sulfonyl, durch Niederalkansulfonyl, wie Methan- oder Ethansulfonyl, durch Arylsulfonyl (Aryl-SO₂-), worin Aryl 6 - 10 Kohlenstoffatome hat und z. B. aus Phenyl und Naphthyl ausgewählt ist, und unsubstituiert oder insbesondere durch Niederalkyl, wie Methyl, oder Niederalkoxy, wie Methoxy, substituiert ist, wie p-Toluolsulfonyl, oder durch Heterocyclylsulfonyl (Heterocyclyl-SO₂-), worin Heterocyclyl vorzugsweise aus Pyrrolyl, Furyl, Thienyl, Imidazolyl, Pyrazolyl, Oxazolyl, Thiazolyl, Pyridyl, Pyrazinyl, Pyrimidinyl, Indolyl, Chinolyl und Isochinolyl, die auch ganz oder teilweise gesättigt vorliegen können, aus Morpholinyl und aus Thiomorpholinyl ausgewählt ist und unsubstituiert oder substituiert, insbesondere durch Niederalkyl, wie Methyl, sein kann, wie Morpholinosulfonyl, Thiomorpholinosulfonyl, Piperidinosulfonyl oder Piperazinosulfonyl (wobei Heterocyclylsulfonyl als Substituent in bevorzugten Ausführungsformen fehlt); und/oder eine Hydroxygruppe der Seitenkette in veretherter oder veresterter Form, z. B. als Niederalkoxy, wie Methoxy oder tert-Butoxy, als Arylniederalkoxy, wie Benzyloxy, als Niederalkanoyloxy, wie Acetoxy, oder als Niederalkyloxycarbonyloxy, z. B. tert-Butyloxycarbonyloxy, vorliegt.

Insbesondere sind über die Carbonylgruppe ihrer Carboxyfunktion gebundene Acylgruppen R₁ oder R₉ einer unsubstituierten oder substituierten Aminosäure ausgewählt aus Phenylalanin, N-(Benzyloxycarbonyl)-phenylalanin, Tyrosin, Tyrosin-O-methylether, N-Morpholinocarbonyl-glycin, N-(N-(2-, 3- oder 4-Pyridyl)methyl-N-methylaminocarbonyl)-glycin, Valin, N-(Trifluoracetyl)-valin, N-Phenylacetyl-valin, N-Acetyl-valin, N-(3-Phenylpropionyl)-valin, N-(2-Carbamoyl-3-phenyl-propionyl)-valin, wie N-(2(R,S)-Carbamoyl-3-phenyl-propionyl)-valin, N-(2- oder 3-Pyridylacetyl)-valin, N-Tetrahydrofurylmethoxycarbonyl-valin, N-(2-Methoxy)ethoxycarbonyl-valin, N-3-(Tetrazol-1-yl)-propionyl-valin, N-(Indol-2-ylcarbonyl)-valin, N-(Chinolin-2-ylcarbonyl)-valin, N-Methoxycarbonyl-valin, N-Ethoxycarbonylvalin, N-Isobutoxycarbonyl-valin, N-tert-Butoxycarbonyl-valin, N-Benzyloxycarbonyl-valin, N-(2-Furylmethoxycarbonyl)valin, N-Allyloxycarbonyl-valin, N-(Morpholinocarbonyl)-valin, N-(Thiomorpholinocarbonyl)-valin, N-(S,S-Dioxothiomorpholinocarbonyl)-valin, N-(N-2-Pyridylmethyl-N-methylaminocarbonyl)-valin, N-(N-3-Pyridylmethyl-aminocarbonyl)-valin, N-(N-2-Pyridylmethyl-aminocarbonyl)-valin, N-Morpholino-carbonylamino-acetyl-valin, N-Methansulfonyl-valin, N-Morpholinosulfonyl-valin, N-Acetyl-leucin, N-(4-Thiomorpholinocarbonyl)-leucin, N-(4-(S,S-Dioxothiomorpholino)carbonyl)-leucin, N-(Benzyloxycarbonyl)-leucin, N-Acetyl-isoleucin, N-Propionyl-isoleucin, N-(Benzyloxycarbonyl)-isoleucin, N-(tert-Butoxycarbonyl)-norleucin, N-Benzyloxycarbonyl-glutaminsäure, Asparagin, Glutamin, N-Benzyloxycarbonyl-asparagin, Chinolin-2-ylcarbonyl-asparagin und N-(Morpholinocarbonyl)-asparagin bevorzugt;
wobei die Aminosäurereste vorzugsweise in der (L)- oder (D,L)-Form, im Falle von Valin auch in der (D)-Form vorliegen.

Unsubstituiertes oder substituiertes Alkyl R₁, R₂, R₈ oder R₉ enthält einen Alkylrest mit 1 - 20, vorzugsweise bis zu 10 Kohlenstoffatomen, ist verzweigt oder unverzweigt, und ist z. B. Methyl, Ethyl, n-Propyl, Isopropyl, n-Butyl, Isobutyl, sec-Butyl, tert-Butyl, n-Pentyl, Isopentyl, Neopentyl, tert-Pentyl, n-Hexyl, Isohexyl, n- Heptyl, n-Octyl, n-Nonyl oder n-Decyl. Bevorzugt ist Niederalkyl, z. B. Methyl, Ethyl, n-Propyl, Isopropyl, n-Butyl, Isobutyl, sec-Butyl, tert-Butyl, n-Pentyl, Isopentyl, Neopentyl, tert-Pentyl, n-Hexyl, Isohexyl oder n- Heptyl, das unsubstituiert oder substituiert ist.

Als Substituenten in substituiertem Alkyl R₁, R₂, R₈ oder R₉, vorzugsweise substituiertem Niederalkyl, kommen die für Niederalkanoyl R₁ und R₉ genannten Reste in Frage.

Besonders bevorzugt ist unsubstituiertes Niederalkyl, wie Methyl, Ethyl, Propyl, Isopropyl, n-Butyl, Isobutyl oder tert-Butyl.

Durch Alkyl substituiertes Sulfonyl R₁ oder R₉ (Alkyl-SO₂-) enthält vorzugsweise einen unter Alkyl R_{1,} R₂, R₈ und R₉ genannten unsubstituierten oder substituierten Alkylrest und ist insbesondere
Niederalkansulfonyl, wie Methansulfonyl, Ethansulfonyl, n-Propansulfonyl oder S-tert-Butylsulfonyl, oder
durch Arylniederalkyl substituiertes Sulfonyl (Arylniederalkyl-SO₂-), das beispielsweise einen unsubstituierten oder substituierten Arylrest enthält, wie für durch Aryl substituiertes Niederalkyl R₁, R₂, R₈ und R₉ definiert, und insbesondere ausgewählt ist aus Phenylmethan-, 4-Chlor-phenylmethan-, 4-Methoxy-phenylmethan oder 4-Nitrophenylmethan-, Naphthylmethan-, z.B. α- oder β-Naphthylmethan-, 2-Phenylethan-, 2-α-Naphthylethan-, 2-β-Naphthylethan-, 2-(4-Methylphenyl)ethan-, 2-(4-Methoxyphenyl)ethan-, 3-Phenylpropan-, 3-(p-Hydroxyphenyl)-propan-, 2,2-Diphenylethan- und 2,2-Di-(4-methoxyphenyl)-ethan-sulfonyl.

Durch Aryl substituiertes Sulfonyl R₁ oder R₉ (Aryl-SO₂-) enthält vorzugsweise einen bei der Definition von Aryl als Substituent von Niederalkanoyl R₁ oder R₉ genannten unsubstituierten oder substituierten Arylrest und ist insbesondere unsubstituiertes oder durch Niederalkyl ein- oder zweifach substituiertes Benzol- oder 1- oder 2-Naphthalin-sulfonyl, wie Benzolsulfonyl, 2- oder 4-Toluolsulfonyl oder 1- oder 2- Naphthalinsulfonyl.

Durch Heterocyclyl substituiertes Sulfonyl R₁ oder R₉ (Heterocyclyl-SO₂-) enthält vorzugsweise Heterocyclyl, welches aus Pyrrolyl, Furyl, Thienyl, Imidazolyl, Pyrazolyl, Oxazolyl, Thiazolyl, Pyridyl, Pyrazinyl, Pyrimidinyl, Indolyl, Chinolyl und Isochinolyl, die auch ganz oder teilweise gesättigt vorliegen können, aus Morpholinyl und aus Thiomorpholinyl ausgewählt ist und unsubstituiert oder substituiert, insbesondere durch Niederalkyl, wie Methyl, sein kann, wie Morpholinosulfonyl, Thiomorpholinosulfonyl, Piperidinosulfonyl oder Piperazinosulfonyl. In besonders bevorzugten Ausführungsformen der Erfindung kann Heterocyclylsulfonyl als Substituent abwesend sein.

Acyloxy R₅ hat z.B. bis zu 25, bevorzugt bis zu 19 C-Atome, und ist in erster Linie die über ihr Carbonyl an den bindenden Sauerstoff gebundene Acyloxygruppe einer Carbonsäure oder einer unsubstituierten oder substituierten Aminosäure, ferner eine Aminocarbonyloxygruppe, eine N-substituierte Aminocarbonyloxygruppe oder ein über seine Carbonylgruppe an den bindenden Sauerstoff geknüpfter Acylrest eines Halbesters der Kohlensäure.

Bevorzugte Acyloxygruppe R₅ einer Carbonsäure ist beispielsweise unsubstituiertes C₁-C₂₀-Alkanoyloxy, z.B. n-Decanoyloxy oder Palmitoyloxy, C₃-C₂₀-Alkenoyloxy oder C₃-C₂₀-Alkinoyloxy, oder substituiertes C₁-C₂₀-Alkanoyloxy, C₃-C₂₀-Alkenoyloxy oder C₃-C₂₀-Alkinoyloxy, insbesondere Niederalkanoyloxy, Octanoyloxy, Nonanoyloxy, Decanoyloxy, Undecanoyloxy oder Dodecanoyloxy; C₃-C₇-Alkenoyloxy; oder C₃-C₇-Alkinoyloxy; oder substituiertes Niederalkanoyloxy, worin die Substituenten beispielsweise aus einem oder mehreren Resten, vorzugsweise aus bis zu drei Resten, insbesondere aus einem Rest ausgewählt aus der Gruppe Hydroxy, Niederalkoxy, Phenoxy, Naphthoxy, Niederalkanoyloxy, Phenylniederalkanoyloxy, wie Benzoyloxy oder Phenylacetyloxy, Halogen, wie Fluor, Chlor, Brom oder Iod, insbesondere Fluor oder Chlor, Carboxy, Niederalkoxycarbonyl, Phenylniederalkoxycarbonyl, wie Benzyloxycarbonyl, Carbamoyl, Niederalkylcarbamoyl, Hydroxyniederalkylcarbamoyl, Diniederalkylcarbamoyl, Bis(hydroxyniederalkyl)carbamoyl, Cyano, Oxo, C₃-C₈-Cycloalkyl, wie Cyclopropyl, Cyclobutyl, Cyclopentyl oder Cyclohexyl, C₆-C₁₂-Bicycloalkyl, wie Decahydronaphth-2-yl, C₉-C₁₄-Tricycloalkyl, wie 1- oder 2-Adamantyl, C₄-C₈-Cycloalkenyl, wie 1-Cyclohexenyl oder 1,4-Cyclohexadienyl, Heterocyclyl, welches vorzugsweise einen gesättigten, teilweise gesättigten oder ungesättigten einfachen Ring bedeutet, der 3 bis 7, vorzugsweise 5 - 7 Ringatome enthält, und bis zu vier Heteroatome ausgewählt aus Stickstoff, Schwefel und/oder Sauerstoff enthält, vorzugsweise 1 oder 2 der genannten Heteroatome; wobei der Ring entweder als solcher vorliegt oder bis zu zweifach, vorzugsweise einfach benzanneliert, cyclopenta-, cyclohexa- oder cyclohepta-anneliert sein kann; und unsubstituiert oder, insbesondere durch Niederalkyl, Niederalkanoyl, Hydroxy, Niederalkoxy, Phenylniederalkoxy, wie Benzyloxy, Hydroxyniederalkyl, wie Hydroxymethyl, Halogen, Cyano und/oder Trifluormethyl, substituiert sein kann, z. B. Pyrrolyl, 2,5-Dihydropyrrolyl, Furyl, Thienyl, Tetrahydrofuryl, Cyclohepta[b]pyrrolyl, Pyrrolidinyl, Imidazolyl, Imidazolidinyl, Pyrazolinyl, Pyrazolidinyl, Triazolyl, wie 1,2,3-, 1,2,4- oder 1,3,4-Triazolyl, Tetrazolyl, wie 1- oder 2-Tetrazolyl, Tetrahydro-oxazolyl, Tetrahydro-isoxazolyl, Tetrahydro-thiazolyl, Tetrahydro-isothiazolyl, Indolyl, Isoindolyl, Chinolyl, Isochinolyl, Benzimidazolyl, Benzofuranyl, Pyridyl, Pyrimidinyl, Piperidinyl, Piperazin-1-yl, Morpholino, Thiomorpholino, S,S-Dioxothiomorpholino, 1,2-Dihydro- oder 1,2,3,4-Tetrahydrochinolyl, oder 1,2-Dihydro- oder 1,2,3,4-Tetrahydroisochinolyl, wobei die genannten Reste unsubstituiert oder wie oben substituiert sind, insbesondere durch Niederalkyl, z. B. in 4-Niederalkyl-piperazin-1-yl, wie 4-Methyl- oder 4-Ethyl-piperazin-1-yl, durch Niederalkanoyl, z. B. in 4-Niederalkanoyl-piperazin-1-yl, wie 4-Acetyl-piperazin-1-yl, oder durch Hydroxyniederalkyl, z.B. in 5-Hydroxymethylfuran-2-ylcarbonyl, und Aryl, vorzugsweise C₆-C₁₄-Aryl, z.B. Phenyl, Naphthyl, wie 1- oder 2-Naphthyl, oder Fluorenyl, wie Fluoren-9-yl, wobei Aryl unsubstituiert oder beispielsweise durch Niederalkyl, z.B. Methyl, Halogenniederalkyl, wie Trifluormethyl oder Chlor- oder Brommethyl, Halogen, z.B. Fluor oder Chlor, Hydroxy, Niederalkoxy, wie Methoxy, Niederalkanoyloxy, Carboxy, Niederalkyloxycarbonyl, Phenylniederalkoxycarbonyl, Carbamoyl, Mono- oder Diniederalkylcarbamoyl, Mono- oder Di-hydroxyniederalkylcarbamoyl, wie Heterocyclylniederalkyl, worin Heterocyclyl wie oben als Substituent von Niederalkanoyloxy R₅ definiert ist, insbesondere Heterocyclylmethyl, worin Heterocyclyl über ein Ringstickstoffatom gebunden ist, z.B. Piperidinomethyl, Piperazin-1-ylmethyl, 4-Niederalkyl-piperazin-1-ylmethyl, wie 4-Methyl- oder 4-Ethyl-piperazin-1-ylmethyl, 4-Niederalkanoyl-piperazin-1-ylmethyl, wie 4-Acetyl-piperazin-1-ylmethyl, Morpholinomethyl oder Thiomorpholinomethyl, Cyano und/oder Nitro ein oder mehrfach, vorzugsweise einfach, substituiert ist, insbesondere Phenyl, welches durch einen der genannten Reste in p-Stellung substituiert ist, ausgewählt sind, z.B. Niederalkanoyloxy, wie Formyloxy, Acetyloxy, Propionyloxy, Pivaloyloxy oder Heptanoyloxy, wie n-Heptanoyloxy, Hydroxyniederalkanoyloxy, z.B. β-Hydroxypropionyloxy, Niederalkoxyniederalkanoyloxy, z.B. Niederalkoxyacetyloxy oder Niederalkoxypropionyloxy, wie Methoxyacetyloxy oder β-Methoxypropionyloxy, Niederalkanoyloxyniederalkanoyloxy, z.B. Niederalkanoyloxyacetyloxy oder Niederalkanoyloxypropionyloxy, wie Acetoxyacetyloxy oder β-Acetoxypropionyloxy, Halogenniederalkanoyloxy, z.B. α-Halogenacetyloxy, wie α-Chlor-, α-Brom-, α-Iod- α,α,α-Trifluor oder α,α,α-Trichloracetyloxy, oder Halogenpropionyloxy, wie β-Chlor- oder β-Brompropionyloxy, Carboxyniederalkanoyloxy, z.B. Carboxyacetyloxy oder 3-Carboxypropionyloxy, Niederalkoxycarbonylniederalkanoyloxy, z.B. Niederalkoxycarbonylacetyloxy oder Niederalkoxycarbonylpropionyloxy, wie Methoxycarbonylacetyloxy, β-Methoxycarbonylpropionyloxy, Ethoxycarbonylacetyloxy, β-Ethoxycarbonylpropionyloxy, tert-Butoxycarbonylacetyloxy oder β-tert-Butoxycarbonylpropionyloxy, Carbamoylniederalkanoyloxy, z.B. Carbamoylacetyloxy oder β-Carbamoylpropionyloxy, Niederalkylcarbamoylniederalkanoyloxy, Diniederalkylcarbamoylniederalkanoyloxy, Hydroxy-carboxy-niederalkanoyloxy, Hydroxy-niederalkoxycarbonyl-niederalkanoyloxy, Dihydroxy-carboxyniederalkanoyloxy, Dihydroxy-niederalkoxycarbonyl-niederalkanoyloxy, Heterocyclylniederalkanoyloxy, beispielsweise Pyrrolylcarbonyloxy, wie 2- oder 3-Pyrrolylcarbonyloxy, Furylcarbonyloxy, z.B. 2-Furylcarbonyloxy, 5-Hydroxymethyl-furan-2-ylcarbonyloxy, Thienylcarbonyloxy, z.B. 2-Thienylcarbonyloxy, Imidazolylcarbonyloxy, wie 4-Imidazolylcarbonyloxy, Imidazolylacetoxy, wie 4-Imidazolylacetoxy, Imidazolylpropionyloxy, wie 3-(4-Imidazolylpropionyloxy, Pyridylcarbonyloxy, z.B. 2-, 3- oder 4-Pyridylcarbonyloxy, Indolylcarbonyloxy, z.B. 2-, 3- oder 5-Indolylcarbonyloxy, 1-Methyl-, 5-Methyl-, 5-Methoxy-, 5-Benzyloxy-, 5-Chlor- oder 4,5-Dimethylindolyl-2-carbonyloxy, Chinolylcarbonyloxy, wie Chinolin-2-ylcarbonyloxy, Pyrrolidinylcarboxy, wie Pyrrolidinyl-3-carbonyloxy, Piperidinylcarbonyloxy, z.B. 2-, 3- oder 4-Piperidinylcarbonyloxy, Morpholinocarbonyloxy, Thiomorpholinocarbonyloxy, Morpholinoacetyloxy, Thiomorpholinoacetyloxy, oder 4-Niederalkyl-1-piperazinoacetyloxy, wie 4-Methyl-piperazinoacetyloxy, Niederalkenoyloxy, z.B. Acryloyloxy, Vinylacetyloxy, Crotonoyloxy oder 3- oder 4-Pentenoyloxy, Niederalkinoyloxy, z.B. Propioloyloxy oder 2- oder 3-Butinoyloxy, C₃-C₈-Cycloalkylcarbonyloxy oder C₃-C₈-Cycloalkylacetoxy, z.B. Cyclopropyl-, Cyclobutyl-, Cyclopentyl- oder Cyclohexylcarbonyloxy, Cyclopropylacetyloxy, Cyclopentylacetyloxy oder Cyclohexylacetyloxy, Phenylniederalkanoyloxy, z.B. Benzoyloxy, Phenylacetoxy oder 3-Phenylpropionyloxy, worin Phenyl unsubstituiert, mono- oder mehrfach substituiert durch Niederalkyl, z.B. Methyl, Haloniederalkyl, wie Chlor- oder Brommethyl, Halogen, z.B. Fluor oder Chlor, Hydroxy, Niederalkoxy, z.B. Methoxy, Piperidinomethyl, Piperazin-1-ylmethyl, 4-Niederalkyl-piperazin-1-ylmethyl, wie 4-Methyl- oder 4-Ethyl-piperazin-1-ylmethyl, 4-Niederalkanoyl-piperazin-1-ylmethyl, wie 4-Acetyl-piperazin-1-ylmethyl, Morpholinoniederalkyl, wie Morpholinomethyl, Thiomorpholinomethyl, Cyano und/oder Nitro, z.B. 4-Chlormethyl-, 4-Brommethyl-, 4-Fluor-, 4-Chlor-, 4-Methoxy-, 4-Morpholinomethyl-, 4-Thiomorpholinomethyl-, 4-Cyano- oder 4-Nitrobenzoyloxy, oder Niederalkylphenylacetyloxy, wie 4-Methylphenylacetyloxy, ist.

Bevorzugtes Acyloxy R₅ eines über seine Carbonylgruppe an den bindenden Sauerstoff geknüpften Acylrestes eines Halbesters der Kohlensäure ist z.B. unsubstituiertes oder substituiertes Alkyloxycarbonyloxy, insbesondere unsubstituiertes oder substituiertes Niederalkoxycarbonyloxy, z.B. Methoxy-, Ethoxy- oder tert-Niederalkoxycarbonyloxy, wie tert-Butoxycarbonyloxy, 2-Halogenniederalkoxycarbonyloxy, z.B. 2-Chlor-, 2-Brom-, 2-Iod- oder 2,2,2-Trichlorethoxycarbonyloxy; Arylniederalkoxycarbonyloxy, z.B. Arylmethoxy-carbonyloxy, worin Aryl vorzugsweise 6 bis 14 Kohlenstoffatome hat, unsubstituiert oder beispielsweise durch Niederalkyl, z.B. Methyl, Halogenniederalkyl, wie Trifluormethyl oder Chlor- oder Brommethyl, Halogen, z.B. Fluor oder Chlor, Hydroxy, Niederalkoxy, wie Methoxy, Niederalkanoyloxy, Carboxy, Niederalkyloxycarbonyl, Phenylniederalkoxycarbonyl, Carbamoyl, Mono- oder Diniederalkylcarbamoyl, Mono- oder Di-hydroxyniederalkylcarbamoyl, Heterocyclylniederalkyl, worin Heterocyclyl wie oben als Substituent für Niederalkanoyloxy R₅ definiert ist, insbesondere Heterocyclylmethyl, worin Heterocyclyl über ein Ringstickstoffatom gebunden ist, z.B. Piperidinomethyl, Piperazin-1-ylmethyl, 4-Niederalkyl-piperazin-1-ylmethyl, wie 4-Methyl- oder 4-Ethyl-piperazin-1-ylmethyl, 4-Niederalkanoyl-piperazin-1-ylmethyl, wie 4-Acetyl-piperazin-1-ylmethyl, Morpholinomethyl oder Thiomorpholinomethyl, Cyano und/oder Nitro ein oder mehrfach, vorzugsweise einfach, substituiert ist, und insbesondere Phenyl, 1- oder 2-Naphthyl, Fluorenyl oder durch Niederalkyl, z.B. Methyl oder tert-Butyl, Niederalkoxy, z.B. Methoxy, Ethoxy oder tert-Butoxy, Hydroxy, Halogen, z.B. Fluor, Chlor oder Brom, und/oder Nitro mono- oder mehrfach substituiertes Phenyl ist, z.B. Phenylniederalkoxycarbonyloxy, wie Benzyloxycarbonyloxy, 4-Methoxybenzyloxycarbonyloxy, 4-Nitrobenzyloxycarbonyloxy, Diphenylniederalkoxycarbonyloxy, wie Diphenylmethoxycarbonyloxy, Di-(4-methoxyphenyl)-methoxycarbonyloxy, Trityloxycarbonyloxy oder Fluorenylniederalkoxycarbonyloxy, wie 9-Fluorenylmethoxycarbonyloxy; oder ferner Heterocyclylniederalkoxycarbonyloxy, worin Heterocyclyl wie oben als Substituent von Niederalkanoyloxy R₅ definiert ist, z.B. Furan-2-ylmethoxycarbonyloxy oder Pyridin-2-, -3- oder -4-ylmethoxycarbonyl. Die jeweils unter die Definition von Acyloxygruppen R₅ eines Halbesters der Kohlensäure fallenden Definitionen können vorzugsweise bei allen vor- und nachstehend genannten Definitionen von Verbindungen der Formel I weggelassen werden.

Eine bevorzugte N-substituierte Aminocarbonyloxygruppe als Acyloxy R₅ trägt am Stickstoff 1 bis 2 Substituenten, die unabhängig voneinander aus unsubstituiertem oder substituiertem Niederalkyl, wobei die Substituenten aus den oben für substituiertes Niederalkanoyloxy R₅ genannten ausgewählt sind und in der dort definierten Anzahl vorhanden sein können, vorzugsweise Substituenten ausgewählt aus Hydroxy, Niederalkoxy, Niederalkanoyloxy, Phenylniederalkanoyloxy, wie Benzoyloxy oder Phenylacetyloxy, Halogen, wie Fluor, Chlor, Brom oder Iod, insbesondere Fluor oder Chlor, Carboxy, Niederalkoxycarbonyl, Phenylniederalkoxycarbonyl, wie Benzyloxycarbonyl, Cyano, Oxo und Phenyl oder Naphthyl, welche unsubstituiert oder beispielsweise durch Niederalkyl, z.B. Methyl, Halogenniederalkyl, wie Trifluormethyl oder Chlor-oder Brommethyl, Halogen, z.B. Fluor oder Chlor, Hydroxy, Niederalkoxy, wie Methoxy, Niederalkanoyloxy, Carboxy, Niederalkyloxycarbonyl, Phenylniederalkoxycarbonyl, Cyano und/oder Nitro ein oder mehrfach, vorzugsweise einfach, substituiert sind, insbesondere Phenyl, welches durch einen der genannten Reste in p-Stellung substituiert ist; insbesondere aus unsubstituiertem Niederalkyl, wie Methyl oder Ethyl; und Aryl, welches vorzugsweise 6 bis 14 Kohlenstoffatome hat und unsubstituiert oder beispielsweise durch Niederalkyl, z.B. Methyl, Halogenniederalkyl, wie Chlor- oder Brommethyl, Halogen, z.B. Fluor oder Chlor, Hydroxy, Niederalkoxy, wie Methoxy, Niederalkanoyloxy, Carboxy, Niederalkyloxycarbonyl, Phenylniederalkoxycarbonyl, Haloniederalkyl, wie Trifluormethyl, Cyano und/oder Nitro ein oder mehrfach, vorzugsweise einfach, substituiert ist, ausgewählt sind, wobei der Stickstoff der Carbamoylgruppe nicht mehr als einen Arylrest trägt; insbesondere handelt es sich bei einer Acyloxygruppe R₅ einer N-substituierten Carbaminsäure um Mono- oder Diniederalkylaminocarbonyloxy, wie N-Methyl-, N-Ethyl-, N,N-Dimethyl- oder N,N-Diethylaminocarbonyloxy, oder Phenylniederalkylaminocarbonyloxy, worin Phenyl unsubstituiert oder durch Niederalkyl, z.B. Methyl, Halogenniederalkyl, wie Chlor- oder Brommethyl oder Trifluormethyl, Halogen, z.B. Fluor oder Chlor, Hydroxy, Niederalkoxy, wie Methoxy, Carboxy, und/oder Cyano, vorzugsweise durch bis zu drei unabhängig voneinander ausgewählte dieser Substituenten, insbesondere einen davon, z.B. in p-Stellung, substituiert ist, wie in N-Benzyl-, N-(4-Fluorbenzyl)-, N-(4-Chlorbenzyl)-, N-(4-Trifluormethylbenzyl)- oder N-(4-Cyanobenzyl)-aminocarbonyloxy; besonders bevorzugt ist durch nur einen Rest am Stickstoff substituiertes Aminocarbonyloxy, z.B. N-Niederalkylaminocarbonyloxy, wie N-Methyl-oder N-Ethyl-aminocarbonyloxy, oder Phenylniederalkylaminocarbonyloxy, worin Phenyl unsubstituiert oder durch Niederalkyl, wie Methyl, Halogenniederalkyl, wie Chlor-oder Brommethyl oder Trifluormethyl, Halogen, wie Fluor oder Chlor, Hydroxy, Niederalkoxy, wie Methoxy, Carboxy, und/oder Cyano substituiert ist, vorzugsweise durch bis zu drei unabhängig voneinander ausgewählte dieser Substituenten, insbesondere einen davon, z.B. in p-Stellung, wie in N-Benzyl-, N-(4-Fluorbenzyl)-, N-(4-Chlorbenzyl)-, N-(4-Trifluormethylbenzyl)- oder N-(4-Cyanobenzyl)-aminocarbonyloxy. Die jeweils unter die Definition von Acyloxygruppen R₅ einer N-substituierten Carbaminsäure fallenden Definitionen und der Rest Aminocarbonyloxy R₅ können vorzugsweise bei allen vor- und nachstehend genannten Definitionen von Verbindungen der Formel I weggelassen werden.

Eine über ihr Carbonyl an den bindenden Sauerstoff gebundene unsubstituierte oder substituierte Aminosäure in Acyloxy R₅ wird vorzugsweise gebildet durch die über das Carbonyl ihrer Carboxygruppe und ein Sauerstoffatom gebundenen Aminosäurereste (Aminoacyloxy) einer α-, β-, γ- oder δ-Aminosäure, insbesondere
einer natürlichen α-Aminosäure mit der L-Konfiguration, wie sie normalerweise in Proteinen vorkommen, oder eines Epimeren einer solchen Aminosäure, d. h. mit der unnatürlichen D-Konfiguration, oder deren D,L-Isomerengemisch, eines Homologen einer solchen Aminosäure, z. B. worin die Aminosäurenseitenkette um eine oder zwei Methylengruppen verlängert oder verkürzt ist, worin die Aminogruppe in β-, γ- oder δ-Stellung vorliegt und/oder worin eine Methylgruppe durch Wasserstoff ersetzt ist, einer substituierten aromatischen Aminosäure, worin der aromatische Rest 6 - 14 Kohlenstoffatome hat, z. B. eines substituierten Phenylalanins oder Phenylglycins, worin der Phenyl-Substituent Niederalkyl, z. B. Methyl, Hydroxy, Niederalkoxy, z. B. Methoxy, Niederalkanoyloxy, z. B. Acetoxy, Amino, Niederalkylamino, z. B. Methylamino, Diniederalkylamino, z. B. Dimethylamino, Niederalkanoylamino, z. B. Acetylamino oder Pivaloylamino, Niederalkoxycarbonylamino, z. B. tert-Butoxycarbonylamino, Arylmethoxycarbonylamino, worin Aryl vorzugsweise 6 - 14 Kohlenstoffatome hat, z. B. Benzyloxycarbonylamino oder 9-Fluorenylmethoxycarbonylamino, Halogen, z. B. Fluor, Chlor, Brom oder Jod, Carboxy und/oder Nitro sein kann und ein- oder mehrfach vorkommt, eines benzannelierten Phenylalanins oder Phenylglycins, wie α-Naphthylalanins, oder eines hydrierten Phenylalanins oder Phenylglycins, wie Cyclohexylalanins oder Cyclohexylglycins.

Diese Aminosäurereste können an freien Amino- oder Hydroxyfunktionen substituiert sein, wie oben für Aminosäurereste R₁ oder R₉ beschrieben.

Besonders bevorzugt ist das über das Carbonyl seiner Carboxygruppe und ein Sauerstoffatom gebundene Radikal einer Aminosäure ausgewählt aus Glycin (H-Gly-OH), Alanin (H-Ala-OH), 2-Aminobuttersäure, 3-Aminobuttersäure, 4-Aminobuttersäure, 3-Aminopentansäure, 4-Aminopentansäure, 5-Aminopentansäure, 3-Aminohexansäure, 4-Aminohexansäure oder 5-Aminohexansäure, Valin (H-Val-OH), Norvalin (α-Aminovaleriansäure), Leucin, (H-Leu-OH), Isoleucin (H-Ile-OH), Norleucin (α-Aminohexansäure, H-Nle-OH), Serin (H-Ser-OH), Homoserin (α-Amino-γ-hydroxybuttersäure), Threonin (H-Thr-OH), Methionin (H-Met-OH), Cystein (H-Cys-OH), Prolin (H-Pro-OH), trans-3- und trans-4-Hydroxyprolin, Phenylalanin (H-Phe-OH), Tyrosin (H-Tyr-OH), 4-Aminophenylalanin, 4-Chlorphenylalanin, 4-Carboxyphenylalanin, β-Phenylserin (β-Hydroxyphenylalanin), Phenylglycin, α-Naphthylalanin (H-Nal-OH), Cyclohexylalanin (H-Cha-OH), Cyclohexylglycin, Tryptophan (H-Trp-OH), Indolin-2-carbonsäure, 1,2,3,4-Tetrahydroisochinolin-3-carbonsäure, Asparaginsäure (H-Asp-OH), Asparagin (H-Asn-OH), Aminomalonsäure, Aminomalonsäure-monoamid, Glutaminsäure (H-Glu-OH), Glutamin (H-Gln-OH), Histidin (H-His-OH), Arginin (H-Arg-OH), Lysin (H-Lys-OH), δ-Hydroxylysin, Ornithin (α,δ-Diaminovaleriansäure), 3-Aminopropansäure, α,γ-Diaminobuttersäure und α,β-Diaminopropionsäure, besonders bevorzugt der Rest einer aliphatischen Aminosäure ausgewählt aus Alanin, Valin, Norvalin, Leucin, 3-Aminopropionsäure, 2-Aminobuttersäure, 3-Aminobuttersäure, 4-Aminobuttersäure, 3-Aminopentansäure, 4-Aminopentansäure, 5-Aminopentansäure, 3-Aminohexansäure, 4-Aminohexansäure oder 5-Aminohexansäure und Isoleucin oder einer Aminosäure ausgewählt aus Glycin, Asparagin, Glutamin, Methionin, Lysin, Histidin, Prolin und Phenylalanin, wobei (ausser in Fällen, wo kein asymmetrisches Kohlenstoffatom vorliegt, z.B. bei Glycin) jede der genannten Aminosäuren in der D-, L- oder (D,L)-, vorzugsweise in der L-Form vorliegen kann, und
eine Aminogruppe unsubstituiert oder ein- oder zweifach N-alkyliert, z. B. durch Niederalkyl, wie Methyl, n-Propyl oder n-Butyl, durch Pyridylniederalkyl, wie 2-,3- oder 4-Pyridylmethyl, und/oder durch Phenylniederalkyl, wie Benzyl, und/oder N-acyliert, z. B. durch unsubstituiertes oder substituiertes Niederalkanoyl, wie oben für Niederalkanoyloxy R₅ definiert, vor allem durch Acetyl, Propionyl oder Pivaloyl, Arylniederalkanoyl, z.B. Phenylniederalkanoyl, wie Benzoyl oder Phenylacetyl, durch Niederalkoxycarbonyl, wie tert-Butoxycarbonyl, oder durch Arylniederalkoxycarbonyl, z.B. Phenylniederalkoxycarbonyl, wie Benzyloxycarbonyl; vorliegt.

Von den zuletzt genannten Resten sind Acyloxygruppen R₅ einer unsubstituierten oder substituierten Aminosäure ausgewählt aus Aminoacetyloxy (Glycyloxy), N-Niederalkylaminoacetyloxy, N,N-Diniederalkylaminoacetyloxy, N-Niederalkyl-N-phenylniederalkylaminoacetyloxy, N-Niederalkyl-N-niederalkoxycarbonylaminoacetyloxy und N-Phenylniederalkoxycarbonyl-N-niederalkylaminoacetyloxy, z.B. N-Methylaminoacetyloxy, N,N-Dimethylaminoacetyloxy, N-Methyl-N-(n-butyl)aminoacetyloxy, N-Methyl-N-benzylaminoacetyloxy, N-Methyl-N-[(2-, 3- oder 4-)pyridylmethyl]-aminoacetyloxy, wie N-Methyl-N-3-pyridylmethylaminoacetyloxy, N-Methyl-N-tert-butoxycarbonylaminoacetyloxy, N-Benzyloxycarbonyl-N-niederalkylaminoacetyloxy, Prolyloxy, Histidyloxy, Glutamyloxy und Asparagyloxy bevorzugt, wobei die Aminosäurereste (ausser in Fällen, wo kein asymmetrisches Kohlenstoffatom vorliegt, z.B. bei Gly) vorzugsweise in der (L)-oder ferner der (D)- oder (D,L)-Form vorliegen.

Unsubstituiertes oder substituiertes Alkyl R₃, R₄ oder R₇ ist vorzugsweise einer der unter Alkyl R₁, R₂, R₈ und R₉ genannten Reste und ist unsubstituiert oder substituiert, in erster Linie durch die für Niederalkanoyl R₁ oder R₉ genannten Substituenten, insbesondere einen dieser Substituenten, und ist insbesondere ausgewählt aus
Niederalkyl, z. B. Methyl, Ethyl, Propyl, Isopropyl, n-Butyl, Isobutyl oder tert-Butyl,
Cycloalkylniederalkyl, worin Cycloalkyl z.B. 3 bis 7 Kohlenstoffatome hat, wie Cyclopropyl, Cyclobutyl, Cyclopentyl, Cyclohexyl oder Cycloheptyl, wobei Cycloalkyl unsubstituiert oder durch Niederalkyl, wie Isopropyl, Halogenniederalkyl, wie Tri-fluormethyl, Hydroxy, Niederalkoxy, Amino, Mono- oder Diniederalkylamino, Halogen, wie Fluor, Chlor oder Brom, Nitro und/oder Cyano ein- oder bis zu dreifach substituiert ist und an Niederalkyl, insbesondere Methyl, Ethyl, n-Propyl, Isopropyl, N-Butyl, Isobutyl oder tert-Butyl, vorzugsweise endständig, gebunden ist, z.B. Cyclobutyl-, Cyclopentyl-, Cyclohexyl- oder Cycloheptyl-niederalkyl, wie -methyl oder -ethyl, besonders bevorzugt Cyclohexylniederalkyl, wie Cyclohexylmethyl, und
Arylniederalkyl, worin Aryl beispielsweise unabhängig definiert ist wie Aryl als Substituent von Niederalkanoyl R₁ oder R₉, welches unsubstituiert oder wie dort substituiert ist, in erster Linie Phenylniederalkyl, wie Benzyl, 2-Phenylethyl, 3-Phenylpropyl, 4-Fluorbenzyl, 4-Cyanobenyzl, 4-Trifluorbenyzl, 4-Hydroxybenzyl oder 4-Methoxybenyzl, besonders bevorzugt Phenylniederalkyl, vor allem wie zuletzt definiert; oder (ferner oder insbesondere)
Thienylmethyl, wie 2-Thienylmethyl.

Cycloalkyl R₃, R₄ oder R₇ ist vorzugsweise wie bei der Definition als Substituent von Niederalkanoyl R₁ oder R₉ definiert und ist z. B. Cyclopropyl, Cyclobutyl, Cyclopentyl, Cyclohexyl oder Cycloheptyl, wobei Cycloalkyl unsubstituiert oder durch Niederalkyl, wie Isopropyl, Halogenniederalkyl, wie Trifluormethyl, Hydroxy, Niederalkoxy, Amino, Mono- oder Diniederalkylamino, Halogen, wie Fluor, Chlor oder Brom, Nitro und/oder Cyano ein- oder bis zu dreifach substituiert ist, wie Cyclobutyl, Cyclopentyl, Cyclohexyl oder Cycloheptyl, besonders bevorzugt Cyclohexyl.

Aryl R₃, R₄ oder R₇ ist vorzugsweise unabhängig wie bei der Definition als Substituent von Niederalkanoyl R₁ oder R₉ definiert und wie dort unsubstituiert oder substituiert, und ist insbesondere Phenyl, das unsubstituiert oder durch Niederalkyl, wie Isopropyl, Halogenniederalkyl, wie Trifluormethyl, Hydroxy, Niederalkoxy, Halogen, wie Fluor, Chlor oder Brom, Nitro und/oder Cyano ein- oder bis zu dreifach substituiert ist, wie Phenyl, 4-Metoxyphenyl, 4-Fluorphenyl, 4-Trifluormethylphenyl oder 4-Cyanophenyl.

Wenn bei den Verbindungen der Formel I Stickstoffatome mit freiem Wasserstoff und/oder Hydroxygruppen vicinal zu Doppel- oder Dreifachbindungen stehen, sind stets auch die entsprechenden tautomeren Imino- beziehungsweise Oxo-Verbindungen mit umfasst.

Salze von Verbindungen der Formel I sind insbesondere Säureadditionssalze, Salze mit Basen oder bei Vorliegen mehrerer salzbildender Gruppen gegebenenfalls auch Mischsalze oder innere Salze.

Salze sind in erster Linie die pharmazeutisch verwendbaren, nichttoxischen Salze von Verbindungen der Formel I.

Solche Salze werden beispielsweise von Verbindungen der Formel I mit einer sauren Gruppe, z. B. einer Carboxy-, Sulfo- oder durch eine oder zwei Hydroxygruppen substituierten Phosphorylgruppe, gebildet und sind beispielsweise deren Salze mit geeigneten Basen, wie nicht-toxische, von Metallen der Gruppe Ia, Ib, IIa und IIb des Periodensystems der Elemente abgeleitete Metallsalze, in erster Linie geeignete Alkalimetall-, z. B. Lithium-, Natrium- oder Kalium-, oder Erdalkalimetallsalze, z. B. Magnesium- oder Calciumsalze, ferner Zinksalze oder Ammoniumsalze, auch solche Salze, welche mit organischen Aminen, wie gegebenenfalls durch Hydroxy substituierten Mono-, Di- oder Trialkylaminen, insbesondere Mono-, Di- oder Triniederalkylaminen, oder mit quaternären Ammoniumverbindungen gebildet werden, z. B. mit N-Methyl-N-ethylamin, Diethylamin, Triethylamin, Mono-, Bis- oder Tris-(2-hydroxyniederalkyl)-aminen, wie Mono-, Bis- oder Tris-(2-hydroxyethyl)-amin, 2-Hydroxy-tert-butylamin oder Tris-(hydroxymethyl)-methylamin, N,N-Diniederalkyl-N-(hydroxyniederalkyl)-aminen, wie N,N-Dimethyl-N-(2-hydroxyethyl)-amin oder Tri-(2-hydroxyethyl)-amin, N-Methyl-D-glucamin oder quaternären Ammoniumsalzen, wie Tetrabutylammoniumsalzen. Die Verbindungen der Formel I mit einer basischen Gruppe, z. B. einer Aminogruppe, können Säureadditionssalze bilden, beispielsweise mit anorganischen Säuren, z. B. Halogenwasserstoffsäure, wie Salzsäure, Schwefelsäure oder Phosphorsäure, oder mit organischen Carbon-, Sulfon-, Sulfo- oder Phosphosäuren oder N-substituierter Sulfaminsäuren, wie, z. B. Essigsäure, Propionsäure, Glykolsäure, Bernsteinsäure, Maleinsäure, Hydroxymaleinsäure, Methylmaleinsäure, Fumarsäure, Aepfelsäure, Weinsäure, Gluconsäure, Glucarsäure, Glucuronsäure, Zitronensäure, Benzoesäure, Zimtsäure, Mandelsäure, Salicylsäure, 4-Aminosalicylsäure, 2-Phenoxybenzoesäure, 2-Acetoxybenzoesäure, Embonsäure, Nicotinsäure oder Isonicotinsäure, ferner mit Aminosäuren, wie z. B. den weiter vorn genannten α-Aminosäuren, insbesondere Glutaminsäure und Asparaginsäure, sowie mit Methansulfonsäure, Ethansulfonsäure, 2-Hydroxyethansulfonsäure, Ethan-1,2-disulfonsäure, Benzolsulfonsäure, 4-Methylbenzolsulfonsäure, Naphthalin-2-sulfonsäure, 2- oder 3-Phosphoglycerat, Glucose-6-phosphat, N-Cyclohexylsulfaminsäure (unter Bildung der Cyclamate) oder mit anderen sauren organischen Verbindungen, wie Ascorbinsäure. Verbindungen der Formel I mit sauren und basischen Gruppen können auch innere Salze bilden.

Zur Isolierung oder Reinigung können auch pharmazeutisch ungeeignete Salze Verwendung finden.

Die Verbindungen der Formel I haben wertvolle pharmakologische Eigenschaften. Sie sind antiretroviral wirksam, insbesondere gegen AIDS, z. B. gegen HIV-1 und HIV-2. Sie dienen als metabolische Vorstufen für Verbindungen der Formel II 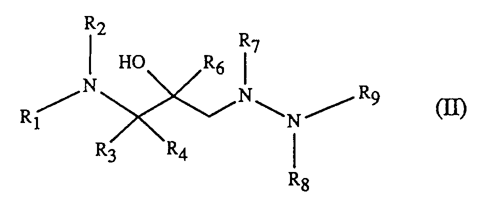 worin die Reste die für Verbindungen der Formel I genannten Bedeutungen haben (Analoge der Verbindungen der Formel I, worin anstelle von R₅ Hydroxy steht), welche als Hemmer retroviraler Aspartatproteasen, insbesondere als Hemmstoffe der gag-Protease von HIV-1 oder HIV-2 (und gegebenenfalls der Aspartoproteasen weiterer Retroviren, die AIDS-analoge Symptome hervorrufen), und damit zur Behandlung von retroviralen Erkrankungen, wie AIDS oder dessen Vorstufen, geeignet sind. Die Verbindungen der Formel II (mit Hydroxy anstelle von R₅ in Formel I) werden dabei im Körper des zu behandelnden Tieres, vorzugsweise eine Warmblüters einschliesslich des Menschen, aus den Verbindungen der Formel I freigesetzt.

Die Verbindungen der Formel I weisen vorzugsweise gegenüber den Verbindungen der Formel II vorteilhafte pharmakodynamische Eigenschaften auf, die beispielsweise wie folgt belegt werden können:

Die zu untersuchenden Verbindungen der Formel I oder als Kontrolle die Vergleichsverbindung der Formel II werden in Dimethylsulfoxid (DMSO) in einer Konzentration von 240 mg/ml gelöst. Die resultierenden Lösungen werden mit 20% (w/v) Hydroxypropyl-β-cyclodextrin (HPβCD) verdünnt, um eine Konzentration der Testsubstanz von 12 mg/ml zu erhalten. Diese Lösung wird Mäusen durch künstliche Sonderernährung in einer Dosis von 120 mg/kg verabreicht. 60, 90 und 120 min nach der Verabreichung werden die Tiere getötet und Blut entnommen. Pro Zeitpunkt werden drei bis vier Tiere untersucht. Das Blut wird heparinisiert und für die Analyse wie folgt aufgearbeitet: Ein interner Standard wird zum heparinisierten Blut in einer Endkonzentration von 4 µM zugegeben. Das Blut wird zentrifugiert. 0,25 ml Plasma werden abgenommen und mit einem gleichen Volumen Acetonitril deproteinisiert. Nach Zentrifugation wird der Überstand unter Vakuum eingetrocknet und der Rückstand in 20 µl 3M NaCl-Lösung und 100 µl 0,05M Phthalat-Puffer mit einem pH von 3,0 suspendiert. Die Suspension wird erst mit 1 ml, dann mit 0,2 ml Diisopropylether extrahiert. Die Diisopropylether-Lösung wird zur Trockene eingedampft und der Rückstand in 50 % (v/v) wässrigem Acetonitril gelöst. Diese Lösung wird durch Reversed-Phase-HPLC untersucht.

Die Analyse mittels Reversed-Phase-HPLC wird mit einer 125 x 4,6 mm Nucleosil® C₁₈-Säule (Reversed-Phase-Material der Firma Macherey-Nagel, Düren, Bundesrep. Deutschland auf der Basis von mit Kohlenwasserstoffresten von 18 Kohlenstoffatomen derivatisiertem Kieselgel) durchgeführt, welche äquilibriert ist mit einer mobilen Phase von 50% Acetonitril in Wasser/0,1% Trifluoressigsäure. Die Flussrate ist 1 ml/min. Detektion erfolgt bei 215 nm. Standards für die Verbindungen in Blut werden analog den Blutproben aufgearbeitet und zur Erstellung von Standardkurven verwendet, aufgrund derer die in-vivo-Konzentrationen ermittelt werden.

Beim Vergleich der Verbindungen der Formel I mit denen der Formel II (aktive Komponente, worin anstelle von Acyloxy R₅ Hydroxy steht) sind folgende Resultate erhältlich: Die Konzentration der aktiven Komponente der Formel II im Blut von Mäusen kann nach oraler Verabreichung einer Verbindung der Formel I, beispielsweise der Verbindung der Formel I, worin R₁ Acetyl bedeutet, zu den meisten Zeitpunkten, vorzugsweise zu allen oben genannten Zeitpunkten, deutlich höher als bei Verabreichung der Verbindung der Formel II in unveresterter Form sein, beispielsweise mehr als dreimal so hoch, insbesondere mehr als 10 mal so hoch, in erster Linie etwa 20 bis etwa 150 mal so hoch. Alternativ oder ergänzend hierzu kann die Absorption der Verbindung der Formel I, beispielsweise derjenigen, worin R₁ Acetyl bedeutet, deutlich höher als die Absorption der Verbindung der Formel II sein, beispielsweise mehr als viermal so hoch. Auch ist es möglich, längere Zeit mit einer Verbindung der Formel I einen höheren Blutspiegel zu erhalten als mit der Verbindung der Formel II.

Vorzugsweise liegt die erreichbare Blutkonzentration des Wirkstoffes der Formel II bei den genannten Zeiten deutlich über der im Zelltest für die entsprechende Verbindung der Formel II ermittelten ED90 (siehe unten).

Die Verbindungen der Formel I, wie auch die Verbindungen der Formel II, können auch als Standards zum Vergleich verschiedener Testsysteme an unterschiedlichen Tierarten verwendet werden, was eine weitere kommerzielle Verwendbarkeit darstellt. Über den Vergleich von Blutspiegeln bei verschiedenen Tierarten können so beispielsweise verschiedene Tiermodelle verglichen werden.

Die aus den Verbindungen der vorliegenden Erfindung unter physiologischen Bedingungen freisetzbaren oder als Ausgangsverbindungen zur Herstellung von Verbindungen der Formel I dienenden Verbindungen der Formel II, oder deren Salze, weisen Hemmwirkung auf virale Aspartatproteasen, insbesondere gag-Protease-hemmende Wirkungen auf. In erster Linie hemmen sie in den nachfolgend beschriebenen Tests in Konzentrationen von 10⁻⁶ bis 10⁻⁹ mol/l die Wirkung der gag-Protease von HIV-1 und HIV-2 und sind daher geeignet als Mittel gegen durch diese oder verwandte Retroviren verursachte Krankheiten, wie z. B. gegen AIDS oder dessen Vorstufen.

Die Blutspiegel dieser Verbindungen der Formel II können analog den oben für Verbindungen der Formel I genannten Verfahren bestimmt werden.

Die Fähigkeit der Verbindungen der Formel II, die proteolytische Aktivität von z. B. HIV-1 Protease zu inhibieren, lässt sich beispielsweise gemäss dem von J. Hansen et al., The EMBO Journal 7, 1785-1791 (1988), beschriebenen Verfahren demonstrieren. Dabei wird die Hemmung der Wirkung der gag-Protease auf ein Substrat gemessen, das ein in E. coli exprimiertes Fusionsprotein aus dem gag-Vorläuferprotein und MS-2 ist. Das Substrat und seine Spaltprodukte werden durch Polyacrylamid-Gelelektrophorese getrennt und durch Immunoblotting mit monoklonalen Antikörpern gegen MS-2 sichtbar gemacht.

In einem noch einfacher zu handhabenden Test, der genaue quantitative Aussagen ermöglicht, wird als Substrat für die gag-Protease ein synthetisches Peptid eingesetzt, das der Spaltstelle des gag-Vorläuferproteins entspricht. Dieses Substrat und seine Spaltprodukte können durch Hochdruckflüssig-Chromatographie (HPLC) gemessen werden.

Beispielsweise wird analog zur von Richards, A.D, et al. , J. Biol. Chem. 265,(14), 7733-7736 (1990) beschriebenen Methode als Substrat für eine rekombinante HIV-1-Protease (Herstellung gemäss Billich, S., et al., J. Biol. Chem. 263(34), 17905 - 17908 (1990)) ein synthetisches chromophores Peptid (z. B. HKARVL[NO₂]FEANleS (Bachem, Schweiz) oder ein Icosapeptid wie RRSNQVSQNYPIVQNIQGRR (hergestellt durch Peptidsynthese nach bekannten Verfahren: J. Schneider et al., Cell 54, 363-368 (1988)) eingesetzt, das einer der Spaltstellen des gag-Vorläuferproteins entspricht. Dieses Substrat und seine Spaltprodukte können durch Hochdruckflüssig-Chromatographie (HPLC) gemessen werden.

Hierzu wird ein zu testender Hemmstoff der Formel II in Dimethylsulfoxid gelöst; der Enzymtest wird durchgeführt, indem geeignete Verdünnungen des Hemmstoffes in 20 mM β-Morpholinoethansulfonsäure (MES)-Puffer pH 6,0 zum Assay-Mix aus 67,2 µM des oben genannten chromophoren Peptides in 0,3 M Natriumacetat, 0,1 M NaCl pH 7,4 oder 122 µM des oben genannten Icosapeptids in 20 mM MES-Puffer pH 6,0 zugegeben werden. Die Grösse der Ansätze beträgt 100 µl. Die Reaktion wird gestartet durch Zugabe von im ersten Fall 2 µl, im zweiten Fall 10 µl HIV-I-Protease und im ersten Fall nach 15 min durch Zugabe von 100 µl 0,3 M HClO₄, im zweiten Fall nach einer Stunde Inkubation bei 37 °C durch Zugabe von 10 µl 0,3 M HClO₄ gestoppt. Die Reaktionsprodukte werden nach Abzentrifugieren der Probe für 5 min bei 10 000 x g in 100 µl (Ansatz mit chromophorem Peptid) bzw. 20 µl (Icosapeptid-Ansatz) des erhaltenen Überstandes und nach Auftragen auf eine 125 x 4,6 mm Nucleosil® C18-5µ-HPLC-Säule (Macherey & Nagel, Düren) und Elution quantifiziert durch Ausmessung der Peaks für das Spaltprodukt der Formel II bei 280 nm (Ansatz mit chromophorem Peptid) oder bei 215 nm (Ansatz mit Icosapeptid), Gradient: 100 % El.1 -> 50 % El.1/50 % El.2 (El.1: 75 % Acetonitril, 90 % H₂O, 0,1 % Trifluoressigsäure (TFA); El.2: 75 % Acetonitril, 25 % H₂O, 0,08 % TFA) innerhalb von 15 min; Durchflussrate 1 ml/min.

Hierbei werden vorzugsweise IC₅₀-Werte (IC₅₀ = diejenige Konzentration, welche die Aktivität der HIV-1-Protease gegenüber einer Kontrolle ohne Hemmstoff um 50 % senkt) von etwa 10⁻⁶ bis 10⁻⁹ M, insbesondere von etwa 10 ⁻⁷ bis etwa 10⁻⁸ M, ermittelt.

In einem weiteren Test kann gezeigt werden, dass die aus den Verbindungen der Formel I freisetzbaren Verbindungen der Formel II Zellen, die normalerweise von HIV infiziert werden, vor einer solchen Infektion schützen oder zumindest eine solche Infektion verlangsamen. Dabei wird die menschliche T-Zell-Leukämie Zellinie MT-2 (Science 229, 563 (1985)), die extrem empfindlich für den zytopathogenen Effekt von HIV ist, mit HIV allein oder mit HIV in Gegenwart der erfindungsgemässen Verbindungen inkubiert und nach einigen Tagen die Lebensfähigkeit der so behandelten Zellen beurteilt.

Hierzu werden die MT-2-Zellen in RPMI 1640-Medium (Gibco, Schweiz; RPMI 1640 enthält ein Aminosäurengemisch ohne L-Gln), das mit 10% hitzeinaktiviertem fetalem Kälberserum, L-Glutamin, Hepes (2-[4-(2-Hydroxyethyl)-1-piperazino]-ethansulfonsäure) und Standardantibiotika supplementiert ist, bei 37 °C in befeuchteter Luft mit 5% CO₂ gehalten. 50 µl der jeweiligen Testverbindung in Kulturmedium und 100 µl HIV-1 in Kulturmedium (800 TCID50/ml) (TCID50 = Tissue Culture Infectious Dose 50 = Dosis, die 50% der MT-2-Zellen infiziert) werden zu 4x10³ exponentiell wachsenden MT-2-Zellen in 50 µl Kulturmedium pro Vertiefung auf 96-Loch-Mikrotiterplatten gegeben. Parallele Ansätze auf einer weiteren Mikrotiterplatte mit Zellen und Testverbindung erhalten 100 µl Kulturmedium ohne Virus. Nach 4 Tagen Inkubation wird in 10 µl Zellüberstand die Reverse-Transkriptase (RT)-Aktivität ermittelt. Die RT-Aktivität wird bestimmt in 50 mM Tris (α,α,α-Tris(hydroxymethyl)-methylamin, Ultra pur, Merck, Bundesrepublik Deutschland) pH 7,8; 75 mM KCl, 2 mM Dithiothreitol, 5 mM MgCl₂; 0,05% Nonidet P-40 (Detergens; Sigma, Schweiz); 50 µg/ml Polyadenylic Acid (Pharmacia, Schweden); 1,6 µg/ml dT(12-18) (Sigma, Schweiz). Die Mischung wird durch einen Acrodisc-Filter (0,45 µ; Gellman Science Inc, Ann Arbor) abfiltriert und bei -20 °C aufbewahrt. Zu Aliquoten dieser Lösung werden 0,1% (v/v) [alpha-³²P]dTTP zum Erzielen einer radioaktiven Endaktivität von 10 µCi/ml zugegeben. 10 µl des Kulturüberstandes werden auf eine neue 96-Loch-Mikrotiterplatte übertragen und hierzu 30 µl des genannten RT-Cocktails gegeben. Nach Mischen wird die Platte für 1,5 bis 3 h bei 37 °C inkubiert. 5 µl dieser Reaktionsmischung werden auf Whatman DE81-Papier (Whatman) überführt. Die getrockneten Filter werden 3-mal für 5 min mit 300 mM NaCl/25 mM Tri-Natriumcitrat und 1-mal mit 95% Ethanol gewaschen und erneut luftgetrocknet. Die Auswertung erfolgt in einem Matrix Packard 96well counter (Packard). Die ED90-Werte werden errechnet und als die niedrigste Konzentration der jeweiligen Testverbindung definiert, welche die RT-Aktivität um 90% im Vergleich zu nicht mit der Testsubstanz behandelten Zellansätzen senkt. Die RT-Aktivität ist dabei ein Mass für die HIV-1-Vermehrung.

Die Verbindungen mit Hydroxy anstelle von R₅ zeigen hierbei eine ED90 von etwa 10⁻⁵ bis 10⁻⁸ M, vorzugsweise von etwa 5 x 10⁻⁷ bis 5 x 10⁻⁸ M.

Bei den im folgenden genannten Gruppen von Verbindungen der Formel I können in sinnvoller Weise, z. B. zur Ersetzung allgemeinerer durch speziellere Definitionen, Definitionen von Resten aus den oben genannten allgemeinen Definitionen eingesetzt werden.

Bevorzugt ist eine Verbindung der Formel I, worin
R₁ und R₉ unabhängig voneinander
   (a) Wasserstoff;
   (b) Niederalkanoyl, wie Formyl, Acetyl, Propionyl, Butyryl oder Pivaloyl, insbesondere Acetyl;
   (c) Arylniederalkanoyl, worin Aryl 6 - 14 Kohlenstoffatome hat, vorzugsweise als Phenyl, Indenyl, Indanyl, Naphthyl, Anthryl, Phenanthryl, Acenaphthyl oder Fluorenyl, und unsubstituiert oder insbesondere durch Niederalkyl, z. B. Methyl, Ethyl oder Propyl, Halogenniederalkyl, z. B. Trifluormethyl, Phenyl, 1- oder 2-Naphthyl, Hydroxy, Niederalkoxy, z. B. Methoxy, Carbamoylniederalkoxy, N-Niederalkylcarbamoylniederalkoxy oder N,N-Diniederalkylcarbamoylniederalkoxy, Amino, Mono- oder Diniederalkylamino, Niederalkanoylamino, z. B. Pivaloylamino, Halogen, z. B. Fluor, Chlor oder Brom, Carboxy, Niederalkoxycarbonyl, wie tert-Butoxycarbonyl, Phenyl-, Naphthyl- oder Fluorenylniederalkoxycarbonyl, wie Benzyloxycarbonyl, Niederalkanoyl, Sulfo, Niederalkylsulfonyl, z. B. Methylsulfonyl, Phosphono, Hydroxyniederalkoxy-phosphoryl oder Diniederalkoxyphosphoryl, Carbamoyl, Mono- oder Diniederalkylcarbamoyl, Sulfamoyl, Mono- oder Diniederalkylaminosulfonyl, Nitro und/oder Cyano ein- oder bis zu dreifach substituiert sein kann,
      und worin Niederalkanoyl unsubstituiert oder substituiert ist durch Carbamoyl oder durch einen oder zwei aus Niederalkyl, Carboxyniederalkyl, Niederalkoxycarbonyl-niederalkyl, Diniederalkylamino-niederalkyl, Hydroxyniederalkyl und Diniederalkoxyniederalkyl ausgewählte Reste am Stickstoff substituiertes Carbamoyl, vorzugsweise, wie unter Arylniederalkanoyl oben bei den allgemeinen Definitionen beschrieben,
      beispielsweise 4-Chlor-, 4-Methoxy- oder 4-Nitrobenzoyl, Naphthylcarbonyl, wie α- oder β-Naphthylcarbonyl oder über beide Carbonylgruppen an die Aminogruppe gebundenes 1,8-Naphthalindicarbonyl, Phenylniederalkanoyl, wie Phenylacetyl oder 3-Phenylpropionyl, Niederalkylphenyl-acetyl, wie 4-Methylphenylacetyl, Niederalkoxyphenylacetyl, wie 4-Methoxyphenylacetyl, 3-(p-Hydroxyphenyl)-propionyl, Diphenylacetyl, Di-(4-methoxyphenyl)-acetyl, Triphenylacetyl, 2,2-Dibenzylacetyl, 3-α- oder 3-β-Naphthylpropionyl, Phenylniederalkanoyl, worin der Niederalkanoylrest durch Carbamoyl substituiert ist, wie 2-Carbamoyl-3-phenylpropionyl, z.B. 2-(R,S)-Carbamoyl-3-phenylpropionyl, 3-Phenyl- oder 3-α-Naphthyl-2-carbamoylpropionyl, 3-Phenyl- oder 3-α-Naphthyl-2-tert-butylcarbamoyl-propionyl, 3-Phenyl- oder 3-α-Naphthyl-2-(2-dimethylaminoethyl)carbamoyl-propionyl, in erster Linie Phenylniederalkanyol, wie Phenylacetyl, oder Phenyl-niederalkanoyl, worin der Niederalkanoylrest durch Carbamoyl substituiert ist, wie 2-Carbamoyl-3-phenyl-propionyl, z.B. 2(R,S)-Carbamoyl-3-phenylpropionyl;
   (d) Heterocyclylniederalkanoyl, worin Heterocyclyl Thienyl, Furyl, Pyrrolyl, Imidazolyl, Pyrazolyl, Oxazolyl, Thiazolyl, Tetrazolyl, Pyridyl, Pyrazinyl, Pyrimidinyl, Pyridazinyl, Indolyl, Benzimidazolyl, Chinolyl, Isochinolyl, 3,1-Benzfuranyl, Cyclohexa[b]pyrrolyl, Cyclohexa[b]pyridyl, Cyclohexa[b]pyrazinyl, Cyclohexa[b]pyrimidinyl, Pyrrolidinyl, Pyrrolinyl, Imidazolidyl, Piperidyl, Piperazinyl, Morpholinyl, Thiomorpholinyl, S,S-Dioxo-thiomorpholinyl, Indolinyl, Isoindolinyl, 4,5,6,7-Tetrahydroindolyl, 1,2,3,4-Tetrahydrochinolyl oder 1,2,3,4-Tetrahydroisochinolyl bedeutet, welches über ein Ringkohlenstoffatom oder ein Ringstickstoffatom, im Falle von gesättigten Heterocyclen vorzugsweise über ein Ringstickstoffatom gebunden ist, insbesondere Indolylcarbonyl, wie 2-, 3- oder 5-Indolylcarbonyl, Chinolylniederalkanoyl, z.B. Chinolylcarbonyl, wie 2-, 3- oder 4-Chinolylcarbonyl, Piperidylcarbonyl, wie Piperidinocarbonyl oder 2-, 3- oder 4-Piperidylcarbonyl, Piperazinylcarbonyl, wie Piperazin-1-yl-carbonyl, Morpholinylniederalkanoyl, z.B. Morpholinoniederalkanoyl, z.B. Morpholinocarbonyl, wie Morpholinocarbonyl, Thiomorpholinylniederalkanoyl, z.B. Thiomorpholinoniederalkanoyl, wie Thiomorpholinocarbonyl, wie Thiomorpholinocarbonyl, S,S-Dioxothiomorpholinylcarbonyl, wie S,S-Dioxothiomorpholinocarbonyl, Tetrazolylniederalkanoyl, wie 3-(Tetrazol-1-yl)-propionyl, und Pyridylniederalkanoyl, z. B. Pyrridylcarbonyl, wie 2-, 3- oder 4-Pyridylcarbonyl, oder Pyridylacetyl, wie 2-, 3- oder 4-Pyridylacetyl, in erster Linie ausgewählt aus Morpholinoniederalkanoyl, wie Morpholinocarbonyl, Thiomorpholinoniederalkanoyl, wie Thiomorpholino-carbonyl, Pyridylniederalkanoyl, wie 2-, 3- oder 4-Pyridylacetyl, und Tetrazolylniederalkanoyl, wie 3-Tetrazol-1-yl-propionyl;
   (e) (Niederalkoxyniederalkoxy)niederalkanoyl;
   (f) durch Heterocyclylniederalkanoyl am Aminostickstoff substituiertes Aminoniederalkanoyl, worin Heterocyclylniederalkanoyl unabhängig wie oben für Heterocyclylniederalkanoyl R₁ oder R₉ definiert ist, insbesondere durch N-Morpholino- oder N-Thiomorpholinocarbonyl am Aminostickstoff substituiertes Aminoniederalkanoyl, in erster Linie N-Morpholino- oder N-Thiomorpholino-carbonylamino-niederalkanoyl, wie N-Morpholino- oder N-Thiomorpholino-carbonylamino-acetyl;
   (g) Halogenniederalkanoyl, welches bis zu drei Halogenatome enthält, in erster Linie α-Halogenacetyl, wie α-Fluor-, α-Chlor-, α-Brom-, α-Iod-, α,α,α Trifluor- oder α,α,α-Trichloracetyl, oder Halogenpropionyl, wie β-Chlor- oder β-Brompropionyl, insbesondere Trifluoracetyl;
   (h) (N-Heterocyclylniederalkyl-carbamoyl)-niederalkanoyl, worin Heterocyclyl vorzugsweise aus Pyrrolyl, Furyl, Thienyl, Imidazolyl, Pyrazolyl, Oxazolyl, Thiazolyl, Pyridyl, Pyrazinyl, Pyrimidinyl, Indolyl, Chinolyl und Isochinolyl, die auch ganz oder teilweise gesättigt sein können, aus Morpholinyl und aus Thiomorpholinyl ausgewählt ist, in erster Linie 2-(N-Morpholinoniederalkyl-carbamoyl)-niederalkanoyl, wie 2(R,S)-(N-(2-morpholinoethyl)-carbamoyl)-3-methyl-butyryl, oder 2-(N-(Pyridylniederalkyl)-carbamoyl)-niederalkanoyl, wie 2(R,S)-(N-(2-pyridylmethyl)-carbamoyl)-3-methyl-butyryl;
   (i) Niederalkoxycarbonyl, in erster Linie Methoxy-, Ethoxy-, Isopropoxy-, Isobutoxy- oder tert-Niederalkoxycarbonyl, z.B. Methoxy-carbonyl, tert-Butoxycarbonyl oder Isobutoxycarbonyl;
   (j) Arylniederalkoxycarbonyl, worin Aryl Phenyl, Biphenylyl, 1- oder 2-Naphthyl, Fluorenyl oder durch Niederalkyl, z. B. Methyl oder tert-Butyl, Hydroxy, Niederalkoxy, z. B. Methoxy, Ethoxy oder tert-Butoxy, Halogen, z. B. Chlor oder Brom, und/oder Nitro mono- oder mehrfach, vorzugsweise bis zu dreifach, vor allem einfach substituiertes Phenyl ist, z. B. Phenylniederalkoxycarbonyl, wie Benzyloxycarbonyl oder 4-Methoxybenzyloxycarbonyl, in erster Linie Phenylniederalkoxycarbonyl, wie Benzyloxycarbonyl;
   (k) Heterocyclylniederalkoxycarbonyl, worin Heterocyclyl aus Pyrrolyl, Furyl, Thienyl, Imidazolyl, Pyrazolyl, Oxazolyl, Thiazolyl, Pyridyl, Pyrazinyl, Pyrimidinyl, Indolyl, Chinolyl und Isochinolyl, die auch ganz oder teilweise gesättigt vorliegen können, aus Morpholinyl und aus Thiomorpholinyl ausgewählt ist und unsubstituiert oder durch Niederalkyl, wie durch Methyl, substituiert ist, z.B. 2-Furylmethoxycarbonyl, oder Tetrahydrofurylniederalkoxycarbonyl, wie 2-Tetrahydrofuryl-methoxycarbonyl, in erster Linie Tetrahydrofuryl-niederalkyloxycarbonyl, wie 2(R,S)-Tetrahydrofurylmethoxycarbonyl;
   (l) Niederalkenyloxycarbonyl, worin der Niederalkenylrest an den Sauerstoff über ein gesättigtes Kohlenstoffatom gebunden ist, z.B. Allyloxycarbonyl;
   (m) Niederalkoxyniederalkoxycarbonyl, wie 2-Methoxyethoxycarbonyl;
   (n) (Niederalkoxyniederalkoxy)niederalkoxycarbonyl, wie 2-(2-Methoxyethoxy)ethoxycarbonyl,
   (o) Niederalkansulfonyl , z.B. Methan- oder Ethansulfonyl, vor allem Methansulfonyl;
   (p) Heterocyclylsulfonyl (Heterocyclyl-SO₂-), worin Heterocyclyl aus Pyrrolyl, Furyl, Thienyl, Imidazolyl, Pyrazolyl, Oxazolyl, Thiazolyl, Pyridyl, Pyrazinyl, Pyrimidinyl, Indolyl, Chinolyl und Isochinolyl, die auch ganz oder teilweise gesättigt vorliegen können, aus Morpholinyl und aus Thiomorpholinyl ausgewählt ist und unsubstituiert oder durch Niederalkyl, wie Methyl, substituiert sein kann, wie Morpholinosulfonyl, Thiomorpholinosulfonyl, Piperidinosulfonyl, oder Piperazinosulfonyl;
   (q) Carbamoyl;
   (r) N-Heterocyclylniederalkyl-N-niederalkyl-carbamoyl, worin Heterocyclyl unabhängig einen der oben bei der Definition von Heterocyclylniederalkanoyl R₁ oder R₉ genannten Reste, insbesondere Pyridyl, wie 2-, 3- oder 4-Pyridyl, bedeutet, vorzugsweise N-Pyridylniederalkyl-N-niederalkyl-carbamoyl, wie N-(2-, 3- oder 4-Pyridylmethyl)-N-methyl-carbamoyl; oder (s) einen Acylrest einer Aminosäure, deren Aminofunktion frei oder durch einen der übrigen bisher für R₁ und R₉ genannten Reste ausser einem der genannten Aminoacylreste acyliert ist, bedeuten, wobei die Aminosäurereste aus den über das Carbonyl ihrer 1-Carboxygruppe gebundenen Radikalen der Aminosäuren Glycin, Alanin, Valin, Norvalin, Leucin, Isoleucin, Norleucin, Serin, Homoserin, Threonin, Methionin, Cystein, Prolin, trans-3- und trans-4-Hydroxyprolin, Phenylalanin, Tyrosin, 4-Aminophenylalanin, 4-Chlorphenylalanin, 4-Carboxyphenylalanin, β-Phenylserin, Phenylglycin, α-Naphthylalanin, Cyclohexylalanin, Cyclohexylglycin, Tryptophan, Indolin-2-carbonsäure, 1,2,3,4-Tetrahydroisochinolin-3-carbonsäure, Asparaginsäure, Asparagin, Aminomalonsäure, Aminomalonsäure-monoamid, Glutaminsäure, Glutamin, Histidin, Arginin, Lysin, δ-Hydroxylysin, Ornithin, 3-Aminopropansäure, α,γ-Diaminobuttersäure und α,β-Diaminopropionsäure, ausgewählt sind, noch stärker bevorzugt aus den Resten einer Aminosäure ausgewählt aus Valin, Alanin, Leucin, Isoleucin, Glycin, Glutaminsäure und Asparagin, wobei (ausser Glycin) jede der genannten Aminosäuren in der D-, L- oder (D,L)-, vorzugsweise (ausser Val, das auch in der (D)- oder (D,L)-Form vorliegen kann) in der L-Form vorliegen kann; und wobei die α-Aminogruppe unsubstituiert oder durch einen der oben unter (a) bis (r) für R₁ und R₉ genannten Reste N-acyliert ist, vor allem durch Niederalkanoyl, Phenylniederalkanyol, wie Phenylacetyl, Phenylniederalkanoyl, worin der Niederalkanoylrest durch Carbamoyl substituiert ist, wie 2(R,S)-Carbamoyl-3-phenyl-propionyl, Morpholinoniederalkanoyl, wie Morpholinocarbonyl, Thiomorpholinoniederalkanoyl, wie Thiomorpholino-carbonyl, Pyridylniederalkanoyl, wie 2-, 3- oder 4-Pyridylacetyl, Chinolinyl-niederalkanoyl, wie Chinolin-2-ylcarbonyl, Tetrazolylniederalkanoyl, wie 3-Tetrazol-1-yl-propionyl, Niederalkoxyniederalkoxyniederalkanoyl, durch N-Morpholino- oder N-Thiomorpholinocarbonyl am Aminostickstoff substituiertes Aminoniederalkanoyl, z. B. N-Morpholino- oder N-Thiomorpholino-carbonylamino-niederalkanoyl, wie N-Morpholino- oder N-Thiomorpholino-carbonylamino-acetyl, Halogenniederalkanoyl, welches bis zu drei Halogenatome enthält, z. B. α-Halogenacetyl, wie α-Fluor-, α-Chlor-, α-Brom-, α-Iod-, α,α,α-Trifluor- oder α,α,α-Trichloracetyl, oder Halogenpropionyl, wie β-Chlor- oder β-Brompropionyl, insbesondere Trifluoracetyl, 2-(N-Morpholinoniederalkyl-carbamoyl)-niederalkanoyl, wie 2(R,S)-(N-(2-Morpholinoethyl)-carbamoyl-3-methyl-butyryl, 2-(N-(Pyridylniederalkyl)-carbamoyl)-niederalkanoyl, wie 2(R,S)-(N-(2-Pyridylmethyl)-carbamoyl)-niederalkanoyl, Niederalkoxycarbonyl, Phenylniederalkoxycarbonyl, Tetrahydrofuryl-niederalkyloxycarbonyl, wie 2(R,S)-Tetrahydrofurylmethoxycarbonyl, Niederalkenyloxycarbonyl, worin der Niederalkenylrest an den bindenden Sauerstoff über ein gesättigtes Kohlenstoffatom gebunden ist, Niederalkoxyniederalkoxycarbonyl, (Niederalkoxyniederalkoxy)niederalkoxycarbonyl, Niederalkansulfonyl, Morpholinosulfonyl, Thiomorpholinosulfonyl, Piperidinosulfonyl, 4-Methylpiperazinylsulfonyl oder Piperazinosulfonyl oder N-Pyridylniederalkyl-N-niederalkyl-carbamoyl, wie N-(2-, 3- oder 4-Pyridylmethyl)-N-methyl-carbamoyl; bedeuten,
   mit der Massgabe, dass höchstens einer der beiden Reste R₁ oder R₉ Wasserstoff bedeuten kann,
R₂, R₄, R₆ und R₈ Wasserstoff bedeuten,
R₃
   (i) Niederalkyl, wie Isobutyl oder n-Butyl;
   (ii) C₃-C₇Cycloalkylniederalkyl, worin C₃-C₇Cycloalkyl unsubstituiert oder durch Niederalkyl, wie Isopropyl, Halogenniederalkyl, wie Trifluormethyl, Hydroxy, Niederalkoxy, Amino, Mono- oder Diniederalkylamino, Halogen, wie Fluor, Chlor oder Brom, Nitro und/oder Cyano ein- oder bis zu dreifach substituiert ist und an Niederalkyl, insbesondere Methyl, Ethyl, n-Propyl, Isopropyl, N-Butyl, Isobutyl oder tert-Butyl, vorzugsweise endständig, gebunden ist, wie Cyclobutyl-, Cyclopentyl-, Cyclohexyl- oder Cycloheptyl-niederalkyl, wie -methyl oder -ethyl, in erster Linie Cyclohexylniederalkyl, vor allem Cyclohexylmethyl; oder
   (iii) Arylniederalkyl bedeutet, worin Aryl unabhängig wie in Arylniederalkanoyl R₁ oder R₉ definiert ist; und insbesondere Phenyl bedeutet, welches unsubstituiert oder durch Niederalkyl, z. B. Methyl, Ethyl oder Isopropyl, Halogenniederalkyl, wie Trifluormethyl, Phenyl, 1- oder 2-Naphthyl, Hydroxy, Niederalkoxy, z. B. Methoxy, Carbamoylniederalkoxy, N-Niederalkylcarbamoylniederalkoxy oder N,N-Diniederalkylcarbamoylniederalkoxy, Amino, Mono- oder Diniederalkylamino, Niederalkanoylamino, z. B. Pivaloylamino, Halogen, z. B. Fluor oder Chlor, Carboxy, Niederalkoxycarbonyl, Benzyl-, Naphthyl- oder Fluorenylniederalkoxycarbonyl, Niederalkanoyl, Sulfo, Niederalkylsulfonyl, Phosphono, Hydroxy-niederalkoxy-phosphoryl oder Diniederalkoxyphosphoryl, Carbamoyl, Mono- oder Diniederalkylcarbamoyl, Sulfamoyl, Mono- oder Diniederalkylsulfamoyl, Nitro und/oder Cyano ein- oder bis zu dreifach substituiert sein kann, in erster Linie Phenylniederalkyl, welches unsubstituiert oder durch die genannten Substituenten substituiert ist, vor allem Benzyl, 2-Phenylethyl, 3-Phenylpropyl, 4- Fluor-, 4-Trifluormethyl-, 4-Cyano-, 4-Methoxy- oder 4-Hydroxybenzyl, besonders bevorzugt Phenylniederalkyl, vor allem wie zuletzt definiert, oder ferner oder insbesondere
      Thienylmethyl, wie 2-Thienylmethyl, bedeutet,
R₅ Niederalkanoyloxy, Octanoyloxy, Nonanoyloxy, Decanoyloxy, Undecanoyloxy, Dodecanoyloxy, Hydroxyniederalkanoyloxy, Niederalkoxyniederalkanoyloxy, Niederalkanoyloxyniederalkanoyloxy, Halogenniederalkanoyloxy, z.B. α-Halogenacetyloxy, wie α-Chlor-, α-Brom-, α-Iod- α,α,α-Trifluor oder α,α,α-Trichloracetyloxy, Carboxyniederalkanoyloxy, Niederalkoxycarbonylniederalkanoyloxy, Carbamoylniederalkanoyloxy, Niederalkylcarbamoylniederalkanoyloxy, Diniederalkylcarbamoylniederalkanoyloxy, Hydroxy-carboxy-niederalkanoyloxy, Hydroxy-niederalkoxycarbonyl-niederalkanoyloxy, Dihydroxy-carboxy-niederalkanoyloxy, Dihydroxy-niederalkoxycarbonyl-niederalkanoyloxy, Pyrrolylcarbonyloxy, wie 2- oder 3-Pyrrolylcarbonyloxy, Furylniederalkanoyloxy, z.B. Furylcarbonyloxy, wie 2-Furylcarbonyloxy, Thienylcarbonyloxy, z.B. 2-Thienylcarbonyloxy, Imidazolylniederalkanoyloxy, z.B. Imidazolylcarbonyloxy, wie 4-Imidazolylcarbonyloxy, Imidazolylacetoxy, wie 4-Imidazolylacetoxy, Imidazolylpropionyloxy, wie 3-(4-Imidazolylpropionyloxy, Pyridylniederalkanoyloxy, wie Pyridylcarbonyloxy, z.B. 2-, 3- oder 4-Pyridylcarbonyloxy, Indolylcarbonyloxy, z.B. 2-, 3- oder 5-Indolylcarbonyloxy, Chinolylniederalkanoyloxy, wie Chinolinylcarbonyloxy, wie Chinolin-2-ylcarbonyloxy, Pyrrolidinylcarboxy, wie Pyrrolidinyl-3-carbonyloxy, Piperidinylcarbonyloxy, z.B. 2-, 3- oder 4-Piperidinylcarbonyloxy, Morpholinocarbonyloxy, Thiomorpholinocarbonyloxy, Morpholinoacetyloxy, Thiomorpholinoacetyloxy oder 4-Niederalkyl-1-piperazinoacetyloxy, wie 4-Methyl-piperazinoacetyloxy, Niederalkenoyloxy (worin der Niederalkenoylrest vorzugsweise an das bindende Sauerstoffatom über ein gesättigtes Kohlenstoffatom gebunden ist), z.B. Acryloyloxy, Vinylacetyloxy, Crotonoyloxy oder 3- oder 4-Pentenoyloxy, Niederalkinoyloxy, z.B. Propioloyloxy oder 2- oder 3-Butinoyloxy, C₃-C₈-Cycloalkylcarbonyloxy, C₃-C₈-Cycloalkylacetoxy, Phenylniederalkanoyloxy, z.B. Benzoyloxy, Phenylacetoxy oder 3-Phenylpropionyloxy, welches im Phenylrest unsubstituiert, mono- oder mehrfachsubstituiert durch Niederalkyl, z.B. Methyl, Haloniederalkyl, wie Chlor- oder Brommethyl, Halogen, z.B. Fluor oder Chlor, Hydroxy, Niederalkoxy, z.B. Methoxy, Piperidinomethyl, Piperazin-1-ylmethyl, 4-Niederalkyl-piperazin-1-ylmethyl, wie 4-Methyl- oder 4-Ethyl-piperazin-1-ylmethyl, 4-Niederalkanoyl-piperazin-1-ylmethyl, wie 4-Acetyl-piperazin-1-ylmethyl, Morpholinoniederalkyl, wie Morpholinomethyl, Thiomorpholinomethyl, Cyano und/oder Nitro, insbesondere durch einen der genannten Substituenten monosubstituiert sein kann, oder das über eine Carbonyloxygruppe, welche das Carbonyl aus der Carboxygruppe der betreffenden Aminosäure enthält, gebundene Radikal einer Aminosäure bedeutet, welche aus Glycin, Alanin, 2-Aminobuttersäure, 3-Aminobuttersäure, 4-Aminobuttersäure, 3-Aminopentansäure, 4-Aminopentansäure, 5-Aminopentansäure, 3-Aminohexansäure, 4-Aminohexansäure, 5-Aminohexansäure, Valin, Norvalin, Leucin, Isoleucin, Norleucin, Serin, Homoserin, Threonin, Methionin, Cystein, Prolin, Phenylalanin, Tyrosin, Cyclohexylalanin, Tryptophan, Asparaginsäure, Asparagin, Glutaminsäure, Glutamin, Histidin, Arginin, Lysin, Ornithin, 3-Aminopropansäure, α,γ-Diaminobuttersäure und α,β-Diaminopropionsäure ausgewählt ist; besonders bevorzugt den über Carbonyloxy gebundenen Rest einer aliphatischen Aminosäure ausgewählt aus Alanin, Valin, Norvalin, Leucin, 3-Aminopropionsäure, 2-Aminobuttersäure, 3-Aminobuttersäure, 4-Aminobuttersäure, 3-Aminopentansäure, 4-Aminopentansäure, 5-Aminopentansäure, 3-Aminohexansäure, 4-Aminohexansäure, 5-Aminohexansäure und Isoleucin oder einer Aminosäure ausgewählt aus Glycin, Asparagin, Glutamin, Methionin, Lysin, Histidin, Prolin und Phenylalanin, wobei (ausser in Fällen, wo kein asymmetrisches Kohlenstoffatom vorliegt, z.B. bei Glycin) jede der genannten Aminosäuren in der D-, L- oder (D,L)-, vorzugsweise in der L-Form vorliegen kann; und worin eine Aminogruppe unsubstituiert oder ein- oder zweifach N-alkyliert durch Niederalkyl, wie Methyl, n-Propyl oder n-Butyl, durch Pyridylniederalkyl, wie 2-,3- oder 4-Pyridylmethyl, und/oder durch Phenylniederalkyl, wie Benzyl, und/oder N-acyliert durch Niederalkanoyl, vor allem durch Acetyl, Propionyl oder Pivaloyl, durch Phenylniederalkanoyl, wie Benzoyl oder Phenylacetyl, durch Niederalkoxycarbonyl, wie tert-Butoxycarbonyl, oder durch Phenylniederalkoxycarbonyl, wie Benzyloxycarbonyl; vorliegt;
   und
R₇ unabhängig von R₃ einen der dort definierten Reste bedeutet, in erster Linie Niederalkyl, vor allem Isobutyl oder n-Butyl, C₃-C₇-Cycloalkylniederalkyl, vor allem Cyclohexylniederalkyl, wie Cyclohexylmethyl, oder Arylniederalkyl, wie für Arylniederalkyl R₃ beschrieben, vor allem Phenylniederalkyl, welches unsubstituiert oder durch die genannten Substituenten substituiert ist, wie Benzyl, 2-Phenylethyl, 3-Phenylpropyl, 4- Fluor-, 4-Trifluormethyl-, 4-Cyano-, 4-Methoxy- oder 4-Hydroxybenzyl, bedeutet, besonders bevorzugt Phenylniederalkyl, vor allem wie zuletzt definiert,
oder ein Salz davon, sofern wenigstens eine salzbildende Gruppe vorliegt.

In erster Linie bevorzugt ist eine Verbindung der Formel I, worin R₁ und R₉ unabhängig voneinander
(a) Wasserstoff,
(b) Niederalkanoyl, wie Acetyl oder Propionyl,
(c) Phenylniederalkanoyl, wie Phenylacetyl,
(d) Phenylniederalkanoyl, worin der Niederalkanoylrest durch Carbamoyl substituiert ist, wie 2-Carbamoyl-3-phenyl-propionyl,
(e) Morpholinoniederalkanoyl, wie Morpholinocarbonyl,
(f) Thiomorpholinoniederalkanoyl, wie Thiomorpholino-carbonyl,
(g) Pyridylniederalkanoyl, wie 2-, 3- oder 4-Pyridylacetyl, (h) Chinolylniederalkanoyl, wie Chinolin-2-ylcarbonyl,
(i) Tetrazolylniederalkanoyl, wie 3-Tetrazol-1-yl-propionyl,
(j) durch N-Morpholino- oder N-Thiomorpholinocarbonyl am Aminostickstoff substituiertes Aminoniederalkanoyl, z.B. N-Morpholino- oder N-Thiomorpholinocarbonylamino-niederalkanoyl, wie N-Morpholino- oder N-Thiomorpholinocarbonylamino-acetyl,
(k) Halogenniederalkanoyl, welches bis zu drei Halogenatome enthält, wie Trifluoracetyl,
(l) 2-(N-Morpholinoniederalkyl-carbamoyl)-niederalkanoyl, wie 2(R,S)-(N-(2-Morpholinoethyl)-carbamoyl-3-methyl-butyryl, (m) 2-(N-Pyridylniederalkyl-carbamoyl)-niederalkanoyl, wie 2(R,S)-(N-(2-pyridylmethyl)-carbamoyl)-3-methyl-butyryl,
(n) Niederalkoxycarbonyl, wie Methoxy-, Ethoxy, Isobutoxy- oder tert-Niederalkoxycarbonyl,
(o) Phenylniederalkoxycarbonyl, wie Benzyloxycarbonyl,
(p) Tetrahydrofuryl-niederalkoxycarbonyl, wie 2-Tetrahydrofuryl-methoxycarbonyl,
(q) Niederalkenyloxycarbonyl (vorzugsweise mit über ein gesättigtes Kohlenstoffatom an den bindenden Sauerstoff gebundenem Niederalkenyl), wie Allyloxycarbonyl,
(r) Niederalkoxyniederalkoxycarbonyl, wie 2-Methoxyethoxycarbonyl,
(s) (Niederalkoxyniederalkoxy)niederalkoxycarbonyl, wie 2-(2-Methoxyethoxy)ethoxycarbonyl,
(t) Niederalkansulfonyl, z.B. Methan- oder Ethansulfonyl,
(u) Morpholinosulfonyl,
(v) Thiomorpholinosulfonyl,
(w) N-Pyridylniederalkyl-N-niederalkyl-carbamoyl, wie N-(2-Pyridylmethyl)-N-methyl-aminocarbonyl, oder
(x) einen über das Carbonyl seiner Carboxygruppe gebundenen Acylrest einer Aminosäure ausgewählt aus Glycin, Alanin, Valin, Leucin, Isoleucin, Glutaminsäure und Asparagin in der (D)-, (L)- oder (D,L)-Form (ausser Glycin), bedeuten, worin die α-Aminogruppe unsubstituiert oder durch einen der übrigen unter (a) bis (w) genannten Reste R₁ oder R₉, ausser einem Acylrest einer Aminosäure acyliert ist, wobei die Acylreste von N-Morpholinocarbonyl-glycin, N-(N-(2-, 3- oder 4-Pyridyl)methyl-N-methylaminocarbonyl)-glycin, Valin, N-(Trifluoracetyl)-valin, N-Phenylacetyl-valin, N-Acetyl-valin, N-(2-Carbamoyl-3-phenyl-propionyl)-valin, N-(2-, 3- oder 4-Pyridylacetyl)-valin, N-2-Tetrahydrofuryl-[2H]-methoxycarbonyl-valin, N-(2-Methoxy)ethoxycarbonyl-valin, N-(2-Methoxyethoxy)ethoxycarbonyl-valin, N-(3-(Tetrazol-1-yl)-propionyl)-valin, N-(Chinolin-2-ylcarbonyl)-valin, N-Methoxycarbonyl-valin, N-Isobutoxycarbonyl-valin, N-tert-Butoxycarbonyl-valin, N-Benzyloxycarbonyl-valin, N-(Morpholinocarbonyl)-valin, N-(N-(Morpholinocarbonyl)aminoacetyl)-valin, N-(Thiomorpholinocarbonyl)valin, N-(N-2-Pyridylmethyl-N-methylaminocarbonyl)-valin, N-Morpholino-carbonylamino-acetyl-valin, N-Methylsulfonyl-valin, Morpholinosulfonyl-valin, N-Acetyl-isoleucin, N-Propionylisoleucin, N-(Benzyloxycarbonyl)-isoleucin, Glutaminsäure, N-Benzyloxycarbonyl-glutaminsäure, Asparagin, N-Benzyloxycarbonyl-asparagin und/oder Chinolin-2-ylcarbonyl-asparagin am stärksten bevorzugt sind, worin die Aminosäurereste jeweils vorzugsweise in der (L)- oder (D,L)-Form, im Falle von Valin auch in der (D)-Form, vorliegen; mit der Massgabe, dass höchstens einer der Reste R₁ und R₉ Wasserstoff bedeutet,
   R₂, R₄, R₆ und R₈ Wasserstoff bedeuten,
   R₃ Niederalkyl, wie n-Butyl oder Isobutyl, Cyclohexylniederalkyl, wie Cyclohexylmethyl, oder Phenylniederalkyl, welches unsubstituiert oder durch Halogen, wie Fluor, Niederalkoxy, wie Methoxy, oder Cyano substituiert ist, vor allem Benzyl, 4-Fluorbenzyl oder 4-Cyanobenzyl,
   R₅ Niederalkanoyloxy, wie Acetyloxy, Propionyloxy, Butyryloxy, Pentanoyloxy oder Pivaloyloxy, Octanoyloxy, Decanoyloxy, Dodecanoyloxy, Carboxyniederalkanoyloxy, wie 3-Carboxypropionyloxy, Furylniederalkanoyloxy, wie 2-Furylcarbonyloxy, Imidazolylniederalkanoyloxy, wie 4-Imidazolylcarbonyloxy, 4-Imidazolylacetoxy oder 3-(4-Imidazolyl)-propionyloxy, Pyridylniederalkanoyloxy, wie 2-, 3- oder 4-Pyridylcarboxy, 2-Pyridylacetoxy oder 3-(2-Pyridyl)propionyloxy, Chinolylniederalkanoyloxy, wie Chinolin-2-ylcarbonyloxy, Aminoacetyloxy (Glycyloxy), N-Niederalkylaminoacetyloxy, wie N-Methylaminoacetyloxy, N,N-Diniederalkylaminoacetyloxy, wie N,N-Dimethylaminoacetyloxy, N-Niederalkyl-N-phenylniederalkoxycarbonylaminoacetyloxy, wie N-Benzyloxycarbonyl-N-methyl-aminoacetyloxy, Phenylniederalkanoyloxy, wie Benzoyloxy, 4-Morpholinoniederalkylbenzoyloxy, wie 4-Morpholinomethylbenzoyloxy, 4-Halogenmethylbenzoyloxy, Histidyloxy oder Prolyloxy bedeutet und
   R₇ dieselben Bedeutungen hat, wie für R₃ zuletzt definiert, insbesondere Niederalkyl, wie Isobutyl oder n-Butyl; Cyclohexylniederalkyl; oder Phenylniederalkyl, welches unsubstituiert oder durch Halogen, wie Fluor, Niederalkoxy, wie Methoxy, oder Cyano substituiert ist; wie zuletzt für R₃ definiert, bedeutet,
   oder ein Salz davon, sofern wenigstens eine salzbildende Gruppe vorliegt, wobei diejenigen Verbindungen noch stärker bevorzugt sind, in denen R₁ und/oder R₉ nicht Morpholinosulfonyl oder Thiomorpholinosulfonyl bedeuten.

Besonders bevorzugt ist eine Verbindung der Formel I, worin R₁ Niederalkoxycarbonyl, Halogenniederalkoxycarbonyl, Phenylniederalkoxycarbonyl, das über Carbonyl gebundene monovalente Radikal einer aliphatischen Aminosäure ausgewählt aus Valin, Alanin, Leucin und Isoleucin oder das über Carbonyl gebundene Radikal einer am Aminostickstoff durch einen der Reste Phenylniederalkanoyl, Morpholinylniederalkanoyl, Thiomorpholinylniederalkanoyl, Pyridylniederalkanoyl, Niederalkoxycarbonyl oder Phenylniederalkoxycarbonyl acylierten, wie oben definierten aliphatischen Aminosäure, wobei alle genannten Aminosäuren in der D-, D,L- oder L-Form vorliegen, bevorzugt in der L-Form, bedeutet, R₂ Wasserstoff, R₃ Phenylniederalkyl, 4-Fluorphenylniederalkyl oder Cyclohexylniederalkyl, R₄ Wasserstoff, R₅ Niederalkanoyloxy, Octanoyloxy, Decanoyloxy, Dodecanoyloxy, Carboxyniederalkanoyloxy, Furylniederalkanoyloxy, Imidazolylniederalkanoyloxy, Pyridylniederalkanoyloxy, Chinolylniederalkanoyloxy, Aminoacetyloxy (Glycyloxy), N-Niederalkylaminoacetyloxy, N,N-Diniederalkylaminoacetyloxy, N-Niederalkyl-N-phenylniederalkoxycarbonylaminoacetyloxy, Phenylniederalkanoyloxy, 4-Morpholinomethylbenzoyloxy, 4-Halogenmethylbenzoyloxy, Histidyloxy oder Prolyloxy (=Pyrrolidin-2-ylcarbonyloxy), R₆ Wasserstoff, R₇ Niederalkyl, Cyclohexylniederalkyl, Phenylniederalkyl, 4-Cyanophenylniederalkyl oder 4-Fluorphenylniederalkyl, R₈ Wasserstoff und R₉ einen der für R₁ genannten Reste bedeuten und die die Reste R₃ und R₅ tragenden asymmetrischen Kohlenstoffatome in der S-Konfiguration vorliegen, sowie pharmazeutisch verwendbare Salze davon.

Sehr bevorzugt ist eine Verbindung der Formel I, worin R₁ und R₉ N-Methoxycarbonylvalyl bedeuten, R₂, R₄, R₆ und R₈ Wasserstoff bedeuten, R₃ Benzyl oder ferner Cyclohexylmethyl bedeutet, R₅ Niederalkanoyloxy, insbesondere Acetyloxy, oder Pyridylcarbonyloxy, insbesondere 2-Pyridylcarbonyloxy, bedeutet und R₇ Cyclohexylmethyl oder ferner Benzyl bedeutet, sowie pharmazeutisch verwendbare Salze davon, insbesondere ein Isomeres dieser Verbindung, worin das R₃ und das R₅ tragende Kohlenstoffatom in der (S)-Konfiguration vorliegen.

Ganz besonders bevorzugt ist
1-[2(S)-(Methoxy-acetoxy)-3(S)-(N-(methoxy-carbonyl)-(L)-valyl)amino-4-phenyl-butyl]-1-[cyclohexylmethyl]-2-[N-methoxy-carbonyl-(L)-valyl]hydrazin der Formel I, oder ein pharmazeutisch verwendbares Salz davon.

Ganz besonders bevorzugt sind vor allem Verbindungen der Formel I ausgewählt aus
1-[2(S)-Propionyloxy-3(S)-(N-(methoxycarbonyl)-(L)-valyl)amino-4-phenyl-butyl]-1-[cyclohexylmethyl]-2-[N-(methoxycarbonyl)-(L)-valyl]hydrazin;
1-[2(S)-Butyryloxy-3(S)-(N-(methoxycarbonyl)-(L)-valyl)amino-4-phenyl-butyl]-1-[cyclohexylmethyl]-2-[N-(methoxycarbonyl)-(L)-valyl]hydrazin;
1-[2(S)-Pentanoyloxy-3(S)-(N-(methoxycarbonyl)-(L)-valyl)amino-4-phenyl-butyl]-1-[cyclohexylmethyl]-2-[N-(methoxycarbonyl)-(L)-valyl]hydrazin;
1-[2(S)-Octanoyloxy-3(S)-(N-(methoxycarbonyl)-(L)-valyl)amino-4-phenyl-butyl]-1-[cyclohexylmethyl]-2-[N-(methoxycarbonyl)-(L)-valyl]hydrazin;
1-[2(S)-Decanoyloxy-3(S)-(N-(methoxycarbonyl)-(L)-valyl)amino-4-phenyl-butyl]-1-[cyclohexylmethyl]-2-[N-(methoxycarbonyl)-(L)-valyl]hydrazin;
1-[2(S)-Dodecanoyloxy-3(S)-(N-(methoxycarbonyl)-(L)-valyl)amino-4-phenyl-butyl]-1-[cyclohexylmethyl]-2-[N-(methoxycarbonyl)-(L)-valyl]hydrazin;
1-[2(S)-Pivaloyloxy-3(S)-(N-(methoxycarbonyl)-(L)-valyl)amino-4-phenyl-butyl]-1-[cyclohexylmethyl]-2-[N-(methoxycarbonyl)-(L)-valyl]hydrazin;
1-[2(S)-(2-Furylcarbonyl)oxy-3(S)-(N-(methoxycarbonyl)-(L)-valyl)amino-4-phenyl-butyl]-1-[cyclohexylmethyl]-2-[N-methoxycarbonyl)-(L)-valyl]hydrazin;
1-[2(S)-(4-Imidazolylcarbonyl)oxy-3(S)-(N-(methoxycarbonyl)-(L)-valyl)amino-4-phenyl-butyl]-1-[cyclohexylmethyl]-2-[N-(methoxycarbonyl)-(L)-valyl]hydrazin;
1-[2(S)-(4-Imidazolylacetyl)oxy-3(S)-(N-(methoxycarbonyl)-(L)-valyl)amino-4-phenylbutyl]-1-[cyclohexylmethyl]-2-[N-(methoxycarbonyl)-(L)-valyl]hydrazin;
1-[2(S)-(3-(4-Imidazolyl)-propionyl)oxy-3(S)-(N-(methoxycarbonyl)-(L)-valyl)amino-4-phenyl-butyl]-1-[cyclohexylmethyl]-2-[N-(methoxycarbonyl)-(L)-valyl]hydrazin;
1-[2(S)-Benzoyloxy-3(S)-(N-(methoxycarbonyl)-(L)-valyl)amino-4-phenyl-butyl]-1-[cyclohexylmethyl]-2-[N-(methoxycarbonyl)-(L)-valyl]hydrazin;
1-[2(S)-(2-Pyridylacetyl)oxy-3(S)-(N-(methoxycarbonyl)-(L)-valyl)amino-4-phenylbutyl]-1-[cyclohexylmethyl]-2-[N-(methoxycarbonyl)-(L)-valyl]hydrazin;
1-[2(S)-(3-(Pyridin-2-yl)-propionyl)oxy-3(S)-(N-(methoxycarbonyl)-(L)-valyl)amino-4-phenylbutyl)]-1-[cyclohexylmethyl]-2-[N-(methoxycarbonyl)-(L)-valyl]hydrazin;
1-[2(S)-(Chinolin-2-ylcarbonyl)oxy-3(S)-(N-(methoxycarbonyl)-(L)-valyl)amino-4-phenyl-butyl]-1-[cyclohexylmethyl]-2-[N-(methoxycarbonyl)-(L)-valyl]hydrazin;
1-[2(S)-(Aminoacetyl)oxy-3(S)-(N-(methoxycarbonyl)-(L)-valyl)amino-4-phenyl-butyl]-1-[cyclohexylmethyl]-2-[N-(methoxycarbonyl)-(L)-valyl]hydrazin;
1-[2(S)-(N-Methylaminoacetyl)oxy-3(S)-(N-(methoxycarbonyl)-(L)-valyl)amino-4-phenyl-butyl]-1-[cyclohexylmethyl]-2-[N-(methoxycarbonyl)-(L)-valyl]hydrazin;
1-[2(S)-(N,N-Dimethylaminoacetyl)oxy-3(S)-(N-(methoxycarbonyl)-(L)-valyl)amino-4-phenyl-butyl]-1-[cyclohexylmethyl]-2-[N-(methoxycarbonyl)-(L)-valyl]hydrazin;
1-[2(S)-(N-Benzyloxycarbonyl-N-methyl-aminoacetyl)oxy-3(S)-(N-(methoxycarbonyl)-(L)-valyl)amino-4-phenyl-butyl]-1-[cyclohexylmethyl]-2-[N-(methoxycarbonyl)-(L)-valyl]hydrazin;
1-[2(S)-Prolyloxy -3(S)-(N-(methoxycarbonyl)-(L)-valyl)amino-4-phenyl-butyl]-1-[cyclohexylmethyl]-2-[N-(methoxycarbonyl)-(L)-valyl]hydrazin;
1-[2(S)-(4-Morpholinomethylbenzoyl)oxy-3(S)-(N-(methoxycarbonyl)-(L)-valyl)amino-4-phenyl-butyl]-1-[cyclohexylmethyl]-2-[N-(methoxycarbonyl)-(L)-valyl]hydrazin;
1-[2(S)-(4-Chlormethylbenzoyl)oxy-3(S)-(N-(methoxycarbonyl)-(L)-valyl)amino-4-phenyl-butyl]-1-[cyclohexylmethyl]-2-[N-(methoxycarbonyl)-(L)-valyl]hydrazin; und
1-[2(S)-(3-Carboxypropionyl)oxy-3(S)-(N-(methoxycarbonyl)-(L)-valyl)amino-4-phenyl-butyl]-1-[cyclohexylmethyl]-2-[N-(methoxycarbonyl)-(L)-valyl]hydrazin, oder pharmazeutisch verwendbare Salze davon.

Zuallererst bevorzugt sind die in den Beispielen genannten Verbindungen oder ihre Salze.

Die Verbindungen der Formel I und Salze von solchen Verbindungen mit mindestens einer salzbildenden Gruppe werden nach an sich bekannten Verfahren erhalten, z. B. indem man
a) eine Hydroxyverbindung der Formel II, 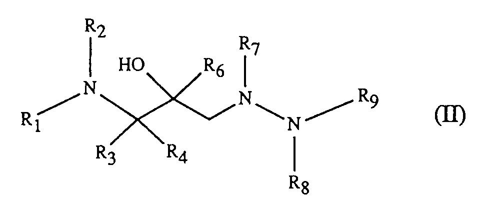 worin die Reste die für Verbindungen der Formel I genannten Bedeutungen haben, mit einer Carbonsäure der Formel III,

   R₅-H (III)

   oder einem reaktionsfähigen Säurederivat davon, worin R₅ die für Verbindungen der Formel I genannten Bedeutungen hat, acyliert, wobei freie funktionelle Gruppen in den Ausgangsmaterialien der Formeln II und III, die nicht an der Reaktion teilnehmen sollen, erforderlichenfalls in geschützter Form vorliegen, und vorhandene Schutzgruppen abspaltet, oder
b) zur Herstellung von Verbindungen der Formel I, worin R₉ Acyl, Sulfo oder durch unsubstituiertes oder substituiertes Alkyl, Aryl oder Heterocyclyl substituiertes Sulfonyl bedeutet und die übrigen Reste die genannten Bedeutungen haben, eine Aminoverbindung der Formel 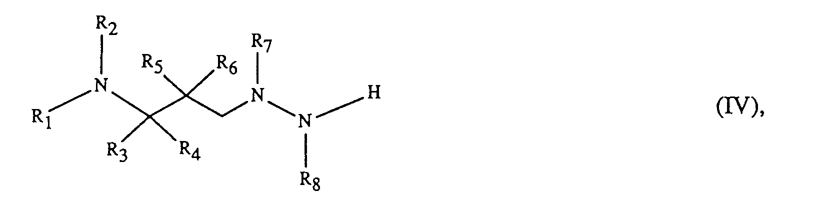 worin die Reste die gerade genannten Bedeutungen haben, mit einer Säure der Formel

   R₉'-OH (V)

   oder reaktionsfähigen Säurederivaten davon, worin R₉' die für R₉ genannten Bedeutungen ausser Wasserstoff und unsubstituiertem oder substituiertem Alkyl hat, kondensiert, wobei freie funktionelle Gruppen mit Ausnahme der an der Reaktion beteiligten erforderlichenfalls in geschützter Form vorliegen, und vorhandene Schutzgruppen abspaltet, oder
c) zur Herstellung von Verbindungen der Formel I, worin R₁ Acyl, Sulfo oder durch unsubstituiertes oder substituiertes Alkyl, Aryl oder Heterocyclyl substituiertes Sulfonyl bedeutet und die übrigen Reste die genannten Bedeutungen haben, eine Aminoverbindung der Formel 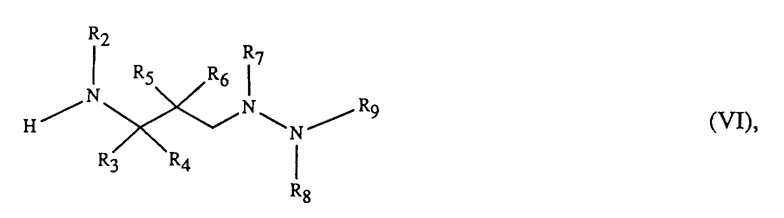 worin die Reste die gerade genannten Bedeutungen haben, mit einer Säure der Formel

   R₁'-OH (VII)

   oder reaktionsfähigen Säurederivaten davon, worin R₁' die für R₁ genannten Bedeutungen ausser Wasserstoff und unsubstituiertem oder substituiertem Alkyl hat, kondensiert, wobei freie funktionelle Gruppen mit Ausnahme der an der Reaktion beteiligten erforderlichenfalls in geschützter Form vorliegen, und vorhandene Schutzgruppen abspaltet, oder
d) zur Herstellung von Verbindungen der Formel I, worin R₁ und R₉ zwei gleiche Reste ausgewählt aus Acyl, Sulfo und durch unsubstituiertes oder substituiertes Alkyl, Aryl oder Heterocyclyl substituiertes Sulfonyl bedeuten und die übrigen Reste die genannten Bedeutungen haben, eine Diaminoverbindung der Formel 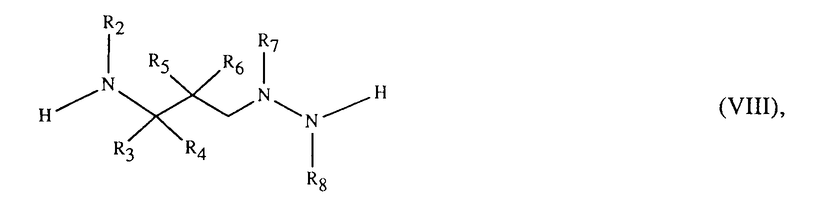 worin die Reste die gerade genannten Bedeutungen haben, mit einer zur Einführung der identischen Reste R₁ und R₉ geeigneten Säure oder reaktionsfähigen Säurederivaten davon, worin R₁ und R₉ die gerade genannten Bedeutungen haben, kondensiert, wobei freie funktionelle Gruppen mit Ausnahme der an der Reaktion beteiligten erforderlichenfalls in geschützter Form vorliegen, und vorhandene Schutzgruppen abspaltet, oder
e) zur Herstellung einer Verbindung der Formel I, worin an Stelle von R₇ der Rest R₇'' vorliegt, welcher unsubstituiertes oder substituiertes Alkyl oder Cycloalkyl bedeutet, in einer Verbindung der Formel I', 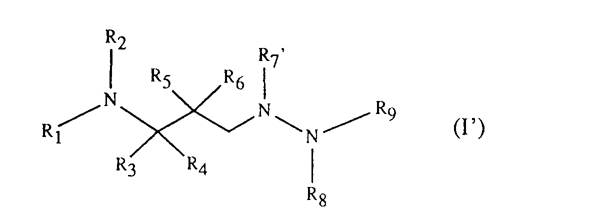 worin R₇' Wasserstoff bedeutet und die übrigen Reste die genannten Bedeutungen haben, durch Substitution mit einer Verbindung der Formel XII,

   R₇''-X (XII)

   worin X eine Abgangsgruppe ist und R₇'' unsubstituiertes oder substituiertes Alkyl oder Cycloalkyl bedeutet, den Rest R₇'' einführt, wobei freie funktionelle Gruppen mit Ausnahme der an der Reaktion beteiligten gegebenenfalls in geschützter Form vorliegen, und gegebenenfalls vorhandene Schutzgruppen abspaltet,
   und gewünschtenfalls eine nach einem der vorstehenden Verfahren a) bis e) erhältliche Verbindung der Formel I mit mindestens einer salzbildenden Gruppe in ihr Salz oder ein erhältliches Salz in die freie Verbindung oder in ein anderes Salz überführt und/oder gegebenenfalls erhältliche Isomerengemische auftrennt und/oder eine erfindungsgemässe Verbindung der Formel I in eine andere erfindungsgemässe Verbindung der Formel I umwandelt.
   Die Abspaltung von Schutzgruppen erfolgt, indem man
f) in einer Verbindung der Formel I, worin die Substituenten die oben genannten Bedeutungen haben mit der Massgabe, dass in der betreffenden Verbindung der Formel I mindestens eine funktionelle Gruppe durch Schutzgruppen geschützt ist, vorhandene Schutzgruppen abspaltet.

Die genannten Verfahren werden nachfolgend näher beschrieben; sofern nichts anderes angegeben ist, haben die Reste R₁, R₂, R₃, R₄, R₅, R₆, R₇, R₈ und R₉ die für Verbindungen der Formel I genannten Bedeutungen:

### Verfahren a) (Acylierung einer Hydroxygruppe)

Die Acylierung der Hydroxygruppe erfolgt beispielsweise in an sich bekannter Weise unter Verwendung einer Säure der Formel III, worin R₅ die genannten Bedeutungen ausser Aminocarbonyloxy oder dem über seine Aminocarbonyloxygruppe gebundenen Rest einer N-substituierten Carbaminsäure hat, oder eines reaktionsfähigen Derivates davon. Als reaktionsfähiges Derivat kommt beispielsweise eine Carbonsäure der Formel IX

R₅'-Z₁ (IX)

in Frage, worin R₅' einen der in Acyloxy, wie oben definiert, vorkommenden Acylreste bedeutet und worin Z₁ reaktionsfähig aktiviertes Hydroxy bedeutet (die Verbindung der Formel IX enthält somit anstelle einer an die Carbonylgruppe gebundenen Hydroxyfunktion reaktionsfähig aktiviertes Hydroxy, vorzugsweise wie nachfolgend definiert). Die freie Carbonsäure der Formel III kann beispielsweise durch starke Säuren , wie Halogenwasserstoff-, Schwefel-, Sulfon-, oder Carbonsäure oder saure Ionenaustauscher, z.B. durch Chlor-, Bromwasserstoff- oder Iodwasserstoffsäure, Schwefelsäure, eine gegebenenfalls, z.B. durch Halogen, substituierte Alkancarbonsäure oder durch eine Säure der Formel III, vorzugsweise mit einem Überschuss der Säure der Formel III, erforderlichenfalls unter Bindung von entstehendem Reaktionswasser durch wasserbindende Mittel, unter azeotroper Abdestillation des Reaktionswassers oder unter extraktiver Veresterung, durch Säureanhydride, insbesondere anorganische oder vor allem organische Säureanhydride, z.B. Carbonsäureanhydride, wie Niederalkancarbonsäureanhydride (ausser Ameisensäureanhydrid), z.B. Acetanhydrid, oder durch geeignete Aktivierungs- oder Kupplungsreagentien der nachstehend aufgeführten Art, insbesondere auch in situ, aktiviert werden. R₅'-Z₁ kann insbesondere auch für ein Carbonsäureazid (Z₁ = Azido; erhältlich beispielsweise durch Umsetzung eines entsprechenden Säureesters über das entsprechende Hydrazid und dessen Behandlung mit salpetriger Säure); für ein Carbonsäurehalogenid (Z₁ = Halogen, vor allem Chlor oder Brom), insbesondere ein Säurechlorid oder -bromid, welches beispielsweise durch Umsetzung mit organischen Säurehalogeniden, insbesondere mit Oxalyldihalogeniden, wie Oxalyldichlorid, mit anorganischen Säurehalogeniden, z.B. mit Säurehalogeniden des Phosphors oder Schwefels, wie Phosphortrichlorid, Phosphortribromid, Phosphorpentachlorid, Phosphorpentabromid, Phosphoroxidchlorid, Phosphoroxidbromid, Thionylchlorid oder Thionylbromid, oder vor allem unter schonenden Bedingungen mit Tetraniederalkyl-α-halogenoenaminen, z.B. Tetramethyl-α-halogenoenaminen, insbesondere 1-Chlor-N,N,2-trimethyl-1-propenamin (vorzugsweise durch Umsetzung in inerten Lösungsmitteln, insbesondere chlorierten Kohlenwasserstoffen, wie Methylenchlorid oder Chloroform, oder Ethern, wie Diethylether, Dioxan oder Tetrahydrofuran, bei bevorzugten Temperaturen zwischen -78 und 50 °C, insbesondere bei -60 bis 30 °C, z.B. zwischen -10 °C und Raumtemperatur (vgl. Devos, A., et al., J. C. S. Chem. Commun. 1979, 1180-81 und Haveaux, B., et al., Org. Synth. 59, 26 (1980))), wobei das erhaltene Säurehalogenid, z.B. das Säurechlorid der Formel IX, worin Z₁ Chlor bedeutet, auch direkt in situ weiter verwendet werden kann, beispielsweise durch Umsetzung mit der Verbindung der Formel II in Gegenwart von tertiären Stickstoffbasen, wie Pyridin und/oder Dimethylaminopyridin (DMAP, welches vorzugsweise in katalytischen Mengen zugefügt wird), bei bevorzugten Temperaturen zwischen - 20 und 50 ⁰C, insbesondere zwischen 0 °C und Raumtemperatur) erhalten werden kann; für einen aktivierten Ester, wobei Z₁ den Rest eines Alkoholes mit elektronenziehenden Substituenten, insbesondere Cyanmethoxy oder Aryloxy, worin Aryl vorzugsweise Phenyl oder Naphthyl ist, welches durch Halogen, Nitro und/oder Cyano ein- oder mehrfach substituiert ist, z.B. Nitrophenoxy, wie 4-Nitrophenoxy oder 2,4-Dinitrophenyoxy, oder Polyhalogenphenoxy, wie Pentachlorphenoxy, bedeutet; oder für ein symmetrisches oder vorzugsweise asymmetrisches Säureanhydrid stehen, welches beispielsweise durch Einwirkung eines Salzes, z.B. eines Alkalimetallsalzes einer Säure der Formel III oder ihres Reaktionspartners, vorzugsweise einer Niederalkancarbonsäure, wie Essigsäure, wie des Natrium- oder Kaliumsalzes, auf ein jeweils komplementäres Säurehalogenid, insbesondere im Falle der Reaktion mit einem Salz einer Carbonsäure der Formel III ein Carbonsäurehalogenid, z.B. -Chlorid, wie Acetylchlorid, und im Falle der Reaktion eines Carbonsäurehalogenides der Formel IX, worin Z₁ Halogen, z.B. Chlor oder Brom, bedeutet, mit einem Salz einer Niederalkancarbonsäure, insbesondere Natrium- oder Kaliumacetat, erhalten werden kann. Als Aktivierungs- und Kupplungsreagentien zur Aktivierung von Carbonsäuren der Formel III in situ können insbesondere Carbodiimide, z.B. N,N'-Di-C₁-C₄-alkyl- oder N,N'-Di-C₅-C₇-cycloalkyl-carbodiimid, wie Diisopropylcarbodiimid oder N,N'-Dicyclohexyl-carbodiimid, vorteilhaft unter Zusatz eines Aktivierungskatalysators, wie N-Hydroxysuccinimid oder gegebenenfalls, z.B. durch Halogen, C₁-C₇-Alkyl oder C₁-C₇-Alkoxy, substituiertes N-Hydroxy-benzotriazol oder N-Hydroxy-5-norbornen-2,3-dicarboxamid, C₁-C₄-Alkylhalogenformiat, z.B. Isobutylchlorformiat, geeignete Carbonylverbindungen, z.B. N,N-Carbonyldiimidazol, geeignete 1,2-Oxazoliumverbindungen, z.B. 2-Ethyl-5-phenyl-1,2-oxazolium-3'-sulfonat oder 2-tert-Butyl-5-methyl-isoxazolium-perchlorat, geeignete Acylaminoverbindungen, z.B. 2-Ethoxy-1-ethoxycarbonyl-1,2-dihydrochinolin, oder geeignete Phosphorylcyanamide bzw. -azide, z.B. Diethylphosphorylcyanamid oder Diphenylphosphorylazid, ferner Triphenylphosphin-disulfid oder 1-C₁-C₄-Alkyl-2-halogeno-pyridinium-halogenide, z.B. 1-Methyl-2-chlorpyridinium-iodid, eingesetzt werden.

Falls in der Verbindung der Formel III zwei freie Carboxygruppen vorliegen, beispielsweise in Carboxyniederalkansäuren, wie 3-Carboxypropansäure, kann als aktiviertes Säurederivat auch ein inneres Anhydrid vorliegen, z.B. Succinanhydrid.

Z₁ bedeutet bevorzugt Halogen, wie Chlor oder Brom, sowie Acyloxy, z.B. Niederalkanoyloxy, wie Acetyloxy.

Für den Spezialfall der Einführung eines über seine Carbonylgruppe an den bindenden Sauerstoff geknüpften Acylrestes eines Halbesters der Kohlensäure eignen sich insbesondere die Verbindungen der Formel IX, worin Z₁ Halogen, wie Chlor, bedeutet, welche z.B. hergestellt werden können durch Umsetzung der komplementären Alkohole, beispielsweise unsubstituierter oder substituierter Alkylalkohole, Arylniederalkylalkohole oder Heterocyclylniederalkylalkohole, wie bei der Definition von unsubstituiertem oder substituiertem Alkoxycarbonyloxy, Arylniederalkyloxycarbonyloxy oder Heterocyclylniederalkyloxycarbonyloxy R₅ definiert, mit Phosgen oder ferner Analogen davon, welche statt Chlor andere Halogenatome, insbesondere Brom, enthalten, vorzugsweise in Gegenwart von tertiären Stickstoffbasen, wie Pyridin oder Triethylamin, und in inerten Lösungsmitteln, z.B. chlorierten Kohlenwasserstoffen, wie Methylenchlorid oder Chloroform, Ethern, wie Diethylether, Tetrahydrofuran oder Dioxan, oder Carbonsäureamiden, wie Dimethylformamid. Auch entsprechende N-Carbonylazolide der Formel IX (Z₁ = N-haltiger Heterocyclus, wie 1-Imidazolido) sind geeignet, welche beispielsweise durch Umsetzung mit den entsprechenden N,N'-Carbonyldiazoliden, wie N,N'-Carbonyldiimidazol, erhalten werden unter Bedingungen, wie gerade für Phosgen und Analoge mit anderen Halogenatomen beschrieben. Die Reaktion der Verbindungen der Formel II mit der entsprechenden Verbindungen der Formel IX findet dann ebenfalls unter diesen Bedingungen statt (vgl. Staab, H. A., Angew. Chemie 74, 407 (1962).

Für den Spezialfall der Einführung von Aminocarbonyloxy R₅ oder einer N-substituierten Aminocarbonyloxygruppe R₅ eignet sich als aktiviertes Säurederivat insbesondere das entsprechende Isocyanat der Formel IX',

Q-N=C=O (IX')

worin Q eine Aminoschutzgruppe, z.B. Trihalogenacetyl, wie Trifluor- oder Trichloracetyl, oder einen der oben bei der Definition von Aminocarbonyloxy R₅, worin die Aminogruppe 1 bis 2 Substituenten trägt, genannten unsubstituierten oder substituierten Niederalkylreste oder Arylreste bedeutet, wobei man, wenn Q eine Aminoschutzgruppe bedeutet, nach der Umsetzung mit der Verbindung der Formel II die entsprechende Verbindung der Formel I, worin R₅ freies Aminocarbonyloxy bedeutet, durch Abspaltung der Schutzgruppe Q erhalten kann, wie unten bei der Freisetzung von durch Acyl geschütztem Amino beschrieben, insbesondere durch saure Hydrolyse, oder, wenn Q einen der genannten substituierten oder unsubstituierten Niederalkylreste oder Arylreste bedeutet, eine entsprechende Verbindung der Formel I mit am Stickstoff monosubstituiertem Aminocarbonyloxy R₅. Sowohl Aminocarbonyloxy als auch N-monosubstituiertes Aminocarbonyloxy R₅ kann in N-disubstituiertes Aminocarbonyloxy überführt werden, indem man mit einem weiteren unsubstituierten oder substituierten Niederalkylrest unter Verwendung geeigneter Ausgangsmaterialien und analogen Bedingungen alkyliert, wie unten bei den zusätzlichen Verfahrensmassnahmen beschrieben.

Die Reaktionen können unter an sich bekannten Reaktionsbedingungen, bei üblichen Temperaturen, in An- oder, insbesondere im Falle der Verwendung von Niederalkanoylanhydriden zur Aktivierung der Carbonsäure der Formel III, Abwesenheit von inerten Lösungs- oder Verdünnungsmitteln durchgeführt werden, beispielsweise in Säureamiden, z.B. Carbonsäuramiden, wie Dimethylformamid, Dimethylacetamid oder 1,3-Dimethyl-3,4,5,6-tetrahydro-2(1H)-pyrimidinon (DMPU), oder Amiden anorganischer Säuren, wie Hexamethylphosphorsäuretriamid, Ethern, z.B. cyclischen Ethern, wie Tetrahydrofuran oder Dioxan, oder acyclischen Ethern, wie Diethylether oder Ethylenglykoldimethylether, halogenierten Kohlenwasserstoffen, wie Haloniederalkanen, z.B. Methylenchlorid oder Chloroform, Ketonen, wie Aceton, Nitrilen, wie Acetonitril, Säureanhydriden, wie Acetanhydrid, Estern, wie Essigsäureethylester, Bisalkansulfinen, wie Dimethylsulfoxid, Stickstoffheterocyclen, wie Pyridin, oder Gemischen dieser Lösungsmittel, insbesondere in wasserfreien Lösungsmitteln oder Lösungsmittelgemischen, wobei die jeweils geeigneten Lösungsmittel für die oben genannten Reaktionen ausgewählt werden können, soweit sinnvoll und zweckmässig, unter Verwendung von Salzen der eingesetzten Verbindungen, insbesondere von Metallsalzen eingesetzter Cabonsäuren, wie den Alkali- oder Erdalkalimetallsalzen, z.B. Natrium- oder Kaliumsalzen, in Ab- oder Anwesenheit von Katalysatoren, wie Dimethylaminopyridin, Kondensationsmitteln oder neutralisierenden Agentien, wie tertiären Stickstoffbasen, z.B. Pyridin, Triethylamin, N-Methylmorpholin, Dimethylaminopyridin oder Ethyldiisopropylamin, je nach Art der Reaktion und/oder der Reaktionsteilnehmer unter atmosphärischem Druck oder in einem geschlossenen Gefäss, unter Normaldruck oder ferner unter erhöhtem Druck, z.B. bei dem Druck, der im Reaktionsgemisch unter den Reaktionsbedingungen in einem geschlossenen Rohr entsteht, und/oder in einer inerten Atmosphäre, z.B. unter einer Argon- oder Stickstoffatmosphäre. Bevorzugt sind Reaktionsbedingungen, die jewelis spezifisch genannt sind oder in erster Linie den in den Beispielen genannten analog sind. Der Reaktionsablauf wird zweckmässig mittels üblicher analytischer Methoden, insbesondere mit Dünnschichtchromatographie, verfolgt. Aus diesen Reaktionsbedingungen können die jeweils geeigneten für alle im vorliegenden Text beschriebenen Reaktionen ausgewählt werden, wobei spezifisch genannte Reaktionsbedingungen besonders bevorzugt sind.

Die erfindungsgemässe Umsetzung wird vorzugsweise unter milden Bedingungen, insbesondere bei Temperaturen zwischen -10 °C und 60 °C, z.B. bei 0 °C bis Raumtemperatur oder leicht erhöhten Temperaturen bis etwa 50 °C, z.B. bei etwa 0 °C bis Raumtemperatur, durchgeführt. Sowohl bei der Umsetzung mit einem Carbonsäurehalogenid der Formel IX, worin Z₁ Halogen, wie Chlor oder Brom bedeutet, als auch bei der Umsetzung mit einem Anhydrid, insbesondere einem symmetrischen Anhydrid (Z₁ = O-R₅'), verwendet man insbesondere die gegenüber der Verbindung der Formel II etwa äquimolare Menge oder einen Ueberschuss der entsprechenden Verbindung der Formel IX (Halogenid bzw. R₅'-O-R₅'), beispielsweise die 0,95- bis 10-fache molare Menge.

Bevorzugt als Verbindungen der Formel II für Verfahren a) sind die Ausgangsverbindungen der Formel 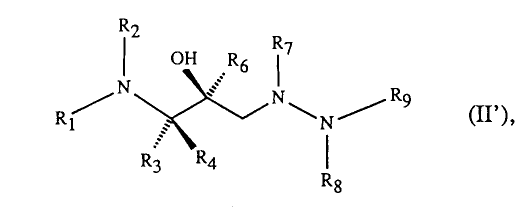
worin die Reste die für Verbindungen der Formel I genannten Bedeutungen haben, sowie die Salze der genannten Verbindungen, sofern salzbildende Gruppen vorliegen.

Funktionelle Gruppen in Ausgangsmaterialien, deren Umsetzung vermieden werden soll, insbesondere Carboxy-, Amino-, Hydroxy-, Mercapto- und Sulfogruppen, können durch geeignete Schutzgruppen (conventional protecting groups) geschützt sein, die üblicherweise bei der Synthese von Peptid-Verbindungen, aber auch von Cephalosporinen und Penicillinen sowie Nucleinsäurederivaten und Zuckern verwendet werden. Diese Schutzgruppen können bereits in den Vorstufen vorhanden sein und sollen die betreffenden funktionellen Gruppen gegen unerwünschte Nebenreaktionen wie Acylierungen, Veretherungen, Veresterungen, Oxidationen, Solvolyse etc. schützen. In bestimmten Fällen können die Schutzgruppen darüber hinaus einen selektiven, beispielsweise stereoselektiven Verlauf von Umsetzungen bewirken. Charakteristisch für Schutzgruppen ist, dass sie leicht, d. h. ohne unerwünschte Nebenreaktionen abspaltbar sind, beispielsweise solvolytisch, reduktiv, photolytisch oder auch enzymatisch, z. B. auch unter physiologischen Bedingungen und vor allem, dass sie in den Endstoffen nicht mehr vorhanden sind.

Der Schutz von funktionellen Gruppen durch solche Schutzgruppen, die Schutzgruppen selbst, sowie ihre Abspaltungsreaktionen sind beispielsweise in Standardwerken wie J. F. W. McOmie, "Protective Groups in Organic Chemistry", Plenum Press, London und New York 1973, in Th. W. Greene, "Protective Groups in Organic Synthesis", Wiley, New York 1981, in "The Peptides"; Band 3 (E. Gross und J. Meienhofer, Herausg.), Academic Press, London und New York 1981, in "Methoden der organischen Chemie", Houben-Weyl, 4. Auflage, Band 15/I, Georg Thieme Verlag, Stuttgart 1974, in H.-D. Jakubke und H. Jescheit, "Aminosäuren, Peptide, Proteine", Verlag Chemie, Weinheim, Deerfield Beach und Basel 1982, und in Jochen Lehmann, "Chemie der Kohlenhydrate: Monosaccharide und Derivate", Georg Thieme Verlag, Stuttgart 1974, beschrieben.

Eine Carboxygruppe ist z. B. als eine Estergruppe geschützt, die unter schonenden Bedingungen selektiv spaltbar ist. Eine in veresterter Form geschützte Carboxygruppe ist in erster Linie durch eine Niederalkylgruppe verestert, welche bevorzugt in 1-Stellung der Niederalkylgruppe verzweigt oder in 1- oder 2-Stellung der Niederalkylgruppe durch geeignete Substituenten substituiert ist.

Eine geschützte Carboxygruppe, welche durch eine Niederalkylgruppe verestert ist, ist beispielsweise Methoxycarbonyl oder Ethoxycarbonyl.

Eine geschützte Carboxygruppe, welche durch eine Niederalkylgruppe verestert ist, die in 1-Stellung der Niederalkylgruppe verzweigt ist, ist beispielsweise tert-Niederalkoxycarbonyl, z. B. tert-Butoxycarbonyl.

Eine geschützte Carboxygruppe, welche durch eine Niederalkylgruppe verestert ist, welche in 1- oder 2-Stellung der Niederalkylgruppe durch geeignete Substituenten substituiert ist, ist beispielsweise Arylmethoxycarbonyl mit einem oder zwei Arylresten, worin Aryl unsubstituiertes oder z. B. durch Niederalkyl, z. B. tert-Niederalkyl, wie tert-Butyl, Niederalkoxy, z. B. Methoxy, Hydroxy, Halogen, z. B. Chlor, und/oder Nitro mono-, di- oder trisubstituiertes Phenyl bedeutet, beispielsweise Benzyloxycarbonyl, durch die genannten Substituenten substituiertes Benzyloxycarbonyl, z. B. 4-Nitrobenzyloxycarbonyl oder 4-Methoxybenzyloxycarbonyl, Diphenylmethoxycarbonyl oder durch die genannten Substituenten substituiertes Diphenylmethoxycarbonyl, z. B. Di-(4-methoxyphenyl)-methoxycarbonyl, ferner durch eine Niederalkylgruppe verestertes Carboxy, wobei die Niederalkylgruppe in 1- oder 2-Stellung durch geeignete Substituenten substituiert ist, wie 1-Niederalkoxyniederalkoxycarbonyl, z. B. Methoxymethoxycarbonyl, 1-Methoxyethoxycarbonyl oder 1-Ethoxyethoxycarbonyl, 1-Niederalkylthioniederalkoxycarbonyl, z. B. 1-Methylthiomethoxycarbonyl oder 1-Ethylthioethoxycarbonyl, Aroylmethoxycarbonyl, worin die Aroylgruppe gegebenenfalls beispielsweise durch Halogen, wie Brom, substituiertes Benzoyl darstellt, z. B. Phenacyloxycarbonyl, 2-Halogenniederalkoxycarbonyl, z. B. 2,2,2-Trichlorethoxycarbonyl, 2-Bromethoxycarbonyl oder 2-Jodethoxycarbonyl, sowie 2-(trisubstituiertes Silyl)-niederalkoxycarbonyl, worin die Substituenten unabhängig voneinander je einen gegebenenfalls, z. B. durch Niederalkyl, Niederalkoxy, Aryl, Halogen, und/oder Nitro substituierten aliphatischen, araliphatischen, cycloaliphatischen oder aromatischen Kohlenwasserstoffrest bedeuten, beispielsweise gegebenenfalls wie oben substituiertes Niederalkyl, Phenylniederalkyl, Cycloalkyl oder Phenyl, z. B. 2-Triniederalkylsilylniederalkoxycarbonyl, wie 2-Triniederalkylsilylethoxycarbonyl, z. B. 2-Trimethylsilylethoxycarbonyl oder 2-(Di-n-butyl-methyl-silyl)-ethoxycarbonyl, oder 2-Triarylsilylethoxycarbonyl, wie Triphenylsilylethoxycarbonyl.

Eine Carboxygruppe kann auch als organische Silyloxycarbonylgruppe geschützt sein. Eine organische Silyloxycarbonylgruppe ist beispielsweise eine Triniederalkylsilyloxycarbonylgruppe, z. B. Trimethylsilyloxycarbonyl. Das Siliciumatom der Silyloxycarbonylgruppe kann auch durch zwei Niederalkylgruppen, z. B. Methylgruppen, und die Aminogruppe oder die Carboxygruppe eines zweiten Moleküls der Formel I substituiert sein. Verbindungen mit solchen Schutzgruppen lassen sich z. B. mit Dimethylchlorsilan als Silylierungsmittel herstellen.

Eine geschützte Carboxygruppe ist vorzugsweise tert-Niederalkoxycarbonyl, z. B. tert-Butoxycarbonyl, Benzyloxycarbonyl, 4-Nitrobenzyloxycarbonyl, 9-Fluorenylmethoxycarbonyl oder Diphenylmethoxycarbonyl.

Eine geschützte Aminogruppe kann durch eine Aminoschutzgruppe geschützt, z. B. in Form einer Acylamino-, Arylmethylamino-, veretherten Mercaptoamino-, 2-Acyl-niederalk-1-enylamino oder Silylaminogruppe oder als Azidogruppe, vorliegen.

In einer entsprechenden Acylaminogruppe ist Acyl beispielsweise der Acylrest einer organischen Carbonsäure mit z. B. bis zu 18 Kohlenstoffatomen, insbesondere einer gegebenenfalls, z.B durch Halogen oder Aryl, substituierten Niederalkancarbonsäure oder gegebenenfalls, z. B. durch Halogen, Niederalkoxy oder Nitro, substituierten Benzoesäure, oder bevorzugt eines Kohlensäurehalbesters. Solche Acylgruppen sind beispielsweise Niederalkanoyl, wie Formyl, Acetyl, Propionyl oder Pivaloyl, Halogenniederalkanoyl, z. B. 2-Halogenacetyl, wie 2-Chlor-, 2-Brom-, 2-Jod-, 2,2,2-Trifluor- oder 2,2,2-Trichloracetyl, gegebenenfalls z. B. durch Halogen, Niederalkoxy oder Nitro substituiertes Benzoyl, wie Benzoyl, 4-Chlorbenzoyl, 4-Methoxybenzoyl oder 4-Nitrobenzoyl, Niederalkoxycarbonyl, vorzugsweise in 1-Stellung des Niederalkylrestes verzweigtes oder in 1- oder 2-Stellung geeignet substituiertes Niederalkoxycarbonyl, z. B. tert-Niederalkoxycarbonyl, wie tert-Butoxycarbonyl, Arylmethoxycarbonyl mit einem, zwei oder drei Arylresten, die gegebenenfalls, z. B. durch Niederalkyl, insbesondere tert-Niederalkyl, wie tert-Butyl, Niederalkoxy, wie Methoxy, Hydroxy, Halogen, wie Chlor, und/oder Nitro mono- oder polysubstituiertes Phenyl darstellen, z. B. Benzyloxycarbonyl, 4-Nitrobenzyloxycarbonyl, Diphenylmethoxycarbonyl, 9-Fluorenylmethoxycarbonyl oder Di-(4-methoxyphenyl)-methoxycarbonyl, Aroylmethoxycarbonyl, worin die Aroylgruppe vorzugsweise gegebenenfalls, z. B. durch Halogen, wie Brom, substituiertes Benzoyl darstellt, z. B. Phenacyloxycarbonyl, 2-Halogen-niederalkoxycarbonyl, z. B. 2,2,2-Trichlorethoxycarbonyl, 2-Bromethoxycarbonyl oder 2-Jodethoxycarbonyl, 2-(trisubstituiertes Silyl)-niederalkoxycarbonyl, z. B. 2-Triniederalkylsilylniederalkoxycarbonyl, wie 2-Trimethylsilylethoxycarbonyl oder 2-(Di-n-butyl-methyl-silyl)-ethoxycarbonyl, oder Triarylsilylniederalkoxycarbonyl, z. B. 2-Triphenylsilylethoxycarbonyl.

In einer Arylmethylaminogruppe, die z. B. eine Mono-, Di- oder insbesondere Triarylmethylaminogruppe darstellt, sind die Arylreste insbesondere gegebenenfalls substituierte Phenylreste. Solche Gruppen sind z. B. Benzyl-, Diphenylmethyl- oder insbesondere Tritylamino.

In einer veretherten Mercaptoaminogruppe liegt die Mercaptogruppe in erster Linie als substituiertes Arylthio oder Arylniederalkylthio, worin Aryl beispielsweise gegebenenfalls z. B. durch Niederalkyl, wie Methyl oder tert.-Butyl, Niederalkoxy, wie Methoxy, Halogen, wie Chlor, und/oder Nitro substituiertes Phenyl ist, z. B. 4-Nitrophenylthio, vor.

In einem als Aminoschutzgruppe verwendbaren 2-Acyl-niederalk-1-enylrest ist Acyl z. B. der entsprechende Rest einer Niederalkancarbonsäure, einer gegebenenfalls z. B. durch Niederalkyl, wie Methyl oder tert-Butyl, Niederalkoxy, wie Methoxy, Halogen, wie Chlor, und/oder Nitro substituierten Benzoesäure, oder insbesondere eines Kohlensäurehalbesters, wie eines Kohlensäure-niederalkylhalbesters. Entsprechende Schutzgruppen sind in erster Linie 1-Niederalkanoyl-niederalk-1-en-2-yl, z. B. 1-Niederalkanoyl-prop-1-en-2-yl, wie 1-Acetyl-prop-1-en-2-yl, oder Niederalkoxycarbonyl-niederalk-1-en-2-yl, z. B. Niederalkoxycarbonyl-prop-1-en-2-yl, wie 1-Ethoxycarbonyl-prop-1-en-2-yl.

Eine Silylaminogruppe ist beispielsweise eine Triniederalkylsilylaminogruppe, z. B. Trimethylsilylamino oder tert-Butyl-dimethylsilylamino. Das Siliciumatom der Silylaminogruppe kann auch nur durch zwei Niederalkylgruppen, z. B. Methylgruppen, und die Aminogruppe oder Carboxylgruppe eines zweiten Moleküls der Formel I substituiert sein. Verbindungen mit solchen Schutzgruppen lassen sich z. B. mit den entsprechenden Chlorsilanen, wie Dimethylchlorsilan, als Silylierungsmittel herstellen.

Eine Aminogruppe kann auch durch Überführung in die protonierte Form geschützt werden; als entsprechende Anionen kommen in erster Linie diejenigen von starken anorganischen Säuren, wie von Schwefelsäure, Phosphorsäure oder Halogenwasserstoffsäuren, z. B. das Chlor- oder Bromanion, oder von organischen Sulfonsäuren, wie p-Toluolsulfonsäure, in Frage.

Bevorzugte Aminoschutzgruppen sind Niederalkoxycarbonyl, Phenylniederalkoxycarbonyl, Fluorenylniederalkoxycarbonyl, 2-Niederalkanoyl-niederalk-1-en-2-yl oder Niederalkoxycarbonyl-niederalk-1-en-2-yl.

Eine Hydroxygruppe kann beispielsweise durch eine Acylgruppe, z. B. durch Halogen, wie Chlor, substituiertes Niederalkanoyl, wie 2,2-Dichloracetyl, oder insbesondere durch einen für geschützte Aminogruppen genannten Acylrest eines Kohlensäurehalbesters geschützt sein. Eine bevorzugte Hydroxyschutzgruppe ist beispielsweise 2,2,2-Trichlorethoxycarbonyl, 4-Nitrobenzyloxycarbonyl, Diphenylmethoxycarbonyl oder Triphenylmethoxycarbonyl. Eine Hydroxygruppe kann ferner durch Triniederalkylsilyl, z. B. Trimethylsilyl, Triisopropylsilyl oder tert-Butyl-dimethylsilyl, eine leicht abspaltbare verethernde Gruppe, z. B. eine Alkylgruppe, wie tert-Niederalkyl, z. B. tert-Butyl, einen oxa- oder einen thiaaliphatischen oder -cycloaliphatischen, insbesondere 2-oxa- oder 2-thiaaliphatischen oder -cycloaliphatischen, Kohlenwasserstoffrest, beispielsweise 1-Niederalkoxyniederalkyl oder 1 -Niederalkylthioniederalkyl, wie Methoxymethyl, 1-Methoxyethyl, 1-Ethoxyethyl, Methylthiomethyl, 1-Methylthioethyl oder 1-Ethylthioethyl, oder 2-Oxa- oder 2-Thiacycloalkyl mit 5-7 Ringatomen, wie 2-Tetrahydrofuryl oder 2-Tetrahydropyranyl, oder ein entsprechendes Thiaanaloges, sowie durch 1-Phenylniederalkyl, wie Benzyl, Diphenylmethyl oder Trityl, wobei die Phenylreste beispielsweise durch Halogen, z. B. Chlor, Niederalkoxy, z. B. Methoxy, und/oder Nitro substituiert sein können, geschützt sein.

Zwei in einem Molekül vorkommende, insbesondere benachbarte Hydroxylgruppen oder eine benachbarte Hydroxy- und Aminogruppe können beispielsweise durch bivalente Schutzgruppen, wie eine vorzugsweise, etwa durch ein oder zwei Niederalkylreste oder Oxo, substituierte Methylengruppe, geschützt sein, z. B. durch unsubstituiertes oder substituiertes Alkyliden, z. B. Niederalkyliden, wie Isopropyliden, Cycloalkyliden, wie Cyclohexyliden, eine Carbonylgruppe oder Benzyliden.

Eine Mercaptogruppe, wie z. B. in Cystein, kann insbesondere durch S-Alkylierung mit gegebenenfalls substituierten Alkyfresten, Silylierung, Thioacetalbildung, S-Acylierung oder durch die Bildung asymmetrischer Disulfidgruppierungen geschützt sein. Bevorzugte Mercaptoschutzgruppen sind beispielsweise gegebenenfalls im Phenylrest, z. B. durch Methoxy oder Nitro, substituiertes Benzyl, wie 4-Methoxybenzyl, gegebenenfalls im Phenylrest, z. B. durch Methoxy, substituiertes Diphenylmethyl, wie Di-(4-methoxyphenyl)-methyl, Triphenylmethyl, Pyridyldiphenylmethyl, Trimethylsilyl, Benzylthiomethyl, Tetrahydropyranyl, Acylaminomethyl, wie Acetamidomethyl, iso-Butyrylacetamidomethyl oder 2-Chloracetamidomethyl, Benzoyl, Benzyloxycarbonyl oder Alkyl-, insbesondere Niederalkylaminocarbonyl, wie Ethylaminocarbonyl, sowie Niederalkylthio, wie S-Ethylthio oder S-tert-Butylthio, oder S-Sulfo.

Eine Sulfogruppe kann beispielsweise durch Niederalkyl, z. B. Methyl oder Ethyl, durch Phenyl oder als Sulfonamid, beispielsweise als Imidazolid, geschützt sein.

Als Schutzgruppe, beispielsweise Carboxyschutzgruppe, im Sinne dieser Anmeldung ist ausdrücklich auch ein in leicht abspaltbarer Weise mit der zu schützenden funktionellen Gruppe, beispielsweise Carboxygruppe, verbundener polymerer Träger zu verstehen, wie er z. B. für die Merrifield-Synthese geeignet ist. Ein solcher geeigneter polymerer Träger ist beispielsweise ein durch Copolymerisation mit Divinylbenzol schwach vernetztes Polystyrolharz, das zur reversiblen Bindung geeignete Brückenglieder trägt.

Die Freisetzung von geschützten Gruppen erfolgt gegebenenfalls nach den unter Verfahren f) (Schutzgruppenabspaltung) beschriebenen Methoden.

### Verfahren b) (Kondensation zur Herstellung einer Amidbindung)

In Ausgangsmaterialien der Formel IV und V sind funktionelle Gruppen mit Ausnahme der Gruppen, die an der Reaktion teilnehmen sollen oder die unter den Reaktionsbedingungen nicht reagieren, unabhängig voneinander durch eine der unter Verfahren a) genannten Schutzgruppen geschützt.

Die Säuren der Formel V enthalten eine freie Carboxy- oder Sulfogruppe oder reaktionsfähige Derivate davon, so dass auch reaktionsfähige Säurederivate der Verbindungen der Formel V vorliegen können, z. B. die abgeleiteten aktivierten Ester oder reaktionsfähigen Anhydride, ferner reaktionsfähige cyclische Amide. Die reaktionsfähigen Säurederivate können auch in situ gebildet werden.

Aktivierte Ester von Carbonsäuren der Formel V mit sind insbesondere am Verknüpfungskohlenstoffatom des veresternden Restes ungesättigte Ester, z. B. vom Vinylester-Typ, wie Vinylester (erhältlich z. B. durch Umesterung eines entsprechenden Esters mit Vinylacetat; Methode des aktivierten Vinylesters), Carbamoylester (erhältlich z. B. durch Behandeln der entsprechenden Säure mit einem Isoxazoliumreagens; 1,2-Oxazolium- oder Woodward-Methode), oder 1-Niederalkoxyvinylester (erhältlich z. B. durch Behandeln der entsprechenden Säure mit einem Niederalkoxyacetylen; Ethoxyacetylen-Methode), oder Ester vom Amidinotyp, wie N,N'-disubstituierte Amidinoester (erhältlich z. B. durch Behandeln der entsprechenden Säure mit einem geeigneten N,N'-disubstituierten Carbodiimid, z. B. N,N'-Dicyclohexylcarbodiimid; Carbodiimid-Methode), oder N,N-disubstituierte Amidinoester (erhältlich z. B. durch Behandeln der entsprechenden Säure mit einem N,N-disubstituierten Cyananmid; Cyanamid-Methode), geeignete Arylester, insbesondere durch elektronenanziehende Substituenten geeignet substituierte Phenylester (erhältlich z. B. durch Behandeln der entsprechenden Säure mit einem geeignet substituierten Phenol, z. B. 4-Nitrophenol, 4-Methylsulfonylphenol, 2,4,5-Trichlorphenol, 2,3,4,5,6-Pentachlorphenol oder 4-Phenyldiazophenol, in Gegenwart eines Kondensationsmittels, wie N,N'-Dicyclohexylcarbodiimid; Methode der aktivierten Arylester), Cyanmethylester (erhältlich z. B. durch Behandeln der entsprechenden Säure mit Chloracetonitril in Gegenwart einer Base; Cyanmethylester-Methode), Thioester, insbesondere gegebenenfalls, z. B. durch Nitro, substituierte Phenylthioester (erhältlich z. B. durch Behandeln der entsprechenden Säure mit gegebenenfalls, z. B. durch Nitro, substituierten Thiophenolen, u. a. mit Hilfe der Anhydrid- oder Carbodiimid-Methode; Methode der aktivierten Thiolester), oder insbesondere Amino- oder Amidoester (erhältlich z. B. durch Behandeln der entsprechenden Säure mit einer N-Hydroxyamino- bzw. N-Hydroxyamidoverbindung, z. B. N-Hydroxysuccinimid, N-Hydroxypiperidin, N-Hydroxyphthalimid, N-Hydroxy-5-norbornen-2,3-dicarbonsäureimid, 1-Hydroxybenztriazol oder 3-Hydroxy-3,4-dihydro-1,2,3-benztriazin-4-on, z. B. nach der Anhydrid- oder Carbodiimid-Methode; Methode der aktivierten N-Hydroxyester). Es sind auch innere Ester, z. B. γ-Lactone, einsetzbar.

Anhydride von Carbonsäuren der Formel V können symmetrische oder vorzugsweise gemischte Anhydride dieser Säuren sein, z. B. Anhydride mit anorganischen Säuren, wie Säurehalogenide, insbesondere Säurechloride (erhältlich z. B. durch Behandeln der entsprechenden Säure mit Thionylchlorid, Phosphorpentachlorid, Phosgen, 1-Chlor-N,N,2-trimethyl-1-propenamin (Reaktionsbedingungen unter Verfahren a) genannt) oder Oxalylchlorid; Säurechloridmethode), Azide (erhältlich z. B. aus einem entsprechenden Säureester über das entsprechende Hydrazid und dessen Behandlung mit salpetriger Säure; Azidmethode), Anhydride mit Kohlensäurehalbestern, z. B. Kohlensäureniederalkylhalbestern (erhältlich z. B. durch Behandeln der entsprechenden Säure mit Chlorameisensäureniederalkylestern oder mit einem 1-Niederalkoxycarbonyl-2-niederalkoxy-1,2-dihydrochinolin; Methode der gemischten O-Alkylkohlensäureanhydride), oder Anhydride mit dihalogenierter, insbesondere dichlorierter Phosphorsäure (erhältlich z. B. durch Behandeln der entsprechenden Säure mit Phosphoroxychlorid; Phosphoroxychloridmethode), Anhydride mit anderen Phosphorsäurederivaten (z. B. solchen, die man mit Phenyl-N-phenylphosphoramidochloridat erhalten kann oder durch Umsetzung von Alkylphosphorsäureamiden in Gegenwart von Sulfonsäureanhydriden und/oder racemisierungssenkenden Additiven, wie N-Hydroxybenztriazol, oder in Gegenwart von Cyanphosphonsäurediethylester) oder mit Phosphorigsäurederivaten, oder Anhydride mit organischen Säuren, wie gemischte Anhydride mit organischen Carbonsäuren (erhältlich z. B. durch Behandeln der entsprechenden Säure mit einem gegebenenfalls substituierten Niederalkan- oder Phenylniederalkancarbonsäurehalogenid, z. B. Phenylessigsäure-, Pivalinsäure- oder Trifluoressigsäurechlorid; Methode der gemischten Carbonsäureanhydride) oder mit organischen Sulfonsäuren (erhältlich z. B. durch Behandeln eines Salzes, wie eines Alkalimetallsalzes, der entsprechenden Säure mit einem geeigneten organischen Sulfonsäurehalogenid, wie Niederalkan- oder Aryl-, z. B. Methan- oder p-Toluolsulfonsäurechlorid; Methode der gemischten Sulfonsäureanhydride) sowie symmetrische Anhydride (erhältlich z. B. durch Kondensation der entsprechenden Säure in Gegenwart eines Carbodiimides oder von 1-Diethylaminopropin; Methode der symmetrischen Anhydride).

Geeignete cyclische Amide von Carbonsäuren der Formel V sind insbesondere Amide mit fünfgliedrigen Diazacyclen aromatischen Charakters, wie Amide mit Imidazolen, z. B. Imidazol (erhältlich z. B. durch Behandeln der entsprechenden Säure mit N,N'-Carbonyldiimidazol; Imidazol-Methode), oder Pyrazol, z. B. 3,5-Dimethylpyrazol (erhältlich z. B. über das Säurehydrazid durch Behandeln mit Acetylaceton; Pyrazolid-Methode).

Wie erwähnt, können Derivate von Carbonsäuren der Formel V, die als Acylierungsmittel verwendet werden, auch in situ gebildet werden. So kann man z. B. N,N'-disubstituierte Amidinoester in situ bilden, indem man das Gemisch des Ausgangsmaterials der Formel IV und der als Acylierungsmittel verwendeten Säure in Gegenwart eines geeigneten N,N'-disubstituierten Carbodiimids, z. B. N,N'-Cyclohexylcarbodiimid, zur Reaktion bringt. Weiter kann man Amino- oder Amidoester der als Acylierungsmittel verwendeten Säuren in Gegenwart des zu acylierenden Ausgangsmaterials der Formel IV bilden, indem man das Gemisch der entsprechenden Säure- und Amino-Ausgangsstoffe in Gegenwart eines N,N'-disubstituierten Carbodiimids, z. B. N,N'-Dicyclohexylcarbodiimid, und eines N-Hydroxyamins oder N-Hydroxy-amids, z. B. N-Hydroxysuccinimid, gegebenenfalls in Anwesenheit einer geeigneten Base, z. B. 4-Dimethylamino-pyridin, umsetzt. Ferner kann man durch Umsetzung mit N,N,N',N'-Tetraalkyluroniumverbindungen, wie O-Benztriazol-1yl-N,N,N',N'-tetra-methyl-uronium-hexafluorphosphat, O-(1,2-Dihydro-2-oxo-1-pyridyl)-N,N,N',N'-tetramethyluronium-tetrafluorborat oder O-(3,4-Dihydro-4-oxo-1,2,3-benz-triazolin-3-yl)-N,N,N',N'-tetramethyluronium-tetrafluorborat, in situ aktivieren. Schliesslich können Phosphorsäureanhydride der Carbonsäuren der Formel V in situ hergestellt werden, indem man ein Alkylphosphorsäureamid, wie Hexamethylphosphorsäuretriamid, in Gegenwart eines Sulfonsäureanhydrides, wie 4-Toluolsulfonsäureanhydrid, mit einem Salz, wie einem Tetrafluoroborat, z. B. Natriumtetrafluoroborat, oder mit einem anderen Abkömmling des Hexamethylphosphorsäuretriamides, wie Benzotriazol-1-yl-oxy-tris-(dimethylamino)-phosphonium-hexafluorid, vorzugsweise in Gegenwart eines racemisierungssenkenden Additives, wie N-Hydroxybenztriazol, umsetzt.

In analoger Weise lassen sich viele der oben für Carbonsäuren der Formel V aufgeführten Reaktionstypen auch für Verbindungen der Formel V mit endständiger Sulfonylgruppe bei der Kondensation mit Verbindungen der Formel IV zu Sulfonamiden durchführen.

So können beispielsweise aktivierte Sulfonsäureester eingesetzt werden, z. B. die entsprechenden, insbesondere durch Nitrogruppen substituierten, Arylester, wie Phenylester, wobei die Aminkomponente der Formel IV auch als Alkalimetallamid, z. B. Alkalimetallarylamid, wie Natriumanilinamid, oder Alkalimetallsalz von stickstoffhaltigen Heterocyclen, z. B. Kalium-pyrrolid, eingesetzt werden kann.

Ferner können reaktionsfähige Anhydride zum Einsatz kommen, wie etwa die entsprechenden symmetrischen (herstellbar z. B. durch Reaktion der alkylsulfonsauren Silbersalze mit Alkylsulfonylchloriden) oder vorzugsweise asymmetrischen Säureanhydride, z. B. Anhydride mit anorganischen Säuren, wie Sulfonylhalogenide, insbesondere Sulfonylchloride (erhältlich z. B. durch Umsetzung der entsprechenden Sulfonsäuren mit anorganischen Säurechloriden, z. B. Thionylchlorid, Phosphorpentachlorid), mit organischen Carbonsäuren (erhältlich z. B. durch Behandeln eines Sulfonsäurehalogenides mit dem Salz einer Carbonsäure, wie einem Alkalimetallsalz, analog der oben genannten Methode der gemischten Sulfonsäureanhydride), oder Azide (erhältlich z. B. aus einem entsprechenden Sulfonsäurechlorid und Natriumazid oder über das entsprechende Hydrazid und dessen Behandlung mit salpetriger Säure analog der oben genannten Azidmethode).

Die Aminogruppe von Verbindungen der Formel IV, die an der Reaktion teilnimmt, trägt vorzugsweise mindestens ein reaktionsfähiges Wasserstoffatom, insbesondere, wenn die damit reagierende Carboxy- oder Sulfonylgruppe in reaktionsfähiger Form vorliegt; sie kann aber auch selbst derivatisiert sein, z. B. durch Reaktion mit einem Phosphit, wie Diethylchlorphosphit, 1,2-Phenylenchlorphosphit, Ethyldichlorphosphit, Ethylenchlorphosphit oder Tetraethylpyrophosphit. Ein Derivat einer solchen Verbindung mit einer Aminogruppe ist z. B. auch ein Carbaminsäurehalogenid oder ein Isocyanat, wobei die an der Reaktion teilnehmende Aminogruppe durch Halogencarbonyl, z. B. Chlorcarbonyl, substituiert bzw. als Isocyanatgruppe abgewandelt ist, wobei im letzteren Falle nur Verbindungen der Formel I zugänglich sind, die am Stickstoffatom der durch die Reaktion gebildeten Amidgruppe ein Wasserstoffatom tragen (R₈=H).

Die Kondensation zur Herstellung einer Amidbindung kann in an sich bekannter Weise durchgeführt werden, beispielsweise wie in Standardwerken, wie "Houben-Weyl, Methoden der organischen Chemie", 4. Auflage, Band 15/II (1974), Band IX (1955) Band E 11 (1985), Georg Thieme Verlag, Stuttgart, "The Peptides" (E. Gross und J. Meienhofer, Hg.), Band 1 und 2, Academic Press, London und New York, 1979/1980, oder M. Bodansky, "Principles of Peptide Synthesis", Springer-Verlag, Berlin 1984, beschrieben.

Die Kondensation einer freien Carbonsäure der Formel V mit dem entsprechenden Amin der Formel IV kann insbesondere in Gegenwart eines der üblichen Kondensationsmittel durchgeführt werden, oder unter Verwendung von Carbonsäureanhydriden oder Carbonsäurehalogeniden, wie -chloriden, oder von aktivierten Carbonsäureestern, wie p-Nitrophenylestern. Übliche Kondensationsmittel sind z. B. Carbodiimide, beispielsweise Diethyl-, Dipropyl-, N-Ethyl-N'-(3-dimethylaminopropyl)-carbodiimid oder insbesondere Dicyclohexylcarbodiimid, ferner geeignete Carbonylverbindungen, beispielsweise N,N'-Carbonyldiimidazol, 1,2-Oxazoliumverbindungen, z. B.
2-Ethyl-5-phenyl-1,2-oxazolium-3'-sulfonat und
2-tert-Butyl-5-methylisoxazoliumperchlorat, oder eine geeignete Acylaminoverbindung, z. B. 2-Ethoxy-1-ethoxycarbonyl-1,2-dihydrochinolin, N,N,N',N'-Tetraalkyluroniumverbindungen, wie O-Benztriazol-1-yl-N,N,N',N'-tetra-methyluronium-hexafluorphosphat, ferner aktivierte Phosphorsäurederivate, z. B. Diphenylphosphorylazid, Diethylphosphorylcyanid, Phenyl-N-phenylphosphoroamidochloridat, Bis-(2-oxo-3-oxazolidinyl)-phosphinsäurechlorid oder 1-Benztriazolyloxy-tris-(dimethylamino)-phosphonium-hexafluorophosphat.

Gewünschtenfalls wird eine organische Base zugegeben, vorzugsweise ein tertiäres Amin, z. B. ein Triniederalkylamin mit voluminösen Resten, wie Ethyldiisopropylamin oder Triethylamin, und/oder eine heterocyclische Base, z. B. 4-Dimethylaminopyridin oder bevorzugt N-Methylmorpholin oder Pyridin.

Die Kondensation aktivierter Ester, reaktionsfähiger Anhydride oder reaktionsfähiger cyclischer Amide mit den entsprechenden Aminen wird üblicherweise in Gegenwart einer organischen Base, z. B. einfachen Triniederalkylaminen, z. B. Triethylamin oder Tributylamin, oder einer der vorstehend genannten organischen Basen durchgeführt. Gewünschtenfalls wird zusätzlich noch ein Kondensationsmittel verwendet, wie für freie Carbonsäuren beschrieben.

Die Kondensation von Säureanhydriden mit Aminen kann z. B. in Gegenwart von anorganischen Carbonaten, z. B. Ammonium- oder Alkalimetallcarbonaten oder -hydrogencarbonaten, wie Natrium- oder Kaliumcarbonat oder -hydrogencarbonat (gewünschtenfalls zusammen mit einem Sulfat), erfolgen.

Carbonsäurechloride, beispielsweise die von der Säure der Formel V abgeleiteten Chlorkohlensäurederivate, oder Sulfonsäurechloride werden mit den entsprechenden Aminen vorzugsweise in Gegenwart eines organischen Amins, z. B. der vorstehend genannten Triniederalkylamine oder heterocyclischen Basen, gegebenenfalls in Gegenwart eines Hydrogensulfates, kondensiert.

Die Kondensation wird vorzugsweise in einem inerten, aprotischen, vorzugsweise wasserfreien, Lösungsmittel oder Lösungsmittelgemisch durchgeführt, beispielsweise in einem Carbonsäureamid, z. B. Formamid oder Dimethylformamid, einem halogenierten Kohlenwasserstoff, z. B. Methylenchlorid, Tetrachlorkohlenstoff oder Chlorbenzol, einem Keton, z. B. Aceton, einem cyclischen Ether, z. B. Tetrahydrofuran, einem Ester, z. B. Essigsäureethylester, oder einem Nitril, z. B. Acetonitril, oder in einer Mischung davon, gegebenenfalls bei erniedrigter oder erhöhter Temperatur, z. B. in einem Temperaturbereich von etwa -40 °C bis etwa +100 °C, bevorzugt von etwa -10 °C bis etwa +50 °C, im Falle der Verwendung von Arylsulfonylestern auch bei etwa +100 °C bis +200 °C, und gegebenenfalls unter Inertgas-, z. B. Stickstoff- oder Argonatmosphäre.

Auch wässrige, beispielsweise alkoholische, z. B. Ethanol, oder aromatische Lösungsmittel, z. B. Benzol oder Toluol, sind möglich. Bei Gegenwart von Alkalihydroxiden als Basen kann gegebenenfalls auch Aceton zugesetzt werden.

Die Kondensation kann auch gemäss der als Festphasen-Synthese bekannten Technik erfolgen, die auf R. Merrifield zurückgeht und beispielsweise in Angew. Chem. 97, 801 - 812 (1985), Naturwissenschaften 71, 252 - 258 (1984) oder in R. A. Houghten, Proc. Natl. Acad. Sci. USA 82, 5131 - 5135 (1985) beschrieben ist.

Für den Spezialfall der Einführung eines Restes eines Halbesters der Kohlensäure R₉ eignen sich insbesondere die Verbindungen der Formel V',

Rₓ-O-(C=O)-Z₂ (V')

worin Rₓ insbesondere Niederalkyl, Halogenniederalkyl, Arylniederalkyl, Heterocyclylniederalkyl, Niederalkenyl, Niederalkoxyniederalkyl oder (Niederalkoxyniederalkoxy)-niederalkyl bedeutet, wie bei der Definition von Acylgruppen R₉ eines Halbesters der Kohlensäure genannt, und worin Z₂ Halogen, wie Chlor, bedeutet, welche z.B. hergestellt werden können durch Umsetzung der komplementären Alkohole, wie Niederalkyl-, Halogenniederalkyl-, Arylniederalkyl-, Heterocyclylniederalkyl-, Niederalkenyl-, Niederalkoxyniederalkyl-oder (Niederalkoxyniederalkoxy)-niederalkylalkohole, mit Phosgen oder ferner Analogen davon, welche statt Chlor andere Halogenatome, insbesondere Brom, enthalten, vorzugsweise in Gegenwart von tertiären Stickstoffbasen, wie Pyridin oder Triethylamin, und in inerten Lösungsmitteln, z.B. chlorierten Kohlenwasserstoffen, wie Methylenchlorid oder Chloroform, Ethern, wie Diethylether, Tetrahydrofuran oder Dioxan, oder Carbonsäureamiden, wie Dimethylformamid. Auch entsprechende N-Carbonylazolide der Formel V' (Z₂ = N-haltiger Heterocyclus, wie 1-Imidazolido) sind geeignet, welche beispielsweise durch Umsetzung mit den entsprechenden N,N'-Carbonyldiazoliden, wie N,N'-Carbonyldiimidazol, erhalten werden unter Bedingungen, wie gerade für Phosgen und Analoge mit anderen Halogenatomen beschrieben. Die Reaktion der Verbindungen der Formel IV mit der entsprechenden Verbindungen der Formel V' findet dann ebenfalls unter diesen Bedingungen statt (vgl. Staab, H. A., Angew. Chemie 74, 407 (1962).

Für den Spezialfall der Einführung eines Restes R₉ einer unsubstituierten oder substituierten Carbaminsäure, wie Carbamoyl oder unsubstituiertes oder subtituiertes N-Alkylcarbamoyl, eignet sich als aktiviertes Säurederivat insbesondere das entsprechende Isocyanat der Formel V'',

W-N=C=O (V'')

worin W eine Aminoschutzgruppe, z.B. Trihalogenacetyl, wie Trifluor- oder Trichloracetyl, oder einen der oben bei der Definition von Acylgruppen R₉ einer substituierten Carbaminsäure genannten unsubstituierten oder substituierten Alkylreste bedeutet, wobei man, wenn W eine Aminoschutzgruppe bedeutet, nach der Umsetzung mit der Verbindung der Formel IV die entsprechende Verbindung der Formel I, worin R₉ Carbamoyl bedeutet, durch Abspaltung der Schutzgruppe W erhalten kann, wie unten bei der Freisetzung von geschütztem Amino beschrieben, insbesondere durch saure Hydrolyse, oder, wenn W einen der genannten substituierten oder unsubstituierten Niederalkylreste bedeutet, eine entsprechende Verbindung der Formel I mit am Stickstoff monosubstituiertem Aminocarbonyl R₉. Sowohl Aminocarbonyl als auch N-monosubstituiertes Aminocarbonyl R₉ kann in N-disubstituiertes Aminocarbonyl überführt werden, indem man mit einem weiteren unsubstituierten oder substituierten Niederalkylrest unter Verwendung geeigneter Ausgangsmaterialien alkyliert unter analogen Bedingungen, wie unten bei den zusätzlichen Verfahrensmassnahmen beschrieben.

Die Umsetzungen mit den Verbindungen der Formeln V' und V'' erfolgen unter analogen Reaktionsbedingungen zu den für die Umsetzung von Verbindungen der Formel IX und IX' mit solchen der Formel II unter Verfahren a) genannten.

Je nach verwendeten Ausgangsverbindungen können die Reste R₁ und R₉ in den erhältlichen Verbindungen der Formel I identisch oder voneinander verschieden sein.

Die Freisetzung von geschützten Gruppen erfolgt gegebenenfalls nach den unter Verfahren f) (Schutzgruppenabspaltung) beschriebenen Methoden.

### Verfahren c) (Herstellung einer Amidbindung)

In Ausgangsmaterialien der Formel VI und VII sind funktionelle Gruppen mit Ausnahme der Gruppen, die an der Reaktion teilnehmen sollen oder die unter den den Reaktionsbedingungen nicht reagieren, unabhängig voneinander durch eine der unter Verfahren a) genannten Schutzgruppen geschützt.

Das Verfahren ist vollständig analog zu dem unter Verfahren b) genannten, wenn man anstelle von Verbindungen der Formel IV die der Formel VI und anstelle von Verbindungen der Formel V die der Formel VII einsetzt.

Je nach verwendeten Ausgangsverbindungen können die Reste R₁ und R₉ in den erhältlichen Verbindungen der Formel I identisch oder voneinander verschieden sein.

Bei den Umsetzungen in Verfahren b), aber auch in c) und d) kann es in einzelnen Fällen auch zu Acylwanderungen des Acylrestes in Acyloxy R₅ an das Stickstoffatom kommen, an das R₉ angekoppelt werden soll; analoge Nebenreaktionen sind bei Verfahren c) oder d) möglich.

Die Freisetzung von geschützten Gruppen erfolgt gegebenenfalls nach den unter Verfahren f) (Schutzgruppenabspaltung) beschriebenen Methoden.

### Verfahren d) (Herstellung einer Amidbindung)

In Ausgangsmaterialien der Formel VIII und der zur Einführung der identischen Reste R₁ und R₉' geeigneten Säure oder deren reaktionsfähigen Derivaten sind funktionelle Gruppen, die nicht an der Reaktion teilnehmen sollen oder die unter den Reaktionsbedingungen nicht reagieren, unabhängig voneinander durch eine der unter Verfahren a) genannten Schutzgruppen geschützt.

Die zur Einführung der identischen Reste R₁ und R₉ geeignete Säure hat vorzugsweise eine der Formeln V oder VII, oder sie liegt als reaktionsfähiges Derivat einer derartigen Säure vor, wie oben beschrieben.

Bevorzugt als gegebenenfalls durch Schutzgruppen geschützte Ausgangsverbindungen der Formel VIII sind diejenigen, die im Abschnitt über Ausgangsverbindungen als bevorzugt beschrieben sind.

Die Bedingungen für das Verfahren sind analog zu den unter Verfahren b) genannten, wobei man anstelle von Verbindungen der Formel IV die der Formel VIII und anstelle von Verbindungen der Formel V die der Formel V oder VII einsetzt.

Die Freisetzung von geschützten Gruppen erfolgt gegebenenfalls nach den unter Verfahren f) (Schutzgruppenabspaltung) beschriebenen Methoden.

### Verfahren e) (Alkylierung eines sekundären Stickstoffatomes)

In Ausgangsmaterialien der Formel I' und der zur Einführung des Restes R₇'' geeigneten Verbindung der Formel XII oder deren reaktionsfähigen Derivaten sind funktionelle Gruppen, die nicht an der Reaktion teilnehmen sollen oder die unter den Reaktionsbedingungen nicht reagieren, unabhängig voneinander durch eine der unter Verfahren a) genannten Schutzgruppen geschützt.

Eine Abgangsgruppe X ist insbesondere eine nukleofuge Abgangsgruppe ausgewählt aus mit einer starken anorganischen oder organischen Säure verestertem Hydroxy, wie mit einer Mineralsäure, z. B. Halogenwasserstoffsäure, wie Chlorwasserstoff-, Bromwasserstoff- oder Jodwasserstoffsäure, oder mit einer starken organischen Sulfonsäure, wie einer gegebenenfalls, z. B. durch Halogen, wie Fluor, substituierten Niederalkansulfonsäure oder einer aromatischen Sulfonsäure, z. B. einer gegebenenfalls durch Niederalkyl, wie Methyl, Halogen, wie Brom, und/oder Nitro substituierten Benzolsulfonsäure, z. B. einer Methansulfon-, p-Bromtoluolsulfonsäure oder p-Toluolsulfonsäure verestertes Hydroxy oder mit Stickstoffwasserstoffsäure verestertes Hydroxy.

Die Substitution kann unter den Bedingungen einer nukleophilen Substitution erster oder zweiter Ordnung ablaufen.

Beispielsweise kann man eine Verbindung der Formeln XII, insbesondere eine, worin X für eine Abgangsgruppe mit hoher Polarisierbarkeit der Elektronenhülle, z. B. für Jod, steht, in einem polaren aprotischen Lösungsmittel, z. B. Aceton, Acetonitril, Nitromethan, Dimethylsulfoxid oder Dimethylformamid, einsetzen. Die Reaktion kann auch in Wasser, dem gegebenenfalls als Lösungsvermittler ein organisches Lösungsmittel, z. B. Ethanol, Tetrahydrofuran oder Aceton, beigemischt ist, durchgeführt werden. Die Substitutionsreaktion wird bei Raumtemperatur oder bei erniedrigter oder erhöhter Temperatur, z. B. in einem Temperaturbereich von etwa -40 ° bis etwa 100 °C, bevorzugt von etwa - 10 ° bis etwa 50 °C, und gegebenenfalls unter Inertgas, z. B. Stickstoff- oder Argonatmosphäre, durchgeführt.

Die Freisetzung von geschützten Gruppen erfolgt gegebenenfalls nach den unter Verfahren f) (Schutzgruppenabspaltung) beschriebenen Methoden.

### Verfahren f) (Schutzgruppenabspaltung)

Die Abspaltung der Schutzgruppen, die nicht Bestandteil des gewünschten Endproduktes der Formel I sind, z. B. der Carboxy-, Amino-, Hydroxy-, Mercapto- und/oder Sulfoschutzgruppen, erfolgt in an sich bekannter Weise, z. B. mittels Solvolyse, insbesondere Hydrolyse, Alkoholyse oder Acidolyse, oder mittels Reduktion, insbesondere Hydrogenolyse oder chemischer Reduktion, sowie Photolyse, gegebenenfalls stufenweise oder gleichzeitig, wobei auch enzymatische Methoden verwendet werden können. Die Abspaltung der Schutzgruppen ist beispielsweise in den unter Verfahren a) im Abschnitt über Schutzgruppen genannten Standardwerken beschrieben.

So kann beispielsweise geschütztes Carboxy, z. B. tert-Niederalkoxycarbonyl, in 2-Stellung durch eine trisubstituierte Silylgruppe oder in 1-Stellung durch Niederalkoxy oder Niederalkylthio substituiertes Niederalkoxycarbonyl oder gegebenenfalls substituiertes Diphenylmethoxycarbonyl durch Behandeln mit einer geeigneten Säure, wie Ameisensäure, Chlorwasserstoff oder Trifluoressigsäure, gegebenenfalls unter Zugabe einer nukleophilen Verbindung, wie Phenol oder Anisol, in freies Carboxy überführt werden. Aus Niederalkoxycarbonyl kann auch durch Basen, wie Hydroxide, z. B. Alkalimetallhydroxide, wie NaOH oder KOH, Carboxy freigesetzt werden. Gegebenenfalls substituiertes Benzyloxycarbonyl kann z. B. mittels Hydrogenolyse, d.h. durch Behandeln mit Wasserstoff in Gegenwart eines metallischen Hydrierkatalysators, wie eines Palladium-katalysators, freigesetzt werden. Ferner kann geeignet substituiertes Benzyloxycarbonyl, wie 4-Nitrobenzyloxycarbonyl, auch durch Reduktion, z. B. durch Behandeln mit einem Alkalimetall, wie Natrium-dithionit, oder mit einem reduzierenden Metall, z. B. Zink, oder einem reduzierenden Metallsalz, wie einem Chrom-II-salz, z. B. Chrom-II-chlorid, üblicherweise in Gegenwart eines Wasserstoff-abgebenden Mittels, das zusammen mit dem Metall nascierenden Wasserstoff erzeugen kann, wie einer Säure, in erster Linie einer geeigneten Carbonsäure, wie einer gegebenenfalls, z. B. durch Hydroxy, substituierten Niederalkancarbonsäure, z. B. Essigsäure, Ameisensäure, Glycolsäure, Diphenylglycolsäure, Milchsäure, Mandelsäure, 4-Chlormandelsäure oder Weinsäure, oder eines Alkohols oder Thiols, wobei man vorzugsweise Wasser zugibt, in freies Carboxy überführt werden. Durch Behandeln mit einem reduzierenden Metall oder Metallsalz, wie oben beschrieben, kann auch 2-Halogenniederalkoxycarbonyl (gegebenenfalls nach Umwandlung einer 2-Bromniederalkoxycarbonylgruppe in eine entsprechende 2-Iodniederalkoxycarbonylgruppe) oder Aroylmethoxycarbonyl in freies Carboxy umgewandelt werden. Aroylmethoxycarbonyl kann ebenfalls durch Behandeln mit einem nukleophilen, vorzugsweise salzbildenden Reagens, wie Natriumthiophenolat oder Natriumjodid, gespalten werden. 2-(trisubstituiertes Silyl)-niederalkoxycarbonyl, wie 2-Triniederalkylsilylniederalkoxycarbonyl, kann auch durch Behandeln mit einem das Fluoridanion liefernden Salz der Fluorwasserstoffsäure, wie einem Alkalimetallfluorid, z. B. Natrium- oder Kaliumfluorid, gegebenenfalls in Anwesenheit eines macrocyclischen Polyethers ("Kronenether"), oder mit einem Fluorid einer organischen quaternären Base, wie Tetraniederalkylammoniumfluorid oder Triniederalkylarylniederalkylammoniumfluorid, z. B. Tetraethyl-ammoniumfluorid oder Tetrabutylammoniumfluorid, in Gegenwart eines aprotischen, polaren Lösungsmittels, wie Dimethylsulfoxid oder N,N-Dimethylacetamid, in freies Carboxy überführt werden. Als organisches Silyloxycarbonyl, wie Triniederalkylsilyloxycarbonyl, z. B. Trimethylsilyloxycarbonyl, geschütztes Carboxy kann in üblicher Weise solvolytisch, z. B. durch Behandeln mit Wasser, einem Alkohol oder Säure, oder ausserdem Fluorid, wie oben beschrieben, freigesetzt werden. Verestertes Carboxy kann auch enzymatisch freigesetzt werden, beispielsweise durch Esterasen oder geeignete Peptidasen, z. B. verestertes Arginin oder Lysin, wie Lysinmethylester, mittels Trypsin.

Eine geschützte Aminogruppe setzt man in an sich bekannter und je nach Art der Schutzgruppen in verschiedenartiger Weise, vorzugsweise mittels Solvolyse oder Reduktion, frei. Niederalkoxycarbonylamino, wie tert-Butoxycarbonylamino, kann in Gegenwart von Säuren, beispielsweise Mineralsäuren, z. B. Halogenwasserstoff, wie Chlorwasserstoff oder Bromwasserstoff, oder von Schwefel- oder oder Phosphorsäure, vorzugsweise von Chlorwasserstoff, oder von starken organischen Säuren, wie Trihalogenessigsäure, z. B. Trifluoressigsäure, oder Ameisensäure, in polaren Lösungsmitteln, wie Wasser, oder Ethern, bevorzugt cyclischen Ethern, wie Dioxan, 2-Halogenniederalkoxycarbonylamino (gegebenenfalls nach Umwandlung einer 2-Bromniederalkoxycarbonylaminogruppe in eine 2-Iodniederalkoxycarbonylaminogruppe), oder direkt gelöst in einer flüssigen organischen Carbonsäure, wie Ameisensäure, Aroylmethoxycarbonylamino oder 4-Nitrobenzyloxycarbonylamino kann z. B. durch Behandeln mit einem geeigneten Reduktionsmittel, wie Zink in Gegenwart einer geeigneten Carbonsäure, wie wässriger Essigsäure, gespalten werden. Aroylmethoxycarbonylamino kann auch durch Behandeln mit einem nucleophilen, vorzugsweise salzbildenden Reagenz, wie Natriumthiophenolat, und 4-Nitrobenzyloxycarbonylamino auch durch Behandeln mit einem Alkalimetall-, z. B. Natrium-dithionit, gespalten werden. Gegebenenfalls substituiertes Diphenylmethoxycarbonylamino, tert-Niederalkoxycarbonylamino oder 2-(trisubstituiertes Silyl)-niederalkoxycarbonylamino, wie 2-Triniederalkylsilylniederalkoxycarbonylamino, kann durch Behandeln mit einer geeigneten Säure, z. B. Ameisen- oder Trifluoressigsäure, gegebenenfalls substituiertes Benzyloxycarbonylamino z. B. mittels Hydrogenolyse, d.h. durch Behandeln mit Wasserstoff in Gegenwart eines geeigneten Hydrierkatalysators, wie eines Platin- oder Palladium-katalysators, gegebenenfalls substituiertes Triarylmethylamino oder Formylamino z. B. durch Behandeln mit einer Säure, wie Mineralsäure, z. B. Chlorwasserstoffsäure, oder einer organischen Säure, z. B. Ameisen-, Essig-oder Trifluoressigsäure, gegebenenfalls in Gegenwart von Wasser, gespalten werden, und eine als Silylamino geschützten Aminogruppe z. B. mittels Hydrolyse oder Alkoholyse freigesetzt werden. Eine durch 2-Halogenacetyl, z. B. 2-Chloracetyl, geschützte Aminogruppe kann durch Behandeln mit Thioharnstoff in Gegenwart einer Base, oder mit einem Thiolatsalz, wie einem Alkalimetallthiolat des Thioharnstoffs, und anschliessende Solvolyse, wie Alkoholyse oder Hydrolyse, des entstandenen Substitutionssproduktes freigesetzt werden, aus Trifluoracetylamino wird beispielsweise durch Hydrogenolyse mit Basen, wie Alkalimetallhydroxiden oder -carbonaten, wie Na₂CO₃ oder K₂CO₃, in polaren Lösungsmitteln, z. B. Alkoholen, wie Methanol, bei Temperaturen zwischen 0 und 100 °C, insbesondere bei 40 bis 80 °C, Amino freigesetzt. Eine durch 2-(trisubstituiertes Silyl)-niederalkoxycarbonyl, wie 2-Triniederalkylsilylniederalkoxycarbonyl, geschützte Aminogruppe kann auch durch Behandeln mit einem Fluoridanionen liefernden Salz der Fluorwasserstoffsäure, wie oben im Zusammenhang mit der Freisetzung einer entsprechend geschützten Carboxygruppe angegeben, in die freie Aminogruppe überführt werden. Ebenso kann man direkt an ein Heteroatom, wie Stickstoff, gebundenes Silyl, wie Trimethylsilyl, mittels Fluoridionen abspalten.

In Form einer Azidogruppe geschütztes Amino wird z. B. durch Reduktion in freies Amino überführt, beispielsweise durch katalytische Hydrierung mit Wasserstoff in Gegenwart eines Hydrierkatalysators, wie Platinoxid, Palladium oder Raney-Nickel, durch Reduktion mittels Mercaptoverbindungen, wie Dithiothreitol oder Mercaptoethanol, oder auch durch Behandeln mit Zink in Gegenwart einer Säure, wie Essigsäure. Die katalytische Hydrierung wird vorzugsweise in einem inerten Lösungsmittel, wie einem halogenierten Kohlenwasserstoff, z. B. Methylenchlorid, oder auch in Wasser oder einem Gemisch von Wasser und einem organischen Lösungsmittel, wie einem Alkohol oder Dioxan, bei etwa 20 °C bis 25 °C, oder auch unter Kühlen oder Erwärmen, durchgeführt.

Eine durch eine geeignete Acylgruppe, eine Triniederalkylsilylgruppe oder durch gegebenenfalls substituiertes 1-Phenylniederalkyl geschützte Hydroxy- oder Mercaptogruppe wird analog einer entsprechend geschützten Aminogruppe freigesetzt. Eine durch 2,2-Dichloracetyl geschützte Hydroxy- bzw. Mercaptogruppe wird z. B. durch basische Hydrolyse, eine durch tert-Niederalkyl oder durch einen 2-oxa- oder 2-thia-aliphatischen oder -cycloaliphatischen Kohlenwasserstoffrest geschützte Hydroxy- bzw. Mercaptogruppe durch Acidolyse, z. B. durch Behandeln mit einer Mineralsäure oder einer starken Carbonsäure, z. B. Trifluoressigsäure, freigesetzt. Durch Pyridyldiphenylmethyl geschütztes Mercapto kann z. B. durch Quecksilber-II-salze bei pH 2-6 oder durch Zink/Essigsäure oder elektrolytische Reduktion, Acetamidomethyl und iso-Butyrylamidomethyl z. B. durch Reaktion mit Quecksilber-II-salzen bei pH 2-6, 2-Chloracetamidomethyl z. B. durch 1-Piperidinothiocarboxamid, S-Ethylthio, S-tert-Butylthio und S-Sulfo z. B. durch Thiolyse mit Thiophenol, Thioglycolsäure, Natrium-thiophenolat oder 1,4-Dithiothreitol freigesetzt werden. Zwei Hydroxygruppen oder eine benachbarte Amino- und Hydroxygruppe, die zusammen mittels einer bivalenten Schutzgruppe, vorzugsweise z. B. einer durch Niederalkyl ein- oder zweifach substituierten Methylengruppe, wie durch Niederalkyliden, z. B. Isopropyliden, Cycloyalkyliden, z. B. Cyclohexyliden, oder Benzyliden, geschützt sind, können durch saure Solvolyse, besonders in Gegenwart einer Mineralsäure oder einer starken organischen Säure, freigesetzt werden. Eine Triniederalkylsilylgruppe wird ebenfalls durch Acidolyse, z. B. durch Mineralsäure, bevorzugt Fluorwasserstoffsäure, oder eine starke Carbonsäure abgespalten. 2-Halogenniederalkoxycarbonyl wird durch die oben genannten Reduktionsmittel, z. B. reduzierendes Metall, wie Zink, reduzierende Metallsalze, wie Chrom-II-salze, oder durch Schwefelverbindungen, beispielsweise Natriumdithionit oder bevorzugt Natriumsulfid und Schwefelkohlenstoff, entfernt.

Eine als Sulfonsäureester oder Sulfonamid geschützte Sulfogruppe wird beispielsweise durch saure Hydrolyse, z. B. in Gegenwart von Mineralsäure, oder bevorzugt durch basische Hydrolyse, z. B. mit Alkalimetallhydroxid oder Alkalimetallcarbonat, beispielsweise Natriumcarbonat, freigesetzt.

Beim Vorhandensein von mehreren geschützten funktionellen Gruppen können, wenn erwünscht, die Schutzgruppen so gewählt werden, dass gleichzeitig mehr als eine solche Gruppe abgespalten werden kann, beispielsweise acidolytisch, wie durch Behandeln mit Trifluoressigsäure, oder mit Wasserstoff und einem Hydrierkatalysator, wie einem Palladium-Kohle-Katalysator. Umgekehrt können die Gruppen auch so gewählt werden, dass sie nicht alle gleichzeitig, sondern in gewünschter Reihenfolge abgespalten werden können, wobei die entsprechenden Zwischenprodukte erhalten werden.

### Zusätzliche Verfahrensmassnahmen

Bei den zusätzlichen Verfahrensmassnahmen, die gewünschtenfalls durchgeführt werden, können funktionelle Gruppen der Ausgangsverbindungen, die nicht an der Reaktion teilnehmen sollen, ungeschützt oder in geschützter Form, beispielsweise durch eine oder mehrere der oben unter Verfahren a) genannten Schutzgruppen, vorliegen. Die Schutzgruppen können in den Endprodukten erhalten bleiben oder ganz oder teilweise nach einer der unter Verfahren f) genannten Methoden abgespalten werden.

Salze von Verbindungen der Formel I mit mindestens einer salzbildenden Gruppe können in an sich bekannter Weise hergestellt werden. So kann man Salze von Verbindungen der Formel I mit sauren Gruppen z. B. durch Behandeln mit Metallverbindungen, wie Alkalimetallsalzen von geeigneten organischen Carbonsäuren, z. B. dem Natriumsalz der 2-Ethyl-hexansäure, mit organischen Alkali- oder Erdalkalimetallverbindungen, wie den entsprechenden Hydroxiden, Carbonaten oder Hydrogencarbonaten, wie Natrium-und Kaliumhydroxid, -carbonat oder -hydrogencarbonat, mit entsprechenden Calciumverbindungen oder mit Ammoniak oder einem geeigneten organischen Amin bilden, wobei man vorzugsweise stöchiometrische Mengen oder nur einen kleinen Überschuss des salzbildenden Mittels verwendet. Säureadditionssalze von Verbindungen der Formel I erhält man in üblicher Weise, z. B. durch Behandeln mit einer Säure oder einem geeigneten Anionenaustauscherreagenz. Innere Salze von Verbindungen der Formel I, welche saure und basische salzbildende Gruppen enthalten, z. B. eine freie Carboxygruppe und eine freie Aminogruppe, können z.B. durch Neutralisieren von Salzen, wie Säureadditionssalzen, auf den isoelektrischen Punkt, z. B. mit schwachen Basen, oder durch Behandeln mit Ionenaustauschern gebildet werden.

Salze können in üblicher Weise in die freien Verbindungen überführt werden, Metall-und Ammoniumsalze beispielsweise durch Behandeln mit geeigneten Säuren, und Säureadditionssalze beispielsweise durch Behandeln mit einem geeigneten basischen Mittel.

Stereoisomerengemische, also Gemische von Diastereomeren und/oder Enantiomeren, wie beispielsweise racemische Gemische, können in an sich bekannter Weise durch geeignete Trennverfahren in die entsprechenden Isomeren aufgetrennt werden. So können Diastereomerengemische durch fraktionierte Kristallisation, Chromatographie, Lösungsmittelverteilung etc. in die einzelnen Diastereomeren aufgetrennt werden. Racemate können nach Überführung der optischen Antipoden in Diastereomere, beispielsweise durch Umsetzung mit optisch aktiven Verbindungen, z. B. optisch aktiven Säuren oder Basen, durch Chromatographie an mit optisch aktiven Verbindungen belegten Säulenmaterialien oder durch enzymatische Methoden, z. B. durch selektive Umsetzung nur eines der beiden Enantiomeren, voneinander getrennt werden. Diese Trennung kann sowohl auf der Stufe eines der Ausgangsprodukte als auch bei den Verbindungen der Formel I selbst erfolgen.

An einzelnen Chiralitätszentren in einer Verbindung der Formel I kann die Konfiguration gezielt umgekehrt werden. Beispielsweise kann man die Konfiguration asymmetrischer Kohlenstoffatome, welche nukleophile Substituenten, wie Amino oder Hydroxy, tragen, durch nukleophile Substitution zweiter Ordnung, gegebenenfalls nach Überführung des gebundenen nukleophilen Substituenten in eine geeignete nucleofuge Abgangsgruppe und Reaktion mit einem den ursprünglichen Substituenten einführenden Reagenz, umkehren.

In einer Verbindung der Formel I kann man einen in R₇ und/oder R₃ vorhandenen Arylrest, insbesondere einen Phenylrest, hydrieren, beispielsweise durch katalytische Hydrierung, insbesondere in Gegenwart von Schwermetalloxiden, wie Rhodium/Platin-Mischoxiden, z. B. mit dem Nishimura-Katalysator, vorzugsweise in einem polaren Lösungsmittel, wie einem Alkohol, z. B. Methanol oder Ethanol, bei Temperaturen zwischen 0 und 80 °C, insbesondere zwischen 10 und 40 °C, und bei einem Wasserstoffdruck von 1 bis 10 atm, vorzugsweise etwa bei Normaldruck.

In einer Verbindung der Formel I kann man einen in R₁ und/oder R₉ vorhandenen C₃-C₇-Alkenylrest oder C₃-C₇-Alkinylrest, insbesondere einen Allylrest, z.B. in Allyloxycarbonyl, hydrieren, beispielsweise durch katalytische Hydrierung in Gegenwart von Metall-, wie Platin- oder insbesondere Palladiumkatalysatoren, welche frei oder vorzugsweise an einen Träger, wie Kohle oder Kieselgel, vorliegen, insbesondere in Gegenwart von Palladium auf Aktivkohle, vorzugsweise in einem polaren Lösungsmittel, wie einem Niederalkanol, z.B. Methanol oder Ethanol, bei Temperaturen zwischen 0 und 80 °C, insbesondere zwischen 10 und 40 °C, und bei einem Wasserstoffdruck von maximal 10 atm, vorzugsweise etwa bei Normaldruck.

In einer erhältlichen Verbindung der Formel I kann man eine Amino- oder Carboxamidgruppe substituieren oder eine in freier oder in reaktionsfähiger Form vorliegende Carboxygruppe verestern oder amidieren.

Die Substitution einer Carboxamidgruppe oder einer anderen primären oder sekundären Aminogruppe, beispielsweise zur Einführung von Resten wie unsubstituiertem oder substituiertem Alkyl R₁, R₂, R₈ und/oder R₉ in Verbindungen der Formel I, in denen einer dieser Reste Wasserstoff bedeutet, zur Einführung von unsubstituiertem oder substituiertem Alkyl, wie für N-substituiertes Aminocarbonyloxy R₅ definiert, als Substituent am Stickstoff von Aminocarbonyloxy R₅, oder zur Einführung von unsubstituiertem oder substituiertem Alkyl als Substituent in unsubstituiertem oder substituiertem N-Alkyl- oder N,N-Dialkylcarbamoyl R₉ oder R₁ erfolgt z. B. durch Alkylierung mit geeigneten Ausgangsmaterialien.

Geeignete Mittel zur Alkylierung einer Carboxamidgruppe in einer Verbindung der Formel I sind z. B. Diazoverbindungen, wie Diazomethan. Man kann Diazomethan in einem inerten Lösungsmittel zersetzen, wobei das gebildete freie Methylen mit der Carboxamidgruppe in der Verbindung der Formel I reagiert. Die Zersetzung von Diazomethan erfolgt vorzugsweise katalytisch, z. B. in Gegenwart eines Edelmetalls in fein verteilter Form, wie Kupfer, oder eines Edelmetallsalzes, z. B. Kupfer(I)-chlorid oder Kupfer(II)-sulfat.

Weitere Alkylierungsmittel sind ausgewählt aus Verbindungen der Formeln

R₁''-X (Xa)

R₂'-X (Xb),

R₉''-X (XIa) und

R₈'-X (XIb),

worin der Rest X eine Abgangsgruppe ist und R₁'' sowie R₉'' die für R₁ oder R₉ genannten Bedeutungen ausser Wasserstoff, Acyl oder unsubstituiertes oder wie oben substituiertes Sulfo haben und R₂' sowie R₈' die für R₂ und R₈ genannten Bedeutungen ausser Wasserstoff haben. Eine Abgangsgruppe X ist insbesondere eine nukleofuge Abgangsgruppe ausgewählt aus mit einer starken anorganischen oder organischen Säure verestertem Hydroxy, wie mit einer Mineralsäure, z. B. Halogenwasserstoffsäure, wie Chlorwasserstoff-, Bromwasserstoff- oder Jodwasserstoffsäure, oder mit einer starken organischen Sulfonsäure, wie einer gegebenenfalls, z. B. durch Halogen, wie Fluor, substituierten Niederalkansulfonsäure oder einer aromatischen Sulfonsäure, z. B. einer gegebenenfalls durch Niederalkyl, wie Methyl, Halogen, wie Brom, und/oder Nitro substituierten Benzolsulfonsäure, z. B. einer Methansulfon-, Trimethansulfon- oder p-Toluolsulfonsäure verestertes Hydroxy oder mit Stickstoffwasserstoffsäure verestertes Hydroxy.

Die Reaktion kann unter den Bedingungen einer nukleophilen Substitution erster oder zweiter Ordnung ablaufen.

Beispielsweise kann man eine der Verbindungen der Formeln Xa, Xb, XIa oder XIb, insbesondere, wenn der Rest X für eine Abgangsgruppe mit hoher Polarisierbarkeit der Elektronenhülle, z. B. für Jod, steht, in einem polaren aprotischen Lösungsmittel, z. B. Aceton, Acetonitril, Nitromethan, Dimethylsulfoxid oder Dimethylformamid einsetzen. Die Reaktion kann auch in Wasser, dem gegebenenfalls als Lösungsvermittler ein organisches Lösungsmittel, z. B. Ethanol, Tetrahydrofuran oder Aceton, beigemischt ist, durchgeführt werden. Die Substitutionsreaktion wird gegebenenfalls bei erniedrigter oder erhöhter Temperatur, z. B. in einem Temperaturbereich von etwa -40 ° bis etwa 100 °C, bevorzugt von etwa -10 ° bis etwa 50 °C, und gegebenenfalls unter Inertgas, z. B. Stickstoff- oder Argonatmosphäre, durchgeführt.

Zur Veresterung oder Amidierung einer Carboxygruppe in einer Verbindung der Formel I kann man, wenn erwünscht, die freie Säure verwenden oder die freie Säure in eines der oben genannten reaktionsfähigen Derivate überführen und mit einem Alkohol, Ammoniak, einem primären oder einem sekundären Amin umsetzen, oder man kann zur Veresterung die freie Säure oder ein reaktionsfähiges Salz, z. B. das Cäsiumsalz, mit einem reaktionsfähigen Derivat eines Alkohols umsetzen. Beispielsweise kann man das Cäsiumsalz einer Carbonsäure mit einem dem Alkohol entsprechenden Halogenid oder Sulfonsäureester umsetzen. Die Veresterung der Carboxygruppe kann auch mit anderen üblichen Alkylierungsmitteln erfolgen, z. B. mit Diazomethan, Alkylhalogeniden, Sulfonsäureestern, Meerweinsalzen oder 1-substituierten 3-Aryltriazenen.

In einer Verbindung der Formel I kann man eine vorhandene freie Aminogruppe acylieren, beispielsweise zur Einführung eines der für R₁ oder R₉ genannten Reste Acyl, Sulfo oder substituiertes Sulfonyl. Die Acylierung erfolgt nach einer der oben unter Verfahren b), c) oder d) für Kondensationen oder einer der für Schutzgruppen genannten Methoden oder beispielsweise nach einem der im Organikum, 17. Auflage, VEB Deutscher Verlag der Wissenschaften, Berlin (Ost) 1988, genannten Verfahren.

In einer erhältlichen Verbindung der Formel I, worin die Substituenten die genannten Bedeutungen haben, mindestens eine freie Hydroxygruppe vorhanden ist und die übrigen funktionellen Gruppen in geschützter Form vorliegen, kann man die freie Hydroxygruppe acylieren oder verethern.

Die Acylierung kann erfolgen mit geeigneten acylierenden Reagentien nach einer der unter Verfahren a) bis d) oder nach einer der für Schutzgruppen genannten Methoden.

Die Veretherung kann mit den oben genannten Alkylierungsmitteln und unter den gleichen Reaktionsbedingungen erfolgen, z. B. mit Diazomethan, Alkylhalogeniden, Sulfonsäureestern, Meerweinsalzen, 1-substituierten 3-Aryltriazenen, etc.

In einer erhältlichen Verbindung der Formel I kann man aus einer Thiogruppe durch Oxidation eine Sulfinyl- oder Sulfonylgruppe herstellen, aus einem Sulfid das entsprechende Sulfoxid oder Sulfon.

Die Oxidation zur Sulfonylgruppe oder zum Sulfon kann mit den meisten der üblichen Oxidationsmittel durchgeführt werden. Besonders bevorzugt verwendet man solche Oxidationsmittel, welche die Thiogruppe oder den Sulfidschwefel selektiv in Gegenwart anderer funktioneller Gruppen der jeweiligen Verbindung der Formel I, z. B. von Amino-oder Hydroxygruppen, oxidieren, beispielsweise aromatische oder aliphatische Peroxycarbonsäuren, z. B. Peroxybenzoesäure, Monoperphthalsäure, m-Chlorperbenzoesäure, Peressigsäure, Perameisensäure oder Trifluorperessigsäure. Die Oxidation mit Peroxycarbonsäuren erfolgt in den üblichen dafür geeigneten Lösungsmitteln, beispielsweise Chlorkohlenwasserstoffen, z. B. Methylenchlorid oder Chloroform, Ethern, wie Diethylether, Estern, wie Essigester oder dergleichen, bei Temperaturen zwischen -78 °C und Raumtemperatur, z. B. zwischen -20 °C und +10 °C, bevorzugt um 0 °C. Die Peroxycarbonsäure kann auch in situ gebildet werden, z. B. mit Wasserstoffperoxid in Essigsäure oder Ameisensäure, die gegebenenfalls Essigsäureanhydrid enthält, z. B. mit 30 % oder 90 % Wasserstoffperoxid in Essigsäure/Essigsäureanhydrid. Geeignet sind auch andere Peroxoverbindungen, beispielsweise Kaliumperoxomonosulfat in Niederalkanol/Wasser-Mischungen, z. B. Methanol/Wasser oder Ethanol/Wasser, oder in wässriger Essigsäure bei Temperaturen zwischen -70 °C und +30 °C, z. B. zwischen -20 °C und Raumtemperatur, ferner Natriummetaperjodat in Methanol oder Methanol/Wasser-Gemischen bei Temperaturen zwischen 0 °C und 50 °C, z. B. um Raumtemperatur. Bei Einsatz stöchiometrischer Mengen der genannten Oxidationsmittel können auch die entsprechenden Sulfinsäuren bzw. Sulfoxide erhalten werden. Geeignet sind hierfür beispielsweise Natriummetaperiodat in Methanol oder Methanol-Wasser-Gemischen bei Temperaturen zwischen -15 °C und Raumtemperatur, z. B. um 0°C, m-Chlorperbenzoesäure in Methylenchlorid, Chloroform oder Essigester bei Temperaturen zwischen -78°C und 10°C, bevorzugt zwischen - 30°C und 0°C, ferner tert-Butylhypochlorit in Niederalkanolen, z. B. Methanol, oder Wasserstoffperoxid in Aceton oder Essigsäure bei Temperaturen um 0°C, oder das oben genannte Kaliumperoxomonosulfat bei tiefen Temperaturen.

Gewünschtenfalls kann durch Reduktion einer Sulfonylgruppe oder eines Sulfonrestes in einer erhältlichen Verbindung der Formel I die entsprechende Thioverbindung oder das entsprechende Sulfid erhalten werden, beispielsweise mit Diisobutylaluminiumhydrid in Ether oder Tetrahydrofuran.

In einer erhältlichen Verbindung der Formel I mit einer Sulfinylgruppe kann man diese Gruppe zu einer Thiogruppe reduzieren. Bevorzugt sind selektive Reduktionsmittel, die andere funktionelle Gruppen der Verbindung der Formel I, z. B. die Amidfunktion, unverändert lassen. Beispiele für solche selektiven Reduktionsmittel sind Dichlorboran, das vorzugsweise in Tetrahydrofuran oder Dimethoxyethan bei Temperaturen zwischen - 30°C und +10°C eingesetzt wird, Triphenylphosphin in siedendem Tetrachlorkohlenstoff, Trichlorsilan oder Hexachlordisilan, Eisenpentacarbonyl, ferner Natriumhydrogensulfit in wässrig-alkoholischen Lösungsmitteln, z. B. Wasser-Methanol, Wasser-Ethanol oder auch Wasser-Tetrahydrofuran, bei Temperaturen zwischen -10°C und +50°C, ferner Natriumborhydrid in Gegenwart von Kobalt(II)chlorid oder auch Wasserstoff in Gegenwart von katalytischen Mengen Palladium, z. B. Palladium/Kohle in siedendem Ethanol.

In einer Verbindung der Formel I kann man vorhandene Schutzgruppen oder geeignete Reste R₁ oder R₉, d. h. insbesondere solche, die Acyl bedeuten, nach einem der unter Verfahren f) genannten Verfahren abspalten, insbesondere durch Hydrolyse, beispielsweise in Gegenwart von Basen, wie Alkali- oder Erdalkalihydroxiden, z. B. Natriumhydroxid, oder Säuren, wie organischen Säuren oder Mineralsäuren, z. B. Halogenwasserstoff, wie Chlorwasserstoff. Die Hydrolyse erfolgt unter den üblichen Bedingungen, beispielsweise in wässriger Lösung oder in wasserfreien Lösungsmitteln, insbesondere in Ethern, wie Dioxan, bei Temperaturen zwischen -50 °C und der Rückflusstemperatur der entsprechenden Reaktionsgemische, z. B. zwischen 0 °C und 50 °C, vorzugsweise in Gegenwart eines Schutzgases, wie Argon oder Stickstoff.

### Ausgangsmaterialien:

Neue Ausgangsmaterialien und/oder Zwischenprodukte sowie Verfahren zu ihrer Herstellung sind ebenfalls Gegenstand der vorliegenden Erfindung. Vorzugsweise werden solche Ausgangsstoffe verwendet und die Reaktionsbedingungen so gewählt, dass man zu den als bevorzugt aufgeführten Verbindungen gelangt.

Es sei angemerkt, dass bei Ausgangsverbindungen der Formeln IV, VI und VIII Wanderungen des Acylrestes von Acyloxy R₅ an einen Stickstoff erfolgen können - daher sind die oben genannten Verfahren b), c) und d) unter diesem Vorbehalt zu sehen. Verfahren a), e) und f) und deren Ausgangsverbindungen sind bevorzugt, insbesondere Verfahren a) und f).

Bei der Herstellung aller Ausgangsmaterialien können freie funktionelle Gruppen, die nicht an der jeweiligen Reaktion teilnehmen sollen, ungeschützt oder in geschützter Form vorliegen, beispielsweise geschützt durch die oben unter Verfahren a) genannten Schutzgruppen. Diese Schutzgruppen können zu geeigneten Zeitpunkten durch die unter Verfahren f) beschriebenen Reaktionen freigesetzt werden. Die Einführung der Schutzgruppen erfolgt beispielsweise, wie bei der Beschreibung von Verfahren a) dargestellt.

Die als Ausgangsmaterialien verwendeten Hydroxyverbindungen der Formel II und weitere Ausgangsverbindungen werden analog den in der Europäischen Patentanmeldung mit der Publikationsnummer EP 0 521 827 (publiziert am 07. Januar 1993) beschriebenen Verfahren erhalten, vorzugsweise, indem man die entsprechend substituierten Verbindungen einsetzt, beispielsweise, indem man ein Hydrazinderivat der Formel 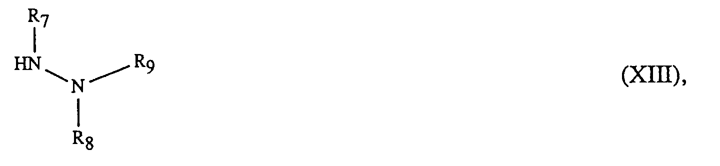
worin die Reste die oben genannten Bedeutungen haben, an ein Epoxid der Formel 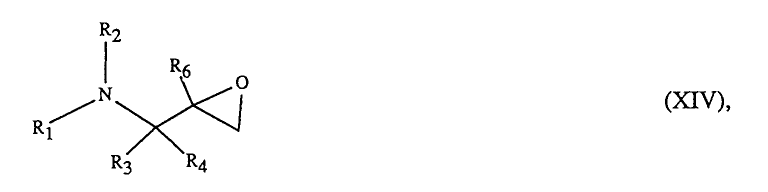
vorzugsweise der Formel 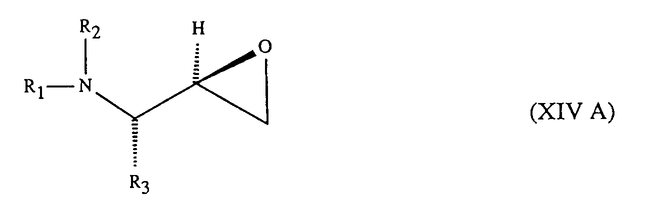
worin jeweils die Reste die oben genannten Bedeutungen haben, addiert, wobei freie funktionelle Gruppen mit Ausnahme der an der Reaktion beteiligten erforderlichenfalls in geschützter Form vorliegen, und vorhandene Schutzgruppen abspaltet. Im Falle der Verwendung der Verbindung der Formel XIV A erhält man die bevorzugten Ausgangsmaterialien der Formel II', wie unter Verfahren a) genannt. Die an der Reaktion teilnehmende Aminogruppe der Hydrazinderivate der Formel XIII hat je nach Bedeutung von R₇ vorzugsweise mindestens ein freies Wasserstoffatom; sie kann aber auch selbst derivatisiert sein, um die Reaktionsfähigkeit des Hydrazinderivates zu steigern.

Die Addition der Verbindungen der Formel XIII and die Epoxide (Oxirane) der Formel XIV erfolgt vorzugsweise unter den zur Addition von Nukleophilen an Epoxide übliche Bedingungen.

Die Addition erfolgt insbesondere in wässriger Lösung und/oder in Gegenwart von polaren Lösungsmitteln, wie Alkoholen, z.B. Methanol, Ethanol oder Ethylenglykol, Ethern, wie Dioxan, Amiden, wie Dimethylformamid, oder Phenolen, wie Phenol, auch unter wasserfreien Bedingungen, in apolaren Lösungsmitteln, wie Benzol oder Toluol, oder in Benzol/Wasser-Emulsionen, gegebenenfalls in Gegenwart von sauren oder basischen Katalysatoren, z.B. von Laugen, wie Natronlauge, oder in Gegenwart von mit dem Hydrazin dotierten Festphasenkatalysatoren, wie Aluminiumoxid, in Ethern, z.B. Diethylether, allgemein bei Temperaturen von etwa 0 °C bis zur Rückflusstemperatur, vorzugsweise zwischen 20 °C und 130 °C, gegebenenfalls unter Rückfluss, unter erhöhtem Druck, z.B. im Bombenrohr, wobei die Siedetemperatur des Reaktionsgemisches auch überschritten werden kann, und/oder unter Inertgas, wie Stickstoff oder Argon, wobei jede einzelne der beiden Verbindungen der Formel XIII und XIV im Überschuss vorliegen kann, beispielsweise im Molverhältnis 1:1 bis 1:100, vorzugsweise im Molverhältnis 1:1 bis 1:10, insbesondere im Verhältnis 1:1 bis 1:3.

Die Ausgangsmaterialien der Formeln XIII und XIV sind bekannt oder können, falls sie neu sind, nach an sich bekannten Verfahren hergestellt werden, z. B. aus Hydrazin oder dessen geeigneten Derivaten die Verbindungen der Formel XIII, aus geeigneten Aminosäuren oder deren Analoga, beispielsweise mit einer der genannten Seitenketten R₃, die Verbindungen der Formel XIV.

Die Verbindungen der Formel XIII sind beispielsweise zugänglich aus Hydrazin oder dessen geeigneten Derivaten der Formel

H₂N-NH-R₁₁ (XV),

worin R₁₁ Wasserstoff oder eine Aminoschutzgruppe, wie oben unter Verfahren a) beschrieben, insbesondere tert-Niederalkoxycarbonyl, wie tert-Butoxycarbonyl, Arylniederalkoxycarbonyl, wie Benzyloxycarbonyl oder 9-Fluorenylmethoxycarbonyl, oder eine der oben genannten Acyl-Aminoschutzgruppen bedeutet, indem man diese (zur über die nachfolgenden Stufen verlaufenden Herstellung einer Verbindung der Formel I, worin anstelle von R₇ der Rest R₇'' steht, welcher für unsubstituiertes oder substituiertes Alkyl oder Cycloalkyl steht), unter Einführung von R₇'', wie unter Verfahren e) definiert, alkyliert mit einer Verbindung der Formel XII unter Bedingungen, wie unter Verfahren e) beschrieben, oder einen Rest R₇ durch Reaktion geeigneter Carbonylverbindungen mit der freien Aminogruppe der Verbindung der Formel XV oder ihrer acylierten Derivate und anschliessende Reduktion des erhaltenen Hydrazones einführt unter Bildung von Hydrazinderivaten der Formel

R₇-NH-NH-R₁₁ (XVI),

wobei die Reste in allen genannten Verbindungen die oben angegebenen Bedeutungen haben und funktionelle Gruppen in den beteiligten Reagenzien, die nicht an der Reaktion teilnehmen sollen, gegebenenfalls geschützt sind, und die Schutzgruppe R₁₁, sofern sie nicht einem der Reste R₉ in den Verbindungen der Formel I entspricht, abspaltet und anschliessend einen Rest R₉ ausser Wasserstoff gewünschtenfalls durch Kondensation unter den oben für Verfahren b) genannten Bedingungen mit Säuren der Formel V oder durch Alkylierung mit einer Verbindung der Formel XIa, wie oben definiert, und/oder einen Rest R₈ ausser Wasserstoff durch Alkylierung mit einer Verbindung der Formel XIb, wie oben definiert, unter analogen Bedingungen, wie oben für Alkylierungen bei den zusätzlichen Verfahrensmassnahmen genannt, einführt.

Die zur Herstellung der Verbindungen der Formel XVI verwendeten, zur Einführung von R₇ geeigneten Carbonylverbindungen sind bekannte, nach an sich bekannten Verfahren herstellbare oder kommerziell erhältliche Aldehyde oder Ketone, deren reaktive Carbonylgruppe nach der Reaktion mit Verbindungen der Formel XV und anschliessender Reduktion Bestandteil eines der genannten Reste R₇ sind, vorzugsweise Niederalkanaldehyde, Cyclohexylniederalkalkanaldehyde oder Phenylniederalkanaldehyde.

Die Reaktion dieser Carbonylverbindungen mit den Verbindungen der Formel XVI zu den entsprechenden Hydrazonen erfolgt unter den zur Reaktion von Carbonylverbindungen mit Aminen üblichen Bedingungen, vorzugsweise in polaren organischen Lösungsmitteln, z. B. Ethern, wie Tetrahydrofuran oder Diethylether, Alkoholen, wie Methanol oder Ethanol, Carbonsäureamiden, wie Dimethylformamid, oder Estern, wie Essigsäureethylester, oder in wässriger Lösung, bevorzugt in Methanol, ferner in Anwesenheit oder Abwesenheit von sauren Katalysatoren, z. B. Carbonsäuren, wie Ameisensäure oder Essigsäure, oder Sulfonsäuren, wie p-Toluolsulfonsäure, bei Temperaturen zwischen 0 °C und der Rückflusstemperatur des Reaktionsgemisches, vorzugsweise bei Temperaturen von 20 °C bis zur Rückflusstemperatur des Reaktionsgemisches.

Die Reduktion der erhaltenen Hydrazone erfolgt vorzugsweise durch Hydrierung in Gegenwart eines geeigneten Katalysators. Als zur Hydrierung geeignete Katalysatoren werden Metalle, wie Nickel, Eisen, Cobalt oder Ruthenium, oder Edelmetalle bzw. deren Oxide, wie Palladium oder Rhodium bzw. deren Oxide, verwendet, gegebenenfalls z. B. auf geeignetem Trägermaterial, wie Bariumsulfat, Aluminiumoxid oder Aktivkohle aufgezogen oder als Skelettkatalysatoren, wie Raney-Nickel. Gebräuchliche Lösungsmittel für die katalytische Hydrierung sind beispielsweise Wasser, Alkohole, wie Methanol oder Ethanol, Ester, wie Essigsäureethylester, Ether, wie Dioxan, Chlorkohlenwasserstoffe, wie Dichlormethan, Carbonsäureamide, wie Dimethylformamid, oder Carbonsäuren, wie Eisessig, oder Gemische dieser Lösungsmittel. Die Hydrierung erfolgt bei Temperaturen von 10 bis 250 °C, bevorzugt von Raumtemperatur bis 100 °C, und bei Wasserstoffdrucken von 1 bis 200 bar, vorzugsweise von 1 bis 10 bar, in den üblichen Apparaturen.

Besonders bevorzugt für die Herstellung der Verbindungen der Formel XV sind Reaktionsbedingungen, welche den in J. Chem. Soc. Perkin I, 1712 (1975) beschriebenen analog sind.

Die Verbindungen der Formel XIV sind beispielsweise zugänglich durch Reduktion von an sich bekannten, kommerziell erhältlichen oder nach an sich bekannten Verfahren herstellbaren Aminosäuren oder deren Analoga der Formel 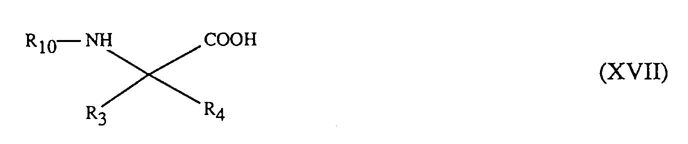
worin R₁₀ Wasserstoff oder eine der unter Verfahren a) genannten Aminoschutzgruppen, insbesondere tert-Niederalkoxycarbonyl, wie tert-Butoxycarbonyl, Arylniederalkoxycarbonyl, wie Benzyloxycarbonyl oder 9-Fluorenylmethoxycarbonyl, oder eine der dort genannten Acyl-Aminoschutzgruppen, bedeutet und R₃ und R₄ die für Verbindungen der Formel I genannten Bedeutungen haben, vorzugsweise von Aminosäuren der Formel 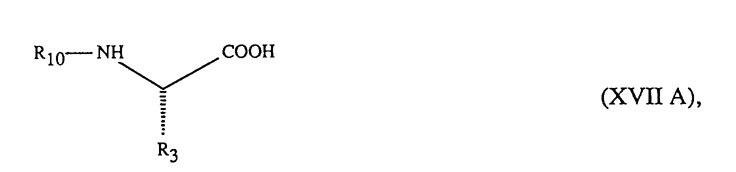
in denen die Reste die genannten Bedeutungen haben, zu Aldehyden der Formel 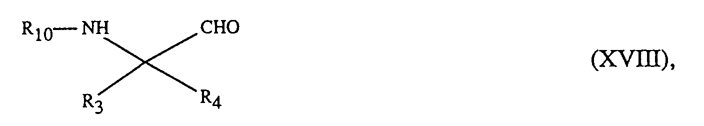
worin die Reste die genannten Bedeutungen haben, vorzugsweise zu den Aldehyden der Formel 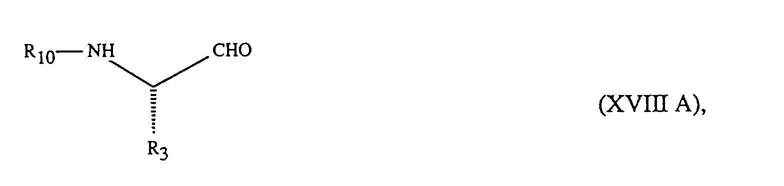
worin die Reste die genannten Bedeutungen haben (erhältlich beispielsweise aus Verbindungen der Formel XVII A), durch Umsetzung dieser Aldehyde mit einer Ylid-Verbindung, vorzugsweise einer Schwefel-Ylid-Verbindung, zu einem Epoxid der Formel 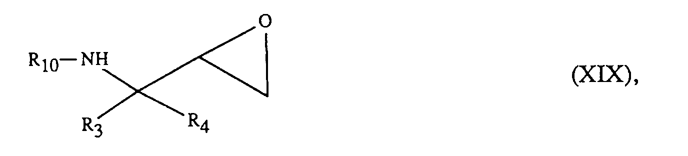
worin die Reste die genannten Bedeutungen haben, vorzugsweise zu Verbindungen der Formel 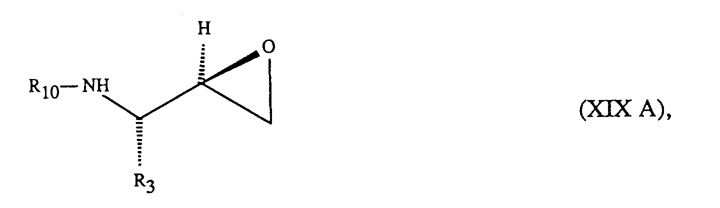
(erhältlich beispielsweise aus Verbindungen der Formel XVIII A), worin die Reste die genannten Bedeutungen haben, Abspaltung der Schutzgruppe R₁₀, sofern sie nicht einem Rest R₁ in den Verbindungen der Formel I entspricht, und (zur Einführung von R₁ ausser Wasserstoff) durch Acylierung der Aminogruppe der resultierenden Verbindung mit einer Säure der Formel VII, worin R₁' die genannten Bedeutungen hat, unter den für Verfahren b) beschriebenen Bedingungen, oder Alkylierung der Aminogruppe der resultierenden Verbindung mit einem Reagens mit einer nukleofugen Abgangsgruppe X der Formel Xa, worin R₁'' die genannten Bedeutungen hat, und/oder (zur Einführung von R₂ ausser Wasserstoff) durch Alkylierung mit einer Verbindung der Formel Xb, worin R₂' die genannten Bedeutungen hat, unter den für zusätzliche Verfahrensmassnahmen beschriebenen Bedingungen, wobei man die Verbindungen der Formel XIV A vorzugsweise aus einem Ausgangsmaterial der Formel XIX A erhält.

Die Reduktion von Aminosäuren der Formel XVII oder XVII A zu den entsprechenden Aldehyden XVIII und XVIII A erfolgt beispielsweise durch Reduktion zu den entsprechenden Alkoholen und anschliessende Oxidation zu den genannten Aldehyden.

Die Reduktion zu den Alkoholen erfolgt beispielsweise durch Hydrogenierung der Aminosäurehalogenide oder anderer aktivierter Carbonsäurederivate (beispielsweise mit aktiviertem Hydroxy analog Verbindungen der Formel IX, wie unter Verfahren a) definiert) unter den für die Hydrogenierung von aus Verbindungen der Formel XVI erhaltenen Hydrazonen genannten Bedingungen oder mit komplexen Hydriden, wie Natriumborhydrid. Die anschliessende Oxidation der erhaltenen Alkohole erfolgt vorzugsweise mit Oxidationsmitteln, welche es ermöglichen, selektiv (d.h. ohne Weiteroxidation der Aldehyde zu den Carbonsäuren) die Aldehyde der Formel XVIII oder XVIII A zu erhalten, beispielsweise mit Kaliumferrat (K₂FeO₄) in wässrigen sowie Braunstein in organischen Lösungsmitteln, oder organischen Chromsäurederivaten, wie Pyridiniumdichromat oder tert-Butylchromat in inerten organischen Lösungsmitteln, z.B. chlorierten Kohlenwasserstoffen, wie Methylenchlorid oder Chloroform, in Gegenwart oder Abwesenheit von basischen Aminen, z.B. Triniederalkylaminen, wie Triethylamin, bei Temperaturen von -50 bis 100 °C, vorzugsweise bei -10 bis 50 °C, beispielsweise, wie in der Europäischen Patentanmeldung EP-A-0 236 734 beschrieben, oder in erster Linie durch Oxidation der Hydroxygruppe mit einem Sulfoxid, wie Dimethylsulfoxid, in Gegenwart eines die Hydroxygruppe aktivierenden Reagenzes, z.B. eines Carbonsäurechlorides, wie Oxalylchlorid, in einem inerten Lösungsmittel, z. B. einem chlorierten Kohlenwasserstoff, wie Dichlormethan, und/oder einem acyclischen oder cyclischen Ether, wie Tetrahydrofuran, bei - 80 bis 0 °C, z. B. - 78 bis - 50 °C.

Auch die direkte Reduktion der Aminosäuren zu den Aldehyden ist möglich, beispielsweise durch Hydrierung in Gegenwart eines partiell vergifteten Palladiumkatalysators oder durch Reduktion der entsprechenden Aminosäureester, z. B. der Niederalkylester, wie Ethylester, mit komplexen Hydriden, z. B. Borhydriden, wie Natriumborhydrid, oder vorzugsweise Aluminiumhydriden, z. B. Lithiumaluminiumhydrid, Lithium-tri-(tertbutoxy)aluminiumhydrid oder insbesondere Diisobutylaluminiumhydrid, in apolaren Lösungsmitteln, z. B. in Kohlenwasserstoffen oder aromatischen Lösungsmitteln, wie Toluol, bei -100 bis 0 °C, bevorzugt -70 bis -30 °C, und anschliessende Umsetzung zu den entsprechenden Semicarbazonen, z. B. mit den entsprechenden Säuresalzen von Semicarbazonen, wie Semicarbazidhydrochlorid, in wässrigen Lösungsmittelsystemen, wie Alkohol/Wasser, z. B. Ethanol/Wasser, bei Temperaturen zwischen -20 und 60 °C, vorzugsweise 10 bis 30 °C, und Umsetzung des erhaltenen Semicarbazones mit einem reaktiven Aldehyd, z. B. Formaldehyd, in einem inerten Lösungsmittel, beispielsweise einem polaren organischen Lösungsmittel, z. B. einem Carbonsäureamid, wie Dimethylformamid, bei Temperaturen zwischen -30 und 60 °C, bevorzugt 0 bis 30 °C, und dann einer Säure, beispielsweise einer starken Mineralsäure, wie Halogenwasserstoff, in wässriger Lösung, gegebenenfalls in Gegenwart des vorher verwendeten Lösungsmittels, bei Temperaturen zwischen -40 und 50 °C, bevorzugt zwischen -10 und 30 °C. Die entsprechenden Ester werden durch Umsetzung der Aminosäuren mit den entsprechenden Alkoholen, beispielsweise Ethanol, analog den bei der Acylierung unter Verfahren a) verwendeten Bedingungen gewonnen, beispielsweise durch Umsetzung mit anorganischen Säurehalogeniden, wie Thionylchlorid, in organischen Lösungsmittelgemischen, wie Mischungen aus aromatischen und alkoholischen Lösungsmitteln, z. B. Toluol und Ethanol, bei Temperaturen zwischen -50 und 50 °C, bevorzugt zwischen -10 und 20 °C (erforderlichenfalls unter Verwendung von Schutzgruppen).

Die Herstellung der Verbindungen der Formel XVIII oder XVIII A erfolgt in besonders bevorzugter Weise unter Bedingungen analog den in J. Org. Chem. **47**, 3016 (1982) oder J. Org. Chem. **43**, 3624 (1978) genannten Reaktionsbedingungen.

Ein zur Umsetzung von Verbindungen der Formel XVIII oder XVIII A zu den Epoxiden der Formel XIX oder XIX A geeignetes Schwefel-Ylid ist beispielsweise ein Dialkylsulfoniummethylid, z. B. Dimethylsulfoniummethylid, ein Alkyl- oder Phenyl-dialkylaminosulfoxoniummethylid, z. B. Methyl- oder Phenyl-dimethylaminosulfoxoniummethylid, oder ein Dialkylsulfoxoniummethylid, z. B. Dimethyl- oder Diethylsulfoxoniummethylid.

Die betreffende Schwefel-Ylid-Verbindung wird zweckmässigerweise in situ aus dem entsprechenden Sulfonium- bzw- Sulfoxoniumsalz und einer Base, z. B. Natriumhydrid, in einem dipolar aprotischen Lösungsmittel, z. B. Dimethylsulfoxid, oder einem Ether, z. B. Tetrahydrofuran oder 1,2-Dimethoxyethan, hergestellt und anschliessend mit den Verbindungen der Formel XVIII oder XVIII A umgesetzt. Die Umsetzung erfolgt normalerweise bei Raumtemperatur, unter Kühlen z. B. bis auf -20 °C, oder unter leichtem Erwärmen z. B. bis auf 40 °C. Das gleichzeitig gebildete Sulfid, Sulfinamid bzw. Sulfoxid wird bei der anschliessenden wässrigen Aufarbeitung entfernt.

Die Umsetzung mit einem Schwefel-Ylid erfolgt in besonders bevorzugter Weise analog den in J. Org. Chem. 50, 4615 (1985) genannten Bedingungen.

Die Verbindung der Formel XIX (vorzugsweise XIXA) kann auch aus einer Verbindung der Formel XVIII (vorzugsweise XVIIIA), wie oben definiert, durch deren Umsetzung mit einer Triniederalkyl-silylmethyl-Grignard-Verbindung, z. B. hergestellt aus dem entsprechenden Halogen-methyl-Silan, wie Chlormethyl-trimethylsilan, in einem inerten Lösungsmittel, z. B. einem Ether, wie Dioxan oder Diethylether, bei Temperaturen zwischen 0 und 50 °C, z. B. zwischen Raumtemperatur und etwa 40 °C, anschliessende Elimination unter Entfernung des Silylrestes und Bildung einer Doppelbindung, z. B. mittels einer Lewis-Säure, wie BF₃, wobei vorzugsweise auch eine vorliegende Aminoschutzgruppe R₁₀ abgespalten wird, in einem inerten LM, z. B. einem Ether, wie Diethylether, oder einem Halogenkohlenwasserstoff, wie Dichlormethan, oder einem Gemisch davon, bei Temperaturen zwischen -50 °C und der Rückflusstemperatur, insbesondere zwischen 0 und 30 °C, wenn erforderlich, erneute Acylierung unter Einführung von einer Aminoschutzgruppe als R₁₀, wie oben definiert, und Oxidation der erhaltenen Doppelbindung zum Oxiran, vorzugsweise mit einer Percarbonsäure, z. B. m-Chlorperbenzoesäure, in einem inerten Lösungsmittel, z. B. halogenierten Kohlenwasserstoff, wie Dichlormethan, bei Temperaturen zwischen -20 °C und der Rückflusstemperatur des Gemisches, z. B. bei 10 bis 30 °C, erhalten werden.

Das bevorzugte Ausgangsmaterial der Formel II' in Verfahren a), oder ein Salz davon, wird beispielsweise hergestellt, indem man ein Hydrazinderivat der oben definierten Formel XVI an ein Epoxid der oben definierten Formel XIV A addiert, und gewünschtenfalls eine nach dem vorstehenden Verfahren erhältliche Verbindung der Formel II' mit mindestens einer salzbildenden Gruppe in ihr Salz oder ein erhältliches Salz in die freie Verbindung oder in ein anderes Salz überführt und/oder gegebenenfalls erhältliche Isomerengemische auftrennt und/oder vorhandene Schutzgruppen in einer Verbindung der Formel II abspaltet und/oder eine erfindungsgemässe Verbindung der Formel II in eine andere erfindungsgemässe Verbindung der Formel II umwandelt.

Die Herstellung und Umwandlung von Salzen, die Auftrennung von Isomerengemischen, die Abspaltung von Schutzgruppen und die Umwandlung von Verbindungen der Formel II' erfolgen analog den oben für Verbindungen der Formel I beschriebenen Verfahren.

Die Ausgangsmaterialien der Verfahren b), c) und d) können nach an sich bekannten Verfahren hergestellt werden, z. B. können Verbindungen der Formel IV aus Hydrazinderivaten der Formel XIII, worin R₉ Wasserstoff bedeutet und die übrigen Reste die für Verbindungen der Formel IV genannten Bedeutungen haben, und Epoxiden der Formel XIV, worin die Reste die für Verbindungen der Formel IV genannten Bedeutungen haben, und anschliessende Acylierung der erhaltenen Verbindung der Formel IV', 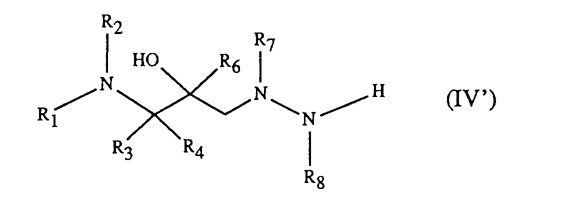
insbesondere der Formel IV'' 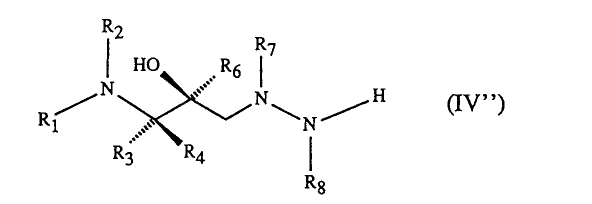
worin die Reste die für Verbindungen der Formel I genannten Bedeutungen haben und worin anstelle des in einer Verbindung der Formel IV vorhandenen Restes R₅ eine freie Hydroxygruppe vorliegt, mit einer Carbonsäure der Formel III oder einem aktivierten Carbonsäurederivat davon, wie unter Verfahren a) beschrieben (ergibt Ausgangsmaterial der Formel IV für Verfahren b); Verbindungen der Formel VI aus Hydrazinderivaten der Formel XIII, worin die Reste die für Verbindungen der Formel VI genannten Bedeutungen haben, und Epoxiden der Formel XIV, worin R₁ Wasserstoff bedeutet und die übrigen Reste die für Verbindungen der Formel VI genannten Bedeutungen haben, und anschliessende Acylierung der erhaltenen Verbindung der Formel VI' 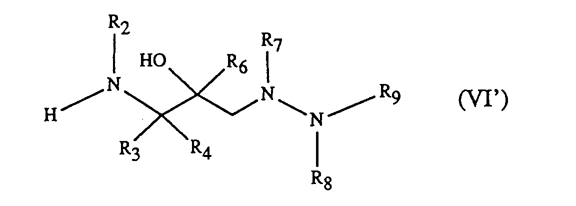
insbesondere der Formel VI'' 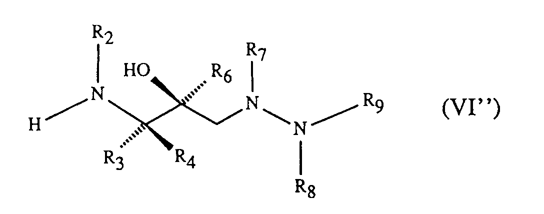
worin die Reste die für Verbindungen der Formel I genannten Bedeutungen haben und anstelle des in einer Verbindung der Formel VI vorhandenen Restes R₅ eine freie Hydroxygruppe vorliegt, mit einer Carbonsäure der Formel III oder einem aktivierten Carbonsäurederivat davon, wie unter Verfahren a) beschrieben (ergibt Ausgangsmaterial der Formel VI für Verfahren c); und Verbindungen der Formel VIII aus Hydrazinderivaten der Formel XIII, worin R₉ Wasserstoff bedeutet und die übrigen Reste die für Verbindungen der Formel VIII genannten Bedeutungen haben und Epoxiden der Formel XIV, worin R₁ Wasserstoff bedeutet und die übrigen Reste die für Verbindungen der Formel IX genannten Bedeutungen haben und anschliessende Acylierung der erhaltenen Verbindung der Formel VIII' 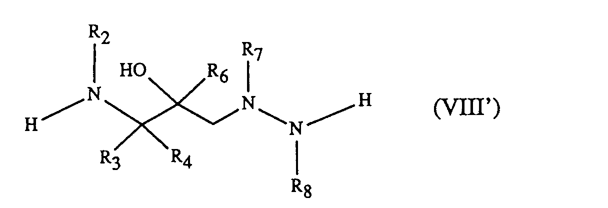
insbesondere der Formel VIII'', 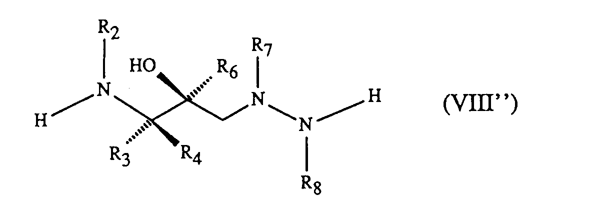
worin die Reste die für Verbindungen der Formel I genannten Bedeutungen haben und worin anstelle des in einer Verbindung der Formel IV vorhandenen Restes R₅ eine freie Hydroxygruppe vorliegt, mit einer Carbonsäure der Formel III oder einem aktivierten Carbonsäurederivat davon, wie unter Verfahren a) beschrieben (ergibt Ausgangsmaterial der Formel VIII für Verfahren d); hergestellt werden in Analogie zu Verfahren a), erforderlichenfalls unter Verwendung und Abspaltung von Schutzgruppen. Vorzugsweise werden bei den genannten Herstellungsverfahren zur Herstellung der bevorzugten Verbindungen der Formel IV (über IV''), VI (über VI'') und VIII (über VIII''), worin die Kohlenstoffatome, welche R₃ bzw. R₅ tragen, beide in der (S)-Konfiguration vorliegen, die Epoxide der Formel XIV A eingesetzt.

Verbindungen der Formel I', in der die Substituenten die oben genannten Bedeutungen haben, lassen sich beispielsweise herstellen aus einer Verbindung der Formel XIII', 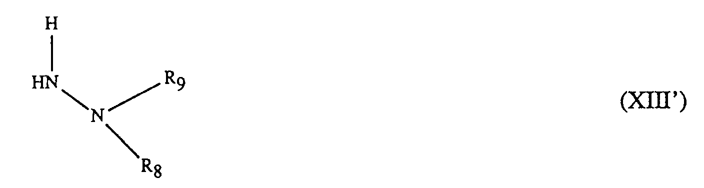
worin die Reste die für Verbindungen der Formel I genannten Bedeutungen haben, durch Umsetzung mit einer Verbindung der Formel XIV, insbesondere XIV A (was zu den bevorzugten Verbindungen der Formel I' führt, worin die Kohlenstoffatome, welche R₃ und R₅ tragen, beide in der (S)-Konfiguration vorliegen), wie für die Umsetzung von Verbindungen der Formel XIII mit solchen der Formel XIV oder XIV A beschrieben, und durch anschliessende Acylierung der erhaltenen Verbindung der Formel I'' 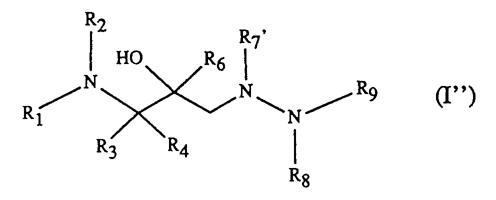
insbesondere der Formel I''', 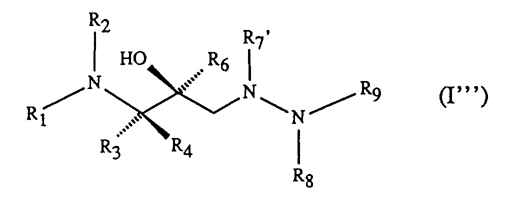
worin die Reste die für Verbindungen der Formel I' genannten Bedeutungen haben, mit einer Verbindung der Formel III oder einem aktivierten Carbonsäurederivat davon (Herstellung des Restes Acyloxy R₅) unter Reaktionsbedingungen analog den unter Verfahren a) beschriebenen (anstelle der Verbindung der Formel II ist dann eine analoge Verbindung einzusetzen, worin anstelle von R₇ der Rest R₇' steht), wobei vorhandene funktionelle Gruppen, die nicht an der Reaktion teilnehmen sollen, wie dort erforderlichenfalls geschützt vorliegen und nach der Reaktion freigesetzt werden können.

Von den Ausgangsverbindungen der Formel II oder II' ist die der Formel 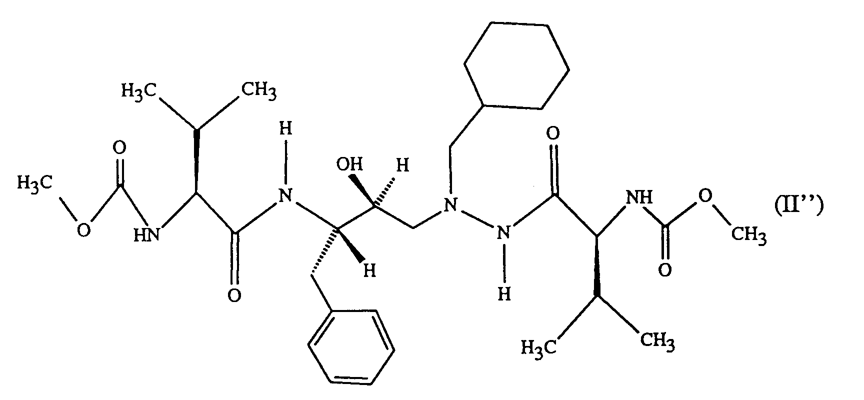
in allererster Linie bevorzugt. Diese Verbindung gehört einerseits zu Verfahren a) als bevorzugte Zwischenverbindung, ist jedoch andererseits selbst eine bevorzugte erfindungsgemässe Verbindung, indem sie eine gute pharmakologische Wirksamkeit aufweist, insbesondere aufgrund einer unerwartet guten Wirksamkeit im oben beschriebenen Zelltest, was auf eine hohe Wirksamkeit in vivo schliessen lässt.

Weitere erfindungsgemässe Verbindungen der Formel II, welche vorteilhafte pharmakologische Eigenschaften haben (insbesondere die eingangs beschriebenen) und daher bevorzugte Gegenstände der vorliegenden Anmeldung sind, sind nachfolgend genannt (auch die entsprechenden Verfahren zur Herstellung dieser Verbindungen, pharmazeutische Präparate mit diesen Verbindungen, diese Verbindungen zur Anwendung in einem therapeutischen Verfahren zur Behandlung des menschlichen oder tierischen Körpers und die Verwendung dieser Verbindungen zur therapeutischen Behandlung des menschlichen oder tierischen Körpers oder zur Herstellung pharmazeutischer Präparate, jeweils analog zu den entsprechenden Erfindungsgegenständen, welche auf Verbindungen der Formel I bezogen sind, gehören, wie auch bei Verbindungen Formel II'', zur vorliegenden Erfindung):
Ganz besonders bevorzugt ist eine Verbindung der Formel II ausgewählt aus den Verbindungen mit der Bezeichnung
1-[2(S)-Hydroxy-3(S)-(N-(chinolin-2-carbonyl)-(L)-asparaginyl)amino-4-phenyl-butyl]-1-[benzyl]-2-[N-benzyloxy-carbonyl)-(L)-valyl]hydrazin,
1-[2(S)-Hydroxy-3(S)-(N-(chinolin-2-carbonyl)-(L)-asparaginyl)amino-4-phenyl-butyl]-1-[4-methoxyphenylmethyl]-2-[N-benzyloxy-carbonyl)-(L)-valyl]hydrazin (besonders bevorzugt),
1-[2(S)-Hydroxy-3(S)-(N-methoxycarbonyl-(L)-valyl)amino-4-phenylbutyl]-
1-[thien-2-ylmethyl]-2-[N-methoxycarbonyl-(L)-valyl]hydrazin;
1-[2(S)-Hydroxy-3(S)-(N-methoxycarbonyl-(L)-valyl)amino-4-phenylbutyl]-
1-[4-methoxyphenylmethyl]-2-[N-methoxycarbonyl-(L)-valyl]hydrazin (besonders bevorzugt);
1-[2(S)-Hydroxy-3(S)-(N-acetyl-(L)-valyl)amino-4-phenylbutyl]-
1-[4-biphenylylmethyl]-2-[N-acetyl-(L)-valyl]hydrazin;
1-[2(S)-Hydroxy-3(S)-(N-methoxycarbonyl-(L)-valyl)amino-4-phenylbutyl]-
1-[4-biphenylylmethyl]-2-[N-methoxycarbonyl-(L)-valyl]hydrazin;
1-[2(S)-Hydroxy-3(S)-(N-ethoxycarbonyl-(L)-valyl)amino-4-phenylbutyl]-
1-[4-biphenylylmethyl]-2-[N-ethoxycarbonyl-(L)-valyl]hydrazin;
Die Herstellung von Verbindungen der Formel II', insbesondere die erfindungsgemässe Herstellung der Verbindung der Formel II'', erfolgt beispielsweise
i) analog der Umsetzung von Ausgangsmaterialien der Formel XIII mit solchen der Formel XIV A, wie bei der Herstellung von Verbindungen der Formel II beschrieben, wobei im Falle von Verbindungen der Formel II' die Reste in den Ausgangsverbindungen die für Verbindungen der Formel I genannten Bedeutungen haben, während im Falle von Verbindungen der Formel II'' in den Ausgangsmaterialien für R₁ und R₉ jeweils N-Methoxycarbonyl-(L)-valyl steht, R₂, R₄, R₆ und R₈ Wasserstoff bedeuten, R₃ Benzyl bedeutet und R₇ Cyclohexylmethyl bedeutet, oder
ii) analog Verfahren b), worin anstelle der Ausgangsmaterialien der Formel IV solche der Formel IV', insbesondere IV'', eingesetzt werden, wie oben definiert, wobei im Falle der Herstellung von Verbindungen der Formel II' die Reste die für Verbindungen der Formel IV' genannten Bedeutungen haben, während im Falle der Herstellung von Verbindungen der Formel II'' in den Ausgangsmaterialien für R₁ und R₉' jeweils N-Methoxycarbonyl-(L)-valyl steht, R₂, R₄, R₆ und R₈ Wasserstoff bedeuten, R₃ Benzyl bedeutet und R₇ Cyclohexylmethyl bedeutet, oder
iii) analog Verfahren c), worin anstelle der Ausgangsmaterialien der Formel VI solche der Formel VI', insbesondere VI'', eingesetzt werden, wie oben definiert, wobei im Falle der Herstellung von Verbindungen der Formel II' die Reste die für Verbindungen der Formel VI' genannten Bedeutungen haben, während im Falle der Herstellung von Verbindungen der Formel II'' in den Ausgangsmaterialien für R₁' und R₉ jeweils N-Methoxycarbonyl-(L)-valyl steht, R₂, R₄, R₆ und R₈ Wasserstoff bedeuten, R₃ Benzyl bedeutet und R₇ Cyclohexylmethyl bedeutet, oder
iv) analog Verfahren d), worin anstelle der Ausgangsmaterialien der Formel VIII solche der Formel VIII', insbesondere VIII'', eingesetzt werden, wie oben definiert, wobei im Falle der Herstellung von Verbindungen der Formel II' die Reste die für Verbindungen der Formel VIII' genannten Bedeutungen haben, während im Falle der Herstellung von Verbindungen der Formel II'' in den Ausgangsmaterialien für R₁' und R₉' jeweils N-Methoxycarbonyl-(L)-valyl steht, R₂, R₄, R₆ und R₈ Wasserstoff bedeuten, R₃ Benzyl bedeutet und R₇ Cyclohexylmethyl bedeutet, oder
v) analog Verfahren e), worin anstelle der Ausgangsmaterialien der Formel IV solche der Formel IV', insbesondere IV'', eingesetzt werden, wie oben definiert, wobei im Falle der Herstellung von Verbindungen der Formel II' die Reste die für Verbindungen der Formel I'' genannten Bedeutungen haben, während im Falle der Herstellung von Verbindungen der Formel II'' in den Ausgangsmaterialien für R₁ und R₉ jeweils N-Methoxycarbonyl-(L)-valyl steht, R₂, R₄, R₆ und R₈ Wasserstoff bedeuten, R₃ Benzyl bedeutet und R₇'' Cyclohexylmethyl bedeutet, oder
vi) im Falle der Verwendung von geschützten Ausgangsmaterialien, beispielsweise in einem der vorgenannten Verfahren, durch Abspaltung der Schutzgruppen analog Verfahren f).

Gewünschtenfalls kann eine nach einem der vorstehenden Verfahren i) bis vi) erhaltene Verbindungen der Formel II', insbesondere II'', in ihr Salz oder ein erhältliches Salz in die freie Verbindung oder in ein anderes Salz überführt und/oder gegebenenfalls erhältliche Isomerengemische aufgetrennt und/oder eine erfindungsgemässe Verbindung der Formel II', insbesondere II'', in eine andere erfindungsgemässe Verbindung der Formel II', insbesondere II'', überführt werden. Die Bedingungen entsprechen dabei den oben für an Verbindungen der Formel I vorgenommene zusätzliche Verfahrensmassnahmen beschriebenen.

Die erfindungsgemässe Herstellung der Verbindungen der Formel II'' erfolgt in besonders bevorzugter Weise aus Verbindungen der Formel VIII'', worin R₂, R₄, R₆ und R₈ Wasserstoff bedeuten, R₃ Benzyl bedeutet und R₇ Cyclohexylmethyl bedeutet, und aus N-Methoxycarbonyl-(L)-valin oder reaktionsfähigen Säurederivaten davon (entspricht der Verbindung der Formel V und VII, worin R₉' und R₁' N-Methoxycarbonyl-(L)-valyl bedeuten), in Analogie zu Verfahren d), wie oben beschrieben.

Die Säuren der Formeln III, V, VII und IX die Säurederivate davon, z.B. der Formeln V', V'' oder IX', sowie die Verbindungen mit nukleofugen Gruppen der Formel Xa, Xb, XIa, XIb und XII sind kommerziell erhältlich, bekannt, oder, wenn sie neu sind, nach an sich bekannten Verfahren herstellbar, beispielsweise analog den in den Beispielen genannten Verfahren durch Verwendung geeigneter Ausgangsmaterialien. Das gleiche gilt für alle weiteren Ausgangsmaterialien.

Als Beispiel erwähnt sei die Herstellung einer durch Heterocyclylmethyl, worin Heterocyclyl über ein Ringstickstoffatom gebunden ist, substituierten Arylniederalkansäure (eine Verbindung der Formel III), welche vorzugsweise durch Umsetzung eines durch Halogenmethyl, wie Chlor- oder Brommethyl, substituierten Arylniederalkanoylrestes, wie Chlormethylbenzoyl oder Brommethylbenzoyl, mit einer entsprechenden heterocyclischen Stickstoffbase, wie Piperidin, Piperazin, 1-Niederalkylpiperazin, 1-Niederalkanyolpiperazin oder insbesondere Morpholin oder Thiomorpholin, unter nukleophiler Substitution des Halogenatomes erfolgt.

Die Herstellung von Aminosäurederivaten der Formeln III, V oder VII, worin die α-Aminogruppe durch einen Rest ausgewählt aus Phenylniederalkyl oder Heterocyclylniederalkyl alkyliert ist, ist beispielsweise möglich durch reduktive Aminierung der (erforderlichenfalls an weiteren Gruppen, die nicht an der Reaktion teilnehmen sollen, geschützten) Aminosäure, welche eine primäre oder sekundäre α-Aminogruppe hat, mit einem Phenylniederalkylketon oder -aldehyd, wie Benzaldehyd, oder Heterocyclylniederalkylketon- oder -aldehyd, beispielsweise Heterocyclylaldehyd, z.B. Furanaldehyd, wie Furan-2-aldehyd, oder Pyridinaldehyd, wie Pyridin-3-aldehyd, beispielsweise unter katalytischer Hydrierung, z.B. in Gegenwart eines Schwermetallkatalysators, wie Raney-Nickel, bei Normaldruck oder Drucken von 1 bis 100 bar, vorzugsweise bei etwa 100 bar, oder unter Reduktion mittels komplexer Borhydride, wie Natriumcyanoborhydrid.

Die Isocyanate der Formeln IX' und V'' lassen sich beispielsweise aus den entsprechenden Aminvorstufen durch Umwandlung der Aminogruppe in die Isocyanatogruppe herstellen, z.B. durch Umsetzung mit Phosgen in der Hitze, z.B. unter Rückflussbedingungen, oder durch Zutropfen des flüssigen oder in einem Lösungsmittel gelösten primären, sekundären oder tertiären Amins zu einem Überschuss von Phosgen in einem geeigneten Lösungsmittel (Toluol, Xylol, Ligroin, Chlorbenzen, α-Chlornaphthalen usw.) unter Kühlung (beispielsweise auf - 50 bis 0°C), wobei sich intermediär eine Mischung aus Carbamoylchlorid und Aminhydrochlorid bildet, die dann bei erhöhter Temperatur (besipielsweise bei 50 ° C bis Rückflusstemperatur) weiter bis zur vollständigen Lösung phosgeniert wird, unter HCl-Eliminierung.

Für alle vor- und nachstehend genannten Verfahren gilt allgemein folgendes:

Infolge der engen Beziehung zwischen den Verbindungen der Formel I und ihren Salzen und Ausgangsmaterialien (Ausgangsstoffe und Zwischenprodukte) in freier Form und in Form ihrer Salze sind vor- und nachstehend unter den freien Verbindungen bzw. ihren Salzen sinn- und zweckmässig gegebenenfalls auch die entsprechenden Salze bzw. freien Verbindungen zu verstehen.

Alle vorstehend angeführten Verfahrensschritte können unter an sich bekannten, vorzugsweise den spezifisch genannten, Reaktionsbedingungen durchgeführt werden, in Ab- oder üblicherweise Anwesenheit von Lösungs- oder Verdünnungsmitteln, vorzugsweise solchen, die gegenüber den verwendeten Reagenzien inert sind und diese lösen, in Ab- oder Anwesenheit von Katalysatoren, Kondensationsmitteln oder neutralisierenden Agentien, z. B. Ionenaustauschern, wie Kationenaustauschern, z. B. in der H⁺-Form, je nach Art der Reaktion und/oder der Reaktionsteilnehmer bei erniedrigter, normaler oder erhöhter Temperatur, z.B. im Temperaturbereich von etwa -100°C bis etwa 190°C, vorzugsweise von etwa -80°C bis etwa 150°C, z.B. bei - 80 bis -60 °C, bei Raumtemperatur, bei - 20 bis 40 °C oder bei Rückflusstemperatur, unter atmosphärischem Druck oder in einem geschlossenen Gefäss, gegebenenfalls unter Druck, und/oder in einer inerten Atmosphäre, z.B. unter einer Argon- oder Stickstoffatmosphäre.

Auf allen Reaktionsstufen können auftretende Isomerengemische in die einzelnen Isomeren, z. B. Diastereomeren oder Enantiomeren, oder in beliebige Gemische von Isomeren, z. B. Racemate oder Diastereomerengemische, aufgetrennt werden, beispielsweise analog zu den Methoden, die unter den "Zusätzlichen Verfahrensmassnahmen" beschrieben sind.

In bestimmten Fällen, beispielsweise bei Hydrierungen, ist es möglich, stereoselektive Reaktionen zu erzielen, so dass z. B. eine erleichterte Gewinnung von einzelnen Isomeren möglich ist.

Zu den Lösungsmitteln, aus denen die für die jeweilige Reaktion geeigneten ausgewählt werden können, zählen beispielsweise Wasser, Ester, wie Niederalkylniederalkanoate, z. B. Essigsäureethylester, Ether, wie aliphatische Ether, z. B. Diethylether, oder cyclische Ether, z. B. Tetrahydrofuran, flüssige aromatische Kohlenwasserstoffe, wie Benzol oder Toluol, Alkohole, wie Methanol, Ethanol oder 1- oder 2-Propanol, Nitrile, wie Acetonitril, Halogenkohlenwasserstoffe, wie Methylenchlorid, Säureamide, wie Dimethylformamid, Basen, wie heterocyclische Stickstoffbasen, z. B. Pyridin, Carbonsäureanhydride, wie Niederalkansäureanhydride, z. B. Acetanhydrid, cyclische, lineare oder verzweigte Kohlenwasserstoffe, wie Cyclohexan, Hexan oder Isopentan, oder Gemische dieser Lösungsmittel, z. B. wässrige Lösungen, soweit bei der Beschreibung der Verfahren nichts anderes angegeben ist. Derartige Lösungsmittelgemische können auch bei der Aufarbeitung, beispielsweise durch Chromatographie oder Verteilung, Verwendung finden.

Die Verbindungen, einschliesslich ihrer Salze, können auch in Form von Hydraten erhalten werden, oder ihre Kristalle können z.B. das zur Kristallisation verwendete Lösungsmittel einschliessen.

Die Erfindung betrifft auch diejenigen Ausführungsformen des Verfahrens, bei denen man von einer auf einer beliebigen Verfahrensstufe als Zwischenprodukt erhältlichen Verbindung ausgeht und die fehlenden Verfahrensschritte durchführt, oder bei denen ein Ausgangsstoff unter den Reaktionsbedingungen gebildet oder in Form eines Derivates, z.B. in geschützter Form oder als Salz, verwendet wird, oder eine nach dem erfindungsgemässen Verfahren erhältliche Verbindung unter den Verfahrensbedingungen erzeugt und in situ weiterverarbeitet. Im Verfahren der vorliegenden Erfindung werden vorzugsweise solche Ausgangsstoffe eingesetzt, die zu den eingangs als besonders wertvoll beschriebenen Verbindungen (der Formel I oder II) führen. In erster Linie sind Reaktionsbedingungen bevorzugt, die den in den Beispielen genannten analog sind.

Soweit erforderlich, könne auf allen Verfahrensstufen geschützte Ausgangsverbindungen eingesetzt und die Schutzgruppen auf geeigneten Reaktionstufen entfernt werden.

Schutzgruppen, ihre Einführung und ihre Freisetzung sind wie unter Verfahren a) und f) beschrieben.

### Pharmazeutische Präparate:

Die Erfindung betrifft auch pharmazeutische Präparate enthaltend Verbindungen der Formel I oder (insbesondere die als bevorzugt genannten Verbindungen) der Formel II.

Die pharmakologisch verwendbaren Verbindungen der vorliegenden Erfindung können z. B. zur Herstellung von pharmazeutischen Präparaten verwendet werden, welche eine wirksame Menge des Wirkstoffes zusammen oder im Gemisch mit einer signifikanten Menge von anorganischen oder organischen, festen oder flüssigen, pharmazeutisch verwendbaren Trägerstoffen enthalten.

Zur Erfindung gehört auch eine pharmazeutische Zusammensetzung (Präparat), die geeignet ist zur Verabreichung an einen Warmblüter, insbesondere Menschen, zur Behandlung oder Verhütung einer Erkrankung, die auf eine Hemmung einer retroviralen Protease, insbesondere einer retroviralen Aspartatprotease, wie HIV-I- oder HIV-II-gag-Protease, anspricht, z. B. einer retroviralen Erkrankung, wie AIDS, umfassend eine zur Hemmung der retroviralen Protease wirksame Menge einer Verbindung der Formel I oder II'', oder eines pharmazeutisch annehmbaren Salzes davon, zusammen mit wenigstens einem pharmazeutisch annehmbaren Trägermaterial.

Bei den erfindungsgemässen pharmazeutischen Präparaten handelt es sich um solche zur enteralen, wie nasalen, rektalen oder oralen, oder parenteralen, wie intramuskulären oder intravenösen, Verabreichung an Warmblüter (Menschen und Tiere), welche eine effektive Dosis des pharmakologischen Wirkstoffs allein oder zusammen mit einer signifikanten Menge eines pharmazeutisch anwendbaren Trägermaterials enthalten. Die Dosierung des Wirkstoffs hängt von der Warmblüter-Spezies, dem Körpergewicht, dem Alter und dem individuellen Zustand, individuellen pharmakokinetischen Gegebenheiten, der zu behandelnden Krankheit sowie der Applikationsweise ab.

Die Erfindung betrifft auch eine Methode zur Behandlung von durch Viren, insbesondere Retroviren verursachten Krankheiten, z. B. von AIDS, dadurch gekennzeichnet, dass eine therapeutisch wirksame Menge von erfindungsgemässen Verbindungen der Formel I oder II'', insbesondere an einen Warmblüter, z. B. Menschen, der wegen einer der genannten Erkrankungen, insbesondere AIDS, einer derartigen Behandlung bedarf, verabreicht wird. Die an Warmblüter, z. B. Menschen von etwa 70 kg Körpergewicht, zu verabreichenden Dosismengen liegen zwischen etwa 3 mg und etwa 3 g, vorzugsweise zwischen etwa 10 mg und etwa 1,5 g, z. B. bei ungefähr 100 mg bis 1000 mg pro Person und Tag, verteilt auf vorzugsweise 1 bis 3 Einzeldosen, die z. B. gleich gross sein können. Ueblicherweise erhalten Kinder die halbe Dosis von Erwachsenen.

Die pharmazeutischen Präparate enthalten von etwa 1 % bis etwa 95 %, vorzugsweise von etwa 20 % bis etwa 90 % des Wirkstoffes. Erfindungsgemässe pharmazeutische Präparate können z. B. in Dosiseinheitsform, wie Ampullen, Vials, Suppositorien, Dragées, Tabletten oder Kapseln, vorliegen.

Die pharmazeutischen Präparate der vorliegenden Erfindung werden in an sich bekannter Weise, z. B. mittels konventioneller Lösungs-, Lyophilisierungs-, Misch-, Granulier- oder Dragierverfahren, hergestellt.

Vorzugsweise verwendet man Lösungen des Wirkstoffes, daneben auch Suspensionen, und zwar insbesondere isotonische wässrige Lösungen oder Suspensionen, wobei diese z. B. bei lyophilisierten Präparaten, welche die Wirksubstanz allein oder zusammen mit einem Trägermaterial, z. B. Mannit, enthalten, vor Gebrauch hergestellt werden können. Die pharmazeutischen Präparate können sterilisiert sein und/oder Hilfsstoffe, z. B. Konservier-, Stabilisier-, Netz- und/oder Emulgiermittel, Löslichkeitsvermittler, Salze zur Regulierung des osmotischen Druckes und/oder Puffer enthalten und werden in an sich bekannter Weise, z. B. mittels konventioneller Lösungs- oder Lyophilisierungsverfahren, hergestellt. Die genannten Lösungen oder Suspensionen können viskositätserhöhende Stoffe, wie Natriumcarboxymethylcellulose, Carboxymethylcellulose, Dextran, Polyvinylpyrrolidon oder Gelatine, enthalten.

Suspensionen in Öl enthalten als ölige Komponente die für Injektionszwecke üblichen vegetabilen, synthetischen oder halbsynthetischen Öle. Als solche sind insbesondere flüssige Fettsäureester zu nennen, die als Säurekomponente eine langkettige Fettsäure mit 8-22, insbesondere 12-22, Kohlenstoffatomen, wie z. B. Laurinsäure, Tridecylsäure, Myristinsäure, Pentadecylsäure, Palmitinsäure, Margarinsäure, Stearinsäure, Arachidinsäure, Behensäure oder entsprechende ungesättigte Säuren, wie z. B. Ölsäure, Elaidinsäure, Erucasäure, Brasidinsäure oder Linolsäure, enthalten, gegebenenfalls unter Zusatz von Antioxidantien, wie z. B. Vitamin E, β-Carotin oder 3,5-Di-tert-butyl-4-hydroxytoluol. Die Alkoholkomponente dieser Fettsäureester hat maximal 6 Kohlenstoffatome und ist ein ein- oder mehrwertiger, z. B. ein-, zwei- oder dreiwertiger Alkohol, z. B. Methanol, Ethanol, Propanol, Butanol oder Pentanol oder deren Isomere, vor allem aber Glycol und Glycerin. Als Fettsäureester sind daher beispielsweise zu nennen: Ethyloleat, Isopropylmyristat, Isopropylpalmitat, "Labrafil M 2375" (Polyoxyethylenglycerintrioleat der Firma Gattefossé, Paris), "Miglyol 812" (Triglycerid gesättigter Fettsäuren der Kettenlänge C₈ bis C₁₂ der Firma Hüls AG, Deutschland), besonders aber vegetabile Öle wie Baumwollsaatöl, Mandelöl, Olivenöl, Ricinusöl, Sesamöl, Sojabohnenöl und vor allem Erdnussöl.

Die Herstellung der Injektionspräparate erfolgt in üblicher Weise unter sterilen Bedingungen, ebenso das Abfüllen in Ampullen oder Vialen sowie das Verschliessen der Behälter.

Pharmazeutische Präparate zur oralen Anwendung können erhalten werden, indem man den Wirkstoff mit festen Trägerstoffen kombiniert, ein erhaltenes Gemisch gegebenenfalls granuliert und das Gemisch, wenn erwünscht oder notwendig, nach Zugabe von geeigneten Hilfsstoffen, zu Tabletten, Dragée-Kernen oder Kapseln verarbeitet. Dabei kann man sie auch in Kunststoffträger einbauen, die die Wirkstoffe dosiert abgeben oder diffundieren lassen.

Geeignete Trägerstoffe sind insbesondere Füllstoffe, wie Zucker, z. B. Lactose, Saccharose, Mannit oder Sorbit, Cellulosepräparate, und/oder Calciumphosphate, z. B. Tricalciumphosphat oder Calciumhydrogenphosphat, ferner Bindemittel, wie Stärkekleister unter Verwendung z. B. von Mais-, Weizen-, Reis- oder Kartoffelstärke, Gelatine, Traganth, ganth, Methylcellulose, Hydroxypropylmethylcellulose, Natriumcarboxymethylcellulose und/oder Polyvinylpyrrolidon, und/oder, wenn erwünscht, Sprengmittel, wie die oben genannten Stärken, ferner Carboxymethylstärke, quervernetztes Polyvinylpyrrolidon, Agar, Alginsäure oder ein Salz davon, wie Natriumalginat. Hilfsmittel sind in erster Linie Fliessregulier- und Schmiermittel, z. B. Kieselsäure, Talk, Stearinsäure oder Salze davon, wie Magnesium- oder Calciumstearat, und/oder Polyethylenglycol. Dragée-Kerne werden mit geeigneten, gegebenenfalls magensaftresistenten Überzügen versehen, wobei man u.a. konzentrierte Zuckerlösungen, welche gegebenenfalls arabischen Gummi, Talk, Polyvinylpyrrolidon, Polyethylenglycol und/oder Titandioxid enthalten, Lacklösungen in geeigneten organischen Lösungsmitteln oder, zur Herstellung von magensaftresistenten Überzügen, Lösungen von geeigneten Cellulosepräparaten, wie Ethylcellulosephthalat oder Hydroxypropylmethylcellulosephthalat, verwendet. Kapseln sind Steckkapseln aus Gelatine sowie weiche, geschlossene Kapseln aus Gelatine und einem Weichmacher, wie Glycerin oder Sorbit. Die Steckkapseln können den Wirkstoff in Form eines Granulats, z. B. mit Füllstoffen, wie Lactose, Bindemitteln, wie Stärken, und/oder Gleitmitteln, wie Talk oder Magnesiumstearat, und gegebenenfalls mit Stabilisatoren, enthalten. In weichen Kapseln ist der Wirkstoff vorzugsweise in geeigneten öligen Hilfsstoffen, wie fetten Ölen, Paraffinöl oder flüssigen Polyethylenglykolen, gelöst oder suspendiert, wobei ebenfalls Stabilisatoren und/oder antibakterielle Mittel zugefügt sein können. Den Tabletten oder Dragée-Überzügen und den Kapselhüllen können Farbstoffe oder Pigmente, z. B. zur Identifizierung oder zur Kennzeichnung verschiedener Wirkstoffdosen, beigefügt werden.

Die folgenden Beispiele dienen zur Illustration der Erfindung, schränken deren Umfang jedoch in keiner Weise ein.

Temperaturen werden in Celsiusgraden (°C) angegeben. Sofern keine Temperaturangabe erfolgt, findet die Reaktion bei Raumtemperatur statt. Die R_{f}-Werte, die das Verhältnis von Laufstrecke der jeweiligen Substanz zur Laufstrecke der Laufmittelfront angeben, werden auf Kieselgeldünnschichtplatten durch Dünnschichtchromatographie (DC) in folgenden Lösungsmittelsystemen ermittelt

### DC-Laufmittelsysteme:

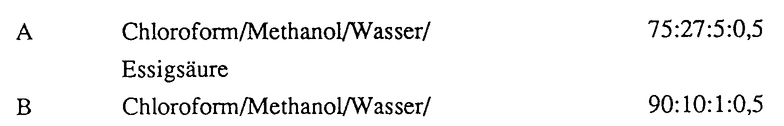

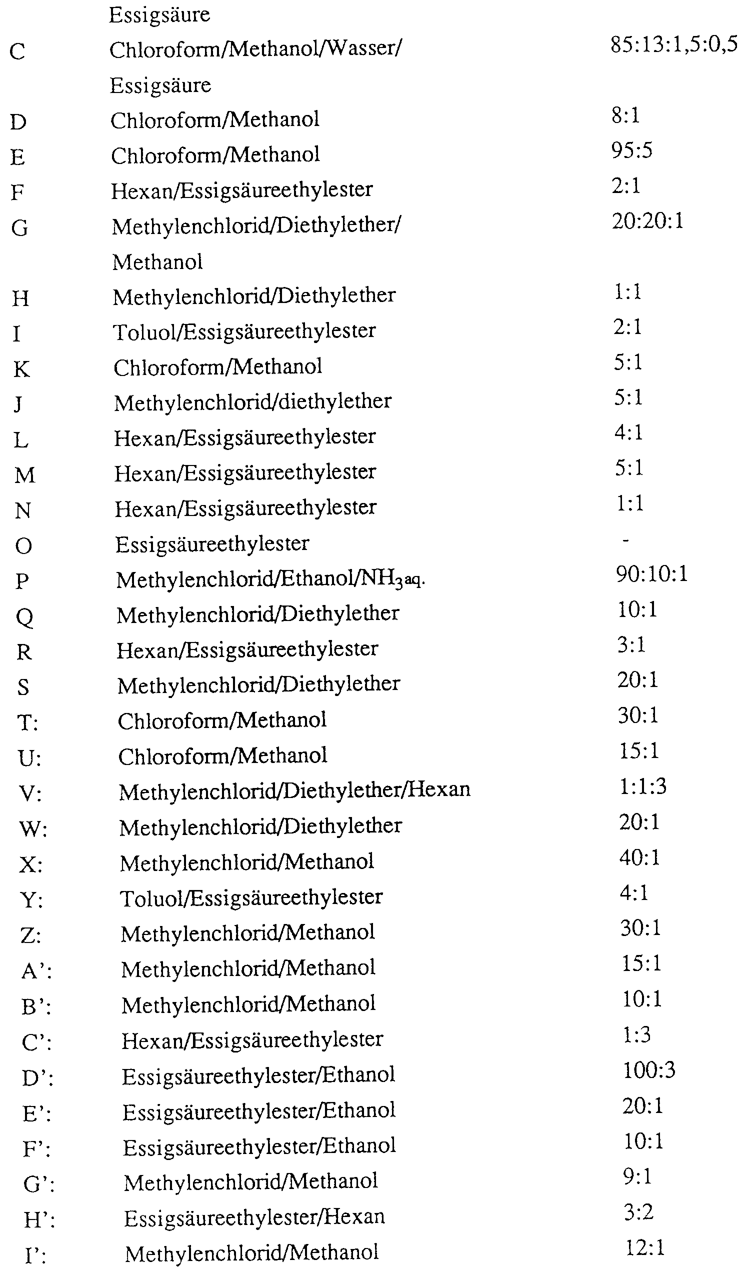

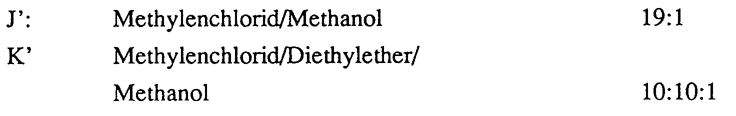

Die Abkürzung "R_{f}(A)" bedeutet beispielsweise, dass der R_{f}-Wert im Lösungsmittelsystem A ermittelt wurde. Das Mengenverhältnis von Lösungsmitteln zueinander ist stets in Volumenanteilen angegeben.

### HPLC-Gradienten:

| | |
|---|---|
| I | 20 % → 100 % a) in b) während 35 min. |
| II | 0 % → 40 % a) in b) während 30 min. |
| III | 20 % → 60 % a) in b) während 60 min. |
| IV | 10 % → 50 % a) in b) während 60 min. |
| V | 20 % → 100 % a) in b) während 20 min. |

Laufmittel a): Acetonitril + 0,05 % TFA; Laufmittel b): Wasser + 0,05 % TFA. Säule (250 x 4,6 mm) gefüllt mit "Reversed-Phase"-Material C₁₈-Nucleosil® (5 µm mittlere Korngrösse, mit Octadecylsilanen kovalent derivatisiertes Silicagel, Macherey & Nagel, Düren, BRD). Detektion durch UV-Absorption bei 215 nm. Die Retentionszeiten (t_{Ret}) werden in Minuten angegeben. Fliessgeschwindigkeit 1 ml/min.

Für die Bezeichnung der Fliessmittel-Systeme werden bei der Flash-Chromatographie und der Mitteldruckchromatographie die gleichen Abkürzungen verwendet.

Die weiteren verwendeten Kurzbezeichnungen und Abkürzungen haben die folgenden Bedeutungen:

| | |
|---|---|
| abs. | absolut (gibt an, dass Lösungsmittel wasserfrei ist) |
| atm | physikalische Atmosphären (Druckeinheit) - 1 atm entspricht 1,013 bar |
| Boc | tert-Butoxycarbonyl |
| BOP | Benzotriazol-1-yl-oxy-tris-(di-methylamino)-phosponium-hexafluor-phosphat |
| DCC | Dicyclohexylcarbodiimid |
| DMAP | Dimethylaminopyridin |
| DIPE | Diisopropylether |
| DMF | Dimethylformamid |
| DMSO | Dimethylsulfoxid |
| EDC | N-Ethyl-N'-(3-dimethylaminopropyl)-carbodiimid-hydrochlorid |
| Ether | Diethylether |
| ges. | gesättigt |
| h | Stunde(n) |
| HBTU | O-Benztriazol-1-yl-N,N,N',N'-tetramethyl-uronium- |
| | hexafluorphosphat |
| HOBt | 1-Hydroxy-benztriazol |
| HV | Hochvakuum |
| min | Minute(n) |
| MS | Massenspektroskopie |
| NMM | N-Methyl-morpholin |
| Ref.-Bsp. | Referenzbeispiel |
| RT | Raumtemperatur |
| RV | Rotationsverdampfer |
| Sole | gesättigte Natriumchloridlösung |
| THF | Tetrahydrofuran |
| TFA | Trifluoressigsäure |
| Z | Benzyloxycarbonyl |

Massenspektroskopische Messwerte werden entweder durch konventionelle MS oder nach der "Fast-Atom-Bombardment" (FAB-MS)-Methode erhalten. Die Massenangaben beziehen sich im ersten Fall auf das unprotonierte Molekülion (M)⁺ oder das protonierte Molekülion (M+H)⁺.

Die Werte für Protonen-Kernresonanzspektroskopie (¹H-NMR) werden in ppm (parts per million) bezogen auf Tetramethylsilan als internen Standard angegeben. s = Singulett, d = Dublett, t = Triplett, q = Quartett, m = Multiplett, dd = Doppeldublett, br = breit.

Die Werte für IR-Spektren werden in cm⁻¹ angegeben, in runden Klammern findet sich das jeweilige Lösungsmittel. Falls angegeben, bedeutet s eine starke, m eine mittlere und w eine schwache Intensität der jeweiligen Bande.

Der Rest mit der Bezeichnung -[Phe^{NN}Phe] bedeutet das zweiwertige Radikal von 3(S)-Amino-4-phenyl-1-(N-benzylhydrazino)-butan-2(S)-ol und hat die Formel

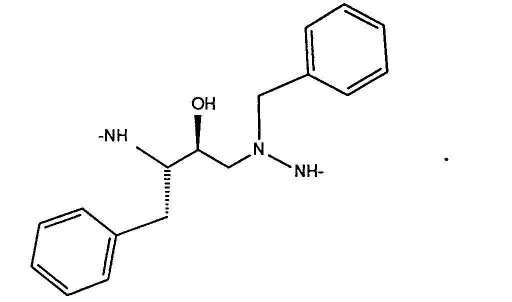

Der Rest mit der Bezeichnung -[Phe^{NN}Cha] bedeutet das zweiwertige Radikal von 3(S)-Amino-4-phenyl-1-(N-cyclohexylmethylhydrazino)-butan-2(S)-ol und hat die Formel

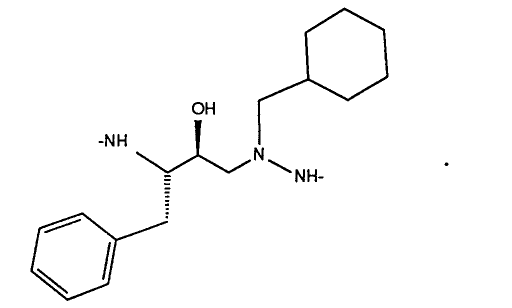

Der Rest mit der Bezeichnung -[Phe^{NN}Leu] bedeutet das zweiwertige Radikal von 3(S)-Amino-4-phenyl-1-(N-isobutylhydrazino)-butan-2(S)-ol und hat die Formel

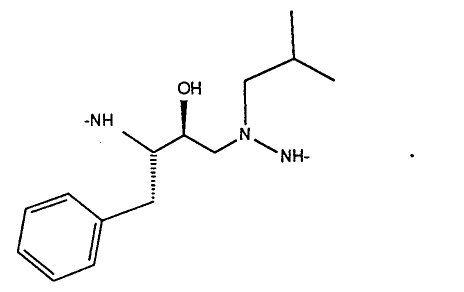

Der Rest mit der Bezeichnung -[Phe^{NN}Nle] bedeutet das Radikal von 3(S)-Amino-4-phenyl-1-(N-n-butylhydrazino)-butan-2(S)-ol und hat die Formel

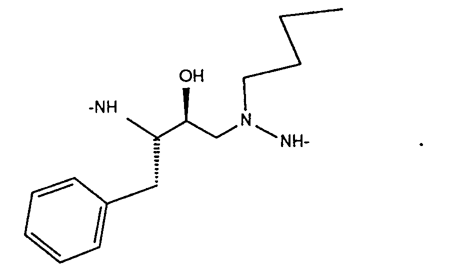

Der Rest mit der Bezeichnung -[Phe^{NN}(p-F)Phe] bedeutet das zweiwertige Radikal von 3(S)-Amino-4-phenyl-1-(N-(p-fluorphenylmethyl)-hydrazino)-butan-2(S)-ol und hat die Formel

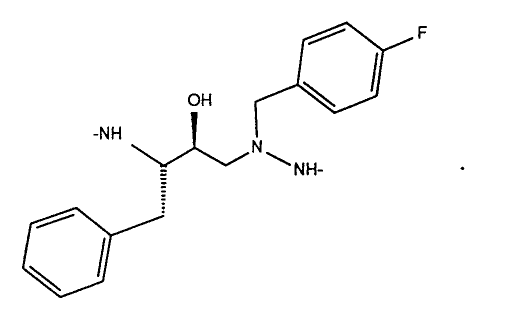

Der Rest mit der Bezeichnung -[(p-F)Phe^{NN}(p-F)Phe] bedeutet das zweiwertige Radikal von 3(S)-Amino-4-(p-fluorphenyl)-1-(N-(p-fluorphenylmethyl)-hydrazino)-butan-2(S)-ol und hat die Formel

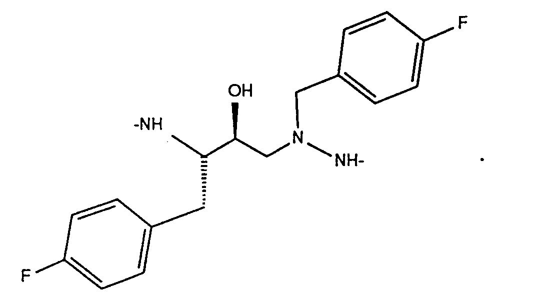

Der Rest mit der Bezeichnung -[Phe^{NN}(p-CN)Phe] bedeutet das zweiwertige Radikal von 3(S)-Amino-4-phenyl-1-(N-(p-cyanophenylmethyl)-hydrazino)-butan-2(S)-ol und hat die Formel

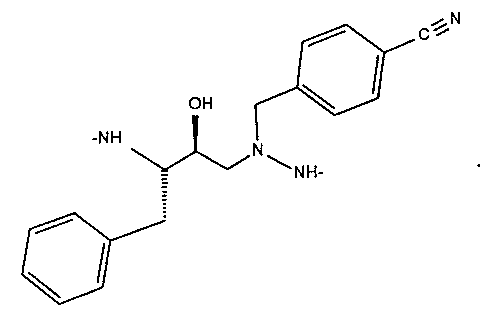

Der Rest mit der Bezeichnung -[Cha^{NN}Leu] bedeutet das zweiwertige Radikal von 3(S)-Amino-4-cyclohexyl-1-(N-isobutyl-hydrazino)-butan-2(S)-ol und hat die Formel

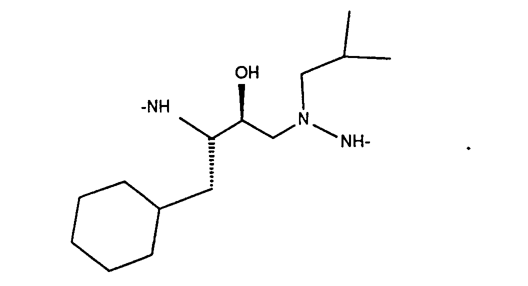

Das zweiwertige Radikal von 1-[2(S)-Acetoxy-3(S)-amino-4-phenylbutyl]-[1-cyclohexylmethyl]hydrazin hat die Formel

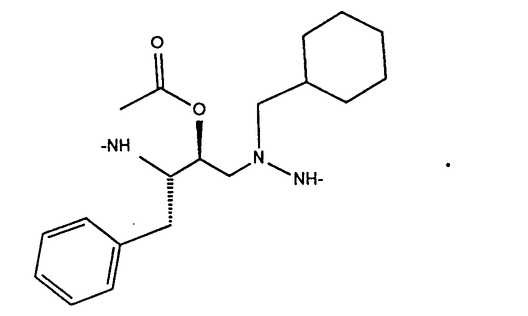

Zur Bezeichnung von zweiwertigen Radikalen von natürlichen α-Aminosäuren werden die in der Peptidchemie üblichen Abkürzungen verwendet. Hierbei haben jedoch Aminosäuren, die in den Verbindungsnamen rechts der Radikale -[Phe^{NN}Phe], -[Phe^{NN}Cha], -[Phe^{NN}Leu], -[Phe^{NN}Nle], -[Phe^{NN}(p-F)Phe], -[(p-F)Phe^{NN}(p-F)Phe], -[Phe^{NN}(p-CN)Phe] oder -[Cha^{NN}Leu] stehen, in Abweichung von der üblichen Peptidnomenklatur, worin links der Amino- und rechts der Carboxyterminus vorliegt, die bindende Carboxygruppe links, was durch einen Pfeil (←), der die Umkehrung der Bindungsrichtung symbolisiert, angedeutet wird (Inversion). Die Konfiguration am α-Kohlenstoffatom wird, wenn sie bekannt ist, durch Voranstellen von (L)- oder (D)-angegeben. An der phenolischen Hydroxygruppe mit dem Rest R veretherte Tyrosin-Radikale werden mit Tyr(OR) bezeichnet. Nle bedeutet das Radikal von Norleucin.

### Referenzbeispiel 1: Boc-[Phe^{NN}Phe]-Boc:

Eine Lösung von 300 mg (1,14 mMol) (2R)-[1'(S)-Boc-amino-2'-phenylethyl]oxiran (J. Org. Chem. **50**, 4615 (1985)) und 253 mg (1,14 mMol) tert-Butyl-3-benzyl-carbazat (J. Chem. Soc., Perkin I, 1712 (1975)) in 4 ml Methanol wird während 12 h unter Rückfluss erhitzt. Nach dem Abkühlen des Reaktionsgemisches auf 0° fällt ein grosser Teil der Titelverbindung aus. Die Mutterlauge wird eingedampft und der Rückstand in wenig Methylenchlorid gelöst. Nach Zutropfen von Hexan fällt weitere Titelverbindung als weisser Niederschlag aus. FAB-MS: (M+H)⁺=486, t_{Ret}(I)=26,8 min, R_{f}(E)=0,70.

### Referenzbeispiel 2: Z-(L)-Val-[Phe^{NN}Phe]←((L)-Val-Z):

Man löst 191 mg (0,76 mMol) Z-(L)-Valin, 336 mg (0,76 mMol) BOP und 103 mg (0,76 mMol) HOBt in 5 ml einer 0,3 M Lösung von NMM in DMF, fügt nach 10 min 100 mg (0,25 mMol) H-[Phe^{NN}Phe]-H · 3HCl zu und rührt während 2 h bei *RT* unter Stickstoffatmosphäre. Die Reaktionsmischung wird eingedampft, der Rückstand in Methylenchlorid gelöst und zweimal mit ges. Natriumbicarbonatlösung gewaschen. Die organischen Phasen werden durch Watte filtriert, eingedampft und der Rückstand mittels Chromatographie auf Kieselgel mit Methylenchlorid/Ether (1:1) gereinigt. Nach Lyophilisation der produkthaltigen Fraktionen aus Dioxan erhält man die Titelverbindung als weissen Festkörper. FAB-MS: (M+H)⁺=752, t_{Ret}(I)=27,8 min, R_{f}(E)=0,45.

Das Ausgangsmaterial wird folgendermassen hergestellt:

### a) H-[Phe^{NN}Phe]-H · 3HCl:

Eine Lösung von 280 mg (0,58 mMol) Boc-[Phe^{NN}Phe]-Boc aus Ref.-Bsp. 1 in 10 ml 4 N Chlorwasserstoff in Dioxan wird während 2 h bei RT unter Stickstoffatmosphäre gerührt und anschliessend lyophilisiert. Erneute Lyophilisation aus Dioxan/tert-Butanol ergibt die Titelverbindung als flockigen Festkörper. FAB-MS: (M+H)⁺=286, t_{Ret}(II)=23,1 min, R_{f}(C)=0,17.

### Referenzbeispiel 3: Boc-(L)-Val-[Phe^{NN}Phe]←((L)-Val-Boc):

Auf analoge Weise wie in Ref.-Bsp. 2 beschrieben erhält man aus 50 mg (0,13 mMol) H-[Phe^{NN}Phe]-H · 3HCl, 83 mg (0,83 mMol) Boc-(L)-Valin, 168 mg (0,38 mMol) BOP, 51 mg (0,38 mMol) HOBt und 2,5 ml 0,3 M NMM in DMF nach chromatographischer Reinigung an Kieselgel mit Chloroform/Methanol (95:5) und Lyophilisation aus Dioxan die Titelverbindung. FAB-MS: (M+H)⁺=684, t_{Ret}(I)=27,4 min, R_{f}(E)=0,38.

### Referenzbeispiel 4: Boc-[Phe^{NN}Cha]-Boc:

Analog Ref.-Bsp. 1 erhält man aus 231 mg (0,88 mMol) (2R,3S)-1-[3-Boc-amino-2-phenylethyl]oxiran und 200 mg (0,88 mMol) tert-Butyl-3-cyclohexylmethyl-carbazat die Titelverbindung als weissen Niederschlag aus Hexan. FAB-MS: (M+H)⁺=492, t_{Ret}(I)=30,4 min, R_{f}(E)=0,78.

Das Ausgangsmaterial wird folgendermassen hergestellt:

### a) tert-Butyl-3-cyclohexylmethyl-carbazat:

10.2 g (45,1 mMol) Cyclohexylcarbaldehyd-tert-butoxycarbonylhydrazon, gelöst in 400 ml Methanol, werden in Gegenwart von 5,1 g 5 % Platin auf Kohle bei RT und 4 atm Wasserstoffdruck hydriert. Nach beendeter Reaktion wird vom Katalysator abfiltriert und das Filtrat eingedampft. Der Rückstand wird in Methylenchlorid gelöst und mit Wasser gewaschen. Nach dem Eindampfen der organischen Phase erhält man die Titelverbindung als farbloses Harz. ¹H-NMR (200 MHz, CDCl₃): 6,1 (s, br, 1H), 3,9 (s, br, 1H), 2,65 (d, 2H), 1,8-0,75 (m, 11H), 1,45 (s, 9H), t_{Ret}(I)=32,0 min, R_{f}(E)=0,75.

### b) Cyclohexylcarbaldehyd-tert-butoxycarbonylhydrazon:

Eine Lösung von 10,8 g (81,2 mMol) tert-Butylcarbazat und 10,1 g (90 mMol) Cyclohexylcarbaldehyd in 400 ml Ethanol wird während 2 h unter Rückfluss erhitzt. Anschliessend wird die Hälfte des Lösungsmittels abdestilliert und die Titelverbindung durch Zugabe von Wasser ausgefällt. Sie wird direkt weiterverwendet in a).

### Referenzbeispiel 5: H-(L)-Val-[Phe^{NN}Phe]←((L)-Val)-H · 3HCl:

Eine Lösung von 40 mg (0,06 mMol) Boc-(L)-Val-[Phe^{NN}Phe]←((L)-Val)-Boc aus Ref.-Bsp. 3 in 4 ml 4 N Chlorwasserstoff in Dioxan wird während 1 h bei RT gerührt. Anschliessend verdünnt man mit Dioxan und erhält nach Lyophilisation die Titelverbindung als Hydrochlorid. FAB-MS: (M+H)⁺=484, t_{Ret}(II)=25,8 min, R_{f}(A)=0,45.

### Referenzbeispiel 6: N-Thiomorpholinocarbonyl-(L)-Val-[Phe^{NN}Phe]←(N-thiomorpholinocarbonyl-(L)-Val):

Eine Lösung von 20 mg (0,03 mMol) H-(L)-Val-[Phe^{NN}Phe]←((L)-Val)-H · 3HCl aus Ref.-Bsp. 5 in 0,5 ml DMF wird bei RT nacheinander mit 35 µl (0,25 mMol) Triethylamin und 16 mg (0,1 mMol) (4-Thiomorpholinylcarbonyl)chlorid versetzt und während 1 h bei RT gerührt. Die Reaktionsmischung wird mit Chloroform verdünnt und mit ges. Natriumbicarbonatlösung gewaschen. Die organische Phase wird durch Watte filtriert, eingedampft und der Rückstand an Kieselgel mit einem Gradienten von Chloroform/Methanol (15:1 -> 8:1) chromatographiert. Die Produktfraktionen werden eingedampft und mit Methylenchlorid/DIPE gefällt. Sie ergeben nach Lyophilisation aus Dioxan die Titelverbindung als flockigen Festkörper. FAB-MS: (M+H)⁺=742, t_{Ret}(I)=21,6 min, R_{f}(D)=0,54.

Das Ausgangsmaterial wird folgendermassen hergestellt:

### a) (4-Thiomorpholinylcarbonyl)chlorid:

Zu einer Lösung von 85 ml (165 mMol) 20 % Phosgen in Toluol wird bei 0° eine Lösung von 10 g (97 mMol) Thiomorpholin in 200 ml Toluol zugetropft und die weisse Suspension während 1 h bei RT gerührt. Ueberschüssiges Phosgen wird durch Einleiten von Stickstoff vertrieben, die Suspension filtriert und das Filtrat eingedampft. Man erhält die Titelverbindung als gelbes Oel. IR (CH₂Cl₂, cm⁻¹): 1735, 1450, 1440, 1405, 1370, 1290, 1180.

### Referenzbeispiel 7: N-Morpholinocarbonyl-(L)-Val-[Phe^{NN}Phe]←(N-morpholinocarbonyl-(L)-Val):

Eine Lösung von 100 mg (0,25 mMol) H-[Phe^{NN}Phe]-H · 3HCl aus Ref.-Bsp. 2a, 163 mg (0,76 mMol) N-Morpholinocarbonyl-(L)-valin und 288 mg (0,76 mMol) HBTU in 2 ml DMF wird mit 210 µl (1.52 mMol) Triethylamin versetzt und bei RT während 16 h unter Stickstoffatmosphäre gerührt. Die Reaktionsmischung wird vollständig eingedampft, der Rückstand in Methylenchlorid gelöst und mit ges. Natriumbicarbonatlösung gewaschen. Die organische Phase wird durch Watte filtriert, eingedampft und an Kieselgel mit Methylenchlorid/Methanol (15:1) chromatographiert. Die Titelverbindung wird aus Methylenchlorid/Hexan gefällt und nach Lyophilisation aus Dioxan/tert-Butanol als flockiger Festkörper erhalten. FAB-MS: (M+H)⁺=710, t_{Ret}(I)=16,3 min, R_{f}(E)=0,16.

Das Ausgangsmaterial wird folgendermassen hergestellt:

### a) N-Morpholinocarbonyl-(L)-valin:

2,7 g (8,4 mMol) N-Morpholinocarbonyl-(L)-valin-benzylester werden in 75 ml Essigester gelöst und in Gegenwart von 500 mg 10 % Palladium auf Kohle bei 1 atm Wasserstoffdruck und RT während 3 h hydriert. Der Katalysator wird abfiltriert, und man erhält nach dem Eindampfen des Lösungsmittels die Titelverbindung als farbloses Oel. ¹H-NMR (300 MHz, CD₃OD): 4,15 (m, 1H), 3,65 (m, 4H), 3,40 (m, 4H), 2,12 (m, 1H), 0,95 (2d, 6H).

### b) N-Morpholinocarbonyl-(L)-valin-benzylester:

Eine Lösung von 4 g (10,5 mMol) (L)-Valin-benzylester-4-toluolsulfonat in 56 ml Methylenchlorid wird mit 0,8 ml (8,1 mMol) (Morpholinocarbonyl)chlorid (Herstellung: J. Med. Chem. **31**, 2277 (1988)) und 4,1 ml (24,1 mMol) N-Ethyldiisopropylamin versetzt und während 24 h bei RT gerührt. Die Reaktionsmischung wird mit Essigester verdünnt und nacheinander mit 1 N Salzsäure, Wasser, ges. Natriumbicarbonatlösung und Sole gewaschen. Die organische Phase wird über Natriumsulfat getrocknet und eingedampft. Sie ergibt nach Chromatographie an Kieselgel mit Essigester den *N*-Morpholinocarbonyl-(L)-valin-benzylester als farbloses Oel. Der Ester wird sofort in a) weiterverwendet.

### Referenzbeispiel 8: Phenylacetyl-(L)-Val-[Phe^{NN}Phe]←(N-phenylacetyl-(L)-Val):

Analog Ref.-Bsp. 7 erhält man aus 100 mg (0,25 mMol) H-[Phe^{NN}Phe]-H · 3HCl aus Ref.-Bsp. 2a, 143 mg (0,61 mMol) Phenylacetyl-(L)-valin (Herstellung: Mem. Tokyo Univ. Agric. **20**, 51 (1978)), 230 mg (0,61 mMol) HBTU und 200 µl (1,42 mMol) Triethylamin nach chromatographischer Reinigung mit Methylenchlorid/Ether/Methanol (20:20:1) und Lyophilisation aus Dioxan/tert-Butanol die Titelverbindung. FAB-MS: (M+H)⁺=720, t_{Ret}(I)=23,7 min, R_{f}(G)=0,21.

### Referenzbeispiel 9: N-(3-Pyridylacetyl)-(L)-Val-[Phe^{NN}Phe]←(N-(3-pyridylacetyl)-(L)-Val):

Analog Ref.-Bsp. 7 erhält man aus 100 mg (0,25 mMol) H-[Phe^{NN}Phe]-H · 3HCl aus Ref.-Bsp. 2a, 576 mg (1,52 mMol) HBTU, 358 mg (1,52 mMol) N-(3-Pyridylacetyl)-(L)-valin und 316 µl (2,3 mMol) Triethylamin nach chromatographischer Reinigung mit Chloroform/Methanol (5:1) und Lyophilisation aus Dioxan/tert-Butanol die Titelverbindung als weissen Festkörper. FAB-MS: (M+H)⁺=722, t_{Ret}(II)=27,9 min, R_{f}(A)=0,71.

Das Ausgangsmaterial wird folgendermassen hergestellt:

### a) N-(3-Pyridylacetyl)-(L)-valin:

3,4 g N-(3-Pyridylacetyl)-(L)-valin-tert-butylester werden in 20 ml Trifluoressigsäure/Methylenchlorid (1:1) gelöst und bei RT während 16 h gerührt. Die Reaktionslösung wird vollständig eingedampft und der Rückstand mit DIPE digeriert. Man erhält die Titelverbindung als weissen, amorphen Festkörper. ¹H-NMR (200 MHz, CD₃OD): 8,9-8,6 (m, breit, 1H), 8,5 (m, 1H), 7,95 (m, 1H), 4,33 (m, 1H), 3,93 (s, 2H), 2,2 (m, 1H), 0,98 (2d, 6H).

### b) N-(3-Pyridylacetyl)-(L)-valin-tert-butylester:

Zu einer Lösung von 3,36 g (16 mMol) (L)-Valin-tert-butylester · HCl, 2 g (14,5 mMol) 3-Pyridylessigsäure und 2,17 ml (14,3 mMol) Cyanphosphonsäure-diethylester in 20 ml DMF werden bei 0° 4,2 ml Triethylamin zugetropft. Die Reaktionsmischung wird während 48 h bei RT gerührt, anschliessend mit Methylenchlorid verdünnt und mit 10 % Citronensäure sowie ges. Natriumbicarbonatlösung gewaschen. Die organische Phase wird durch Watte filtriert und ergibt nach Abdampfen des Lösungsmittels N-(3-Pyridylacetyl)-(L)-valin-tert-butylester, der sofort unter a) weiterverwendet wird.

### Referenzbeispiel 10: Boc-(L)-Val-[Phe^{NN}Cha]←((L)-Val)-Boc:

Analog Ref.-Bsp. 7 erhält man ausgehend von 500 mg (1,25 mMol) H-[Phe^{NN}Cha]-H · 3HCl, 1,08 g (4,98 mMol) Boc-(L)-Valin, 1,89 g (4,98 mMol) HBTU und 1,39 ml (9,96 mMol) Triethylamin nach chromatographischer Reinigung an Kieselgel mit Methylenchlorid/Ether (1:1) und Lyophilisation aus Dioxan die Titelverbindung als flockigen Festkörper. FAB-MS: (M+H)⁺=690, t_{Ret}(I)=29,3 min, R_{f}(H)=0,48.

Das Ausgangsmaterial wird folgendermassen hergestellt:

### a) H-[Phe^{NN}Cha]-H · 3HCl:

1,10 g (2,2 mMol) Boc-[Phe^{NN}Cha]-Boc aus Ref.-Bsp. 4 werden in 20 ml 4 N Chlorwasserstoff in Dioxan gelöst und während 3 h bei RT gerührt. Nach Lyophilisation der Reaktionslösung erhält man die Titelverbindung als Hydrochlorid. FAB-MS: (M+H)⁺=292, t_{Ret}(II)=27,3 min.

### Referenzbeispiel 11: Z-(L)-Val-[Phe^{NN}Cha]←((L)-Val)-Z:

Analog Ref.-Bsp. 2 erhält man aus 50 mg (0,12 mMol) H-[Phe^{NN}Cha]-H · 3HCl aus Ref.-Bsp. 10a, 94 mg (0,37 mMol) Z-(L)-Valin, 165 mg (0,37 mMol) BOP, 51 mg (0,37 mMol) HOBt und 2,5 ml 0,3 M *NMM* in DMF nach chromatographischer Reinigung an Kieselgel mit Methylenchlorid/Ether (1:1) und Lyophilisation aus Dioxan die Titelverbindung. FAB-MS: (M+H)⁺=758, t_{Ret}(I)=29,1 min, R_{f}(H)=0,55.

### Referenzbeispiel 12: Boc-[Phe^{NN}Leu]-Boc:

Analog Ref.-Bsp. 1 erhält man ausgehend von 1,0 g (3,8 mMol) (2R)-[1'(S)-Boc-amino-2'-phenylethyl]oxiran und 715 mg (3,8 mMol) tert-Butyl-3-isobutyl-carbazat (Herstellung: J. Chem. Soc., Perkin I, 1712 (1975)) die Titelverbindung als Niederschlag aus Hexan. FAB-MS: (M+H)⁺=452, t_{Ret}(I)=27,2 min, R_{f}(I)=0,55.

### Referenzbeispiel 13: Z-(L)-Val-[Phe^{NN}Leu]←((L)-Val)-Z:

Analog Ref.-Bsp. 2 erhält man ausgehend von 60 mg (0,17 mMol) H-[Phe^{NN}Leu]-H ·3HCl, 125 mg (0,50 mMol) Z-(L)-Valin, 221 mg (0,50 mMol) BOP, 67 mg (0,50 mMol) HOBt und 3,3 ml 0,3 M NMM in DMF nach chromatographischer Reinigung an Kieselgel mit Methylenchlorid/Ether (1:1) und Lyophilisation aus Dioxan die Titelverbindung. FAB-MS: (M+H)⁺= 718, t_{Ret} (I)=26,8 min, R_{f}(H)=0,38.

Das Ausgangsmaterial wird folgendermassen hergestellt:

### a) H-[Phe^{NN}Leu]-H · 3HCl:

Analog Ref.-Bsp. 10a erhält man aus 1,21 g (2,48 mMol) Boc-[Phe^{NN}Leu]-Boc aus Ref.-Bsp. 12 die Titelverbindung als Lyophilisat. FAB-MS: (M+H)⁺=252, t_{Ret}(II)=20,9 min , R_{f}(K)=0,23.

### Referenzbeispiel 14: H-(L)-Val-[Phe^{NN}Cha]←((L)-Val)-H · 3HCl:

Analog Ref.-Bsp. 10a erhält man aus 632 mg (0,91 mMol)

Boc-(L)-Val-[Phe^{NN}Cha]←((L)-Val)-Boc aus Ref.-Bsp. 10 nach Lyophilisation die Titelverbindung als Hydrochlorid. FAB-MS: (M+H)⁺=490, t_{Ret}(II)=29,4 min, R_{f}(K)=0,23.

### Referenzbeispiel 15: N-(3-Pyridylacetyl)-(L)-Val-[Phe^{NN}Leu]←(N-(3-pyridylacetyl)-(L)-Val):

Analog Ref.-Bsp. 9 erhält man aus 90 mg (0,25 mMol) H-[Phe^{NN}Leu]-H · 3 HCl aus Ref.-Bsp. 13 a), 358 mg (1,52 mMol) N-(3-Pyridylacetyl)-(L)-valin, 576 mg (1,52 mMol) HBTU und 316 µl (2,5 mMol) Triethylamin nach chromatographischer Reinigung mit Methylenchlorid/Methanol (15:1) und Lyophilisation aus Dioxan/tert-Butanol/Wasser die Titelverbindung. FAB-MS: (M+H)⁺= 688, t_{Ret}(IV)= 15,5 min, R_{f}(D)= 0,37.

### Referenzbeispiel 16: N-Trifluoracetyl-[Phe^{NN}(p-F)Phe]-Boc:

Eine Lösung von 4,0 g (15,4 mMol) 2(R)-[1'(S)-(Trifluoracetyl-amino)-2'-phenylethyl]-oxiran und 3,89 g (16,2 mMol) tert-Butyl-3-(p-fluorphenyl-methyl)-carbazat in 35 ml Methanol werden während ca. 20 h im Bombenrohr auf 80°C erhitzt. Das Reaktionsgemisch wird eingedampft, der Rückstand in wenig Dichlormethan gelöst und daraus die Titelverbindung mit Hexan gefällt (Kühlschrank). Weiteres Produkt liefert eine Säulenchromatographie (SiO₂, Methylenchlorid/Ether 95:7). DC R_{f}(J)=0,57; t_{Ret}(I)=24,3 min; FAB-MS (M+H)⁺=500.

Die Ausgangsmaterialien werden wie folgt hergestellt:

### a) N-3(S)-(Boc-amino)-2(R,S)-hydroxy-4-phenyl-1-trimethylsilyl-butan:

Unter N₂-Atmosphäre werden 24,7 g (1,02 Mol) Magnesium in 100 ml abs. Ether vor gelegt und über 35 min mit wenig Jod und gleichzeitig mit 132,5 ml (0,95 Mol) Chlormethyltrimethylsilan und 300 ml Ether versetzt, wobei die Temperatur mittels Eisbad bei 38°C gehalten wird. Das erhaltene Reaktionsgemisch wird dann 1,5 h bei RT gerührt. Nach Abkühlen auf -60°C wird mit einer Suspension von 48,6 g (0,195 Mol) N-Boc-phenylalaninal (Herstellung: D.J. Kempf, J. Org. Chem. 51, 3921 (1986)) in 1,1 l Ether innerhalb 40 min versetzt. Über 90 min wird das Reaktionsgemisch auf RT erwärmt und weitere 90 min bei dieser Temperatur gerührt. Danach wird auf 2 l Eiswasser und 1,5 l 10 %-ige wässrige Zitronensäure gegossen. Die abgetrennte wässrige Phase wird zweimal mit 500 ml Ether extrahiert. Alle Etherextrakte werden mit 500 ml 10 %-iger Zitronensäure und zweimal mit Sole gewaschen. Nach Trocknen über Natriumsulfat wird unter Vakuum eingeengt und die erhaltene Titelverbindung ohne zusätzliche Reinigung weiterverwendet. DC R_{f}(L)= 0,6; FAB-MS (M+H)⁺= 338.

### b) 1-Phenyl-3-buten-2(S)-amin:

Eine Lösung von 18,8 g (0,055 Mol) 3(S)-(Boc-amino)-2(R,S)-hydroxy-4-phenyl-1-trimethylsilyl-butan in 420 ml Methylenchlorid wird bei 5°C innerhalb von 10 min mit 35,6 ml (0,28 Mol) einer ungefähr 48 %-igen Lösung von Bortrifluorid in Ether versetzt. Das Reaktionsgemisch wird dann 16 h bei RT gerührt, auf 10°C gekühlt und innerhalb von 20 min mit 276 ml einer 4 N Natriumhydroxydlösung versetzt. Die wässrige Phase wird abgetrennt und zweimal mit je 400 ml Methylenchlorid extrahiert. Die vereinigten organischen Extrakte werden mit Sole gewaschen und über Natriumsulfat getrocknet. Das Titelprodukt wird ohne zusätzliche Reinigung weiterverwendet. DC R_{f} (C)= 0,15 ; IR (Methylenchlorid) (cm⁻¹): 3370, 3020, 2920, 1640, 1605.

### c) N-Trifluoracetyl-1-phenyl-3-buten-2(S)-amin:

Gelöst in 210 ml Methylenchlorid und 70 ml Pyridin, werden 11,9 g (81 mMol) 1-Phenyl-3-buten-2(S)-amin bei 0°C tropfenweise mit 17,0 ml (121 mMol) Trifluoressigsäureanhydrid versetzt. Nach 0,5 h Rühren bei 0 °C wird 2x mit verd. HCl, Wasser und Sole extrahiert. Die wässrigen Phasen wäscht man noch 2x mit Methylenchlorid, trocknet sie mit Natriumsulfat und dampft sie ein: DC R_{f}(M)=0,4.

### d) 2(R)-[1'(S)-(Trifluoracetyl-amino)-2'-phenylethyl]-oxiran:

Eine Lösung von 14,5 g (60 mMol) N-Trifluoracetyl-1-phenyl-3-buten-2(S)-amin in 600 ml Chloroform versetzt man mit 54,28 g (314 mMol) m-Chlorperbenzoesäure und rührt sie 24 h bei RT aus. Das Reaktionsgemisch wird 2x mit 10 %-iger Natriumsulfitlösung, 2x mit ges. Natriumcarbonatlösung , Wasser und Sole gewaschen. Die wässrigen Phasen werden noch 2x mit Methylenchlorid extrahiert, die vereinigten organischen Phasen mit Natriumsulfat getrocknet und eingedampft, was die Titelverbindung liefert, die ohne weitere Reinigung in der nächsten Stufe eingesetzt wird: DC R_{f}(N)=0,6.

### e) p-Fluorphenylcarbaldehyd-tert-butoxycarbonylhydrazon:

Analog Ref.-Bsp. 4 b) werden 32 g (242 mMol) tert-Butylcarbazat und 30 g (242 mMol) p-Fluorbenzaldehyd in 300 ml Ethanol bei 80°C während 3 h zur Titelverbindung umgesetzt, die beim Abkühlen und Verdünnen mit Wasser auskristallisiert: DC R_{f}(N)=0,48; t_{Ret}(I)=19,4 min.

### f) tert-Butyl-3-(p-fluorphenyl-methyl)-carbazat:

Analog Ref.-Bsp. 4 a) werden 55 g (231 mMol) p-Fluorphenylcarbaldehyd-tert-butoxycarbonylhydrazon in 500 ml THF mit 5,5 g Palladium (5 %) auf Kohle zur Titelverbindung hydriert: ¹H-NMR (200 MHz, CD₃OD): 7,35 (dd, 8 und 6 Hz, 2 H), 7,05 (t, 8 Hz, 2 H), 3,9 (s, 2 H), 1,45 (s, 9 H).

### Referenzbeispiel 17: N-Morpholinocarbonyl-(L)-Val-[Phe^{NN}(p-F)Phe]-Boc:

Eine Gemisch von 185 mg (0,80 mMol) N-Morpholinocarbonyl-(L)-valin (Herstellung siehe Ref.-Bsp. 7 a), 270 mg (0,67 mMol) H-[Phe^{NN}(p-F)Phe]-Boc, 311 mg (0,70 mMol) BOP und 95 mg (0,70 mMol) HOBT wird bei RT in 6,8 ml NMM/DMF 0,3 M gelöst und 5 h bei RT gerührt. Das Reaktionsgemisch wird am HV eingedampft und der Rückstand verteilt zwischen 4 Portionen Methylenchlorid, 2 Portionen 1 M Natriumcarbonatlösung, Wasser und Sole. Die über Natriumsulfat getrockneten vereinigten organischen Phasen werden eingedampft und durch Säulenchromatographie (SiO₂, Essigsäureethylester) gereinigt: DC R_{f}(O)=0,38; t_{Ret}(I)=21,8 min; FAB-MS (M+H)⁺=616.

Das Ausgangsmaterial wird wie folgt hergestellt:

### a) H-[Phe^{NN}(p-F)Phe]-Boc:

Bei 70°C tropft man zu einer Lösung von 0,3 g (0,6 mMol) N-Trifluoracetyl-[Phe^{NN}(p-F)-Phe]-Boc (Herstellung siehe Ref.-Bsp. 16) in 50 ml Methanol unter N₂-Atmosphäre 15 ml einer 1 M wässrigen Lösung von Kaliumcarbonat und rührt 25 h bei dieser Temperatur nach. Das Reaktionsgemisch wird am HV eingedampft, der Rückstand mit Methylenchlorid versetzt und 2x mit Wasser und Sole gewaschen. Die wässrigen Phasen werden 2x mit Methylenchlorid extrahiert, die organischen Phasen mit Natriumsulfat getrocknet und eingedampft. Das Rohprodukt setzt man ohne weitere Reinigung in der nächsten Stufe ein: t_{Ret}(I)=16,2 min.

### Referenzbeispiel 18: N-Morpholinocarbonyl-(L)-Val-[Phe^{NN}(p-F)Phe]←((L)-Val)-Z:

Eine Lösung von 86 mg (0,34 mMol) Z-(L)-Val und 160 mg (0,31 mMol) N-Morpholinocarbonyl-(L)-Val-[Phe^{NN}(p-F)Phe]-H in 2,7 ml NMM/CH₃CN 0,25 M (0,25 M NMM in CH₃CN) wird mit 129 mg (0,34mMol) HBTU versetzt. Nach 4 h bei RT dampft man ein und verteilt den Rückstand zwischen 3 Portionen Methylenchlorid, 2 Portionen ges. Natriumbicarbonatlösung und Sole. Trocknen der organischen Phasen mit Natriumsulfat und Eindampfen liefert die Titelverbindung, die nach Digerieren aus Methylenchlorid/Ether 1:1 rein erhalten wird: DC R_{f}(P)=0,4; t_{Ret}(I)=22,4 min; FAB-MS (M+H)⁺=749.

Das Ausgangsmaterial wird wie folgt hergestellt:

### a) N-Morpholinocarbonyl-(L)-Val-[Phe^{NN}(p-F)Phe]-H:

Gelöst in 105 ml Ameisensäure, werden 210 mg (0,34 mMol) N-Morpholinocarbonyl-(L)-Val-[Phe^{NN}(p-F)Phe]-Boc (Ref.-Bsp. 17) 4 h bei RT gerührt. Anschliessend wird eingedampft, der Rückstand in Methylenchlorid aufgenommen und die Lösung mit ges. Natriumbicarbonatlösung und Sole gewaschen. Extraktion der wässrigen Phasen mit 2 Portionen Methylenchlorid, Trocknen der organischen Phasen mit Natriumsulfat und Eindampfen liefert die Titelverbindung, die ohne weitere Reinigung in der nächsten Stufe eingesetzt wird: t_{Ret}(I)=12,9.

### Referenzbeispiel 19: N-Morpholinocarbonyl-(L)-Val-[Phe^{NN}(p-F)Phe]←((L)-Val)-H:

Bei Normaldruck werden 160 mg (0,21 mMol) N-Morpholinocarbonyl-(L)-Val-[Phe^{NN}(p-F)Phe]←((L)-Val)-Z (Ref.-Bsp. 18) in 6 ml Ethanol mit 40 mg Palladium (10 %) auf Kohle hydriert. Nach Filtration durch Celite® (Kieselgur, Filterhilfsmittel von Fluka, Buchs, Schweiz), Eindampfen und Lyophilisieren aus Dioxan erhält man die Titelverbindung: t_{Ret}(Hydrochlorid, I)=13,4 min; FAB-MS (M+H)⁺=615.

### Referenzbeispiel 20: N-Morpholinocarbonyl-(L)-Val-[Phe^{NN}(p-F)Phe]←((L)-Val)←(N-morpholinocarbonyl-Gly):

Eine Lösung von 26,9 mg (0,143 mMol) N-Morpholinocarbonyl-glycin und 80 mg (0,130 mMol) N-Morpholinocarbonyl-(L)-Val-[Phe^{NN}(p-F)Phe]←((L)-Val)-H in 1,1 ml NMM/CH₃CN 0,25 M wird mit 54 mg (0,143 mMol) HBTU versetzt und 16 h bei RT gerührt. Man dampft ein und verteilt den Rückstand zwischen 3 Portionen Essigsäure-ethylester, Wasser, 2 Portionen ges. Natriumbicarbonatlösung, Wasser und Sole. Trocknen der organischen Phasen mit Natriumsulfat und Eindampfen liefert die Titelverbindung, die nach Lösen in wenig DMF und Fällen mit DIPE rein anfällt: t_{Ret}(I)=15,1 min; FAB-MS (M+H)⁺=785.

Das Ausgangsmaterial wird wie folgt hergestellt:

### a) N-Morpholinocarbonyl-glycin-benzylester:

Analog Ref.-Bsp. 7 b) werden 7,69 g (22,8 mMol) Glycin-benzylester-4-toluolsulfonat und 2,8 g (19 mMol) (Morpholinocarbonyl)chlorid in 118 ml Methylenchlorid und 9 ml (53 mMol) N-Ethyldiisopropylamin während 18 h umgesetzt. Die Titelverbindung fällt nach Extraktion mit Methylenchlorid und Digerieren aus Hexan rein an: t_{Ret}(I)=11,6 min.

### b) N-Morpholinocarbonyl-glycin: Analog Ref.-Bsp. 7 a) werden 4,8 g (18,3 mMol) N-Morpholinocarbonyl-glycin-benzylester in 100 ml Essigsäureethylester mit 1 g Palladium (10 %) auf Kohle zur Titelverbindung hydriert: ¹H-NMR (300 MHz, CDCl₃): 3,88 (s, 2 H), 3,64 (s, 4 H), 3,50 (s, 2 H), 3,35 (s, 4 H).

### Referenzbeispiel 21: Z-(L)-Val-[Phe^{NN}(p-F)Phe]-Boc:

Eine Lösung von 335 mg (1,33 mMol) Z-(L)-Val und 448 mg (1,11 mMol) H-[Phe^{NN}(p-F)Phe]-Boc (Herstellung siehe Ref.-Bsp. 17 a) in 9,4 ml NMM/CH₃CN 0,25 M (0,25 M NMM in CH₃CN) wird mit 463 mg (1,22mMol) HBTU versetzt. Nach 16 h Rühren bei RT dampft man ein und verteilt den Rückstand zwischen 3 Portionen Methylenchlorid, 2 Portionen ges. Natriumbicarbonatlösung und Sole. Trocknen der organischen Phasen mit Natriumsulfat und Eindampfen liefert die Titelverbindung, die durch Säulenchromatographie (SiO₂, Hexan/Essigsäureethylester 4:1→1:1) gereinigt wird: t_{Ret}(I)=26,6 min; FAB-MS (M+H)⁺=637.

### Referenzbeispiel 22: Z-(L)-Val-[Phe^{NN}(p-F)Phe]←((L)-Val)-Boc:

Analog Ref.-Bsp. 18) werden 165 mg (0,76 mMol) Boc-(L)-Val und 371 mg (0,69 mMol) Z-(L)-Val-[Phe^{NN}(p-F)Phe]-H in 6 ml NMM/CH₃CN 0,25 M mit 289 mg (0,76 mMol) HBTU zur Titelverbindung umgesetzt, die direkt aus der Reaktionslösung kristallisiert und abfiltriert werden kann: t_{Ret}(I)=27,2 min; FAB-MS (M+H)⁺=736.

Das Ausgangsmaterial wird wie folgt hergestellt:

### a) Z-(L)-Val-[Phe^{NN}(p-F)Phe]-H:

Analog Ref.-Bsp. 18 a) werden 440 mg (0,69 mMol) Z-(L)-Val-[Phe^{NN}(p-F)Phe]-Boc mit 212 ml Ameisensäure zur Titelverbindung entschützt: t_{Ret}(I)=17,8 min.

### Referenzbeispiel 23: Z-(L)-Val-[Phe^{NN}(p-F)Phe]←((L)-Val)-H:

Analog Ref.-Bsp. 18 a) werden 250 mg (0,34 mMol) Z-(L)-Val-[Phe^{NN}(p-F)Phe]←((L)-Val)-Boc (Ref.-Bsp. 22) mit 50 ml Ameisensäure zur Titelverbindung entschützt: t_{Ret}(I)=18,0 min; FAB-MS (M+H)⁺=636.

### Referenzbeispiel 24: Z-(L)-Val-[Phe^{NN}(p-F)Phe]←((L)-Val)←(N-morpholinocarbonyl-Gly):

Analog Ref.-Bsp. 20) werden 32 mg (0,17 mMol) N-Morpholinocarbonyl-glycin (Ref.-Bsp. 20 b) und 99 mg (0,16 mMol) Z-(L)-Val-[Phe^{NN}(p-F)Phe]←((L)-Val)-H in 1,3 ml NMM/CH₃CN 0,25 M mit 65 mg (0,17 mMol) HBTU zur Titelverbindung umgesetzt, die direkt aus der Reaktionslösung kristallisiert: t_{Ret}(I)=21,1 min; FAB-MS (M+H)⁺=806.

### Referenzbeispiel 25: Z-(L)-Asn-[Phe^{NN}(p-F)Phe]-Boc:

Zu einer Lösung von 2,09 g (5,2 mMol) H-[Phe^{NN}(p-F)Phe]-Boc (Herstellung siehe Ref.-Bsp. 17 a) in 68 ml DMF und 2,7 ml (16 mMol) N-Ethyl-diisopropylamin gibt man 3,0 g (7,8 mMol) Z-(L)-Asparagin-p-nitrophenylester (Bachem, Bubendorf/Schweiz). Nach 16 h Rühren bei RT wird am HV eingedampft, der Rückstand in viel Methylenchlorid aufgenommen (schlecht löslich) und mit 2 Portionen 5 %-iger Kaliumcarbonatlösung gewaschen. Die wässrigen Phasen extrahiert man noch 2x mit viel Methylenchlorid, trocknet die vereinigten organischen Phasen mit Natriumsulfat und dampft sie ein. Nach Lösen des Rohproduktes in wenig Methanol und Fällen durch Zugabe von Toluol bei -20°C erhält man die Titelverbindung: t_{Ret}(I)=21,2 min.

### Referenzbeispiel 26: H-(L)-Asn-[Phe^{NN}(p-F)Phe]-Boc:

Analog Ref.-Bsp. 19) werden 0,40 g (0,61 mMol) Z-(L)-Asn-[Phe^{NN}(p-F)Phe]-Boc in 20 ml Methanol zur Titelverbindung hydriert: t_{Ret}(I)=14,9 min.

### Referenzbeispiel 27: Chinolin-2-carbonyl-(L)-Asn-[Phe^{NN}(p-F)Phe]-Boc:

Analog Ref.-Bsp. 17) werden 134 mg (0,78 mMol) Chinolin-2-carbonsäure (Fluka, Buchs/Schweiz) in 4 ml NMM/DMF 0,3 M mit 344 mg (0,78 mMol) BOP, 105 mg (0,78 mMol) HOBT und 268 mg (0,52 mMol) H-(L)-Asn-[Phe^{NN}(p-F)Phe]-Boc umgesetzt. Da laut HPLC nach 16 h Rühren bei RT noch H-(L)-Asn-[Phe^{NN}(p-F)Phe]-Boc vorhanden ist, werden nochmals 299 mg BOP, 70 mg HOBT, 89 mg Chinaldinsäure und 113 µl NMM zugesetzt. Nach weiteren 16 h wird eingedampft und der Rückstand zwischen 3 Portionen Methylenchlorid, 2 Portionen ges. Natriumbicarbonatlösung und Sole verteilt. Die vereinigten organischen Phasen werden mit Natriumsulfat getrocknet und eingedampft. Lösen des Rohproduktes in wenig DMF, Fällen mit DIPE und Kühlen auf -20°C liefert die Titelverbindung: t_{Ret}(I)=22,8 min; FAB-MS (M+H)⁺=673.

### Referenzbeispiel 28: Z-(L)-Asn-[Phe^{NN}(p-F)Phe]←((L)-Val)-Z:

88 mg (0,35 mMol) Z-(L)-Val in 3,8 ml NMM/DMF 0,3 N werden mit 153 mg (0,35 mMol) BOP und 47 mg (0,35 mMol) HOBT aktiviert und nach 15 min mit 144 mg (0,23 mMol) Z-(L)-Asn-[Phe^{NN}(p-F)Phe]-H·2 HCl versetzt. Nach 14 h Rühren bei RT dampft man das Reaktionsgemisch ein, löst den Rückstand in 2 ml Methanol und verteilt zwischen 3 Portionen Methylenchlorid und 2 Portionen 1 M Natriumcarbonatlösung, trocknet die organischen Phasen mit Natriumsulfat und dampft sie ein. Wiederholtes Lösen des Rohproduktes in wenig DMF und Ausfällen mit DIPE liefert die Titelverbindung: t_{Ret}(I)=22,2 min; FAB-MS (M+H)⁺=785.

Das Ausgangsmaterial wird wie folgt hergestellt:

### a) Z-(L)-Asn-[Phe^{NN}(p-F)Phe]-H·2 HCl:

Unter N₂-Atmosphäre wird eine Lösung von 150 mg (0,23 mMol) Z-(L)-Asn-[Phe^{NN}(p-F)Phe]-Boc (Ref.-Bsp. 25) in 1 ml Dioxan mit 2 ml HCl/Dioxan 4 N (Fluka, Buchs/Schweiz) versetzt. Nach 1,5 h Rühren bei RT nwird das Reaktionsgemisch lyophilisiert und das Lyophilisat sofort weiter umgesetzt.

### Referenzbeispiel 29: Trifluoracetyl-[Phe^{NN}(p-F)Phe]←((L)-Val)-Z:

Analog Ref.-Bsp. 17) werden 239 mg (0,95 mMol) Z-(L)-Val in 10,5 ml NMM/DMF 0,3 M mit 421 mg (0,95 mMol) BOP, 129 mg (0,95 mMol) HOBT und 0,3 g (0,63 mMol) N-Trifluoracetyl-[Phe^{NN}(p-F)Phe]-H während 15 h umgesetzt. Säulenchromatographie (SiO₂, Methylenchlorid/Ether 10:1) und Fällen aus DMF-Lösung mit DIPE liefert die Titelverbindung: DC R_{f}(Q)=0,15; t_{Ret}(I)=25,9 min; FAB-MS (M+H)⁺=633.

Das Ausgangsmaterial wird wie folgt hergestellt:

### a) N-Trifluoracetyl-[Phe^{NN}(p-F)Phe]-H:

Bei 0°C werden 0,20 g (0,40 mMol) N-Trifluoracetyl-[Phe^{NN}(p-F)Phe]-Boc (Herstellung siehe Ref.-Bsp. 16) in 5 ml Methylenchlorid mit 5 ml Trifluoressigsäure versetzt. Nach 4 h Rühren bei 0°C und 2 h bei RT wird das Reaktionsgemisch eingedampft. Lyophilisation des Rückstandes aus Dioxan liefert die Titelverbindung, die ohne Reinigung weiter umgesetzt wird: t_{Ret}(I)=14,7 min.

### Referenzbeispiel 30: Z-(L)-Asn-[Phe^{NN}Phe]-Boc:

Analog Ref.-Bsp. 25) werden 167 mg (0,34 mMol) H-[Phe^{NN}Phe]-Boc in 3,6 ml DMF und 0,18 ml (1 mMol) N-Ethyl-diisopropylamin mit 0,20 g (0,52 mMol) Z-(L)-Asparagin-p-nitrophenylester zur Titelverbindung umgesetzt, die nach Säulenchromatographie (SiO₂, Essigsäureethylester) rein anfällt: DC R_{f}(O)=0,19; t_{Ret}(I)=20,9 min.

Das Ausgangsmaterial wird wie folgt hergestellt:

### a) N-Trifluoracetyl-[Phe^{NN}Phe]-Boc:

Analog Ref.-Bsp. 16) werden 1,82 g (7,0 mMol) 2(R)-[1'(S)-(Trifluoracetyl-amino)-2'-phenylethyl]-oxiran (Ref.-Bsp. 16 d) und 1,58 g (7,1 mMol) tert-Butyl-3-benzyl-carbazat (J. Chem., Perkin I, 1712 (1975)) in 15 ml Methanol im Bombenrohr zur Titelverbindung umgesetzt, die durch Säulenchromatographie (SiO₂, Methylenchlorid/Ether 50:1) isoliert wird: DC R_{f}(J)=0,38; t_{Ret}(I)=24,5 min.

### b) H-[Phe^{NN}Phe]-Boc:

Analog Ref.-Bsp. 17 a) werden 258 mg (0,53 mMol) N-Trifluoracetyl-[Phe^{NN}Phe]-Boc in 60 ml Methanol mit 10,7 ml 1 M Kaliumcarbonatlösung zur Titelverbindung umgesetzt.

### Referenzbeispiel 31: Z-(L)-Val-[(p-F)Phe^{NN}(p-F)Phe]-Boc:

Analog Ref.-Bsp. 21) werden 18 mg (0,070 mMol) Z-(L)-Val und 27 mg (0,064 mMol) H-[(p-F)Phe^{NN}(p-F)Phe]-Boc in 0,6 ml NMM/CH₃CN 0,25 M mit 26,6 mg (0,070m Mol) HBTU zur Titelverbindung umgesetzt, die durch Lösen in wenig Methylenchlorid und Ausfällen mit DIPE gereinigt wird: FAB-MS (M+H)⁺=655.

Das Ausgangsmaterial wird wie folgt hergestellt:

### a) N-Boc-(p-fluorphenylalanin):

In 0,4 l Dioxan/Wasser 1:1 werden 20 g (109 mMol) p-Fluorphenylalanin (Fluka, Buchs, Schweiz) mit 35,5 g (163 mMol) Boc-anhydrid und 150 g (1,09 Mol) Kaliumcarbonat umgesetzt. Nach 4 h wird das Reaktionsgemisch mit Zitronensäurelösung angesäuert und mit 3 Portionen Essigsäureethylester extrahiert. Die organischen Phasen wäscht man mit 10 %-iger Zitronensäure, Wasser und Sole, trocknet sie mit Natriumsulfat und dampft sie ein. Lösen des Rückstands in wenig Methylenchlorid und Kristallisieren durch Zusatz von Hexan liefert die Titelverbindung: t_{Ret}(I)=16,9 min.

### b) N-Boc-(p-fluorphenylalaninol):

Bei -5°C bis -10°C versetzt man eine Lösung von 17,9 g (63 mMol) N-Boc-(p-fluorphenylalanin) in 73 ml abs. THF mit 9,66 ml (69 mMol) Triethylamin und tropft eine Lösung von 9,05 ml (69 mMol) Chlorameisensäureisobutylester in 44 ml abs. THF zu. Nach 0,5 h Rühren bei RT wird der gebildete Niederschlag abgenutscht. Das Filtrat tropft man unter Kühlen zu 4,77 g (126 mMol) Natriumborhydrid in 28 ml Wasser. Nach 4 h Rühren bei RT säuert man mit 10 %-iger Zitronensäure an, dampft das THF am RV teilweise ab und verteilt den Rückstand zwischen 3 Portionen Essigsäureethylester, 2 Portionen 2 N Natriumhydroxidlösung, Wasser, ges. Natriumhydrogencarbonatlösung und Sole. Die mit Natriumsulfat getrockneten und eingedampften organischen Phasen liefern nach Lösen in wenig Methylenchlorid und Kristallisieren durch Zugabe von Hexan die Titelverbindung: DC R_{f}(N)=0,36; t_{Ret}(I)=16,8 min; ¹H-NMR (200 MHz, CD₃OD): 7,24 (dd, 8 und 5 Hz, 2 H), 6,98 (t, 8 Hz, 2 H), 3,73 (m, 1 H), 3,47 (d, 5 Hz, 2 H), 2,88 (dd, 13 und 6 Hz, 1 H), 2,62 (dd, 13 und 8 Hz, 1 H), 1,36 (s, 9 H).

### c) N-Boc-(p-fluorphenylalaninal):

Unter N₂-Atmosphäre werden zu einer auf -60°C abgekühlten Lösung von 4,0 ml (46,8 mMol) Oxalylchlorid in 44 ml Methylenchlorid 4,44 ml (62,4 mMol) DMSO gelöst in 76 ml Methylenchlorid getropft. Nach 15 min Rühren fügt man der klaren Reaktionslösung 8,4 g (31,2 mMol) N-Boc-(p-fluorphenylalaninol) als Lösung in 185 ml Methylenchlorid/THF 1:1 (→ Ausfällung) zu und rührt 25 min nach. Anschliessend setzt man 17,3 ml (124,8 mMol) Triethylamin gelöst in 38 ml Methylenchlorid zu. Nach 30 min Rühren werden 278 ml einer 20 %-igen Kaliumhydrogensulfatlösung zugetropft, gefolgt von 220 ml Hexan. Man lässt auf RT aufwärmen, trennt die wässrige Phase ab und extrahiert sie mit 2 Portionen Ether. Die organischen Phasen ergeben nach Waschen mit ges. Natriumbicarbonatlösung, Wasser und Sole, Trocknen mit Natriumsulfat und Eindampfen die Titelverbindung, die ohne weitere Reinigung in der nächsten Stufe eingesetzt wird: ¹H-NMR (200 MHz, CDCl₃): 9,63 (s,1H), 6,9-7,2 (2m, 4H), 5,04 (m, 1H), 4,42 (m, 1H), 3,10 (m, 2H), 1,43 (s, 9H).

### d) N-3(S)-(Boc-amino)-2(R,S)-hydroxy-4-(p-fluorphenyl)-1-trimethylsilyl-butan:

Analog Ref.-Bsp. 16 a) werden 1,63 g (67 mMol) Magnesium in 33 ml abs. Ether, mit 8,3 ml (60 mMol) Chlormethyltrimethylsilan zur Grignard-Verbindung umgesetzt, die nach Reaktion mit 13 mMol N-Boc-(p-fluorphenylalaninal), Extraktion und Säulenchromatographie (SiO₂, Hexan/Essigsäureethylester 5:1 → 4:1) das Diastereomerengemisch der Titelverbindungen liefert: DC R_{f}(L)=0,32; t_{Ret}(I)=24,9 min (22 %)/ 25,5 min (78 %); FAB-MS (M+H)⁺=356.

### e) 1-(p-Fluorphenyl)-3-buten-2(S)-amin:

Analog Ref.-Bsp. 16 b) werden 1,1 g (3,1 mMol) N-3(S)-(Boc-amino)-2(R,S)-hydroxy-4-(p-fluorphenyl)-1-trimethylsilyl-butan in 22 ml Methylenchlorid mit 1,9 ml (15,5 mMol) einer ungefähr 48 %-igen Lösung von Bortrifluorid in Ether zur Titelverbindung umgesetzt: ¹H-NMR (300 MHz, CDCl₃): 7,2-7,10 und 7,05-6,9 (2m, je 2 H), 5,9-5,8 (m, 1 H), 5,2-5,0 (m, 2 H), 3,57 (m, 1 H), 2,79 (dd, 12 und 6 Hz, 1 H), 2,62 (dd, 12 und 8 Hz, 1 H), 1,7 (sb, 2 H).

### f) N-Trifluoracetyl-1-(p-fluorphenyl)-3-buten-2(S)-amin:

Analog Ref.-Bsp. 16 c) werden 364 mg (2,2 mMol) 1-(p-Fluorphenyl)-3-buten-2(S)-amin in 1,8 ml Methylenchlorid und 5,4 ml Pyridin mit 460 µl (3,3 mMol) Trifluoressigsäureanhydrid zur Titelverbindung umgesetzt, die nach Digerieren in Hexan rein anfällt: DC R_{f}(F)=0,58; MS (M)⁺=261.

### g) 2(R)-[1'(S)-(Trifluoracetyl-amino)-2'-(p-fluorphenyl)ethyl]-oxiran:

Analog Ref.-Bsp. 16 d) werden 359 mg (1,37 mMol) N-Trifluoracetyl-1-(p-fluorphenyl)-3-buten-2(S)-amin in 9 ml Chloroform mit 1,18 g (6,87 mMol) m-Chlorperbenzoesäure zur Titelverbindung oxidiert: DC R_{f}(R)=0,45.

### h) N-Trifluoracetyl-[(p-F)Phe^{NN}(p-F)Phe]-Boc:

Analog Ref.-Bsp. 16) werden 415 mg (1,49 mMol) 2(R)-[1'(S)-(Trifluoracetyl-amino)-2'-(p-fluorphenyl)ethyl]-oxiran und 377 mg (1,57 mMol) tert-Butyl-3-(p-fluorphenylmethyl)-carbazat in 9 ml Methanol zur Titelverbindung umgesetzt: DC R_{f}(S)=0,53; FAB-MS (M+H)⁺=518; ¹H-NMR (300 MHz, CD₃OD): 7,4-7,3 und 7,3-7,2 (2m, je 2 H), 7,05-6,9 (m, 4 H), 4,23 (m, 1 H), 3,90-3,65 (m, 3 H), 3,03-2,78 und 2,74-2,60 (2m, je 2 H), 1,30 (s, 9 H).

### i) H-[(p-F)Phe^{NN}(p-F)Phe]-Boc:

Analog Ref.-Bsp. 17 a) werden 285 mg (0,55 mMol) N-Trifluoracetyl-[(p-F)Phe^{NN}(p-F)Phe]-Boc in 45 ml Methanol mit 14 ml 1 M Kaliumcarbonatlösung zur Titelverbindung umgesetzt: t_{Ret}(I)=16,4 min.

### Referenzbeispiel 32: Z-(L)-Val-[(p-F)Phe^{NN}(p-F)Phe]-H:

Analog Ref.-Bsp. 18 a) werden 215 mg (0,33 mMol) Z-(L)-Val-[(p-F)Phe^{NN}(p-F)Phe]-Boc mit 100 ml Ameisensäure zur Titelverbindung entschützt: FAB-MS (M+H)⁺=555.

### Referenzbeispiel 33: Z-(L)-Val-[(p-F)Phe^{NN}(p-F)Phe]←(N-(N-(2-pyridylmethyl)-N-methyl-aminocarbonyl)-(L)-Val):

Analog Ref.-Bsp. 18) werden 23,6 mg (0,089 mMol) N-(N-(2-pyridylmethyl)-N-methylaminocarbonyl)-(L)-valin (Herstellung siehe EP 402646 A1, 19. Dez. 1990) und 45 mg (0,081 mMol) Z-(L)-Val-[(p-F)Phe^{NN}(p-F)Phe]-H mit 33,8 mg (0,089 mMol) HBTU in 0,76 ml NMM/CH₃CN 0,25 M zur Titelverbindung umgesetzt und mit DMF/DIPE umkristallisiert: DC R_{f}(O)=0,39; FAB-MS (M+H)⁺=802.

### Referenzbeispiel 34: Z-(L)-Val-[(p-F)Phe^{NN}(p-F)Phe]←(N-(2(R,S)carbamoyl-3-phenyl-propionyl)(L)-Val):

Analog Ref.-Bsp. 18) werden 26,0 mg (0,089 mMol) N-(2(R,S)-carbamoyl-3-phenyl-propionyl)-(L)-valin (Herstellung: Synth., Struct., Funct., Proc. Am. Pept. Symp., 7^{th}, 85, (1981)) und 45 mg (0,081 mMol) Z-(L)-Val-[(p-F)Phe^{NN}(p-F)Phe]-H (Ref.-Bsp. 32) mit 33,8 mg (0,089 mMol) HBTU in 0,76 ml NMM/CH₃CN 0,25 M zur Titelverbindung umgesetzt und mit DMF/DIPE umkristallisiert: R_{f}(P)=0,64; FAB-MS (M+H)⁺=829

### Referenzbeispiel 35: Acetyl-Val-[Phe^{NN}Phe]←(N-acetyl-Val):

Analog Ref.-Bsp. 7 erhält man aus 100 mg (0,25 mMol) H-[Phe^{NN}Phe]-H·3 HCl aus Ref.-Bsp. 2a, 121 mg (0,76 mMol) N-Acetyl-(L)-valin, 288 mg (0,76 mMol) HBTU und 0,211 ml (1,52 mMol) Triethylamin in DMF die Titelverbindung nach Lyophilisation aus Dioxan. FAB-MS: (M+H)⁺=568, t_{Ret}(I)=15,0 min., R_{f}(B)=0,46.

### Referenzbeispiel 36: Z-(D)-Val-[Phe^{NN}Phe]←((D)-Val)-Z:

Analog Ref.-Bsp. 2 erhält man aus 50 mg (0,123 mMol) H-[Phe^{NN}Phe]-H·3 HCl aus Ref.-Bsp. 2a, 95 mg (0,38 mMol) Z-(D)-Valin, 168 mg (0,38 mMol) BOP, 51 mg (0,38 mMol) HOBt und 2,53 ml 0,3M NMM in DMF die Titelverbindung nach Lyophilisation aus Dioxan. FAB-MS: (M+H)⁺=752, t_{Ret}(I)=26,4 min, R_{f} (H)=0,21.

### Referenzbeispiel 37: Chinolin-2-carbonyl-Val-[Phe^{NN}Phe]←(N-chinolin-2-carbonyl-Val):

Man löst 145 mg (0,53 mMol) N-(Chinolin-2-carbonyl)-(L)-valin, 235 mg (0,53 mMol) BOP und 72 mg (0,53 mMol) HOBt in 3,5 ml einer 0,3 M Lösung von NMM in DMF, fügt nach 10 min 70 mg (0,18 mMol) H-[Phe^{NN}Phe]-H·HCl (Ref.-Bsp. 2a) zu und rührt während 5 h bei RT unter Stickstoffatmosphäre. Die Reaktionsmischung wird eingedampft, der Rückstand in Methylenchlorid gelöst und zweimal mit ges. Natriumbicarbonatlösung, einmal mit 10 % Zitronensäure und erneut einmal mit ges. Natriumbicarbonatlösung gewaschen. Die organischen Phasen werden durch Watte filtriert, eingedampft und der Rückstand zweimal aus Methylenchlorid/Methanol durch Zugabe von DIPE ausgefällt. Nach Lyophilisation aus Dioxan erhält man die Titelverbindung als weissen Festkörper (Gemisch von zwei im HPLC unterscheidbaren Diastereomeren). FAB-MS: (M+H)⁺=794, t_{Ret}(A)=29,1 und 29,3 min, R_{f}(B)=0,81.

### a) N-(Chinolin-2-carbonyl)-(L)-valin:

Eine Lösung von 2,5 g (14,5 mMol) (L)-Valyl-tert-butylester und 2,5 g (14,5 mMol) Chinolin-2-carbonsäure in 100 ml Methylenchlorid/THF (10:1) wird mit 3,28 g (15,9 mMol) N,N-Dicyclohexylcarbodiimid und 2,0 ml (14,5 mMol) Triethylamin versetzt und während 18h bei RT gerührt. Die Reaktionsmischung wird auf -18° abgekühlt und vom Harnstoff abfiltriert. Das Filtrat wird eingedampft, der Rückstand in Methylenchlorid gelöst und je einmal mit ges. Natriumbicarbonatlösung und Wasser gewaschen. Die organischen Phasen werden durch Watte filtriert, eingedampft und ergeben nach chromatographischer Reinigung auf Kieselgel mit Hexan/Essigsäureethylester (2:1) N-(Chinolin-2-carbonyl)-(L)-valyl-tert-butylester. 2,59 g (12,2 mMol) davon werden während 4,5h bei RT in Methylenchlorid/TFA (1:1) belassen. Nach dem Eindampfen wird der Rückstand durch Chromatographie auf Kieselgel mit Hexan/Essigsäureethylester (2:1) gereinigt. Die produkthaltigen Fraktionen werden eingedampft, erneut in Methylenchlorid gelöst und durch Waschen mit 1N Natronlauge und 1N Salzsäure in das Hydrochlorid der Titelverbindung übergeführt. ¹H-NMR (200 MHz, CD₃OD): 1,05 und 1,07 (2d, J=6Hz, 6H) 2,40 (m, 1H) 4,65 (m, 1H) 7,70 (m, 1H) 7,85 (m, 1H) 8,00 (dxd, 1H) 8,20 (m, 2H) 8,48 (d, 1H).

### Referenzbeispiel 38: Acetyl-(L)-Val-[Phe^{NN}Cha]←(N-acetyl-(L)-Val):

Analog Ref.-Bsp. 37 erhält man aus 160 mg (0,40 mMol) H-[Phe^{NN}Cha]-H·3 HCl aus Ref.-Bsp. 10a, 190 mg (1,19 mMol) N-Acetyl-(L)-valin, 525 mg (1,19 mMol) BOP, 160 mg (1,19 mMol) HOBt und 7,9 ml 0,3M NMM in DMF die Titelverbindung nach Fällung aus Chloroform/Methanol mit DIPE und Lyophilisation aus Dioxan. FAB-MS: (M+H)⁺=574, t_{Ret}(I)=18,1 min, R_{f} (B)=0,30.

### Referenzbeispiel 39: N-(3-Pyridylacetyl)-(L)-Val-[Phe^{NN}Cha]←(N-(3-pyridylacetyl)-(L)-Val) ·3 HCl:

Analog Ref.-Bsp. 7 erhält man aus 100 mg (0,25 mMol) H-[Phe^{NN}Cha]-H·3 HCl aus Ref.-Bsp. 10a, 358 mg (1,52 mMol) N-(3-Pyridylacetyl)-(L)-valin aus Ref.-Bsp. 9a, 576 mg (1,52 mMol) HBTU und 0,316 ml (2,28 mMol) Triethylamin in DMF die Titelverbindung nach chromatographischer Reinigung auf Kieselgel mit Methylenchlorid/Methanol (15:1) und Lyophilisation der produkthaltigen Fraktionen aus Dioxan. FAB-MS: (M+H)⁺=728, t_{Ret}(I)=11,3 min, R_{f} (U)=0,21.

### Referenzbeispiel 40: Acetyl-Ile-[Phe^{NN}Cha]←(N-acetyl-Ile):

Analog Ref.-Bsp. 37 erhält man aus 160 mg (0,40 mMol) H-[Phe^{NN}Cha]-H·3 HCl aus Ref.-Bsp. 10a, 206 mg (1,19 mMol) N-Acetyl-(L)-isoleucin, 525 mg (1,19 mMol) BOP, 160 mg (1,19 mMol) HOBt und 7,9 ml 0,3M NMM in DMF die Titelverbindung nach Ausfällen aus Methylenchlorid/Methanol durch Zugabe von DIPE und Lyophilisation aus Dioxan/tert-Butanol (Gemisch von 2 im HPLC unterscheidbaren Diasteromeren). FAB-MS: (M+H)⁺=602, t_{Ret}(I)=20,4 und 20,7 min, R_{f} (D)=0,33.

### Referenzbeispiel 41: Thiomorpholinocarbonyl-(L)-Val-[Phe^{NN}Cha]←(N-thiomorpholinocarbonyl-(L)-Val):

Analog Ref.-Bsp. 6 erhält man ausgehend von 70 mg (0,12 mMol) H-(L-Val)-[Phe^{NN}Cha]←(N-(L)-Val)-H·3 HCl aus Ref.-Bsp. 14, 58 mg (0,35 mMol) (4-Thiomorpholinylcarbonyl)chlorid aus Ref.-Bsp. 6a und 0,127 ml Triethylamin in 2 ml DMF nach chromatographischer Reinigung auf Kieselgel mit Methylenchlorid/Methanol (95:5) und Lyophilisation der produkthaltigen Fraktionen aus Dioxan die Titelverbindung. FAB-MS: (M+H)⁺=748, t_{Ret}(I)=24,0 min, R_{f} (B)=0,70.

### Referenzbeispiel 42: Z-(L)-Glu-[Phe^{NN}(p-F)Phe]←((L)-Glu)-Z:

Man rührt eine Lösung von 130 mg (0,14 mMol) Z-(L)-Glu(O-tert-butyl)-[Phe^{NN}(p-F)Phe]←((L)-Glu(O-tert-butyl))-Z [(Glu(O-tert-butyl) bedeutet hierin das an der γ-Carboxygruppe mit einem tert-Butylrest veresterte Radikal von Glutaminsäure] in 8 ml Methylenchlorid/TFA (1:1) während 3 h bei RT Das Lösungsmittel wird unter vermindertem Druck abgedampft und der Rückstand aus Methylenchlorid durch Zugabe von DIPE ausgefällt. Man erhält die Titelverbindung nach Lyophilisation aus Dioxan/tert-Butanol. FAB-MS: (M+H)⁺=830, t_{Ret}(I)=19,6 min, R_{f} (B)=0,32.

### a) Z-(L)-Glu(O-tert-butyl)-[Phe^{NN}(p-F)Phe]←((L)-Glu(O-tert-butyl))-Z:

Analog Ref.-Bsp. 37 erhält man aus 100 mg (0,24 mMol) H-[Phe^{NN}(p-F)Phe]-H·3HCl, 245 mg (0,73 mMol) Z-(L)-Glutaminsäure-tert-butylester, 321 mg (0,73 mMol) BOP, 98 mg (0,73 mMol) HOBt und 4,8 ml 0,3M NMM in DMF die Titelverbindung nach chromatographischer Reinigung auf Kieselgel mit Methylenchlorid/Ether (1:1). t_{Ret}(I)=30,2 min, R_{f}(H)=0,17.

### b) H-[Phe^{NN}(p-F)Phe]-H·3 HCl:

Analog Ref.-Bsp. 2a erhält man ausgehend von 1,77 g (3,51 mMol) Boc-[Phe^{NN}(p-F)Phe]-Boc nach Lyophilisation die Titelverbindung. FAB-MS: (M+H)⁺=304, R_{f} (K)=0,19.

### c) Boc-[Phe^{NN}(p-F)Phe]-Boc:

Analog Ref.-Bsp. 1 erhält man ausgehend von 2,0 g (7,60 mMol) (2R)-[1'(S)-Boc-amino-2'-phenylethyl]oxiran und 2,17 g (9,04 mMol) tert-Butyl-3-(4-fluorphenyl-methyl)-carbazat aus Ref.-Bsp. 16 f die Titelverbindung nach chromatographischer Reinigung auf Kieselgel mit Hexan/Essigsäureethylester (2:1). FAB-MS: (M+H)⁺=504, t_{Ret}(I)=26,2 min, R_{f} (F)=0,26.

### Referenzbeispiel 43: N-(2-Pyridylmethyl)-N-methyl-aminocarbonyl-(L)-Val-[Phe^{NN}(p-F)-Phe]←(N-(N-(2-pyridylmethyl)-N-methyl-aminocarbonyl)-(L)-Val):

Analog Ref.-Bsp. 37 erhält man aus 70 mg (0,17 mMol) H-[Phe^{NN}(p-F)Phe]-H·3 HCl aus Ref.-Bsp. 42b, 135 mg (0,51 mMol) N-(N-(2-Pyridylmethyl)-N-methyl-aminocarbonyl)-(L)-valin (Herstellung wie in EP 0 402 646 A1 vom 19. Dec. 1990 beschrieben), 225 mg (0,51 mMol) BOP, 69 mg (0,51 mMol) HOBt und 3,4 ml 0,3M NMM in DMF die Titelverbindung nach Chromatographie auf Kieselgel mit Methylenchlorid/Methanol (15:1) und Lyophilisation der produkthaltigen Fraktionen aus Dioxan. FAB-MS: (M+H)⁺=798, t_{Ret}(IV)=35 min, R_{f} (U)=0,21.

### Referenzbeispiel 44: N-(3-(Tetrazol-1-yl)-propionyl)-Val-[Phe^{NN}(p-F)-Phe]←(N-(3-(tetrazol-1-yl)-propionyl)-Val):

Analog Ref.-Bsp. 37 erhält man aus 100 mg (0,24 mMol) H-[Phe^{NN})p-F)Phe]-H·3 HCl (aus Ref.-Bsp. 42b), 146 mg (0,61 mMol) N-(3-(Tetrazol-1-yl)-propionyl)-(L)-valin, 268 mg (0,61 mMol) BOP, 82 mg (0,61 mMol) HOBt und 4 ml 0,3M NMM in DMF die Titelverbindung nach Ausfällung aus Methylenchlorid durch Zugabe von DIPE und Lyophilisation aus Dioxan (im HPLC 4 Diastereomere unterscheidbar). FAB-MS: (M+H)⁺=750, t_{Ret}(III)=30,8; 31,4; 32,4 und 32,8 min, R_{f} (K)=0,5.

### a) N-(3-(Tetrazol-1-yl)-propionyl)-(L)-valin:

Analog Ref.-Bsp. 9b erhält man ausgehend von 4 g (16,4 mMol) (L)-Valin-benzylester·HCl, 2,1 g (14,9 mmol) 3-(Tetrazol-1-yl)-propionsäure (Herstellung: US 4,794,109 A vom 27. Dec. 1988), 2,4 ml Cyanphosphonsäure-diethylester und 4,4 ml Triethylamin in DMF nach chromatographischer Reinigung auf Kieselgel mit Methylenchlorid/Methanol (30:1) N-(3-(Tetrazol-1-yl)-propionyl)-(L)-valin-benzylester. 2,66 g (8,03 mMol) davon werden in Methanol/Wasser (9:1) in Gegenwart von 530 mg 10 % Palladium auf Kohle bei 1 atm Wasserstoffdruck hydriert und ergeben nach Fällung aus Methanol/DIPE die Titelverbindung. ¹H-NMR (200 MHz, CD₃OD): 0,9 (d, J=7Hz, 6H) 2,1 (m, 1H) 2,95 (m, 2H) 4,29 (d, J=6Hz, 1H) 4,78 (m, 2H) 9,15 (s, 1H).

### Referenzbeispiel 45: Z-(L)-Val-[Phe^{NN}(p-F)Phe]←((L)-Val)-Z:

Analog Ref.-Bsp. 37 erhält man aus 100 mg (0,24 mMol) H-[Phe^{NN}(p-F)Phe]-H·3 HCl (aus Ref.-Bsp. 42b), 182 mg (0,38 mMol) Z-(L)-Valin, 321 mg (0,73 mMol) BOP, 98 mg (0,73 mMol) HOBt und 4,8 ml 0,3M NMM in DMF die Titelverbindung nach Ausfällen aus Methylenchlorid durch Zugabe von DIPE und Lyophilisation aus Dioxan. FAB-MS: (M+H)⁺=770, t_{Ret}(I)=26,3 min, R_{f} (H)=0,25.

### Referenzbeispiel 46: Acetyl-Val-[Phe^{NN}(p-F)Phe]←(N-acetyl-Val):

Analog Ref.-Bsp. 37 erhält man aus 80 mg (0,19 mMol) H-[Phe^{NN}(p-F)Phe]-H·3 HCl aus Ref.-Bsp. 42b), 124 mg (0,78 mMol) N-Acetyl-(L)-valin, 344 mg (0,78 mMol) BOP, 105 mg (0,76 mMol) HOBt und 4,5 ml 0,3M NMM in DMF die Titelverbindung nach zweimaliger Umfällung aus Methylenchlorid/Methanol durch Zugabe von DIPE und Lyophilisation aus Dioxan/tert-Butanol. FAB-MS: (M+H)⁺=586, t_{Ret}(I)=15,8 min, R_{f} (E)=0,32.

### Referenzbeispiel 47: Acetyl-Val-[Phe^{NN}(p-CN)Phe]←(N-acetyl-Val):

Analog Ref.-Bsp. 37 erhält man aus 80 mg (0,19 mMol) H-[Phe^{NN}(p-CN)Phe]-H·3 HCl, 124 mg (0,78 mMol) N-Acetyl-(L)-valin, 344 mg (0,78 mMol) BOP, 105 mg (0,78 mMol) HOBt und 4,5 ml 0,3M NMM in DMF die Titelverbindung nach Fällung aus Methylenchlorid/Methanol durch Zugabe von DIPE und Lyophilisation aus Dioxan als Gemisch von 2 im HPLC unterscheidbaren Diastereomeren. FAB-MS: (M+H)⁺=593, t_{Ret}(I)=14,4 und 14,6 min, R_{f} (D)=0,39.

### a) H-[Phe^{NN}(p-CN)Phe]-H·3 HCl:

Analog Ref.-Bsp. 2a erhält man ausgehend von 2,69 g (5,27 mMol) Boc-[Phe^{NN}(p-CN)Phe]-Boc nach Lyophilisation die Titelverbindung. FAB-MS: (M+H)⁺=311, R_{f} (K)=0,16.

### b) Boc-[Phe^{NN}(p-CN)Phe]-Boc:

Analog Ref.-Bsp. 1 erhält man ausgehend von 2,0 g (7,60 mMol) (2R)-[1'(S)-Boc-amino-2'-phenylethyl]oxiran und 1,87 g (7,6 mMol) tert-Butyl-3-(4-cyanphenyl-methyl)-carbazat die Titelverbindung nach Kristallisation aus Methanol/DIPE. FAB-MS: (M+H)⁺=511, t_{Ret}(I)=25 min, R_{f} (Y)=0,19.

### c) tert-Butyl-3-(4-cyanphenyl-methyl)-carbazat:

Analog Ref.-Bsp. 4b werden 10 g (76,3 mMol) 4-Cyanbenzaldehyd und 10 g (76,3 mMol) tert-Butylcarbazat in Ethanol zum 4-Cyanphenylcarbaldehyd-tert-butoxycarbonylhydrazon umgesetzt. 11,1 g davon werden in 150 ml THF in Gegenwart von 2 g Palladium auf Kohle 10% bei 2 atm Wasserstoffdruck hydriert und ergeben die Titelverbindung. ¹H-NMR (200 MHz, CDCl₃): 7,65 (d, J=8Hz, 2H), 7,45 (d, J=8 Hz, 2H), 6,08 (s, br, 1H), 4,3 (s, br, 1H), 4,02 (s, 2H), 1,45 (s, 9H).

### Referenzbeispiel 48: Z-(L)-Val-[Phe^{NN}(p-CN)Phe]←((L)-Val)-Z:

Analog Ref.-Bsp. 37 erhält man aus 70 mg (0,17 mMol) H-[Phe^{NN}(p-CN)Phe]-H·3 HCl (aus Ref.-Bsp. 47a), 125 mg (0,5 mMol) Z-(L)-Valin, 221 mg (0,5 mMol) BOP, 68 mg (0,5 mMol) HOBt und 3,33 ml 0,3M NMM in DMF die Titelverbindung nach Fällung aus Methylenchlorid durch Zugabe von Hexan und Lyophilisation aus Dioxan. FAB-MS: (M+H)⁺=777, t_{Ret}(I)=25,3 min, R_{f} (D)=0,69.

### Referenzbeispiel 49: Z-(L)-Ile-[Phe^{NN}Leu]←((L)-Ile)-Z:

Analog Ref.-Bsp. 37 erhält man aus 70 mg (0,19 mMol) H-[Phe^{NN}Leu]-H·3 HCl (aus Ref.-Bsp. 13a), 154 mg (0,58 mMol) Z-(L)-Isoleucin, 257 mg (0,58 mMol) BOP, 79 mg (0,58 mMol) HOBt und 3,88 ml 0,3M NMM in DMF die Titelverbindung nach Chromatographie auf Kieselgel mit Methylenchlorid/Ether (3:1) und Fällung der produkthaltigen Fraktionen aus Methylenchlorid/DIPE und Lyophilisation aus Dioxan. FAB-MS: (M+H)⁺=746, t_{Ret}(I)=28,2 min, R_{f} (H)=0,39.

### Referenzbeispiel 50: Isobutoxycarbonyl-(L)-Val-[Phe^{NN}Leu]←(N-isobutoxycarbonyl-(L)-Val):

Analog Ref.-Bsp. 37 erhält man aus 70 mg (0,19 mMol) H-[Phe^{NN}Leu]-H·3 HCl (aus Ref.-Bsp. 13a), 130 mg (0,58 mMol) N-(Isobutoxycarbonyl)-(L)-valin, 256 mg (0,58 mMol) BOP, 78 mg (0,58 mMol) HOBt und 3,9 ml 0,3M NMM in DMF die Titelverbindung nach Chromatographie auf Kieselgel mit Methylenchlorid/Ether (1:1) und Lyophilisation der produkthaltigen Fraktionen aus Dioxan. FAB-MS: (M+H)⁺=650, t_{Ret}(I)=26,4 min, R_{f} (H)=0,38.

### a) N-(Isobutoxycarbonyl)-(L)-valin:

Eine Lösung von 10 g (85,3 mMol) (L)-Valin in 100 ml 2N Natronlauge wird mit 11,2 ml (85,3 mMol) Isobutylchloroformiat versetzt und bei RT während 18h gerührt. Die Reaktionslösung wird mit Methylenchlorid gewaschen, mit 4N Salzsäure angesäuert und mit Methylenchlorid extrahiert. Die organischen Extrakte werden mit Sole gewaschen, durch Watte filtriert und ergeben nach dem Eindampfen die Titelverbindung als farbloses Harz. ¹H-NMR (200 MHz, CD₃OD): 0,95 (m, 12H) 1,9 (m, 1H) 2,15 (m, 1H) 3,85 (d, J=7Hz, 2H) 4,05 (d breit, 1H).

### Referenzbeispiel 51: N-(3-(Tetrazol-1-yl)-propionyl)-(L)-Val-[Phe^{NN}Leu]←(N-3-(tetrazol-1-yl)-propionyl-(L)-Val):

Analog Ref.-Bsp. 37 erhält man aus 150 mg (0,42 mMol) H-[Phe^{NN}Leu]-H·3 HCl (aus Ref.-Bsp. 13a), 251 mg (1,04 mMol) N-(3-(Tetrazol-1-yl-propionyl)-(L)-valin aus Ref.-Bsp. 44a, 460 mg (1,04 mMol) BOP, 140 mg (1,04 mMol) HOBt und 6,9 ml 0,3M N-Methylmorpholin in DMF die Titelverbindung nach Fällung aus Methylenchlorid/DIPE und Lyophilisation aus Dioxan/tert-Butanol/Wasser. FAB-MS: (M+H)⁺=689, t_{Ret}(I)=14,7 min, R_{f} (K)=0,36.

### Referenzbeispiel 52: Acetyl-Val-[Phe^{NN}Leu]←(N-acetyl-Val):

Analog Ref.-Bsp. 37 erhält man aus 70 mg (0,19 mMol) H-[Phe^{NN}Leu]-H·3 HCl (aus Ref.-Bsp. 13a), 184 mg (1,16 mMol) N-Acetyl-(L)-valin, 512 mg (1,16 mMol) BOP, 156 mg (1,16 mMol) HOBt und 7,8 ml 0,3M NMM in DMF die Titelverbindung nach Fällung aus Methylenchlorid/Methanol durch Zugabe von DIPE und Lyophilisation aus Dioxan/tert-Butanol/Wasser (gemäss HPLC 2 Diasteromere unterscheidbar). FAB-MS: (M+H)⁺=534, t_{Ret}(I)=14,7 und 15,1 min, R_{f} (D)=0,35.

### Referenzbeispiel 53: Boc-(L)-Val-[Phe^{NN}Leu]←((L)-Val)-Boc:

Analog Ref.-Bsp. 7 erhält man aus 300 mg (0,83 mMol) H-[Phe^{NN}Leu]-H·3 HCl (aus Ref.-Bsp. 13a), 722 mg (3,33 mMol) Boc-(L)-valin, 1,262 g (3,33 mMol) HBTU und 0,927 ml (6,66 mMol) Triethylamin in DMF die Titelverbindung nach chromatographischer Reinigung auf Kieselgel mit Methylenchlorid/Ether (1:1), Fällung der produkthaltigen Fraktionen und Lyophilisation aus Dioxan. FAB-MS: (M+H)⁺=650, t_{Ret}(I)=26,3 min, R_{f} (H)=0,64.

### Ref.-Bsp. 54: H-(L)-Val-[Phe^{NN}Leu]←((L)-Val)-H·3 HCl:

Analog Ref.-Bsp. 5 erhält man aus 396 mg (0,61 mMol) Boc-(L)-Val-[Phe^{NN}Leu]←((L)-Val(-Boc aus Ref.-Bsp. 53 und 10 ml 4N Chlorwasserstoff in Dioxan nach Lyophilisation der Reaktionslösung die Titelverbindung. FAB-MS: (M+H)⁺=450, t_{Ret}(II)=24,1 min, R_{f} (K)=0,25.

### Referenzbeispiel 55: N-Thiomorpholinocarbonyl-(L)-Val-[Phe^{NN}Leu]←(N-thiomorpholinocarbonyl(L)-Val):

Analog Ref.-Bsp. 6 erhält man ausgehend von 100 mg (0,16 mMol) H-(L)-Val-[Phe^{NN}Leu]←(L)-Val-H·3 HCl, 78,5 mg (0,47 mMol) (4-Thiomorpholinyl-carbonyl)chlorid aus Ref.-Bsp. 6a und 0,172 ml Triethylamin in DMF nach chromatographischer Reinigung auf Kieselgel mit Methylenchlorid/Methanol (95:5), Fällung der produkthaltigen Fraktionen aus Methylenchlorid/Hexan und Lyophilisation aus Dioxan die Titelverbindung als amorphen Festkörper. FAB-MS: (M+H)⁺=708, t_{Ret}(I)=21,4 min, R_{f} (E)=0,45.

### Referenzbeispiel 56: 2(R,S)-Tetrahydrofuryl-methoxycarbonyl-(L)-Val-[Cha^{NN}Leu]←(N-2(R,S)-tetrahydrofuryl-methoxycarbonyl-(L)-Val):

Analog Ref.-Bsp. 37 erhält man aus 80 mg (0,22 mMol) H-[Cha^{NN}Leu]-H·3 HCl, 160 mg (0,65 mMol) N-(2(R,S)-Tetrahydrofuryl-methoxycarbonyl)-(L)-valin, 289 mg (0,65 mMol) BOP, 88 mg (0,65 mMol) HOBt und 4,35 ml 0,3M NMM in DMF die Titelverbindung nach chromatographischer Reinigung auf Kieselgel mit Essigsäureethylester und Lyophilisation der produkthaltigen Fraktionen aus Dioxan. FAB-MS: (M+H)⁺=712, t_{Ret}(I)=22,4 min, R_{f} (E)=0,21.

### a) H-[Cha^{NN}Leu]-H·3 HCl:

Analog Ref.-Bsp. 5 erhält man aus 150 mg (0,33 mMol) Boc-[Cha^{NN}Leu]-Boc und 10 ml 4N Chlorwasserstoff in Dioxan nach Lyophilisation der Reaktionslösung 100 mg (83 %) der Titelverbindung. R_{f} (K)=0,26.

### b) Boc-[Cha^{NN}Leu]-Boc

Eine Lösung von 200 mg (0,24 mMol) Boc-[Phe^{NN}Leu]-Boc (Ref.-Bsp. 12) in 15 ml Methanol wird in Gegenwart von 10 mg Nishimura-Katalysator (Rh(III)- und Pt(IV)-Oxid Monohydrat, Degussa) bei 1 atm Wasserstoffdruck während 4 h hydriert. Man filtriert vom Katalysator ab, dampft das Lösungsmittel vollständig ein und erhält die Titelverbindung nach Kristallisation aus Methylenchlorid/Hexan. t_{Ret}(I)=26,7 min, R_{f} (V)=0,21.

### c) N-(2(R,S)-Tetrahydrofuryl-methoxycarbonyl)-(L)-valin:

Analog Ref.-Bsp. 50a erhält man aus 7 g (60 mMol) (L)-Valin und 9,8 g (60 mMol) 2(R,S)-Tetrahydrofurylmethyl-chloroformiat (Heterocycles 27, 1155 (1988)) in 100 ml 2N Natronlauge und 30 ml Dioxan die Titelverbindung als Gemisch von 2 Diastereomeren. t_{Ret}(II)=23,5 und 23,8 min

### Referenzbeispiel 57: Z-Val-[Phe^{NN}Leu]←(N-(3-(tetrazol-1-yl)-propionyl)-Val):

Analog Ref.-Bsp. 37 erhält man aus 100 mg (0,21 mMol) Z(L)-Val-[Phe^{NN}Leu]-H, 75 mg (0,31 mMol) N-(3-(Tetrazol-1-yl)-propionyl)-(L)-valin aus Ref.-Bsp. 44a, 137 mg (0,31 mMol) BOP, 42 mg (0,31 mMol) HOBt und 2 ml 0,3M NMM in DMF die Titelverbindung nach Fällung aus Methylenchlorid/Hexan und Lyophilisation aus Dioxan/tert-Butanol (2 Diasteromere im HPLC unterscheidbar). FAB-MS: (M+H)⁺=708, t_{Ret}(I)=21,1 und 21,1 min, R_{f} (D)=0,45.

### a) Z-(L)-Val-[Phe^{NN}Leu]-H:

Eine Lösung von 250 mg (0,43 mMol) Z-(L)-Val-[Phe^{NN}Leu]-Boc in 5 ml Ameisensäure wird während 7,5 h bei RT gerührt. Nach dieser Zeit ist bei HPLC-Analyse kein Ausgangsmaterial mehr erkennbar (t_{Ret}(I)=27,5 min), und die Reaktionslösung wird eingedampft. Der Rückstand wird in Chloroform gelöst und mit ges. Natriumbicarbonatlösung gewaschen. Die Chloroform-Phase wird durch Watte filtriert und ergibt die rohe Titelverbindung nach Abdampfen des Lösungsmittels. t_{Ret}(I)=16,7 min, R_{f} (K)=0,21.

### b) Z-(L)-Val-[Phe^{NN}Leu]-Boc:

Analog Ref.-Bsp. 37 erhält man aus 230 mg (0,653 mMol) H-[Phe^{NN}Leu]-Boc, 247 mg (0,98 mMol) Z-(L)-Valin, 434 mg (0,98 mMol) BOP, 133 mg (0,98 mMol) HOBt und 6,5 ml 0,3M NMM in DMF die Titelverbindung nach Fällung aus Methylenchlorid/Methanol durch Zugabe von DIPE. FAB-MS: (M+H)⁺=585, t_{Ret}(I)=27,5 min, R_{f} (C)=0,71.

### c) H-[Phe^{NN}Leu]-Boc:

Analog Ref.-Bsp. 17a erhält man ausgehend von 1,27 g (2,84 mMol) N-Trifluoracetyl-[Phe^{NN}Leu]-Boc und 24 ml 1N wässriger Natriumcarbonatlösung in 90 ml Methanol die Titelverbindung, welche aus Methylenchlorid durch Zugabe von DIPE gefällt wird. t_{Ret}(I)=14,9 min, R_{f} (K)=0,38.

### d) N-Trifluoracetyl-[Phe^{NN}Leu]-Boc:

Analog Ref.-Bsp. 16 erhält man ausgehend von 3 g (11,57 mMol) 2(R)-[1'(S)-(Trifluoracetyl-amino)-2'-phenylethyl]-oxiran aus Ref.-Bsp. 16d und 2,3 g (12,15 mMol) tert-Butyl-3-isobutyl-carbazat (Herstellung: J. Chem. Soc. Perkin I, 1712 (1975)) nach chromatographischer Reinigung auf Kieselgel mit Methylenchlorid/Ether (20:1) die Titelverbindung. t_{Ret}(I)=24,7 min, R_{f} (W)=0,36.

### Referenzbeispiel 58: Acetyl-Val-[Phe^{NN}Leu]←(N-(2(R,S)-carbamoyl-3-phenyl-propionyl)-Val):

Analog Ref.-Bsp. 37 erhält man aus 140 mg (0,3 mMol) Acetyl-(L)-Val-[Phe^{NN}Leu]-H·2HCl, 132 mg (0,45 mMol) N-(2(R,S)-Carbamoyl-3-phenyl-propionyl)-(L)-valin (Herstellung: Synth., Struct., Funct., Proc. Am. Pept. Symp., 7^{th}, 85, (1981)), 199 mg (0,45 mMol) BOP, 61 mg (0,45 mMol) HOBt und 3,5 ml 0,3M NMM in DMF die Titelverbindung nach Ausfällen aus Methylenchlorid/DIPE und Lyophilisation aus Dioxan (im HPLC 2 Diastereomere unterscheidbar). FAB-MS: (M+H)⁺=667, t_{Ret}(I)=17,9 und 18,4 min, R_{f} (D)=0,33.

### a) Acetyl-Val-[Phe^{NN}Leu]-H·2 HCl:

Analog Ref.-Bsp. 2a erhält man ausgehend von 230 mg (0,46 mMol) Acetyl-(L)-Val-[Phe^{NN}Leu]-Boc die Titelverbindung nach Lyophilisation. t_{Ret}(I)=10,5 min, R_{f} (D)=0,38.

### b) Acetyl-Val-[Phe^{NN}Leu]-Boc:

Analog Ref.-Bsp. 37 erhält man aus 250 mg (0,71 mMol) H-[Phe^{NN}Leu]-Boc aus Ref.-Bsp. 57c, 170 mg (1,07 mMol) N-Acetyl-(L)-valin, 471 mg (1,07 mMol) BOP, 144 mg (1,07 mMol) HOBt und 7,1 ml 0,3M NMM in DMF die Titelverbindung nach Ausfällen aus Methylenchlorid durch Zugabe von DIPE und Lyophilisation aus Dioxan. FAB-MS: (M+H)⁺=493, t_{Ret}(I)=20,5 min, R_{f} (D)=0,59.

### Referenzbeispiel 59: N-Morpholinocarbonyl-(L)-Val-[Phe^{NN}Leu]←(N-(3-(tetrazol-1-yl)-propionyl)-Val):

Analog Ref.-Bsp. 37 erhält man aus 100 mg (0,19 mMol) N-Morpholinocarbonyl-(L)-Val-[Phe^{NN}Leu]-H·2 HCl, 67 mg (0,38 mMol) N-(3-(Tetrazol-1-yl)-propionyl)-(L)-valin aus Ref.-Bsp. 44a, 124 mg (0,28 mMol) BOP, 38 mg (0,28 mMol) HOBt und 2,1 ml 0,3M NMM in DMF die Titelverbindung nach Ausfällen aus Methylenchlorid durch Zugabe von DIPE und Lyophilisation aus Dioxan (im HPLC 2 Diastereomere unterscheidbar). FAB-MS: (M+H)⁺=687, t_{Ret}(I)=15,2 und 15,4 min, R_{f} (D)=0,25.

### a) N-Morpholinocarbonyl-(L)-Val-[Phe^{NN}Leu]-H·2 HCl:

Analog Ref.-Bsp. 2a erhält man ausgehend von 279 mg (0,49 mMol) N-Morpholinocarbonyl-(L)-Val-[Phe^{NN}Leu]-Boc nach Lyophilisation die Titelverbindung. FAB-MS: (M+H)⁺=464, t_{Ret}(II)=30,3 min, R_{f} (D)=0,46.

### b) N-Morpholinocarbonyl-(L)-Val-[Phe^{NN}Leu]-Boc:

Analog Ref.-Bsp. 37 erhält man aus 250 mg (0,71 mMol) H-[Phe^{NN}Leu]-Boc (aus Ref.-Bsp. 57c), 265 mg (1,07 mMol) N-Morpholinocarbonyl-(L)-valin aus Ref.-Bsp. 7a, 471 mg (1,07 mMol) BOP, 144 mg (1,07 mMol) HOBt und 7,1 ml 0,3M NMM in DMF die Titelverbindung nach Ausfällen aus Methylenchlorid/Hexan und Lyophilisation aus Dioxan. FAB-MS: (M+H)⁺=564, t_{Ret}(I)=21,5 min, R_{f} (K)=0,69.

### Referenzbeispiel 60: N-Trifluoracetyl-[Phe^{NN}Leu]←(N-(2(R,S)-carbamoyl-3-phenylpropionyl)-(L)-Val):

Analog Ref.-Bsp. 37 erhält man aus 136 mg (0,32 mMol) N-Trifluoracetyl-[Phe^{NN}Leu]-H·2HCl, 142 mg (0,49 mMol) N-(2(R,S)-Carbamoyl-3-phenyl-propionyl)-(L)-valin (Herstellung: Synth., Struct., Funct., Proc. Am. Pept. Symp., 7^{th}, 85, (1981)), 215 mg (0,49 mMol) BOP, 66 mg (0,49 mMol) HOBt und 3,5 ml 0,3M NMM in DMF die Titelverbindung nach chromatographischer Reinigung auf Kieselgel mit Chloroform/Methanol (15:1), Fällung der produkthaltigen Fraktionen aus Methylenchlorid/DIPE und Lyophilisation aus Dioxan/tert-Butanol (im HPLC 2 Diasteromere unterscheidbar). FAB-MS: (M+H)⁺=622, t_{Ret}(I)=21,6 und 22,0 min, R_{f} (K)=0,26.

### a) N-Trifluoracetyl-[Phe^{NN}Leu]-H·2 HCl:

Analog Ref.-Bsp. 2a erhält man ausgehend von 300 mg (0,67 mMol) N-Trifluoracetyl-[Phe^{NN}Leu]-Boc aus Ref.-Bsp. 57d nach Lyophilisation die Titelverbindung. R_{f} (W)<0,1.

### Referenzbeispiel 61: Z-(L)-Val-[Phe^{NN}Nle]←(N-(2(R,S)-(N-(2-morpholinoethyl)-carbamoyl)-3-methyl)-butyryl):

Analog Ref.-Bsp. 37 erhält man aus 100 mg (0,17 mMol) Z-(L)-Val-[Phe^{NN}Nle]-H·2HCl, 69 mg (0,27 mMol) 2(R,S)-(N-(2-Morpholinoethyl)-carbamoyl)-3-methyl-buttersäure (Isopropylmalonsäure-N-(2-morpholinoethyl)monoamid), 119 mg (0,27 mMol) BOP, 36 mg (0,27 mMol) HOBt und 2,1 ml 0,3M NMM in DMF die Titelverbindung nach Fällung aus Methylenchlorid/DIPE und Lyophilisation aus Dioxan (im HPLC 2 Diastereomere unterscheidbar). FAB-MS: (M+H)⁺=725, t_{Ret}(I)=17,2 und 17,6 min, R_{f} (D)=0,56.

### a) Z-(L)-Val-[Phe^{NN}Nle]-H·2 HCl:

Analog Ref.-Bsp. 2a erhält man ausgehend von 310 mg (0,53 mMol) Z-(L)-Val-[Phe^{NN}Nle]-Boc nach Lyophilisation die Titelverbindung. t_{Ret}(I)=16,4 min, R_{f} (U)=0,25.

### b) Z-(L)-Val-[Phe^{NN}Nle]-Boc:

Analog Ref.-Bsp. 37 erhält man aus 250 mg (0,71 mMol) H-[Phe^{NN}Nle]-Boc, 268 mg (1,07 mMol) Z-(L)-Valin, 472 mg (1,07 mMol) BOP, 144 mg (1,07 mMol) HOBt und 7,1 ml 0,3M NMM in DMF die Titelverbindung nach chromatographischer Reinigung auf Kieselgel mit Methylenchlorid/Methanol (40:1) und Fällung der produkthaltigen Fraktionen aus Methylenchlorid/DIPE. t_{Ret}(I)=25,6 min, R_{f} (X)=0,17.

### c) H-[Phe^{NN}Nle]-Boc:

Analog Ref.-Bsp. 17a erhält man ausgehend von 830 mg (1,85 mMol) N-Trifluoracetyl-[Phe^{NN}Nle]-Boc die Titelverbindung nach Fällung aus Methylenchlorid/DIPE. t_{Ret}(I)=15,4 min, R_{f} (K)=0,54.

### d) N-Trifluoracetyl-[Phe^{NN}Nle]-Boc:

Analog Ref.-Bsp. 16 erhält man ausgehend von 1 g 3,86 (mMol) 2(R)-[1'-(S)-(Trifluoracetyl-amino)-2'-phenylethyl]-oxiran aus Ref.-Bsp. 16d und 720 mg (3,86 mMol) tert-Butyl-3-butyl-carbazat nach chromatographischer Reinigung auf Kieselgel mit Methylenchlorid/Ether (20:1) die Titelverbindung. t_{Ret}(I)=25,3 min, R_{f} (Q)=0,43.

### e) tert-Butyl-3-butyl-carbazat:

Analog Ref.-Bsp. 4b erhält man aus 18,0 g (136,2 mMol) tert-Butylcarbazat und 12,3 ml (136,2 (mMol) n-Butanal das entsprechende tert-Butoxycarbonyl-hydrazon (25g, 99 %) als Rohprodukt, welches wie in Ref.-Bsp. 4a beschrieben in Gegenwart von 10 g 5% Platin auf Kohle und 4 atm Wasserstoffdruck hydriert wird. Chromatographische Reinigung des Rohproduktes auf Kieselgel mit Hexan/Essigsäureethylester (1:1) ergibt die Titelverbindung. R_{f} (N)=0,44, ¹H-NMR (200 MHz, CD₃OD). 0,92 (t, J=7Hz, 3H) 1,43 (s, 9H) 1,30 bis 1,50 (m, 4H) 2,75 (t, J=7Hz, 2H).

### f) 2(R,S)-(N-(2-Morpholinoethyl)-carbamoyl)-3-methyl-buttersäure

Analog Ref.-Bsp. 9b erhält man aus 7 g (43,7 mMol) racemischem Isopropylmalonsäure-monomethylester (Chem. Ber. 119, 1196 (1986)), 6,3 ml (48,1 mMol) Aminoethyl-morpholin, 6,6 ml (43,7 mMol) Cyanphosphonsäure-diethylester und 12,8 ml (91,8 mMol) Triethylamin in DMF 2(R,S)-(N-(2-Morpholinoethyl)-carbamoyl)-3-methyl-buttersäure-methylester (Isopropylmalonsäure-N-morpholinoethylamid-methylester). Dieser wird während 5h in einem Gemisch von 28 ml 2N Natronlauge und 28 ml Dioxan bei RT gerührt, mit 2N Salzsäure angesäuert und vollständig eingedampft. Der Rückstand wird mit Ethanol digeriert, abfiltriert und ergibt nach Eindampfen des Filtrats die Titelverbindung. ¹H-NMR (200 MHz, CD₃OD): 0,95 und 1,00 (2d, J=7H, 6H) 2,25 (m, 4H) 2,70 (m, 6H) 2,75 (d, J=8Hz, 1H) 3,45 (m, 2H) 3,75 (m, 4H).

### Referenzbeispiel 62: Z-(L)-Val-[Phe^{NN}Nle]←(3-(tetrazol-1-yl)-propionyl)-Val):

Analog Ref.-Bsp. 37 erhält man aus 100 mg (0,18 mMol) Z-(L)-Val-[Phe^{NN}Nle]-H·2 HCl (aus Ref.-Bsp. 61a), 65 mg (0,27 mMol) N-(3-(Tetrazol-1-yl)-propionyl)-(L)-valin aus Ref.-Bsp. 44a, 119 mg (0,27 mMol) BOP, 36 mg (0,27 mMol) HOBt und 2,1 ml 0,3M N-Methylmorpholin in DMF die Titelverbindung nach Fällung aus Methylenchlorid/DIPE und Lyophilisation aus Dioxan/tert-Butanol (2 Diastereomere im HPLC unterscheidbar). FAB-MS: (M+H)⁺=708, t_{Ret}(I)=20,3 und 20,6 min, R_{f} (D)=0,43.

### Referenzbeispiel 63: Z-(L)-Val-[Phe^{NN}Nle]←(N-(2-(R,S)-(N-(2-pyridylmethyl)-carbamoyl)-3-methyl)-butyryl) (Dibenzolsulfonat):

Analog Ref.-Bsp. 37 erhält man aus 95 mg (0,17 mMol) Z-(L)-Val-[Phe^{NN}Nle]-H·2 HCl aus Ref.-Bsp. 61a, 60 mg (0,26 mMol) (R,S)-Isopropylmalonsäure-N-(2-picolyl)-monoamid, 113 mg (0,26 mMol) BOP, 35 mg (0,26 mMol) HOBt und 2,0 ml 0,3M NMM in DMF die Titelverbindung nach chromatographischer Reinigung auf Kieselgel mit Methylenchlorid/Methanol (15:1) als freies Amin. Dieses wird in Methylenchlorid gelöst, mit 2 Aequivalenten Benzolsulfonsäure versetzt und durch Zugabe von DIPE ausgefällt. Lyophilisation aus tert-Butanol ergibt das Dibenzolsulfonat-Salz (2 Diastereomere im HPLC unterscheidbar). FAB-MS: (M+H)⁺=703, t_{Ret}(I)=17,7 und 18,0 min, R_{f} (D)=0,54.

### a) Isopropylmalonsäure-N-(2-picolyl)monoamid:

Eine Lösung von 15 g (93,6 mMol) Isopropylmalonsäure-monomethylester (Herstellung: Chem. Ber. 119, 1196 (1986)) in 150 ml THF wird mit 10,6 ml (103 mMol) N-Methylmorpholin und anschliessend tropfenweise mit 13,5 ml (103 mmol) Isobutylchloroformiat versetzt. Nach 30 min fügt man 15,3 ml (150 mMol) 2-Picolylamin zu und rührt die entstandene Suspension während 2h. Das Reaktionsgemisch wird verdünnt mit 1N Natronlauge und Wasser und mit Methylenchlorid gewaschen, die organische Phase durch Watte filtriert und eingedampft. Kristallisation des Rückstandes liefert Isopropylmalonsäure-N-(2-picolylamid)-methylester, welcher wie in Ref.-Bsp. 61f beschrieben in 2N Natronlauge und Dioxan zur Titelverbindung hydrolisiert wird. t_{Ret}(II)=16,0 min.

### Referenzbeispiel 64: Z-(L)-Val-[Phe^{NN}(p-F)Phe]←(N-(3-(tetrazol-1-yl)-propionyl)-(L)-Val) (Benzolsulfonat):

Analog Ref.-Bsp. 37 erhält man aus 100 mg (0,16 mMol) Z-(L)-Val-[Phe^{NN}(p-F)Phe]-H aus Ref.-Bsp. 22a, 59 mg (0,25 mMol) N-(3-(Tetrazol-1-yl)-propionyl)-(L)-valin aus Ref.-Bsp. 44a, 109 mg (0,25 mMol) BOP, 33 mg (0,25 mMol) HOBt und 1,19 ml 0,3M N-Methylmorpholin in DMF die Titelverbindung nach Ausfällen aus Methylenchlorid/DIPE als freies Amin. Dieses wird in Methylenchlorid/Methanol gelöst, mit 1 Aequivalent Benzolsulfonsäure versetzt und durch Zugabe von Hexan gefällt. Lyophilisation aus tert-Butanol ergibt die Titelverbindung als Benzolsulfonat-Salz. FAB-MS: (M+H)⁺=760, t_{Ret}(I)=21,6 min, R_{f} (B)=0,49.

### Referenzbeispiel 65: Methylsulfonyl-[Phe^{NN}Phe]←(N-phenylacetyl-(L)-Val):

132 mg (0,28 mMol) Methylsulfonyl-[Phe^{NN}Phe]-H·2 HCl wird analog Ref.-Bsp. 7 mit 197 mg (0,84 mMol) N-Phenylacetyl-(L)-valin, (Herstellung: Mem. Tokyo Univ. Agric. 20, 51 (1978)), 317 mg (0,84 mMol) HBTU und 0,23 ml (1,67 mMol) Triethylamin in DMF umgesetzt und ergibt nach Fällung aus Methanol durch Zugabe von Ether die Titelverbindung. FAB-MS: (M+H)⁺=581, t_{Ret}(I)=20,2 min, R_{f} (B)=0,64.

### a) Methylsulfonyl-[Phe^{NN}Phe]-H·2 HCl:

Analog Ref.-Bsp. 2a erhält man ausgehend von 130 mg (0,28 mMol) Methylsulfonyl-[Phe^{NN}Phe]-Boc die Titelverbindung nach Lyophilisation. FAB-MS: (M+H)⁺=364, t_{Ret}(II)=28,5 min, R_{f} (K)=0,56.

### b) Methylsulfonyl-[Phe^{NN}Phe]-Boc:

Analog Ref.-Bsp. 16a erhält man ausgehend von 1,1 g (4,56 mMol) 2(R)-[1'(S)-(Methylsulfonylamino)-2'-phenylethyl]oxiran und 1,11 g (5,02 mMol) tert-Butyl-3-benzyl-carbazat (Herstellung: J. Chem Soc. Perkin I, 1712 (1975)) die Titelverbindung als Diastereomerengemisch im Verhältnis 4:1. Durch Kristallisation aus Methylenchlorid/Hexan wird das Verhältnis zugunsten des 2S-Diastereomeren auf 10:1 verbessert. FAB-MS: (M+H)⁺=464, t_{Ret}(I)=21,3 min, R_{f} (N)=0,26.

### c) 2(R)-[1'(S)-(Methylsulfonylamino)-2'-phenylethyl]oxiran:

Eine Lösung von 1 g (6,8 mMol)1-Phenyl-3-buten-2(S)-amin aus Ref.-Bsp. 16b in 10 ml Methylenchlorid wird bei 0° mit 2,36 g (13,6 mMol) Methansulfonsäureanhydrid und 1,88 ml (13,6 mMol) Triethylamin versetzt und während 1h gerührt. Das Reaktionsgemisch wird mit Wasser und ges. Natriumbicarbonatlösung gewaschen, die organische Phase wird durch Watte filtriert und eingedampft und ergibt 2(S)-Methylsulfonylamino-1-phenyl-3-buten. 1 g (4,4 mMol) dieses Rohprodukts wird in 30 ml Methylenchlorid gelöst, bei RT mit 3,05 g (17,7 mMol) 4-Chlorperbenzoesäure versetzt und während 18h gerührt. Die Reaktionslösung wird 5x mit 10% wässriger Natriumsulfitlösung gewaschen, durch Watte filtriert und vollständig eingedampft. Das Rohprodukt enthält gemäss ¹H-NMR die beiden (2R) und (2S)-Epimeren im Verhältnis 4:1. ¹H-HMR (200 MHz, CD₃OD): 2,30 und 2,52, (2 s, zusammen 3H) 2,6 bis 3,2 (m, 5H) 3,55 (m, 1H) 7,32 (m, 5H).

### Referenzbeispiel 66: Methoxycarbonyl-(L)-Val-[Phe^{NN}Leu]←(N-methoxycarbonyl-(L)-Val):

Analog Ref.-Bsp. 37 erhält man aus 200 mg (0,55 mMol) H-[Phe^{NN}Leu]-H·3 HCl (aus Ref.-Bsp. 13a), 291 mg (1,66 mMol) N-Methoxycarbonyl-(L)-valin (Herstellung: Chem. Lett. 705, (1980)), 735 mg (1,66 mMol) BOP, 225 mg (1,66 mMol) HOBt und 11 ml 0,3M NMM in DMF die Titelverbindung nach Fällung aus Methylenchlorid/DIPE und Lyophilisation aus Dioxan. FAB-MS: (M+H)⁺=566, t_{Ret}(I)=18,6 min, R_{f} (U)=0,33.

### Referenzbeispiel 67: Methoxycarbonyl-(L)-Val-[Phe^{NN}(p-F)Phe]←(N-methoxycarbonyl-(L)-Val):

Analog Ref.-Bsp. 37 erhält man aus 200 mg (0,48 mMol) H-[Phe^{NN}(p-F)Phe]-H·3 HCl (aus Ref.-Bsp. 42b), 255 mg (1,45 mMol) N-Methoxycarbonyl-(L)-valin (Herstellung: Chem. Lett. 705, (1980)), 643 mg (1,45 mMol) BOP, 196 mg (1,45 mMol) HOBt und 9,7 ml 0,3M NMM in DMF die Titelverbindung nach Fällung aus Methylenchlorid/DIPE und Lyophilisation aus Dioxan. FAB-MS: (M+H)⁺=618, t_{Ret}(I)=19,5 min, R_{f} (U)=0,22.

### Referenzbeispiel 68: Methoxycarbonyl-(L)-Val-[Phe^{NN}(p-CN)Phe]←(N-methoxycarbonyl-(L)-Val):

Analog Ref.-Bsp. 37 erhält man aus 200 mg (0,48 mMol) H-[Phe^{NN}(p-CN)Phe]-H·3 HCl (aus Ref.-Bsp. 47a), 250 mg (1,43 mMol) N-Methoxycarbonyl-(L)-valin (Herstellung: Chem. Lett. 705, (1980)), 631 mg (1,43 mMol) BOP, 193 mg (1,43 mMol) HOBt und 9,5 ml 0,3M NMM in DMF die Titelverbindung nach chromatographischer Reinigung auf Kieselgel mit Methylenchlorid/Methanol (15:1) und Lyophilisation der produkthaltigen Fraktionen aus Dioxan. FAB-MS: (M+H)⁺=625, t_{Ret}(I)=18 min, R_{f} (U)=0,31.

### Referenzbeispiel 69:

### Z-(L)-Val-[(p-F)Phe^{NN}(p-F)Phe]←(N-(2(R,S)-(N-(2-morpholinoethyl)-carbamoyl)-3-methyl)-butyryl):

Analog Ref.-Bsp. 18) werden 23,0 mg (0,089 mMol) 2(R,S)-(N-(2-Morpholinoethyl)-carbamoyl)-3-methyl-buttersäure (Ref.-Bsp. 61 f) und 45 mg (0,081 mMol) Z-(L)-Val-[(p-F)Phe^{NN}(p-F)Phe]-H (Ref.-Bsp. 32) mit 33,8 mg (0,089 mMol) HBTU in 0,76 ml NMM/CH₃CN 0,25 M zur Titelverbindung umgesetzt und mit DMF/DIPE umgefällt: DC R_{f}(P)=0,42; FAB-MS (M+H)⁺=795.

### Referenzbeispiel 70: Z-(L)-Val-[(p-F)Phe^{NN}(p-F)Phe]←(N-(2(R,S)-(N-(2-pyridylmethyl)-carbamoyl)-3-methyl)-butyryl):

Analog Ref.-Bsp. 18) werden 21,0 mg (0,089 mMol) rac. Isopropylmalonsäure-N-(2-picolyl)amid (Ref.-Bsp. 63 a) und 45 mg (0,081 mMol) Z-(L)-Val-[(p-F)Phe^{NN}(p-F)Phe]-H (Ref.-Bsp. 32) mit 33,8 mg (0,089 mMol) HBTU in 0,76 ml NMM/CH₃CN 0,25 M zur Titelverbindung umgesetzt und mit DMF/DIPE umgefällt: DC R_{f}(P)=0,52; FAB-MS (M+H)⁺=773.

### Referenzbeispiel 71:

Analog zu einem der vorgenannten Verfahren können folgende Verbindungen hergestellt werden:
a) Z-(L)-Val-[(p-F)Phe^{NN}(p-F)Phe]←((L)-Val)←(N-morpholinocarbonyl-Gly);
b) N-Morpholinocarbonyl-(L)-Val-[(p-F)Phe^{NN}(p-F)Phe]←((L)-Val)←(N-morpholinocarbonyl-Gly);
c) N-(Chinolin-2-carbonyl)-(L)-Asn-[Phe^{NN}(p-F)Phe]←((L)-Val)-Z
d) N-(Morpholinosulfonyl)-(L)-Val-[Phe^{NN}Leu]←(N-(morpholinosulfonyl)-(L)-Val)
e) N-(Chinolin-2-carbonyl)-(L)-Asn-[Phe^{NN}Cha]←((L)-Val)-Z (= 1-[2(S)-Hydroxy-3(S)-(N-(chinolin-2-carbonyl)-(L)-asparagyl)amino-4-phenyl-butyl]-1-[cyclohexylmethyl]-2-[N-(benzyloxycarbonyl)-(L)-valyl]hydrazin): Unter N₂-Atmosphäre werden 27 mg (0,107 mMol) Z-Valin in 0,59 ml einer 0,3 M Lösung von NMM in DMF mit 47 mg (0,107 mMol) BOP und 14 mg (0,107 mMol) HOBT aktiviert und nach 15 min mit 50 mg (0,089 mMol) 1-[2(S)-Hydroxy-3(S)-(N-(chinolin-2-carbonyl)-(L)-asparagyl)amino-4-phenyl-butyl]-1-[cyclohexylmethyl]-hydrazin versetzt. Das Gemisch rührt man während 18 h bei RT und dampft es am HV ein. Man löst den Rückstand in Methylenchlorid, wäscht mit ges. NaHCO₃-Lösung, Wasser und Sole, extrahiert die Wasserphasen 2x mit Methylenchlorid, trocknet die organischen Phasen mit Na₂SO₄ und dampft sie ein. Eine Säulenchromatographie (SiO₂, Essigsäureethylester/Ethanol 100:3) liefert die reine Titelverbindung: DC R_{f}(D')=0,21; t_{Ret}(V)=16,7 min; FAB-MS (M+H)⁺=794.

Das Ausgangsmaterial wird wie folgt hergestellt:

### i) 1-[2(S)-Hydroxy-3(S)-(N-(chinolin-2-carbonyl)-(L)-asparagyl)amino-4-phenyl-butyl]-1-[cyclohexylmethyl]-hydrazin

Analog Beispiel 27 (s. unten)) werden 921 mg (1,39 mMol) 1-[2(S)-Hydroxy-3(S)-(N-(chinolin-2-carbonyl)-(L)-asparagyl)amino-4-phenyl-butyl]-1-[cyclohexylmethyl]-2-[tert-butoxy-carbonyl]hydrazin (Beispiel 29, s. unten) in 37 ml Ameisensäure zur Titelverbindung umgesetzt und direkt in der nächsten Stufe eingesetzt.

### f) N-(Chinolin-2-carbonyl)-(L)-Asn-[Phe^{NN}(p-F)Phe]←(N-(methoxycarbonyl)-(L)-Val)

### Beispiel 1: 1-[2(S)-Acetoxy-3(S)-(N-(2-methoxyethoxycarbonyl)-(L)-valyl)amino-4-phenyl-butyl]-1-[cyclohexylmethyl]-2-[N-(2-methoxyethoxycarbonyl)-(L)-valyl]hydrazin:

Unter Stickstoffatmosphäre werden 200 mg (0,29 mMol) N-(2-Methoxy-ethoxycarbonyl)-(L)-Val-[Phe^{NN}Cha]←(N-(2-methoxy-ethoxycarbonyl)-(L)-Val) in 4 ml THF und 60 µl (0,43 mMol) Triethylamin in Gegenwart von 0,5 mg (0,003 mMol) DMAP mit 40 µl (0,43 mMol) Acetanhydrid während 3 h bei RT acetyliert. Das Reaktionsgemisch wird verteilt zwischen 3 Portionen Methylenchlorid, Wasser, ges. NaHCO₃-Lsg und Sole. Nach Trocknen mit Na₂SO₄, Eindampfen und Säulenchromatographie (SiO₂, Methylenchlorid/Methanol 30:1) erhält man aus den organischen Phasen die Titelverbindung: DC R_{f}(Z)=0,17; t_{Ret}(I)=22,5 min; FAB-MS (M+H)⁺=736.

Die Ausgangsmaterialien werden wie folgt hergestellt:

### a) N-(2-Methoxy-ethoxycarbonyl)-(L)-Val-[Phe^{NN}Cha]←(N-(2-methoxy-ethoxycarbonyl)-(L)-Val):

Analog Ref.-Bsp. 2) werden 820 mg (3,74 mMol) N-(2-Methoxy-ethyloxycarbonyl)-(L)-Valin mit 1,65 g (3,74 mMol) BOP und 505 mg (3,74 mMol) HOBT in 25 ml einer 0,3 M Lösung von NMM in DMF aktiviert und nach 10 min mit 500 mg (1,25 mMol) H-[Phe^{NN}Cha]-H (Hydrochlorid-Salz) (vgl. Ref.-Bsp. 10a) während 18 h umgesetzt. Das Reaktionsgemisch wird am HV eingedampft, der Rückstand in CHCl₃ gelöst und mit 10 %-iger Zitronensäurelösung, ges. NaHCO₃-Lsg. und Sole gewaschen. Die Wasserphasen extrahiert man mit 2 Portionen CHCl₃, trocknet die organischen Phasen mit Na₂SO₄ und dampft sie ein. Säulenchromatographie (SiO₂, CHCl₃/MeOH 30:1) und Ausfällen mit Hexan aus einer CH₂Cl₂-Lösung liefert die Titelverbindung: D CR_{f}(T)=0,37; t_{Ret}(I)=21,5 min; FAB-MS (M+H)⁺=694.

### b) Chlorameisensäure-(2-methoxy-ethyl)-ester:

Unter Stickstoffatmosphäre tropft man bei 0 bis 5°C zu 100 ml (202 mMol) einer 20 %-igen Lösung von Phosgen in Toluol 13,3 ml (168 mMol) 2-Methoxy-ethanol, rührt 90 min bei 0°C und 18 h bei RT aus. Man extrahiert das Reaktionsgemisch mit Wasser, filtriert die organische Phase durch Watte und dampft sie ein: IR (CH₂Cl₂): u.a. 3055w, 2995w, 2935w, 2895w, 2825w, 1775s, 1167s, 1127s; ¹H-NMR (200 MHz, CDCl₃): 3,38 (s, 3 H), 3,64 und 4,44 (2t, J=5 Hz, je 2 H).

### c) N-(2-Methoxy-ethyloxycarbonyl)-(L)-valin:

Zu 2,59 g (22,1 mMol) L-Valin in 26,4 ml 2 N NaOH gibt man eine Lösung von 3,06 g (22,1 mMol) Chlorameisensäure-(2-methoxy-ethyl)-ester in 18 ml Dioxan und rührt während 18 h bei RT nach. Das Reaktionsgemisch wird mit Chloroform extrahiert, die anorganische Phase mit 4 N HCl angesäuert und wiederum mit Chloroform extrahiert. Trocknen und Eindampfen der zuletzt erhaltenen Chloroform-Phase liefert die Titelverbindung: ¹H-NMR (200 MHz, CDCl₃): 0,92 und 0,99 (2d, J=7 Hz, 6 H), 2,2 (m, 1H), 3,38 (s, 3 H), 3,59 und 4,24 (2m, je 2 H), 4,3 (m, 1 H), 5,4 (d, J=9 Hz, HN), 8,5 (sb, 1 H).

### Beispiel 2: 1-[2(S)-Acetoxy-3(S)-(N-(methoxycarbonyl)-(L)-valyl)amino-4-phenylbutyl]-1-[cyclohexylmethyl]-2-[N-(methoxycarbonyl)-(L)-valyl]hydrazin:

Analog Beispiel 1) werden 200 mg (0,33 mMol) N-(Methoxy-carbonyl)-(L)-Val-[Phe^{NN}Cha]←(N-(methoxy-carbonyl)-(L)-Val) in 4 ml THF und 68 µl (0,50 mMol) Triethylamin in Gegenwart von 1,2 mg (0,01 mMol) DMAP mit 46 µl (0,50 mMol) Acetanhydrid umgesetzt. Fällen mit DIPE aus einer konzentrierten Lösung des Rohproduktes in Methanol liefert die reine Titelverbindung: DC R_{f}(A')=0,42; t_{Ret}(I)=22,6 min; FAB-MS (M+H)⁺=648.

Das Ausgangsmaterial wird wie folgt hergestellt:

### a) N-(Methoxy-carbonyl)-(L)-Val-[Phe^{NN}Cha]←(N-(methoxy-carbonyl)-(L)-Val) (= 1-[2(S)-Hydroxy-3(S)-(N-(methoxycarbonyl)-(L)-valyl)-amino-4-phenyl-butyl]-1-[cyclohexylmethyl]-2-[N-(methoxycarbonyl)-(L)-valyl]hydrazin):

Analog Ref.-Bsp. 2) werden 1,47 g (8,4 mMol) N-(Methoxy-carbonyl)-(L)-Valin mit 3,71 g (8,4 mMol) BOP und 1,13 g (8,4 mMol) HOBT in 54 ml einer 0,3 M Lösung von NMM in DMF aktiviert und nach 15 min mit 1,12 g (2,8 mMol) H-[Phe^{NN}Cha]-H (Hydrochlorid-Salz) (vgl. Ref.-Bsp. 10a) während 18 h umgesetzt. Das Reaktionsgemisch wird am RV bei 50°C eingedampft (→ brauner Rückstand), der Rückstand in Methylenchlorid gelöst und 2x mit ges. NaHCO₃-Lsg. sowie Sole gewaschen. Die Wasserphasen extrahiert man mit 2 Portionen Methylenchlorid, trocknet die organischen Phasen mit Na₂SO₄ und dampft sie ein. Filtration durch Kieselgel (Methylenchlorid/Methanol 15:1) und zweimaliges Fällen mit DIPE aus einer konzentrierten Methylenchlorid-Lösung liefert die Titelverbindung: DC R_{f}(U)=0,33; t_{Ret}(I)=21,5 min; FAB-MS (M+H)⁺=606.

Das Ausgangsmaterial wird wie folgt hergestellt:

### b) N-(Methoxy-carbonyl)-(L)-valin:

Zu 7,0 g (60 mMol) L-Valin in 100 ml 2 N NaOH und 30 ml Dioxan gibt man 5,67 g (60 mMol) Chlorameisensäure-methylester (→ exotherme Reaktion) und rührt während 18 h bei RT nach. Das Reaktionsgemisch wird mit Methylenchlorid extrahiert, die Wasserphase mit 27 ml einer 4 N HCl angesäuert und wiederum mit Methylenchlorid extrahiert. Trocknen und Eindampfen der letzteren Methylenchlorid-Phase liefert die Titelverbindung: t_{Ret}(I)=7,2 min; ¹H-NMR (200 MHz, CD₃OD): 0,96 (t, J=7 Hz, 6 H), 2,16 (m, 1 H), 3,67 (s, 3 H), 4,06 (m, 1 H), 7,07 (d, J=8 Hz, HN_{teilweise ausgetauscht}).

### Beispiel 3: 1-[2(S)-(2-Pyridylcarbonyl)oxy-3(S)-(N-(methoxycarbonyl)-(L)-valyl)amino-4-phenylbutyl]-1-[cyclohexylmethyl]-2-[N-(methoxycarbonyl)-(L)-valyl]hydrazin

Unter Stickstoffatmosphäre werden zu 81 mg (0,66 mMol) 2-Picolinsäure in 4 ml Methylenchlorid bei 0°C 56 µl (0,4 mMol) 1-Chlor-N,N,2-trimethyl-1-propenamin (*B*. *Haveaux*, *A. Dekoker, M. Rens, A.R. Sidani, J. Toye, L. Ghosez, M. Murakami, M. Yoshioka, and W. Nagata,* Organic Syntheses **59**, 26 (1980)) gegeben. Nach 45 min bei RT fügt man 1,3 ml Pyridin, 100 mg (0,165 mMol) N-(Methoxy-carbonyl)-(L)-Val-[Phe^{NN}Cha]←(N-(methoxy-carbonyl)-(L)-Val) (Beispiel 2 a) und eine Spatelspitze DMAP zu und rührt während 18 h bei RT nach. Das dunkle Reaktionsgemisch wird verteilt zwischen 3 Portionen Methylenchlorid, 2 Portionen ges. NaHCO₃-Lsg, Wasser und Sole. Säulenchromatographie (SiO₂, Essigsäureethylester) des Eindampfrückstandes der mit Na₂SO₄ getrockneten Methylenchlorid-Phase liefert die reine Titelverbindung: DC R_{f}(O)=0,23; t_{Ret}(I)=22,5 min; FAB-MS (M+H)⁺=711.

### Beispiel 4:

Nach einem der vorgenannten Verfahren werden hergestellt:
a) 1-[2(S)-Propionyloxy-3(S)-(N-(methoxycarbonyl)-(L)-valyl)amino-4-phenyl-butyl]-1-[cyclohexylmethyl]-2-[N-(methoxycarbonyl)-(L)-valyl]hydrazin;
b) 1-[2(S)-Butyryloxy-3(S)-(N-(methoxycarbonyl)-(L)-valyl)amino-4-phenyl-butyl]-1-[cyclohexylmethyl]-2-[N-(methoxycarbonyl)-(L)-valyl]hydrazin;
c) 1-[2(S)-Pentanoyloxy-3(S)-(N-(methoxycarbonyl)-(L)-valyl)amino-4-phenyl-butyl]-1-[cyclohexylmethyl]-2-[N-(methoxycarbonyl)-(L)-valyl]hydrazin;
d) 1-[2(S)-Octanoyloxy-3(S)-(N-(methoxycarbonyl)-(L)-valyl)amino-4-phenyl-butyl]-1-[cyclohexylmethyl]-2-[N-(methoxycarbonyl)-(L)-valyl]hydrazin;
e) 1-[2(S)-Decanoyloxy-3(S)-(N-(methoxycarbonyl)-(L)-valyl)amino-4-phenyl-butyl]-1-[cyclohexylmethyl]-2-[N-(methoxycarbonyl)-(L)-valyl]hydrazin;
f) 1-[2(S)-Dodecanoyloxy-3(S)-(N-(methoxycarbonyl)-(L)-valyl)amino-4-phenyl-butyl]-1-[cyclohexylmethyl]-2-[N-(methoxycarbonyl)-(L)-valyl]hydrazin;
g) 1-[2(S))-Pivaloyloxy-3(S)-(N-(methoxycarbonyl)-(L)-valyl)amino-4-phenyl-butyl]-1-[cyclohexylmethyl]-2-[N-(methoxycarbonyl)-(L)-valyl]hydrazin;
h) 1-[2(S)-(2-Furylcarbonyl)oxy-3(S)-(N-(methoxycarbonyl)-(L)-valyl)amino-4-phenylbutyl]-1-[cyclohexylmethyl]-2-[N-(methoxycarbonyl)-(L)-valyl]hydrazin;
i) 1-[2(S)-(4-Imidazolylcarbonyl)oxy-3(S)-(N-(methoxycarbonyl)-(L)-valyl)amino-4-phenyl-butyl]-1-[cyclohexylmethyl]-2-[N-(methoxycarbonyl)-(L)-valyl]hydrazin;
j) 1-[2(S)-(4-Imidazolylacetyl)oxy-3(S)-(N-(methoxycarbonyl)-(L)-valyl)amino-4-phenyl-butyl]-1-[cyclohexylmethyl]-2-[N-(methoxycarbonyl)-(L)-valyl]hydrazin;
k) 1-[2(S)-(3-(4-Imidazolyl)-propionyl)oxy-3(S)-(N-(methoxycarbonyl)-(L)-valyl)amino-4-phenyl-butyl]-1-[cyclohexylmethyl]-2-[N-(methoxycarbonyl)-(L)-valyl]hydrazin;
l) 1-[2(S)-Benzoyloxy-3(S)-(N-(methoxycarbonyl)-(L)-valyl)amino-4-phenyl-butyl]-1-[cyclohexylmethyl]-2-[N-(methoxycarbonyl)-(L)-valyl]hydrazin;
m) 1-[2(S)-(2-Pyridylacetyl)oxy-3(S)-(N-(methoxycarbonyl)-(L)-valyl)amino-4-phenylbutyl]-1-[cyclohexylmethyl]-2-[N-(methoxycarbonyl)-(L)-valyl]hydrazin;
n) 1-[2(S)-(3-(Pyridin-2-yl)-propionyl)oxy-3(S)-(N-(methoxycarbonyl)-(L)-valyl)amino-4-phenylbutyl)]-1-[cyclohexylmethyl]-2-[N-(methoxycarbonyl)-(L)-valyl]hydrazin;
o) 1-[2(S)-(Chinolin-2-ylcarbonyl)oxy-3(S)-(N-(methoxycarbonyl)-(L)-valyl)amino-4-phenyl-butyl]-1-[cyclohexylmethyl]-2-[N-(methoxycarbonyl)-(L)-valyl]hydrazin;
p) 1-[2(S)-(Aminoacetyl)oxy-3(S)-(N-(methoxycarbonyl)-(L)-valyl)amino-4-phenylbutyl]-1-[cyclohexylmethyl]-2-[N-(methoxycarbonyl)-(L)-valyl]hydrazin;
q) 1-[2(S)-(N-Methylaminoacetyl)oxy-3(S)-(N-(methoxycarbonyl)-(L)-valyl)amino-4-phenyl-butyl]-1-[cyclohexylmethyl]-2-[N-(methoxycarbonyl)-(L)-valyl]hydrazin;
r) 1-[2(S)-(N,N-Dimethylaminoacetyl)oxy-3(S)-(N-(methoxycarbonyl)-(L)-valyl)amino-4-phenyl-butyl]-1-[cyclohexylmethyl]-2-[N-(methoxycarbonyl)-(L)-valyl]hydrazin;
s) 1-[2(S)-(N-Benzyloxycarbonyl-N-methyl-aminoacetyl)oxy-3(S)-(N-(methoxycarbonyl)-(L)-valyl)amino-4-phenyl-butyl]-1-[cyclohexylmethyl]-2-[N-(methoxycarbonyl)-(L)-valyl]hydrazin;
t) 1-[2(S)-Prolyloxy -3(S)-(N-(methoxycarbonyl)-(L)-valyl)amino-4-phenyl-butyl]-1-[cyclohexylmethyl]-2-[N-(methoxycarbonyl)-(L)-valyl]hydrazin;
u) 1-[2(S)-(4-Morpholinomethylbenzoyl)oxy-3(S)-(N-(methoxycarbonyl)-(L)-valyl)-amino-4-phenyl-butyl]-1-[cyclohexylmethyl]-2-[N-(methoxycarbonyl)-(L)-valyl]hydrazin;
v) 1-[2(S)-(4-Chlormethylbenzoyl)oxy-3(S)-(N-(methoxycarbonyl)-(L)-valyl)amino-4-phenyl-butyl]-1-[cyclohexylmethyl]-2-[N-(methoxycarbonyl)-(L)-valyl]hydrazin;
w) 1-[2(S)-(3-Carboxypropionyl)oxy-3(S)-(N-(methoxycarbonyl)-(L)-valyl)amino-4-phenyl-butyl]-1-[cyclohexylmethyl]-2-[N-(methoxycarbonyl)-(L)-valyl]hydrazin.

### Beispiel 5:

Nach einem der oben genannten Verfahren werden aus den Titelverbindungen der Referenzbeispiele 1 bis 70 die monoacetylierten Derivate hergestellt, welche im jeweils zutreffenden zentralen bivalenten, von Butan-2(S)-ol derivatisierten Radikal mit einer der Bezeichnungen -[Phe^{NN}Phe], -[Phe^{NN}Cha], -[Phe^{NN}Leu], -[Phe^{NN}Nle], -[Phe^{NN}(p-F)Phe], -[(p-F)Phe^{NN}(p-F)Phe], -[Phe^{NN}(p-CN)Phe] oder -[Cha^{NN}Leu] anstelle der freien 2(S)-Hydroxygruppe eine 2(S)-Acetoxygruppe enthalten.

### Beispiel 6:

Nach einem der oben genannten Verfahren werden die folgenden Verbindungen hergestellt (in eckigen Klammern ([]) sind die Ausgangsmaterialien angegeben (z.B. das jeweilige Referenzbeispiel, dessen Titelverbindung als Ausgangsmaterial eingesetzt wird):
a) 1-[2(S)-(2-Furylcarbonyl)oxy-3(S)-(N-(benzyloxycarbonyl)-(L)-valyl)-amino-4-phenylbutyl)]-1-[benzyl]-2-[N-(benzyloxycarbonyl)-(L)-valyl]-hydrazin [aus der Titelverbindung von Ref.-Bsp. 2 und Furan-2-carbonsäurechlorid];
b) 1-[2(S)-Pivaloyloxy-3(S)-(N-(benzyloxycarbonyl)-(L)-valyl)-amino-4-phenyl-butyl]-1-[cyclohexylmethyl]-2-[N-(benzyloxycarbonyl)-(L)-valyl]-hydrazin [aus der Titelverbindung von Ref.-Bsp. 11 und Pivalinsäureanhydrid];
c) 1-[2(S)-(N-Methylaminoacetyl)oxy-3(S)-(N-(morpholinocarbonyl)-(L)-valyl)-amino-4-phenyl-butyl]-1-[p-fluorphenylmethyl]-2-[N-(benzyloxycarbonyl)-(L)-valyl]-hydrazin [aus der Titelverbindung von Ref.-Bsp. 18 und N-Benzyloxycarbonyl-N-methyl-aminoessigsäure mit anschliessender Hydrogenolyse der erhaltenen 2(S)-(N-Benzyloxycarbonyl-N-methylaminoacetylverbindung unter Katalyse mit Pd/C];
d) 1-[2(S)-(N-Benzyloxycarbonyl-N-methyl-aminoacetyl)oxy-3(S)-(N-(benzyloxycarbonyl)-(L)-valyl)-amino-4-phenyl-butyl]-1-[p-fluorphenylmethyl]-2-[N-(N-(morpholinocarbonyl)-glycyl)-(L)-valyl]-hydrazin [aus der Titelverbindung von Ref.-Bsp. 24 und N-Benzyloxycarbonyl-N-methylaminoessigsäurechlorid];
e) 1-[2(S)-(N,N-Dimethyl-aminoacetyl)oxy-3(S)-(N-(chinolin-2-ylcarbonyl)-(L)-aspartoyl)-amino-4-phenyl-butyl]-1-[p-fluorphenylmethyl]-2-[tert-butoxycarbonyl]-hydrazin [aus der Titelverbindung von Ref.-Bsp. 27 und Dimethylaminoessigsäurechlorid];
f) 1-[2(S)-(2-Pyridylcarbonyl)oxy-3(S)-(N-(benzyloxycarbonyl)-(L)-aspartoyl)-amino-4-phenyl-butyl]-1-[p-fluorphenylmethyl]-2-[N-(benzyloxycarbonyl)-(L)-valyl]-hydrazin [aus der Titelverbindung von Ref.-Bsp. 28 und 2-Pyridincarbonsäurechlorid);
g) 1-[2(S)-(4-(Morpholinomethyl)benzoyl)oxy-3(S)-(N-(benzyloxycarbonyl)-(L)-valyl)amino-4-(p-fluorphenyl)-butyl]-1-[p-fluorphenylmethyl]-2-[tert-butoxycarbonyl]hydrazin [aus der Titelverbindung von Ref.-Bsp. 31 und 4-Morpholinomethyl-benzoesäure über das Säurechlorid in Gegenwart von N,N,2-Trimethyl-1-chlor-propen-(1)-amin];
h) 1-[2(S)-Benzoyloxy-3(S)-(N-(benzyloxycarbonyl)-(L)-valyl)-amino-4-(p-fluorphenyl)-butyl]-1-[p-fluorphenylmethyl]-2-[N-(N-(2-pyridylmethyl)-N-methylaminocarbonyl)-(L)-valyl]-hydrazin [aus der Titelverbindung von Ref.-Bsp. 33 und Benzoylchlorid];
i) 1-[2(S)-(4-Chlormethylbenzyloyl)oxy-3(S)-(N-(benzyloxycarbonyl)-(L)-valyl)-amino-4-(p-fluorphenyl)-butyl]-1-[p-fluorphenylmethyl]-2-[N-(2(R,S)-carbamoyl-3-phenyl-propionyl)-(L)-valyl]-hydrazin [aus der Titelverbindung von Ref.-Bsp. 34 und 4-Chlormethylbenzoesäure in Gegenwart von N,N,2-Trimethyl-1-chlor-propen-(1)-amin];
j) 1-[2(S)-(Imidazol-4-ylacetyl)oxy-3(S)-(N-acetyl-valyl)-amino-4-phenyl-butyl]-1-[benzyl]-2-[N-acetyl-valyl]-hydrazin [aus der Titelverbindung von Ref.-Bsp. 35 und 1 -Tritylimidazolyl-4-essigsäure (hergestellt aus Tritylchlorid und 4-Imidazolylessigsäure in Gegenwart von Pyridin) in Gegenwart von N,N,2-Trimethyl-1-chlor-propen-(1)-amin über die tritylgeschützte Zwischenverbindung mit anschliessender acidolytischer Abspaltung der Tritylschutzgruppe, z.B. mit Trifluoressigsäure];
k) 1-[2(S)-(2-Pyridylacetyl)oxy-3(S)-(N-(chinolin-2-ylcarbonyl)-valyl)-amino-4-phenyl-butyl]-1-[benzyl]-2-[N-chinolin-2-yl-carbonyl-valyl]-hydrazin [aus der Titelverbindung von Ref.-Bsp. 37 und 2-Pyridinessigsäure in Gegenwart von N,N,2-Tri methyl-1-chlor-propen-(1)-amin];
l) 1-[2(S)-(3-Pyridylacetyl)oxy-3(S)-(N-acetyl-(L)-valyl)-amino-4-phenyl-butyl]-1-[cyclohexylmethyl]-2-[N-acetyl-(L)-valyl]-hydrazin [aus der Titelverbindung von Ref.-Bsp. 38 und 3-Pyridinessigsäure in Gegenwart von N,N,2-Trimethyl-1-chlor-propen-(1)-amin];
m) 1-[2(S)-(4-Pyridylacetyl)oxy-3(S)-(N-(3-pyridylacetyl)-(L)-valyl)-amino-4-phenyl-butyl]-1-[cyclohexylmethyl]-2-[N-(3-pyridylacetyl)-(L)-valyl]hydrazin [aus der Titelverbindung von Ref.-Bsp. 39 und 4-Pyridinessigsäure in Gegenwart von N,N,2-Trimethyl-1-chlor-propen-(1)-amin];
n) 1-[2(S)-(Chinolin-2-ylcarbonyl)oxy-3(S)-(N-(N-(2-pyridylmethyl)-N-methylaminocarbonyl)-(L)-valyl)-amino-4-phenyl-butyl]-1-[p-fluorphenylmethyl]-2-[N-(N-(2-pyridylmethyl)-N-methylaminocarbonyl)-(L)-valyl]-hydrazin [aus der Titelverbindung von Ref.-Bsp. 43 und Chinolin-2-carbonsäure in Gegenwart von N,N,2-Trimethyl-1-chlorpropen-(1)-amin];
o) 1-[2(S)-(2-Pyrrolidinylcarbonyl)oxy-3(S)-(N-(benzyloxycarbonyl)-(L)-valyl)-amino-4-phenyl-butyl]-1-[p-fluorphenylmethyl]-2-[N-(benzyloxycarbonyl)-(L)-valyl]-hydrazin [aus der Titelverbindung von Ref.-Bsp. 45 und Prolin in Gegenwart von N,N,2-Trimethyl-1-chlor-propen-(1)-amin];
p) 1-[2(S)-Propionyloxy-3(S)-(N-(benzyloxycarbonyl)-(L)-valyl)-amino-4-phenylbutyl]-1-[p-cyanophenylmethyl]-2-[N-(benzyloxycarbonyl)-(L)-valyl]-hydrazin [aus der Titelverbindung von Ref.-Bsp. 48 und Propansäureanhydrid];
q) 1-[2(S)-Butyryloxy-3(S)-(N-(benzyloxycarbonyl)-(L)-isoleucyl)-amino-4-phenylbutyl]-1-[isobutyl]-2-[N-(benzyloxycarbonyl)-(L)-isoleucyl]-hydrazin [aus der Titelverbindung von Ref.-Bsp. 49 und Buttersäureanhydrid];
r) 1-[2(S)-Pentanoyloxy-3(S)-(N-(isobutoxycarbonyl)-(L)-valyl)-amino-4-phenyl-butyl]-1-[isobutyl]-2-[N-(isobutoxycarbonyl)-(L)-valyl]-hydrazin [aus der Titelverbindung von Ref.-Bsp. 50 und Pentansäurechlorid];
s) 1-[2(S)-Decanoyloxy-3(S)-(N-acetyl-valyl)-amino-4-phenyl-butyl]-1-[isobutyl]-2-[N-acetyl-valyl]-hydrazin [aus der Titelverbindung von Ref.-Bsp. 52 und Decansäure in Gegenwart von N,N,2-Trimethyl-1-chlor-propen-(1)-amin];
t) 1-[2(S)-Dodecanoyloxy-3(S)-N-valyl-amino-4-phenyl-butyl]-1-[isobutyl]-2-[N-valyl]hydrazin [aus der an den beiden freien Valyl-Aminogruppen durch Benzyloxycarbonyl geschützten Titelverbindung aus Ref.-Bsp. 54 in Gegenwart von N,N,2-Trimethyl-1-chlor-propen-(1)-amin unter anschliessender hydrogenolytischer Abspaltung der Benzyloxycarbonylschutzgruppen aus der erhältlichen Zwischenverbindung];
u) 1-[2(S)-(3-Carboxypropionyl)oxy-3(S)-(N-(thiomorpholinocarbonyl)-(L)-valyl)amino-4-phenyl-butyl]-1-[isobutyl]-2-[N-(thiomorpholinocarbonyl)-(L)-valyl]hydrazin [hergestellt aus der Titelverbindung von Ref.-Bsp. 55 und Succinsäureanhydrid in Gegenwart von Pyridin];
v) 1-[2(S)-(4-Imidazolylacetyl)oxy-3(S)-(N-(benzyloxycarbonyl)-(L)-valyl)amino-4-phenyl-butyl]-1-[isobutyl]-2-[N-(3-(tetrazol-1-yl)propionyl)-(L)-valyl]hydrazin [hergestellt aus der Titelverbindung aus Ref.-Bsp. 57 und 1-Trityl-4-imidazolylessigsäure analog Beispiel 6 j] ;
w) 1-[2(S)-(Furan-2-ylcarbonyl)oxy-3(S)-(N-acetyl-(L)-valyl)amino-4-phenyl-butyl]-1-[isobutyl]-2-[N-(2(R,S)-carbamoyl-3-phenyl-propionyl)-(L)-valyl]hydrazin [hergestellt aus der Titelverbindung aus Ref.-Bsp. 58 und Furan-2-carbonsäurechlorid];
x) 1-[2(S)-Pivaloyloxy-3(S)-(N-(benzyloxycarbonyl)-(L)-valyl)amino-4-phenyl-butyl]-1-[n-butyl]-2-[2(R,S)-(N-(2-morpholinoethyl)carbamoyl)-3-methylbutyryl]hydrazin [hergestellt aus der Titelverbindung aus Ref.-Bsp. 61 und Pivalinsäureanhydrid];
y) 1-[2(S)-(N-Benzyloxycarbonyl-N-methylaminoacetyl)oxy-3(S)-(N-(benzyloxycarbonyl)-(L)-valyl)amino-4-phenylbutyl]-1-[n-butyl]-2-[N-(3-(tetrazol-1-yl)-propionyl)-valyl]-hydrazin [aus der Titelverbindung von Ref.-Bsp. 62 analog Beispiel 6 d];
z) 1-[2(S)-(N-Methylaminoacetyl)oxy-3(S)-(N-(benzyloxycarbonyl)-(L)-valyl)amino-4-phenyl-butyl]-1-[n-butyl]-2-[N-(2(R,S)-(N-(2-pyridylmethyl)-carbamoyl)-3-methyl)butyryl]-hydrazin [aus der Titelverbindung von Ref.-Bsp. 63 über das 2(S)-N-Benzyloxycarbonyl-N-methylaminoacetyloxy-Analoge durch Hydrogenolyse gemäss Beispiel 6 c].

### Beispiel 7:

Nach einem der oben genannten Verfahren werden die folgenden Verbindungen hergestellt (in eckigen Klammern ([]) sind die Ausgangsmaterialien angegeben (z.B. das jeweilige Referenzbeispiel, dessen Titelverbindung als Ausgangsmaterial eingesetzt wird):
a) 1-[2(S)-(N,N-Dimethyl-aminoacetyl)oxy-3(S)-((N-benzyloxycarbonyl)-(L)-valyl)amino-4-phenyl-butyl]-1-[p-fluorphenylmethyl]-2-[N-(3-(tetrazol-1-yl)-propionyl)-valyl]hydrazin [aus der Titelverbindung von Ref.-Bsp. 64 und Dimethylaminoessigsäure chlorid];
b) 1-[2(S)-(Pyridin-2-ylcarbonyl)oxy-3(S)-(N-methoxycarbonyl)-(L)-valyl)-amino-4-phenyl-butyl]-1-[isobutyl]-2-[N-(methoxycarbonyl)-(L)-valyl]-hydrazin [aus der Titelverbindung von Ref.-Bsp. 66 und 2-Pyridincarbonsäurechlorid].
c) 1-[2(S)-(4-Morpholinomethylbenzoyl)oxy-3(S)-(N-methoxycarbonyl)-(L)-valyl)-amino-4-phenyl-butyl]-1-[p-fluorphenylmethyl]-2-[N-(methoxycarbonyl)-(L)-valyl]-hydrazin [aus der Titelverbindung von Ref.-Bsp. 67 und 4-Morpholinomethyl-benzoesäure (über das Säurechlorid in Gegenwart von N,N,2-Trimethyl-1-chlor-propen-(1)-amin)];
d) 1-[2(S)-Benzoyloxy-3(S)-(N-methoxycarbonyl)-(L)-valyl)-amino-4-phenyl-butyl]-1-[p-cyanophenylmethyl]-2-[N-(methoxycarbonyl)-(L)-valyl]-hydrazin [aus der Titelverbindung von Ref.-Bsp. 68 und Benzoesäureanhydrid];

### Beispiel 8: Analog den vor- und nachstehenden Beispielen und Verfahren erhält man folgende Verbindung:

A) 1-[2(S)-Hydroxy-3(S)-(N-methoxycarbonyl-(L)-valyl)amino-4-phenylbutyl]-1-[thien-2-ylmethyl]-2-[N-methoxycarbonyl-(L)-valyl]hydrazin.

### Beispiel 9: 1-[2(S)-Hydroxy-3(S)-(N-methoxycarbonyl-(L)-valyl)amino-4-phenylbutyl]-1-[4-methoxyphenylmethy]-2-[N-methoxycarbonyl-(L)-valyl]hydrazin:

Analog Referenzbeispiel 37 erhält man aus 200 mg (0,47 mMol) 1-[2(S)-Hydroxy-3(S)-(N-acetyl-(L)-valyl)amino-4-phenylbutyl]-1-[4-methoxyphenylmethyl]-2-[N-acetyl-(L)-valyl]hydrazin, 247 mg (1,41 mMol) N-Methoxycarbonyl-(L)-valin aus Beispiel 2b, 624 mg (1,41 mMol) BOP, 191 mg (1,41 mMol) HOBt und 9,4 ml 0,3M N-Methylmorpholin in DMF die Titelverbindung nach chromatographischer Reinigung (SiO₂, Methylenchlorid/Methanol (19:1)) und Lyophilisation aus Dioxan. FAB-MS: (M+H)⁺=630, t_{Ret}(V)=13,5 min., R_{f}(A')=0,27.

Die Ausgangsmaterialien werden folgendermassen hergestellt:

### a) 1-[2(S)-Hydroxy-3(S)-(N-acetyl-(L)-valyl)amino-4-phenylbutyl]-1-[4-methoxyphenylmethyl]-2-[N-acetyl-(L)-valyl]hydrazin

Analog Referenzbeispiel 37 erhält man aus 200 mg (0,47 mMol) 1-[2(S)-Hydroxy-3(S)-amino-4-phenylbutyl]-1-[4-methoxyphenylmethyl]-hydrazin · 3HCl, 225 mg (1,41 mMol) N-Acetyl-(L)-valin, 624 mg (1,41 mMol) BOP, 191 mg (1,41 mMol) HOBt und 9,4 ml 0,3M N-Methylmorpholin in DMF die Titelverbindung nach chromatographischer Reinigung (SiO₂, Methylenchlorid/Methanol (12:1)) und Lyophilisation aus Dioxan/Wasser/tert.-Butanol. FAB-MS: (M+H)⁺=598, t_{Ret}(V)=11,2 min., R_{f}(I')=0,25.

### b) 1-[2(S)-Hydroxy-3(S)-amino-4-phenylbutyl]-1-[4-methoxyphenylmethyl]-hydrazin·3HCl:

Analog Referenzbeispiel 2a erhält man ausgehend von 2,65 g (5,14 mMol) 1-[2(S)-Hydroxy-3(S)-tert-butoxycarbonylamino-4-phenylbutyl]-1-[4-methoxyphenylmethyl]-2-[tert-butoxycarbonyl]hydrazin nach Lyophilisation die Titelverbindung. ¹H-NMR (200 MHz, CD₃OD): 7,42 - 7,15 (m, 7H), 6,92 (d, J=8Hz, 2H), 4,1 - 3,8 (m, 3H), 3,75 (s, 3H), 3,55 (m, 1H), 3,1 (m, br, 2H), 2,75 (m, br, 2H).

### c) 1-[2(S)-Hydroxy-3(S)-tert-butoxycarbonylamino-4-phenylbutyl]-1-[4-methoxyphenylmethyl]-2-[tert-butoxycarbonyl]hydrazin:

Analog Referenzbeispiel 1 erhält man ausgehend von 3,13 g (11,9 mMol) (2R,3S)-1-[3-Boc-amino-2-phenylethyl]oxiran und 3,0 g (11,9 mMol) tert.-Butyl-3-(4-methoxyphenyl-methyl)-carbazat die Titelverbindung nach Kristallisation aus Methanol/DIPE. ¹H-NMR (200 MHz, CD₃OD): 7,3 - 7,1 (m, 7H), 6,85 (d, J=8Hz, 2H), 3,78 (s, 3H), 3,65 (m, 4H), 2,9 - 2,5 (m, 4H), 1,25 (s, 9H), 1,20 (s, 9H), t_{Ret}(V)=16,6 min.

### d) tert-Butyl-3-(p-methoxyphenyl-methyl)-carbazat:

In Gegenwart von 11,5 g Pd/C 5 % werden 130 g (520 mMol) p-(Methoxy-phenyl)-carbaldehyd-tert-butoxycarbonylhydrazon in 1,3 l THF hydriert. Abfiltrieren des Katalysators durch ®Celite und Eindampfen des Filtrats liefert die Titelverbindung: DC R_{f}(F)=0,3; t_{Ret}(V)=8,9 min; ¹H-NMR (200 MHz, CD₃OD): 1,44 (s, 9 H), 3,77 (s, 3 H), 3,83 (s, 2 H), 6,87 und 7,26 (2d, J=8 Hz, je 2 H).

### e) p-(Methoxy-phenyl)-carbaldehyd-tert-butoxycarbonylhydrazon:

Unter Schutzgas werden 65 ml (534 mMol) frisch destillierter Anisaldehyd in 850 ml Ethanol gelöst, mit 70,6 g (534 mMol) tert-Butylcarbazat versetzt und 3 h auf 80°C erhitzt. Eindampfen des Reaktionsgemisches liefert die Titelverbindung: ¹H-NMR (200 MHz, CD₃OD): 1,53 (s, 9 H), 3,82 (s, 3 H), 6,94 und 7,64 (2d, J=9 Hz, je 2 H), 7,86 (s, 1 H).

### Beispiel 10:

Analog den vor- und nachstehenden Beispielen und Verfahren erhält man folgende Verbindungen:
A) 1-[2(S)-Hydroxy-3(S)-(N-acetyl-(L)-valyl)amino-4-phenylbutyl]-1-[4-biphenylylmethyl]-2-[N-acetyl-(L)-valyl]hydrazin
B) 1-[2(S)-Hydroxy-3(S)-(N-methoxycarbonyl-(L)-valyl)amino-4-phenylbutyl]-1-[4-biphenylylmethyl]-2-[N-methoxycarbonyl-(L)-valyl]hydrazin
C) 1-[2(S)-Hydroxy-3(S)-(N-ethoxycarbonyl-(L)-valyl)amino-4-phenylbutyl]-1-[4-biphenylylmethyl]-2-[N-ethoxycarbonyl-(L)-valyl]hydrazin

### Beispiel 11: 1-[2(S)-Hydroxy-3(S)-(N-(chinolin-2-carbonyl)-(L)-asparaginyl)amino-4-phenyl-butyl]-1-[benzyl]-2-[N-benzyloxy-carbonyl)-(L)-valyl]hydrazin:

Unter N₂-Atmosphäre werden 50 mg (0,198 mMol) Z-(L)-Valin und 100 mg (0,18 mMol) 1-[2(S)-Hydroxy-3(S)-(N-(chinolin-2-carbonyl)-(L)-asparaginyl)amino-4-phenyl-butyl]-1-[phenylmethyl]hydrazin in 1,8 ml einer 0,3 M lösung von NMM in DMF gelöst, mit 75,1 mg (0,198 mMol) HBTU versetzt und 18 h bei RT gerührt. Das Reaktionsgemisch wird am HV eingedampft, der Rückstand in Essigsäureethylester aufgenommen, mit 2 Portionen 10 %-iger Zitronensäure-Lösung, Wasser, ges. NaHCO₃-Lösung und Sole gewaschen. Die wässrigen Phasen werden noch 2x mit Essigsäureethylester extrahiert, die vereinigten organischen Phasen mit Na₂SO₄ getrocknet und eingedampft. Eine Säulenchromatographie (SiO₂, Methylenchlorid/Methanol 19:1) liefert die Titelverbindung: DC R_{f}(J')=0,27; t_{Ret}(V)=15,7 min; FAB-MS (M+H)⁺=788.

Das Ausgangsmaterial wird wie folgt hergestellt:

### a) 1-[2(S)-Hydroxy-3(S)-(N-(chinolin-2-carbonyl)-(L)-asparaginyl)amino-4-phenyl-butyl]-1-[phenylmethyl]hydrazin

In 10 ml Ameisensäure wird 1,0 g (1,53 mMol)
1-[2(S)-Hydroxy-3(S)-(N-(chinolin-2-carbonyl)-(L)-asparaginyl)amino-4-phenyl-butyl]-1-[phenylmethyl]-2-[tert-butoxy-carbonyl]hydrazin unter Schutzgas gelöst und während 16 h bei RT gerührt. Abdampfen der Ameisensäure am HV, Verteilen des Rückstandes zwischen 3 Portionen Essigsäureethylester, ges. NaHCO₃-Lösung und Sole, Trocknen der organischen Phasen mit Na₂SO₄ und Eindampfen liefert die Titelverbindung: t_{Ret}(V)=10,7 min.

### b) 1-[2(S)-Hydroxy-3(S)-(N-(chinolin-2-carbonyl)-(L)-asparaginyl)amino-4-phenyl-butyl]-1-[phenylmethyl]-2-[tert-butoxy-carbonyl]hydrazin:

Bei 0°C werden 4,69 g (12,2 mMol) 1-[2(S)-Hydroxy-3(S)-amino-4-phenyl-butyl]-1-[phenylmethyl]-2-[tert-butoxy-carbonyl]hydrazin in 250 ml THF mit 3,84 g (13,4 mMol) Chinolin-2-carbonyl-(L)-asparagin (Hydrochloridsalz) (Beispiel 11 f)) versetzt. Zur Suspension gibt man 2,18 g (13,4 mMol) HOBT, 2,76 g (13,4 mMol) DCC und 2,14 ml (19,5 mMol) NMM und rührt während 30 min bei 0°C und 17 h bei RT nach. Das Reaktionsgemisch wird filtriert und das Filtrat auf ein Restvolumen von ca. 50 ml eingedampft. Die feine Suspension wird in Methylenchlorid aufgenommen mit NaHCO₃-Lösung und Sole gewaschen und die wässrigen Phasen werden mit 2 Portionen Methylenchlorid extrahiert. Filtrieren der vereinigten organischen Phasen durch Watte, Eindampfen und Fällen aus einer konzentrierten Lösung in Methanol/Methylenchlorld mit DIPE und schliesslich Hexan liefert die reine Titelverbindung: DC R_{f}(P)=0,41; t_{Ret}(V)=14,8 min; FAB-MS (M+H)⁺=655.

### c) 1-[2(S)-Hydroxy-3(S)-(trifluoracetyl-amino)-4-phenyl-butyl]-1-[phenylmethyl]-2-[tert-butoxy-carbonyl]hydrazin: (verbesserte Version für Referenzbeisp. 30a))

Unter N₂-Atmosphäre werden 20,49 g (79 mMol) 2(R)-[1'(S)-(Trifluoracetyl-amino)-2'-phenylethyl]-oxiran (Beispiel 12 g)) und 17,56 g (79 mMol) tert-Butyl-3-benzyl-carbazat (J. Chem., Perkin I, 1712 (1975)) in 300 ml Ethanol während 20 h auf 80°C erhitzt. Abkühlen und teilweises Eindampfen bis zur beginnenden Kristallisation, Filtrieren und Waschen mit wenig Ethanol liefert die reine Titelverbindung: t_{Ret}(V)=16,1 min; FAB-MS (M+H)⁺=482; ¹H-NMR (200 MHz, CD₃OD): 1,30 s, 9 H), 2,70 (m, 2 H), 2,83-3,08 (m, 2 H), 3,76 (m, 1 H), 3,85 (s, 2 H), 4,21 (m, 1 H), 7,2-7,4 (m, 10 H).

### d) 1-[2(S)-Hydroxy-3(S)-amino-4-phenyl-butyl]-1-[phenylmethyl]-2-[tert-butoxy-carbonyl]hydrazin: (verbesserte Version für Referenzbeisp. 30b))

Unter N₂-Atmosphäre werden 6,0 g (12,5 mMol) 1-[2(S)-Hydroxy-3(S)-(trifluoracetyl-amino)-4-phenyl-butyl]-1-[phenylmethyl]-2-[tert-butoxycarbonyl]hydrazin gelöst in 420 ml MeOH auf 80°C erhitzt, tropfenweise mit 125 ml 1 M wässriger K₂CO₃-Lösung versetzt (15 min) und während 18 h bei 80°C gerührt. Das Reaktionsgemisch wird eingedampft und der Rückstand verteilt zwischen 3 Portionen Methylenchlorid, Wasser und Sole. Eindampfen der durch Watte filtrierten organischen Phasen liefert die Titelverbindung: t_{Ret}(V)=11,5 min; ¹H-NMR (200 MHz, CD₃OD): 1,29 (s, 9 H), 2,5-3,05 (m, 5 H), 3,56 (m, 1 H), 3,8-3,95 (AB, 2 H), 7,1-7,4 (m, 10 H).

### e) Chinolin-2-carbonyl-(L)-asparagin-tert-butylester:

Es werden 5,45 g (31,4 mMol) Chinaldinsäure in 161 ml THF mit 7,08 g (34,3 mMol) DCC, 4,63 g (34,3 mMol) HOBT und 5,38 g (28,6 mMol) (L)-Asparagin-tert-butylester (Bachem, Bubendorf/Schweiz) umgesetzt. Filtration, Extraktion (Verteilen des Rückstandes zwischen 3 Portionen Essigsäureethylester, ges. NaHCO₃-lösung, Wasser und Sole, Eindampfen der mit Na₂SO₄ getrockneten organischen Phasen) und Säulenchromatographie (SiO₂, Essigsäureethylester/Hexan 3:1) liefert die reine Titelverbindung: DC R_{f}(C')=0,15; t_{Ret}(V)=12,2 min; FAB-MS (M+H)⁺=344.

### f) Chinolin-2-carbonyl-(L)-asparagin (Hydrochloridsalz):

Unter N₂-Atmosphäre werden 4,0 g (11,6 mMol) Chinolin-2-carbonyl-(L)-asparagin-tert-butylester in 40 ml Dioxan gelöst und mit 40 ml 4 N HCl/Dioxan versetzt. Beim Rühren während 17 h bei RT scheidet sich das Produkt als Festkörper aus. Filtrieren und Waschen mit DIPE liefert die reine Titelverbindung: ¹H-NMR (200 MHz, CD₃OD): 3,02 (d, J=6 Hz, 2 H), 5,09 (t, J=6 Hz, 1 H), 7,92 (m, 1 H), 8,12 (m, 1 H), 8,26 (m, 1 H), 8,4 (m, 2 H), 9,03 (m, 1 H).

### Beispiel 12: 1-[2(S)-Hydroxy-3(S)-(N-(chinolin-2-carbonyl)-(L)-asparaginyl)amino-4-phenyl-butyl]-1-[p-(methoxy-phenyl)methyl]-2-[N-(benzyloxy-carbonyl)-(L)-valyl]-hydrazin:

Unter N₂-Atmosphäre werden 330 mg (1,32 mMol) Z-(L)-Valin und 700 mg (1,197 mMol) 1-[2(S)-Hydroxy-3(S)-(N-(chinolin-2-carbonyl)-(L)-asparaginyl)amino-4-phenyl-butyl]-1-[p-(methoxy-phenyl)methyl]hydrazin in 11,6 ml einer 0,3 M Lösung von NMM in DMF gelöst, mit 0,50 g (1,32 mMol) HBTU versetzt und 18 h bei RT gerührt. Das Reaktionsgemisch wird am HV eingedampft, der Rückstand in Essigsäureethylester aufgenommen, mit ges. NaHCO₃-lösung, Wasser und Sole gewaschen. Die wässrigen Phasen werden noch mit Essigsäureethylester extrahiert und die vereinigten organischen Phasen mit Na₂SO₄ getrocknet und eingedampft. Eine Säulenchromatographie (SiO₂, Essigsäureethylester) liefert nach Kristallisation aus DIPE die Titelverbindung: DC R_{f}(G')=0,50; t_{Ret}(V)=15,4 min.

Das Ausgangsmaterial wird wie folgt hergestellt:

### a) 1-[2(S)-Hydroxy-3(S)-(N-(chinolin-2-carbonyl)-(L)-asparaginyl)amino-4-phenyl-butyl]-1-[p-(methoxy-phenyl)methyl]hydrazin

Analog Beispiel 11 a) werden 4,6 g (6,71 mMol) 1-[2(S)-Hydroxy-3(S)-(N-(chinolin-2-carbonyl)-(L)-asparaginyl)amino-4-phenyl-butyl]-1-[p-(methoxy-phenyl)- methyl]-2-[tert-butoxy-carbonyl]hydrazin in 168 ml Ameisensäure zur Titelverbindung umgesetzt: t_{Ret}(V)=10,8 min.

### b) 1-[2(S)-Hydroxy-3(S)-(N-(chinolin-2-carbonyl)-(L)-asparaginyl )amino-4-phenylbutyl]-1-[p-(methoxy-phenyl)methyl]-2-[tert-butoxy-carbonyl]hydrazin:

Analog Beispiel 11 werden 7,00 g (16,8 mMol) 1-[2(S)-Hydroxy-3(S)-amino-4-phenyl-butyl]-1-[p-(methoxy-phenyl)methyl]-2-[tert-butoxy-carbonyl]hydrazin in 420 ml THF mit 5,3 g (18,5 mMol) Chinolin-2-carbonyl-(L)-asparagin (Hydrochloridsalz) (Beispiel 11 f)), 3,0 g (18,5 mMol) HOBT, 3,8 g (18,5 mMol) DCC und 5 ml NMM umgesetzt. Das Reaktionsgemisch wird filtriert, das Filtrat eingedampft, der Eindampfrückstand in Essigsäureethylester aufgenommen und mit NaHCO₃-lösung und Sole je 2x gewaschen. Die wässrigen Phasen werden mit 2 Portionen Essigsäureethylester extrahiert, die organischen Phasen mit Na₂SO₄ getrocknet und eingedampft. Säulenchromatographie (SiO₂, Essigsäureethylester) liefert die reine Titelverbindung: DC R_{f}(O)=0,59; t_{Ret}(V)=14,5 min; FAB-MS (M+H)⁺=685.

### c) p-(Methoxy-phenyl)-carbaldehyd-tert-butoxycarbonylhydrazon:

Unter Schutzgas werden 65 ml (534 mMol) frisch destillierter Anisaldehyd in 850 ml Ethanol gelöst, mit 70,6 g (534 mMol) tert-Butylcarbazat versetzt und 3 h auf 80°C erhitzt. Eindampfen des Reaktionsgemisches liefert die Titelverbindung: ¹H-NMR (200 MHz, CD₃OD): 1,53 (s, 9H), 3,82 (s, 3H), 6,94 und 7,64 (2d, J=9 Hz, je 2H), 7,86 (s, 1 H).

### d) tert-Butyl-3-(p-methoxyphenyl-methyl)-carbazat:

In Gegenwart von 11,5 g Pd/C 5 % werden 130 g (520 mMol) p-(Methoxy-phenyl)-carbaldehyd-tert-butoxycarbonylhydrazon in 1,3 l THF hydriert. Abfiltrieren des Katalysators durch ®Celite und Eindampfen des Filtrats liefert die Titelverbindung: DC R_{f}(F)=0,3; t_{Ret}(V)=8,9 min; ¹H-NMR (200 MHz, CD₃OD): 1,44 (s, 9 H), 3,77 (s, 3 H), 3,83 (s, 2 H), 6,87 und 7,26 (2d, J=8 Hz, je 2 H).

### e) 1-[2(S)-Hydroxy-3(S)-(trifluoracetyl-amino)-4-phenyl-butyl]-1-[p-(methoxy-phenyl)-methyl]-2-[tert-butoxy-carbonyl]hydrazin:

Unter Schutzgas werden 15 g (57,9 mMol) 2(R)-[1'(S)-(Trifluoracetyl-amino)-2'-phenylethyl]-oxiran und 14,6 g (57,9 mMol) tert-Butyl-3-(p-methoxyphenyl-methyl)-carbazat in 220 ml Ethanol während 18 h auf 80°C erhitzt. Abkühlen, Eindampfen und Digerieren in DIPE liefert die Titelverbindung: DC R_{f}(F)=0,42; t_{Ret}(V)=15,8 min.

### f) 1-[2(S)-Hydroxy-3(S)-amino-4-phenyl-butyl]-1-[p-(methoxy-phenyl)-methyl]-2-[tert-butoxy-carbonyl]hydrazin:

Analog Beispiel 11 d) werden 19,7 g (38,4 mMol) 1-[2(S)-Hydroxy-3(S)-(trifluoracetyl-amino)-4-phenyl-butyl]-1-[p-(methoxy-phenyl)methyl]-2-[tert-butoxy-carbonyl]-hydrazin in 1 l Methanol mit 384 ml 1 M K₂CO₃-Lösung zur Titelverbindung hydrolysiert: DC R_{f}(G')=0,4.

### g) 2(R)-[1'(S)-(Trifluoracetyl-amino)-2'-phenylethyl]-oxiran (Alternative zu Referenzbeispiel 16d):

Eine lösung von 14,5 g (60 mMol) N-Trifluoracetyl-1-phenyl-3-buten-2(S)-amin (Referenzbeispiel 16c) in 600 ml Chloroform versetzt man mit 54,28 g (314 mMol) m-Chlorperbenzoesäure und rührt sie 16 h bei RT aus. Das Reaktionsgemisch wird 2x mit 10 %-iger Natriumsulfitlösung, 2x mit ges. Natriumcarbonatlösung, mit Wasser und zuletzt mit Sole gewaschen. Die wässrigen Phasen werden noch 2x mit Methylenchlorid extrahiert, und die vereinigten organischen Phasen werden mit Natriumsulfat getrocknet und eingedampft. Säulenchromatographie (SiO₂, Hexan/Essigsäureethylester 2:1) liefert die Titelverbindung als 6:1 Gemisch der (2R)- und (2S)-Epimeren: DC R_{f}(F)=0,41, DC R_{f}(N)=0,6; t_{Ret}(V)=12,6 min; ¹H-NMR (200 MHz, CDCl₃): u.a. 4,08 (m, 1/7H, H-C(2(S))), 4,47 (m, 6/7H, H-C(2(R))).

### Beispiel 13: Gelatine-Lösung:

Eine sterilfiltrierte wässrige Lösung mit 20 % Cyclodextrinen als Lösungsvermittler von einer der in den vorausgehenden Beispielen genannten Verbindungen der Formel I als Wirkstoff wird unter Erwärmen mit einer sterilen Gelatinelösung, welche als Konservierungsmittel Phenol enthält, unter aseptischen Bedingungen so vermischt, dass 1,0 ml Lösung die folgende Zusammensetzung hat:

| | |
|---|---|
| Wirkstoff | 3 mg |
| Gelatine | 150,0 mg |
| Phenol | 4,7 mg |
| dest. Wasser mit 20 % Cyclodextrinen als Lösungsvermittler | 1,0 ml |

### Beispiel 14: Sterile Trockensubstanz zur Injektion:

Man löst 5 mg einer der in den vorausgehenden Beispielen genannten Verbindungen der Formel I als Wirkstoff in 1 ml einer wässrigen Lösung mit 20 mg Mannit und 20 % Cyclodextrinen als Lösungsvermittler. Die lösung wird sterilfiltriert und unter aseptischen Bedingungen in eine 2 ml-Ampulle gefüllt, tiefgekühlt und lyophilisiert. Vor dem Gebrauch wird das Lyophilisat in 1 ml destilliertem Wasser oder 1 ml physiologischer Kochsalzlösung gelöst. Die Lösung wird intramuskulär oder intravenös angewendet. Diese Formulierung kann auch in Doppelkammerspritzampullen abgefüllt werden.

### Beispiel 15: Nasenspray:

In einer Mischung von 3,5 ml Myglyol 812® und 0,08 g Benzylalkohol werden 500 mg fein gemahlenes (<5,0 µm) Pulver einer der in den vorausgehenden Beispielen genannten Verbindungen der Formel I als Wirkstoff suspendiert. Diese Suspension wird in einen Behälter mit Dosierventil eingefüllt. Es werden 5,0 g Freon 12® unter Druck durch das Ventil in einen Behälter abgefüllt. Durch Schütteln wird das "Freon" in der Myglyol-Benzylalkoholmischung gelöst. Dieser Spraybehälter enthält ca. 100 Einzeldosen, die einzeln appliziert werden können.

### Beispiel 16: Lacktabletten

Für die Herstellung von 10 000 Tabletten enthaltend je 100 mg Wirkstoff werden folgende Bestandteile verarbeitet:

| | |
|---|---|
| Wirkstoff | 1000 g |
| Maisstärke | 680 g |
| Kolloidale Kieselsäure | 200 g |
| Magnesiumstearat | 20 g |
| Stearinsäure | 50 g |
| Natriumcarboxymethylstärke | 250 g |
| Wasser | quantum satis |

Ein Gemisch von einer der in den vorausgehenden Beispielen genannten Verbindungen der Formel I als Wirkstoff, 50 g Maisstärke und der kolloidalen Kieselsäure wird mit Stärkekleister aus 250 g Maisstärke und 2,2 kg entmineralisiertem Wasser zu einer feuchten Masse verarbeitet. Diese wird durch ein Sieb von 3 mm Maschenweite getrieben und bei 45 ° während 30 min im Wirbelschichttrockner getrocknet. Das getrocknete Granulat wird durch ein Sieb von 1 mm Maschenweite gedrückt, mit einer vorher gesiebten Mischung (1 mm Sieb) von 330 g Maisstärke, des Magnesiumstearats, der Stearinsäure und der Natriumcarboxymethylstärke gemischt und zu schwach gewölbten Tabletten verpresst.

## Patentansprüche

1. Verbindungen der Formel 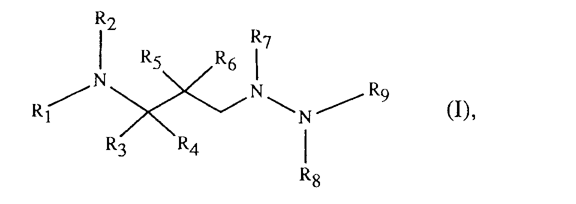 worin R₁ und R₉ unabhängig voneinander Wasserstoff, Acyl, unsubstituiertes oder substituiertes Alkyl; Sulfo; oder durch unsubstituiertes oder substituiertes Alkyl, Aryl oder Heterocyclyl substituiertes Sulfonyl bedeuten, mit der Massgabe, dass höchstens einer der Reste R₁ und R₉ Wasserstoff bedeutet; und R₂ und R₈ jeweils unabhängig voneinander Wasserstoff oder unsubstituiertes oder substituiertes Alkyl bedeuten;
R₃ und R₄ unabhängig voneinander Wasserstoff, unsubstituiertes oder substituiertes Alkyl, unsubstituiertes oder substituiertes Cycloalkyl oder Aryl bedeuten;
R₅ Acyloxy bedeutet;
R₆ Wasserstoff bedeutet;
und R₇ unsubstituiertes oder substituiertes Alkyl, unsubstituiertes oder substituiertes Cycloalkyl oder Aryl bedeutet;
sowie Salze der genannten Verbindungen, sofern salzbildende Gruppen vorliegen;
wobei die Verbindung ausgeschlossen ist, worin R₁ und R₉ jeweils Acetyl bedeuten, R₂, R₃, R₄, R₆ und R₈ jeweils Wasserstoff bedeutet, R₅ Acetyloxy bedeutet und R₇ 2,2-[N-Ethoxycarbonylmethyl)-N-methyl]hydrazin-1-ylcarbonylmethyl bedeutet.

2. Eine Verbindung der Formel I gemäss Anspruch 1, worin
R₁ und R₉ unabhängig voneinander
(a) Wasserstoff;
(b) Niederalkanoyl;
(c) Arylniederalkanoyl, worin Aryl 6 - 14 Kohlenstoffatome hat und unsubstituiert oder durch Niederalkyl, Halogenniederalkyl, Phenyl, 1- oder 2-Naphthyl, Hydroxy, Niederalkoxy, Carbamoylniederalkoxy, N-Niederalkylcarbamoylniederalkoxy, N,N-Diniederalkylcarbamoylniederalkoxy, Amino, Mono- oder Diniederalkylamino, Niederalkanoylamino, Halogen, Carboxy, Niederalkoxycarbonyl, Phenyl-, Naphthyl- oder Fluorenylniederalkoxycarbonyl, Niederalkanoyl, Sulfo, Niederalkylsulfonyl, Phosphono, Hydroxyniederalkoxy-phosphoryl oder Diniederalkoxyphosphoryl, Carbamoyl, Mono- oder Diniederalkylcarbamoyl, Sulfamoyl, Mono- oder Diniederalkylaminosulfonyl, Nitro und/oder Cyano ein- oder bis zu dreifach substituiert sein kann, und worin Niederalkanoyl unsubstituiert oder substituiert ist durch Carbamoyl oder durch einen oder zwei aus Niederalkyl, Carboxyniederalkyl, Niederalkoxycarbonylniederalkyl, Diniederalkylamino-niederalkyl, Hydroxyniederalkyl und Diniederalkoxyniederalkyl ausgewählte Reste am Stickstoff substituiertes Carbamoyl;
(d) Heterocyclylniederalkanoyl, worin Heterocyclyl Thienyl, Furyl, Pyrrolyl, Imidazolyl, Pyrazolyl, Oxazolyl, Thiazolyl, Tetrazolyl, Pyridyl, Pyrazinyl, Pyrimidinyl, Pyridazinyl, Indolyl, Benzimidazolyl, Chinolyl, Isochinolyl, 3,1-Benzfuranyl, Cyclohexa[b]pyrrolyl, Cyclohexa[b]pyridyl, Cyclohexa[b]pyrazinyl, Cyclohexa[b]pyrimidinyl, Pyrrolidinyl, Pyrrolinyl, Imidazolidyl, Piperidyl, Piperazinyl, Morpholinyl, Thiomorpholinyl, S,S-Dioxo-thiomorpholinyl, Indolinyl, Isoindolinyl, 4,5,6,7-Tetrahydroindolyl, 1,2,3,4-Tetrahydrochinolyl oder 1,2,3,4-Tetrahydroisochinolyl bedeutet, welches über ein Ringkohlenstoffatom oder ein Ringstickstoffatom gebunden ist;
(e) (Niederalkoxyniederalkoxy)niederalkanoyl;
(f) durch Heterocyclylniederalkanoyl am Aminostickstoff substituiertes Aminoniederalkanoyl, worin Heterocyclylniederalkanoyl unabhängig wie oben unter (d) für Heterocyclylniederalkanoyl R₁ oder R₉ definiert ist;
(g) Halogenniederalkanoyl, welches bis zu drei Halogenatome enthält;
(h) (N-Heterocyclylniederalkyl-carbamoyl)-niederalkanoyl, worin Heterocyclyl aus Pyrrolyl, Furyl, Thienyl, Imidazolyl, Pyrazolyl, Oxazolyl, Thiazolyl, Pyridyl, Pyrazinyl, Pyrimidinyl, Indolyl, Chinolyl und Isochinolyl, die auch ganz oder teilweise gesättigt sein können, aus Morpholinyl und aus Thiomorpholinyl ausgewählt ist;
(i) Niederalkoxycarbonyl;
(j) Arylniederalkoxycarbonyl, worin Aryl Phenyl, Biphenylyl, 1- oder 2-Naphthyl, Fluorenyl oder durch Niederalkyl, Hydroxy, Niederalkoxy, Halogen und/oder Nitro mono- oder mehrfach substituiertes Phenyl ist;
(k) Heterocyclylniederalkoxycarbonyl, worin Heterocyclyl aus Pyrrolyl, Furyl, Thienyl, Imidazolyl, Pyrazolyl, Oxazolyl, Thiazolyl, Pyridyl, Pyrazinyl, Pyrimidinyl, Indolyl, Chinolyl und Isochinolyl, die auch ganz oder teilweise gesättigt vorliegen können, und aus Morpholinyl und aus Thiomorpholinyl ausgewählt ist und unsubstituiert oder durch Niederalkyl substituiert ist;
(l) Niederalkenyloxycarbonyl, worin der Niederalkenylrest an den Sauerstoff über ein gesättigtes Kohlenstoffatom gebunden ist;
(m) Niederalkoxyniederalkoxycarbonyl;
(n) (Niederalkoxyniederalkoxy)niederalkoxycarbonyl;
(o) Niederalkansulfonyl;
(p) Heterocyclylsulfonyl, worin Heterocyclyl aus Pyrrolyl, Furyl, Thienyl, Imidazolyl, Pyrazolyl, Oxazolyl, Thiazolyl, Pyridyl, Pyrazinyl, Pyrimidinyl, Indolyl, Chinolyl und Isochinolyl, die auch ganz oder teilweise gesättigt vorliegen können, aus Morpholinyl und aus Thiomorpholinyl ausgewählt ist und unsubstituiert oder durch Niederalkyl substituiert sein kann;
(q) Carbamoyl;
(r) N-Heterocyclylniederalkyl-N-niederalkyl-carbamoyl, worin Heterocyclyl unabhängig einen der oben unter (d) bei der Definition von Heterocyclylniederalkanoyl R₁ oder R₉ für Heterocyclyl genannten Reste bedeutet; oder
(s) einen über die Carbonylgruppe der 1-Carboxyfunktion gebundenen Acylrest einer Aminosäure, deren Aminofunktion frei oder durch einen der übrigen bisher für R₁ und R₉ genannten Reste acyliert ist, bedeuten, wobei die Aminosäurereste aus den über das Carbonyl ihrer 1-Carboxygruppe gebundenen Radikalen der Aminosäuren Glycin, Alanin, Valin, Norvalin, Leucin, Isoleucin, Norleucin, Serin, Homoserin, Threonin, Methionin, Cystein, Prolin, trans-3- und trans-4-Hydroxyprolin, Phenylalanin, Tyrosin, 4-Aminophenylalanin, 4-Chlorphenylalanin, 4-Carboxyphenylalanin, β-Phenylserin, Phenylglycin, α-Naphthylalanin, Cyclohexylalanin, Cyclohexylglycin, Tryptophan, Indolin-2-carbonsäure, 1,2,3,4-Tetrahydroisochinolin-3-carbonsäure, Asparaginsäure, Asparagin, Aminomalonsäure, Aminomalonsäure-monoamid, Glutaminsäure, Glutamin, Histidin, Arginin, Lysin, δ-Hydroxylysin, Ornithin, 3-Aminopropansäure, α,γ-Diaminobuttersäure und α,β-Diaminopropionsäure ausgewählt sind,
wobei (ausser Glycin) jede der genannten Aminosäuren in der D-, L- oder (D,L)-Form vorliegen kann;
und wobei die α-Aminogruppe unsubstituiert oder durch einen der oben für R₁ und R₉ unter (a) bis (r) genannten Reste N-acyliert ist,
mit der Massgabe, dass höchstens einer der beiden Reste R₁ oder R₉ Wasserstoff bedeuten kann,
R₂, R₄, R₆ und R₈ Wasserstoff bedeuten,
R₃
(i) Niederalkyl;
(ii) C₃-C₇Cycloalkylniederalkyl, worin C₃-C₇-Cycloalkyl unsubstituiert oder durch Niederalkyl, Halogenniederalkyl, Hydroxy, Niederalkoxy, Amino, Mono- oder Diniederalkylamino, Halogen, Nitro und/oder Cyano ein- oder bis zu dreifach substituiert ist; oder
(iii) Arylniederalkyl bedeutet, worin Aryl unabhängig wie in Arylniederalkanoyl R₁ oder R₉ definiert ist;
R₅ Niederalkanoyloxy, Octanoyloxy, Nonanoyloxy, Decanoyloxy, Undecanoyloxy, Dodecanoyloxy, Hydroxyniederalkanoyloxy, Niederalkoxyniederalkanoyloxy, Niederalkanoyloxyniederalkanoyloxy, Halogenniederalkanoyloxy, Carboxyniederalkanoyloxy, Niederalkoxycarbonylniederalkanoyloxy, Carbamoylniederalkanoyloxy, Niederalkylcarbamoylniederalkanoyloxy, Diniederalkylcarbamoylniederalkanoyloxy, Hydroxy-carboxy-niederalkanoyloxy, Hydroxy-niederalkoxycarbonyl-niederalkanoyloxy, Dihydroxy-carboxy-niederalkanoyloxy, Dihydroxyniederalkoxycarbonyl-niederalkanoyloxy, Pyrrolylcarbonyloxy, Furylniederalkanoyloxy, Thienylcarbonyloxy, Imidazolylniederalkanoyloxy, Pyridylniederalkanoyloxy, Indolylcarbonyloxy, Chinolylniederalkanoyloxy, Pyrrolidinylcarbonyloxy, Piperidinylcarbonyloxy, Morpholinocarbonyloxy, Thiomorpholinocarbonyloxy, Morpholinoacetyloxy, Thiomorpholinoacetyloxy oder 4-Niederalkyl-1-piperazinoacetyloxy, Niederalkenoyloxy, Niederalkinoyloxy, C₃-C₈-Cycloalkylcarbonyloxy, C₃-C₈-Cycloalkylacetoxy, Phenylniederalkanoyloxy, im Phenylrest unsubstituiert, mono- oder mehrfachsubstituiert durch Niederalkyl, Haloniederalkyl, Halogen, Hydroxy, Niederalkoxy, Piperidinomethyl, Piperazin-1-ylmethyl, 4-Niederalkyl-piperazin-1-ylmethyl, 4-Niederalkanoyl-piperazin-1-ylmethyl, Morpholinoniederalkyl, Thiomorpholinomethyl, Cyano und/oder Nitro, oder das über eine Carbonyloxygruppe, welche das Carbonyl aus der Carboxygruppe der betreffenden Aminosäure enthält, gebundene Radikal einer Aminosäure bedeutet, welche aus Glycin, Alanin, 2-Aminobuttersäure, 3-Aminobuttersäure, 4-Aminobuttersäure, 3-Aminopentansäure, 4-Aminopentansäure, 5-Aminopentansäure, 3-Aminohexansäure, 4-Aminohexansäure, 5-Aminohexansäure, Valin, Norvalin, Leucin, Isoleucin, Norleucin, Serin, Homoserin, Threonin, Methionin, Cystein, Prolin, Phenylalanin, Tyrosin, Cyclohexylalanin, Tryptophan, Asparaginsäure, Asparagin, Glutaminsäure, Glutamin, Histidin, Arginin, Lysin, Ornithin, 3-Aminopropansäure, α,γ-Diaminobuttersäure und α,β-Diaminopropionsäure ausgewählt ist,
wobei (ausser in Fällen, wo kein asymmetrisches Kohlenstoffatom vorliegt) jede der genannten Aminosäuren in der D-, L- oder (D,L)-Form vorliegen kann,
und worin eine Aminogruppe unsubstituiert oder ein- oder zweifach N-alkyliert durch Niederalkyl, durch Pyridylniederalkyl, und/oder durch Phenylniederalkyl, und/oder N-acyliert durch Niederalkanoyl, durch Phenylniederalkanoyl, durch Niederalkoxycarbonyl oder durch Phenylniederalkoxycarbonyl vorliegt, und
R₇ unabhängig von R₃ einen der oben für R₃ definierten Reste bedeutet
oder ein Salz davon, sofern wenigstens eine salzbildende Gruppe vorliegt.

3. Eine Verbindung der Formel I gemäss Anspruch 1, worin
R₁ und R₉ unabhängig voneinander
(a) Wasserstoff,
(b) Niederalkanoyl,
(c) Phenylniederalkanoyl,
(d) Phenylniederalkanoyl, worin der Niederalkanoylrest durch Carbamoyl substituiert ist,
(e) Morpholinoniederalkanoyl,
(f) Thiomorpholinoniederalkanoyl,
(g) Pyridylniederalkanoyl,
(h) Chinolylniederalkanoyl,
(i) Tetrazolylniederalkanoyl,
(j) durch N-Morpholino- oder N-Thiomorpholinocarbonyl am Aminostickstoff substituiertes Aminoniederalkanoyl,
(k) Halogenniederalkanoyl, welches bis zu drei Halogenatome enthält,
(l) 2-(N-Morpholinoniederalkyl-carbamoyl)-niederalkanoyl,
(m) 2-(N-Pyridylniederalkyl-carbamoyl)-niederalkanoyl,
(n) Niederalkoxycarbonyl,
(o) Phenylniederalkoxycarbonyl,
(p) Tetrahydrofuryl-niederalkoxycarbonyl,
(q) Niederalkenyloxycarbonyl,
(r) Niederalkoxyniederalkoxycarbonyl,
(s) (Niederalkoxyniederalkoxy)niederalkoxycarbonyl,
(t) Niederalkansulfonyl,
(u) Morpholinosulfonyl,
(v) Thiomorpholinosulfonyl,
(w) N-Pyridylniederalkyl-N-niederalkyl-carbamoyl, oder
(x) einen über das Carbonyl seiner Carboxygruppe gebundenen Acylrest einer Aminosäure ausgewählt aus Glycin, Alanin, Valin, Leucin, Isoleucin, Glutaminsäure und Asparagin in der (D)-, (L)- oder (D,L)-Form (ausser Glycin), bedeuten, worin die α-Aminogruppe unsubstituiert oder durch einen der übrigen, bisher unter (a) bis (w) genannten Reste R₁ oder R₉ acyliert ist;
mit der Massgabe, dass höchstens einer der Reste R₁ und R₉ Wasserstoff bedeutet,
R₂, R₄, R₆ und R₈ Wasserstoff bedeuten,
R₃ Niederalkyl, Cyclohexylniederalkyl oder Phenylniederalkyl, welches unsubstituiert oder durch Halogen, Niederalkoxy oder Cyano substituiert ist, bedeutet,
R₅ Niederalkanoyloxy, Octanoyloxy, Decanoyloxy, Dodecanoyloxy, Carboxyniederalkanoyloxy, Furylniederalkanoyloxy, Imidazolylniederalkanoyloxy, Pyridylniederalkanoyloxy, Chinolylniederalkanoyloxy, Aminoacetyloxy, N-Niederalkylaminoacetyloxy, N,N-Diniederalkylaminoacetyloxy, N-Niederalkyl-N-phenylniederalkoxycarbonylaminoacetyloxy, Phenylniederalkanoyloxy, 4-Morpholinoniederalkylbenzoyloxy, 4-Halogenmethylbenzoyloxy, Histidyloxy oder Prolyloxy bedeutet und R₇ dieselben Bedeutungen hat, wie für R₃ definiert,
oder ein pharmazeutisch verwendbares Salz davon, sofern wenigstens eine salzbildende Gruppe vorliegt.

4. Eine Verbindung der Formel I gemäss Anspruch 1, worin
R₁ Niederalkoxycarbonyl, Halogenniederalkoxycarbonyl, Phenylniederalkoxycarbonyl, das über Carbonyl gebundene monovalente Radikal einer aliphatischen Aminosäure ausgewählt aus Valin, Alanin, Leucin und Isoleucin oder das über Carbonyl gebundene Radikal einer am Aminostickstoff durch einen der Reste Phenylniederalkanoyl, Morpholinylniederalkanoyl, Thiomorpholinylniederalkanoyl, Pyridylniederalkanoyl, Niederalkoxycarbonyl oder Phenylniederalkoxycarbonyl acylierten, wie oben definierten aliphatischen Aminosäure, wobei alle genannten Aminosäuren in der D-, D,L- oder L-Form vorliegen, bedeutet,
R₂ Wasserstoff,
R₃ Phenylniederalkyl, 4-Fluorphenylniederalkyl oder Cyclohexylniederalkyl,
R₄ Wasserstoff,
R₅ Niederalkanoyloxy, Octanoyloxy, Decanoyloxy, Dodecanoyloxy, Carboxyniederalkanoyloxy, Furylniederalkanoyloxy, Imidazolylniederalkanoyloxy, Pyridylniederalkanoyloxy, Chinolylniederalkanoyloxy, Aminoacetyloxy (Glycyloxy), N-Niederalkylaminoacetyloxy, N,N-Diniederalkylaminoacetyloxy, N-Niederalkyl-N-phenylniederalkoxycarbonylaminoacetyloxy, Phenylniederalkanoyloxy, 4-Morpholinomethylbenzoyloxy, 4-Halogenmethylbenzoyloxy, Histidyloxy oder Prolyloxy,
R₆ Wasserstoff,
R₇ Niederalkyl, Cyclohexylniederalkyl, Phenylniederalkyl, 4-Cyanophenylniederalkyl oder 4-Fluorphenylniederalkyl,
R₈ Wasserstoff und
R₉ einen der oben für R₁ genannten Reste
bedeuten
und die die Reste R₃ und R₅ tragenden asymmetrischen Kohlenstoffatome in der S-Konfiguration vorliegen,
sowie pharmazeutisch verwendbare Salze davon.

5. Eine Verbindung der Formel I gemäss Anspruch 1, worin R₁ und R₉ N-Methoxycarbonylvalyl bedeuten, R₂, R₄, R₆ und R₈ Wasserstoff bedeuten, R₃ Benzyl oder Cyclohexylmethyl bedeutet, R₅ Niederalkanoyloxy oder Pyridylcarbonyloxy bedeutet und R₇ Cyclohexylmethyl oder Benzyl bedeutet, sowie pharmazeutisch verwendbare Salze davon.

6. Ein Isomeres einer Verbindung der Formel I gemäss Anspruch 5, worin das R₃ und das R₅ tragende Kohlenstoffatom in der (S)-Konfiguration vorliegen und die Reste R₁, R₂, R₃, R₄, R₅, R₆, R₇, R₈ und R₉ die in Anspruch 6 genannten Bedeutungen haben, oder pharmazeutisch verwendbare Salze davon.

7. 1-[2(S)-Acetoxy-3(S)-(N-(2-methoxyethoxycarbonyl)-(L)-valyl)amino-4-phenyl-butyl]-1-[cyclohexylmethyl]-2-[N-(2-methoxyethoxycarbonyl)-(L)-valyl]hydrazin gemäss Anspruch 1, oder pharmazeutisch verwendbare Salze davon.

8. 1-[2(S)-Acetoxy-3(S)-(N-(methoxycarbonyl)-(L)-valyl)amino-4-phenyl-butyl]-1-[cyclohexylmethyl]-2-[N-(methoxycarbonyl)-(L)-valyl]hydrazin gemäss Anspruch 1, oder pharmazeutisch verwendbare Salze davon.

9. 1-[2(S)-(2-Pyridylcarbonyl)oxy-3(S)-(N(methoxycarbonyl) -(L)-valyl)amino-4-phenyl-butyl]-1-[cyclohexylmethyl]-2-[N-(methoxycarbonyl)-(L)-valyl]hydrazin gemäss Anspruch 1, oder pharmazeutisch verwendbare Salze davon.

10. Eine Verbindung gemäss Anspruch 1 ausgewählt aus
1-[2(S)-Propionyloxy-3(S)-(N-(methoxycarbonyl)-(L)-valyl)amino-4-phenyl-butyl]-1-[cyclohexylmethyl]-2-[N-(methoxycarbonyl)-(L)-valyl]hydrazin;
1-[2(S)-Butyryloxy-3(S)-(N-(methoxycarbonyl)-(L)-valyl)amino-4-phenyl-butyl]-1-[cyclohexylmethyl]-2-[N-(methoxycarbonyl)-(L)-valyl]hydrazin;
1-[2(S)-Pentanoyloxy-3(S)-(N-(methoxycarbonyl)-(L)-valyl)amino-4-phenyl-butyl]-1-[cyclohexylmethyl]-2-[N-(methoxycarbonyl)-(L)-valyl]hydrazin;
1-[2(S)-Octanoyloxy-3(S)-(N-(methoxycarbonyl)-(L)valyl)amino-4-phenyl-butyl]-1-[cyclohexylmethyl]-2-[N-(methoxycarbonyl)-(L)-valyl]hydrazin;
1-[2(S)-Decanoyloxy-3(S)-(N-(methoxycarbonyl)-(L)-valyl)amino-4-phenyl-butyl]-1-[cyclohexylmethyl]-2-[N-(methoxycarbonyl)-(L)-valyl]hydrazin;
1-[2(S)-Dodecanoyloxy-3(S)-(N-(methoxycarbonyl)-(L)-valyl)amino-4-phenyl-butyl]-1-[cyclohexylmethyl]-2-[N-(methoxycarbonyl)-(L)-valyl]hydrazin;
1-[2(S)-Pivaloyloxy-3(S)-(N-(methoxycarbonyl)-(L)-valyl)amino-4-phenyl-butyl]-1-[cyclohexylmethyl]-2-[N-(methoxycarbonyl)-(L)-valyl]hydrazin;
1-[2(S)-(2-Furylcarbonyl)oxy-3(S)-(N-(methoxycarbonyl)-(L)-valyl)amino-4-phenylbutyl]-1-[cyclohexylmethyl]-2-[N-(methoxycarbonyl)-(L)-valyl]hydrazin;
1-[2(S)-(4-Imidazolylcarbonyl)oxy-3(S)-(N-(methoxycarbonyl)-(L)-valyl)amino-4-phenyl-butyl]-1-[cyclohexylmethyl]-2-[N-(methoxycarbonyl)-(L)-valyl]hydrazin;
1-[2(S)-(4-Imidazolylacetyl)oxy-3(S)-(N-(methoxycarbonyl)-(L)-valyl)amino-4-phenylbutyl]-1-[cyclohexylmethyl]-2-[N-(methoxycarbonyl)-(L)-valyl]hydrazin;
1-[2(S)-(3-(4-Imidazolyl)-propionyl)oxy-3(S)-(N-(methoxycarbonyl)-(L)-valyl)amino-4-phenyl-butyl]-1-[cyclohexylmethyl]-2-[N-(methoxycarbonyl)-(L)-valyl]hydrazin;
1-[2(S)-Benzoyloxy-3(S)-(N-(methoxycarbonyl)-(L)-valyl)amino-4-phenyl-butyl]-1-[cyclohexylmethyl]-2-[N-(methoxycarbonyl)-(L)-valyl]hydrazin;
1-[2(S)-(2-Pyridylacetyl)oxy-3(S)-(N-(methoxycarbonyl)-(L)-valyl)amino-4-phenylbutyl]-1-[cyclohexylmethyl]-2-[N-(methoxycarbonyl)-(L)-valyl]hydrazin;
1-[2(S)-(3-(Pyridin-2-yl)-propionyl)oxy-3(S)-(N-(methoxycarbonyl)-(L)-valyl)amino-4-phenylbutyl)]-1-[cyclohexylmethyl]-2-[N-(methoxycarbonyl)-(L)-valyl]hydrazin;
1-[2(S)-(Chinolin-2-ylcarbonyl)oxy-3(S)-(N-(methoxycarbonyl)-(L)-valyl)amino-4-phenyl-butyl]-1-[cyclohexylmethyl]-2-[N-(methoxycarbonyl)-(L)-valyl]hydrazin;
1-[2(S)-(Aminoacetyl)oxy-3(S)-(N-(methoxycarbonyl)-(L)-valyl)amino-4-phenyl-butyl]-1-[cyclohexylmethyl]-2-[N-(methoxycarbonyl)-(L)-valyl]hydrazin;
1-[2(S)-(N-Methylaminoacetyl)oxy-3(S)-(N-(methoxycarbonyl)-(L)-valyl)amino-4-phenyl-butyl]-1-[cyclohexylmethyl]-2-[N-(methoxycarbonyl)-(L)-valyl]hydrazin;
1-[2(S)-(N,N-Dimethylaminoacetyl)oxy-3(S)-(N-(methoxycarbonyl)-(L)-valyl)amino-4-phenyl-butyl]-1-[cyclohexylmethyl]-2-[N-(methoxycarbonyl)-(L)-valyl]hydrazin;
1-[2(S)-(N-Benzyloxycarbonyl-N-methyl-aminoacetyl)oxy-3(S)-(N-(methoxycarbonyl)-(L)-valyl)amino-4-phenyl-butyl]-1-[cyclohexylmethyl]-2-[N-(methoxycarbonyl)-(L)-valyl]hydrazin;
1-[2(S)-Prolyloxy -3(S)-(N-(methoxycarbonyl)-(L)-valyl)amino-4-phenyl-butyl]-1-[cyclohexylmethyl]-2-[N-(methoxycarbonyl)-(L)-valyl]hydrazin;
1-[2(S)-(4-Morpholinomethylbenzoyl)oxy-3(S)-(N-(methoxycarbonyl)-(L)-valyl)-amino-4-phenyl-butyl]-1-[cyclohexylmethyl]-2-[N-(methoxycarbonyl)-(L)-valyl]hydrazin;
1-[2(S)-(4-Chlormethylbenzoyl)oxy-3(S)-(N-(methoxycarbonyl)-(L)-valyl)amino-4-phenyl-butyl]-1-[cyclohexylmethyl]-2-[N-(methoxycarbonyl)-(L)-valyl]hydrazin; und
1-[2(S)-(3-Carboxypropionyl)oxy-3(S)-(N-(methoxycarbonyl)-(L)-valyl)amino-4-phenylbutyl]-1-[cyclohexylmethyl]-2-[N-(methoxycarbonyl)-(L)-valyl]hydrazin, oder ein pharmazeutisch verwendbare Salze davon.

11. 1-[2(S)-Butyryloxy-3(S)-(N-(methoxy-carbonyl)-(L)-valyl)amino-4-phenyl-butyl]-1-[cyclohexylmethyl]-2-[N-methoxy-carbonyl-(L)-valyl]hydrazin der Formel I gemäss Anspruch 1, oder ein pharmazeutisch verwendbares Salz davon.

12. 1-[2(S)-(Methoxy-acetoxy)-3(S)-(N-(methoxy-carbonyl)-(L)-v alyl)amino-4-phenyl-butyl]-1-[cyclohexylmethyl]-2-[N-methoxy-carbonyl-(L)-valyl]hydrazin der Formel I gemäss Anspruch 1, oder ein pharmazeutisch verwendbares Salz davon.

13. Eine Verbindung der Formel I oder ein pharmazeutisch verwendbares Salz davon gemäss einem der Ansprüche 1 bis 12 zur Anwendung in einem Verfahren zur diagnostischen oder therapeutischen Behandlung des menschlichen oder tierischen Körpers.

14. Verwendung einer Verbindung der Formel I gemäss einem der Ansprüche 1 bis 12, oder eines pharmazeutisch verwendbaren Salzes davon, zur Herstellung von pharmazeutischen Präparaten zur Behandlung von AIDS.

15. Pharmazeutische Präparate enthaltend Verbindungen der Formel I gemäss einem der Ansprüche 1 bis 12 oder ein pharmazeutisch verwendbares Salz einer solchen Verbindung mit mindestens einer salzbildenden Gruppe zusammen mit pharmazeutisch verwendbarem Trägermaterial.

16. Verfahren zur Herstellung von Verbindungen der Formel I gemäss Anspruch 1, dadurch gekennzeichnet, dass man entweder
a) eine Hydroxyverbindung der Formel II, 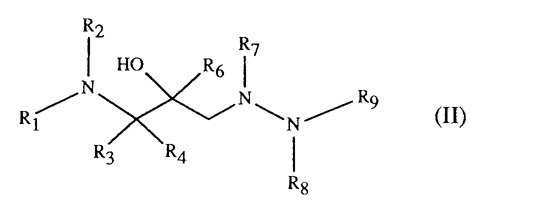 worin die Reste R₁ bis R₄ und R₆ bis R₉ die für Verbindungen der Formel I genannten Bedeutungen haben, mit einer Carbonsäure der Formel III,
R₅-H (III)
oder einem reaktionsfähigen Säurederivat davon, worin R₅ die für Verbindungen der Formel I genannten Bedeutungen hat, acyliert, wobei freie funktionelle Gruppen in den Ausgangsmaterialien der Formeln II und III, die nicht an der Reaktion teilnehmen sollen, erforderlichenfalls in geschützter Form vorliegen, und vorhandene Schutzgruppen abspaltet, oder
b) zur Herstellung von Verbindungen der Formel I, worin R₉ Acyl, Sulfo oder durch unsubstituiertes oder substituiertes Alkyl, Aryl oder Heterocyclyl substituiertes Sulfonyl bedeutet und die übrigen Reste R₁ bis R₈ die für Verbindungen der Formel I genannten Bedeutungen haben, eine Aminoverbindung der Formel 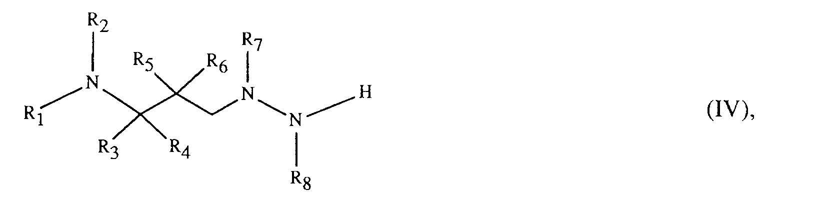 worin die Reste R₁ bis R₈ die für Verbindungen der Formel I genannten Bedeutungen haben, mit einer Säure der Formel
R₉'-OH (V)
oder reaktionsfähigen Säurederivaten davon, worin R₉' die oben unter b) für R₉ genannten Bedeutungen ausser Wasserstoff und unsubstituiertem oder substituiertem Alkyl hat, kondensiert, wobei freie funktionelle Gruppen mit Ausnahme der an der Reaktion beteiligten erforderlichenfalls in geschützter Form vorliegen, und vorhandene Schutzgruppen abspaltet, oder
c) zur Herstellung von Verbindungen der Formel I, worin R₁ Acyl, Sulfo oder durch unsubstituiertes oder substituiertes Alkyl, Aryl oder Heterocyclyl substituiertes Sulfonyl bedeutet und die übrigen Reste R₂ bis R₉ die für Verbindungen der Formel I genannten Bedeutungen haben, eine Aminoverbindung der Formel 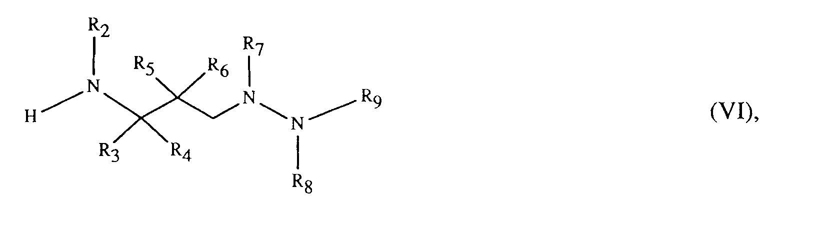 worin die Reste R₂ bis R₉ die für Verbindungen der Formel I genannten Bedeutungen haben, mit einer Säure der Formel
R₁'-OH (VII)
oder reaktionsfähigen Säurederivaten davon, worin R₁' die oben unter c) für R₁ genannten Bedeutungen ausser Wasserstoff und unsubstituiertem oder substituiertem Alkyl hat, kondensiert, wobei freie funktionelle Gruppen mit Ausnahme der an der Reaktion beteiligten erforderlichenfalls in geschützter Form vorliegen, und vorhandene Schutzgruppen abspaltet, oder
d) zur Herstellung von Verbindungen der Formel I, worin R₁ und R₉ zwei gleiche Reste ausgewählt aus Acyl, Sulfo und durch unsubstituiertes oder substituiertes Alkyl, Aryl oder Heterocyclyl substituiertes Sulfonyl bedeuten und die übrigen Reste R₂ bis R₈ die für Verbindungen der Formel I genannten Bedeutungen haben, eine Diaminoverbindung der Formel 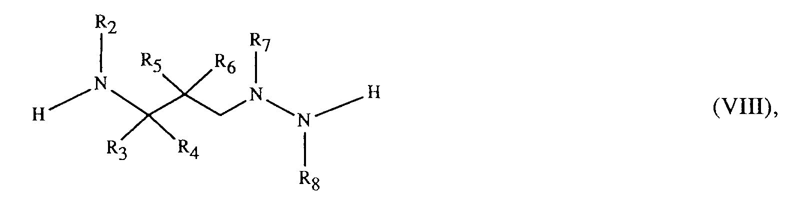 worin die Reste R₂ bis R₈ die für Verbindungen der Formel I genannten Bedeutungen haben, mit einer zur Einführung der identischen Reste R₁ und R₉ geeigneten Säure oder reaktionsfähigen Säurederivaten davon, worin R₁ und R₉ die gerade genannten Bedeutungen haben, kondensiert, wobei freie funktionelle Gruppen mit Ausnahme der an der Reaktion beteiligten erforderlichenfalls in geschützter Form vorliegen, und vorhandene Schutzgruppen abspaltet, oder
e) zur Herstellung einer Verbindung der Formel I, worin R₇ unsubstituiertes oder substituiertes Alkyl oder Cycloalkyl bedeutet und die übrigen Reste R₁ bis R₆, R₈ und R₉ die für Verbindungen der Formel I genannten Bedeutungen haben, in einer Verbindung der Formel I', 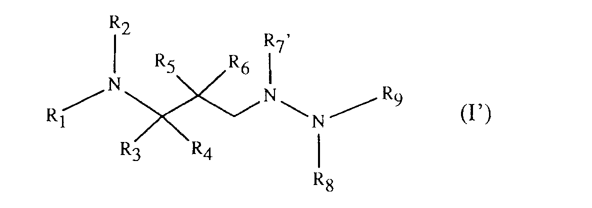 worin R₇' Wasserstoff bedeutet und die übrigen Reste R₁ bis R₆, R₈ und R₉ die für Verbindungen der Formel I genannten Bedeutungen haben, durch Substitution mit einer Verbindung der Formel XII,
R₇-X (XII)
worin X eine Abgangsgruppe ist und R₇ unsubstituiertes oder substituiertes Alkyl oder Cycloalkyl bedeutet, den Rest R₇ einführt, wobei freie funktionelle Gruppen mit Ausnahme der an der Reaktion beteiligten erforderlichenfalls in geschützter Form vorliegen, und gegebenenfalls vorhandene Schutzgruppen abspaltet,
und gewünschtenfalls als zusätzliche Verfahrensmassnahme eine nach einem der vorstehenden Verfahren a) bis e) erhältliche Verbindung der Formel I mit mindestens einer salzbildenden Gruppe in ihr Salz oder ein erhältliches Salz in die freie Verbindung oder in ein anderes Salz überführt und/oder gegebenenfalls erhältliche Isomerengemische auftrennt und/oder eine erfindungsgemässe Verbindung der Formel I in eine andere erfindungsgemässe Verbindung der Formel I umwandelt.

17. Eine Verbindung der in Anspruch 16 gezeigten Formel II, ausgewählt aus den Verbindungen mit der Bezeichnung
1-[2(S)-Hydroxy-3(S)-(N-(methoxycarbonyl)-(L)-valyl)-amino-4-phenyl-butyl]-1-[cyclohexylmethyl]-2-[N-(methoxycarbonyl)-(L)-valyl]hydrazin,
1-[2(S)-Hydroxy-3(S)-(N-(methoxycarbonyl)-(L)-valyl)-amino-4-phenyl-butyl]-1-[thien-2-ylmethyl]-2-[N-(methoxycarbonyl)-(L)-valyl]hydrazin,
1-[2(S)-Hydroxy-3(S)-(N-(chinolin-2-carbonyl)-(L)-asparaginyl)amino-4-phenyl-butyl]-1-[4-methoxyphenylmethyl]-2-[N-benzyloxy-carbonyl)-(L)-valyl]hydrazin,
1-[2(S)-Hydroxy-3(S)-(N-methoxycarbonyl-(L)-valyl)amino-4-phenylbutyl]-1-[4-methoxyphenylmethyl]-2-[N-methoxycarbonyl-(L)-valyl]hydr azin,
1-[2(S)-Hydroxy-3(S)-(N-(methoxycarbonyl)-(L)-valyl)amino-4-phenyl-butyl]-1-[4-biphenylylmethyl]-2-[N-(methoxycarbonyl)-(L)valyl]hydrazin,
1-[2(S)-Hydroxy-3(S)-(N-(ethoxycarbonyl)-(L)-valyl)-amino-4-phenyl-butyl]-1-[4-biphenylylmethyl]-2-[N-(ethoxycarbonyl)-(L)-valyl]hydrazin,
1-[2(S)-Hydroxy-3(S)-(N-(chinolin-2-carbonyl)-(L)-asparaginyl)-amino-4-phenyl-butyl]-1-[benzyl]-2-[N-(benzyloxycarbonyl)-(L)-valyl]hydrazin und
1-[2(S)-Hydroxy-3(S)-(N-acetyl-(L)-valyl)-amino-4-phenyl-butyl]-1-[4-biphenylylmethyl]-2-[N-acetyl-(L)-valyl]hydrazin,
oder ein pharmazeutisch verwendbares Salz davon.

18. Die Verbindung der in Anspruch 16 gezeigten Formel II mit der Bezeichnung 1-[2(S)-Hydroxy-3(S)-(N-(methoxycarbonyl)-(L)-valyl)-amino-4-phenyl-butyl]-1-[cyclohexylmethyl]-2-[N-(methoxycarbonyl)-(L)-valyl]hydrazin, oder ein pharmazeutisch verwendbares Salz davon.

19. Die Verbindung der in Anspruch 16 gezeigten Formel II mit der Bezeichnung 1-[2(S)-Hydroxy-3(S)-(N-(methoxycarbonyl)-(L)-valyl)-amino-4-phenyl-butyl]-1-[4-biphenylylmethyl]-2-[N-(methoxycarbonyl)-(L)-valyl]hydrazin, oder ein pharmazeutisch verwendbares Salz davon.

## Claims

1. A compound of formula 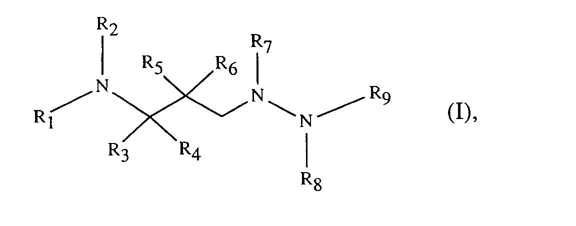 wherein
R₁ and R₉ are each independently of the other hydrogen; acyl; unsubstituted or substituted alkyl; sulfo; or sulfonyl substituted by unsubstituted or substituted alkyl, aryl or heterocyclyl, with the proviso that not more than one of the radicals R₁ and R₉ is hydrogen; and
R₂ and R₈ are each independently of the other hydrogen or unsubstituted or substituted alkyl;
R₃ and R₄ are each independently of the other hydrogen, unsubstituted or substituted alkyl, unsubstituted or substituted cycloalkyl or aryl;
R₅ is acyloxy;
R₆ is hydrogen; and
R₇ is unsubstituted or substituted alkyl, unsubstituted or substituted cycloalkyl or aryl; or a salt of such a compound where a salt-forming group is present, with the exception of the compound wherein R₁ and R₉ are each acetyl, R₂, R₃, R₄, R₆ and
R₈ are each hydrogen, R₅ is acetoxy and R₇ is 2,2-[N-(ethoxycarbonylmethyl)-N-methyl]-hydrazin-1-ylcarbonylmethyl.

2. A compound of formula I according to claim 1, wherein
R₁ and R₉ are each independently of the other
(a) hydrogen;
(b) lower alkanoyl;
(c) aryl-lower alkanoyl wherein aryl has from 6 to 14 carbon atoms and may be unsubstituted or mono- to tri-substituted by lower alkyl, halo-lower alkyl, phenyl, 1- or 2-naphthyl, hydroxy, lower alkoxy, carbamoyl-lower alkoxy, N-lower alkylcarbamoyl-lower alkoxy, N,N-di-lower alkylcarbamoyl-lower alkoxy, amino, mono- or di-lower alkylamino, lower alkanoylamino, halogen, carboxy, lower alkoxycarbonyl, phenyl-, naphthyl- or fluorenyl-lower alkoxycarbonyl, lower alkanoyl, sulfo, lower alkylsulfonyl, phosphono, hydroxy-lower alkoxyphosphoryl or di-lower alkoxyphosphoryl, carbamoyl, mono- or di-lower alkylcarbamoyl, sulfamoyl, mono- or di-lower alkylaminosulfonyl, nitro and/or by cyano, and wherein lower alkanoyl is unsubstituted or substituted by carbamoyl or by carbamoyl substituted at the nitrogen atom by one or two radicals selected from lower alkyl, carboxy-lower alkyl, lower alkoxycarbonyl-lower alkyl, di-lower alkylamino-lower alkyl, hydroxy-lower alkyl and di-lower alkoxy-lower alkyl;
(d) heterocyclyl-lower alkanoyl wherein heterocyclyl is thienyl, furyl, pyrrolyl, imidazolyl, pyrazolyl, oxazolyl, thiazolyl, tetrazolyl, pyridyl, pyrazinyl, pyrimidinyl, pyridazinyl, indolyl, benzimidazolyl, quinolyl, isoquinolyl, 3,1-benzofuranyl, cyclohexa[b]pyrrolyl, cyclohexa[b]pyridyl, cyclohexa[b]pyrazinyl, cyclohexa[b]pyrimidinyl, pyrrolidinyl, pyrrolinyl, imidazolidyl, piperidyl, piperazinyl, morpholinyl, thiomorpholinyl, S,S-dioxothiomorpholinyl, indolinyl, isoindolinyl, 4,5,6,7-tetrahydroindolyl, 1,2,3,4-tetrahydroquinolyl or 1,2,3,4-tetrahydroisoquinolyl, which is bonded *via* a ring carbon atom or a ring nitrogen atom;
(e) (lower alkoxy-lower alkoxy)-lower alkanoyl;
(f) amino-lower alkanoyl substituted at the amino nitrogen atom by heterocyclyl-lower alkanoyl, wherein heterocyclyl-lower alkanoyl is independently as defined above under (d) for heterocyclyl-lower alkanoyl R₁ or R₉;
(g) halo-lower alkanoyl containing up to three halogen atoms;
(h) (N-heterocyclyl-lower alkylcarbamoyl)-lower alkanoyl wherein heterocyclyl is selected from pyrrolyl, furyl, thienyl, imidazolyl, pyrazolyl, oxazolyl, thiazolyl, pyridyl, pyrazinyl, pyrimidinyl, indolyl, quinolyl and isoquinolyl, which may also be fully or partially saturated, from morpholinyl and from thiomorpholinyl;
(i) lower alkoxycarbonyl;
(j) aryl-lower alkoxycarbonyl wherein aryl is phenyl, biphenylyl, 1- or 2-naphthyl, fluorenyl, or phenyl that is mono- or poly-substituted by lower alkyl, hydroxy, lower alkoxy, halogen and/or by nitro;
(k) heterocyclyl-lower alkoxycarbonyl wherein heterocyclyl is selected from pyrrolyl, furyl, thienyl, imidazolyl, pyrazolyl, oxazolyl, thiazolyl, pyridyl, pyrazinyl, pyrimidinyl, indolyl, quinolyl and isoquinolyl, which may also be fully or partially saturated, and from morpholinyl and from thiomorpholinyl and is unsubstituted or substituted by lower alkyl;
(l) lower alkenyloxycarbonyl wherein the lower alkenyl radical is bonded to the oxygen atom *via* a saturated carbon atom;
(m) lower alkoxy-lower alkoxycarbonyl;
(n) (lower alkoxy-lower alkoxy)-lower alkoxycarbonyl;
(o) lower alkanesulfonyl;
(p) heterocyclylsulfonyl wherein heterocyclyl is selected from pyrrolyl, furyl, thienyl, imidazolyl, pyrazolyl, oxazolyl, thiazolyl, pyridyl, pyrazinyl, pyrimidinyl, indolyl, quinolyl and isoquinolyl, which may also be fully or partially saturated, from morpholinyl and from thiomorpholinyl and may be unsubstituted or substituted by lower alkyl;
(q) carbamoyl;
(r) N-heterocyclyl-lower alkyl-N-lower alkylcarbamoyl wherein heterocyclyl is independently one of the radicals mentioned above under (d) for heterocyclyl in the definition of heterocyclyl-lower alkanoyl R₁ or R₉; or
(s) an acyl radical, bonded *via* the carbonyl group of the 1-carboxy function, of an amino acid the amino function of which is free or acylated by one of the other radicals mentioned hitherto for R₁ and R₉, the amino acid residues being selected from the residues, bonded *via* the carbonyl of their 1-carboxy group, of the amino acids glycine, alanine, valine, norvaline, leucine, isoleucine, norleucine, serine, homoserine, threonine, methionine, cysteine, proline, trans-3- and trans-4-hydroxyproline, phenylalanine, tyrosine, 4-aminophenylalanine, 4-chlorophenylalanine, 4-carboxyphenylalanine, β-phenylserine, phenylglycine, α-naphthylalanine, cyclohexylalanine, cyclohexylglycine, tryptophan, indoline-2-carboxylic acid, 1,2,3,4-tetrahydroisoquinoline-3-carboxylic acid, aspartic acid, asparagine, aminomalonic acid, aminomalonic acid monoamide, glutamic acid, glutamine, histidine, arginine, lysine, δ-hydroxylysine, ornithine, 3-aminopropanoic acid, α,γ-diaminobutyric acid and α,β-diaminopropionic acid, it being possible for each of the mentioned amino acids (with the exception of glycine) to be in the D-, L- or (D,L)-form; and the α-amino group being unsubstituted or N-acylated by one of the radicals mentioned above under (a) to (r) for R₁ and R₉,
with the proviso that not more than one of the two radicals R₁ and R₉ may be hydrogen,
R₂, R₄, R₆ and R₈ are hydrogen,
R₃ is
(i) lower alkyl;
(ii) C₃-C₇cycloalkyl-lower alkyl wherein C₃-C₇cycloalkyl is unsubstituted or mono- to tri-substituted by lower alkyl, halo-lower alkyl, hydroxy, lower alkoxy, amino, mono- or di-lower alkylamino, halogen, nitro and/or by cyano; or
(iii) aryl-lower alkyl wherein aryl is independently as defined in aryl-lower alkanoyl R₁ or R₉,
R₅ is lower alkanoyloxy, octanoyloxy, nonanoyloxy, decanoyloxy, undecanoyloxy, dodecanoyloxy, hydroxy-lower alkanoyloxy, lower alkoxy-lower alkanoyloxy, lower alkanoyloxy-lower alkanoyloxy, halo-lower alkanoyloxy, carboxy-lower alkanoyloxy, lower alkoxycarbonyl-lower alkanoyloxy, carbamoyl-lower alkanoyloxy, lower alkylcarbamoyl-lower alkanoyloxy, di-lower alkylcarbamoyl-lower alkanoyloxy, hydroxycarboxy-lower alkanoyloxy, hydroxy-lower alkoxycarbonyl-lower alkanoyloxy, dihydroxy-carboxy-lower alkanoyloxy, dihydroxy-lower alkoxycarbonyl-lower alkanoyloxy, pyrrolylcarbonyloxy, furyl-lower alkanoyloxy, thienylcarbonyloxy, imidazolyl-lower alkanoyloxy, pyridyl-lower alkanoyloxy. indolylcarbonyloxy, quinolyl-lower alkanoyloxy, pyrrolidinylcarbonyloxy, piperidinylcarbonyloxy, morpholinocarbonyloxy, thiomorpholinocarbonyloxy, morpholinoacetoxy, thiomorpholinoacetoxy or 4-lower alkyl-1-piperazinoacetoxy, lower alkenoyloxy, lower alkynoyloxy, C₃-C₈cycloalkylcarbonyloxy, C₃-C₈cycloalkylacetoxy, phenyl-lower alkanoyloxy unsubstituted, mono- or polysubstituted in the phenyl radical by lower alkyl, halo-lower alkyl, halogen, hydroxy, lower alkoxy, piperidinomethyl, piperazin- 1-ylmethyl, 4-lower alkyl-piperazin-1-ylmethyl, 4-lower alkanoyl-piperazin-1-ylmethyl, morpholino-lower alkyl, thiomorpholinomethyl, cyano and/or by nitro, or is the residue, bonded *via* a carbonyloxy group containing the carbonyl from the carboxy group of the amino acid in question, of an amino acid selected from glycine, alanine, 2-aminobutyric acid, 3-aminobutyric acid, 4-aminobutyric acid, 3-aminopentanoic acid, 4-aminopentanoic acid, 5-aminopentanoic acid, 3-aminohexanoic acid, 4-aminohexanoic acid, 5-aminohexanoic acid, valine, norvaline, leucine, isoleucine, norleucine, serine, homoserine, threonine, methionine, cysteine, proline, phenylalanine, tyrosine, cyclohexylalanine, tryptophan, aspartic acid, asparagine, glutamic acid, glutamine, histidine, arginine, lysine, ornithine, 3-aminopropanoic acid, α,γ-diaminobutyric acid and α,β-diaminopropionic acid, it being possible for each of the mentioned amino acids to be in the D-, L- or (D,L)-form (except in cases where there is no asymmetric carbon atom), and wherein an amino group is unsubstituted or is mono- or di-N-alkylated by lower alkyl, by pyridyl-lower alkyl and/or by phenyl-lower alkyl, and/or is N-acylated by lower alkanoyl, by phenyl-lower alkanoyl, by lower alkoxycarbonyl or by phenyl-lower alkoxycarbonyl;
and
R₇ is independently of R₃ one of the radicals defined above for R₃, or a salt thereof where at least one salt-forming group is present.

3. A compound of formula I according to claim 1 wherein
R₁ and R₉ are each independently of the other
(a) hydrogen,
(b) lower alkanoyl,
(c) phenyl-lower alkanoyl,
(d) phenyl-lower alkanoyl wherein the lower alkanoyl radical is substituted by carbamoyl,
(e) morpholino-lower alkanoyl,
(f) thiomorpholino-lower alkanoyl,
(g) pyridyl-lower alkanoyl,
(h) quinolyl-lower alkanoyl,
(i) tetrazolyl-lower alkanoyl,
(j) amino-lower alkanoyl substituted at the amino nitrogen atom by N-morpholino- or N-thiomorpholino-carbonyl,
(k) halo-lower alkanoyl containing up to three halogen atoms,
(l) 2-(N-morpholino-lower alkylcarbamoyl)-lower alkanoyl,
(m) 2-(N-pyridyl-lower alkylcarbamoyl)-lower alkanoyl,
(n) lower alkoxycarbonyl,
(o) phenyl-lower alkoxycarbonyl,
(p) tetrahydrofuryl-lower alkoxycarbonyl,
(q) lower alkenyloxycarbonyl,
(r) lower alkoxy-lower alkoxycarbonyl,
(s) (lower alkoxy-lower alkoxy)-lower alkoxycarbonyl,
(t) lower alkanesulfonyl,
(u) morpholinosulfonyl,
(v) thiomorpholinosulfonyl,
(w) N-pyridyl-lower alkyl-N-lower alkylcarbamoyl, or
(x) an acyl radical, bonded *via* the carbonyl of its carboxy group, of an amino acid selected from glycine, alanine, valine, leucine, isoleucine, glutamic acid and asparagine in the (D)-, (L)- or (D,L)-form (with the exception of glycine), wherein the α-amino group is unsubstituted or acylated by one of the other radicals R₁ or R₉ mentioned hitherto under (a) to (w);
with the proviso that not more than one of the radicals R₁ and R₉ is hydrogen,
R₂, R₄, R₆ and R₈ are hydrogen,
R₃ is lower alkyl, cyclohexyl-lower alkyl or phenyl-lower alkyl that is unsubstituted or substituted by halogen, lower alkoxy or by cyano,
R₅ is lower alkanoyloxy, octanoyloxy, decanoyloxy, dodecanoyloxy, carboxy-lower alkanoyloxy, furyl-lower alkanoyloxy, imidazolyl-lower alkanoyloxy, pyridyl-lower alkanoyloxy, quinolyl-lower alkanoyloxy, aminoacetoxy, N-lower alkylaminoacetoxy, N,N-di-lower alkylaminoacetoxy, N-lower alkyl-N-phenyl-lower alkoxycarbonylaminoacetoxy, phenyl-lower alkanoyloxy, 4-morpholino-lower alkylbenzoyloxy, 4-halomethyl-benzoyloxy, histidyloxy or prolyloxy and
R₇ has the same definitions as R₃,
or a pharmaceutically acceptable salt thereof where at least one salt-forming group is present.

4. A compound of formula I according to claim 1 wherein
R₁ is lower alkoxycarbonyl, halo-lower alkoxycarbonyl, phenyl-lower alkoxycarbonyl, the monovalent residue, bonded *via* carbonyl, of an aliphatic amino acid selected from valine, alanine, leucine and isoleucine or the residue, bonded *via* carbonyl, of an aliphatic amino acid as defined above acylated at the amino nitrogen atom by one of the radicals phenyl-lower alkanoyl, morpholinyl-lower alkanoyl, thiomorpholinyl-lower alkanoyl, pyridyl-lower alkanoyl, lower alkoxycarbonyl and phenyl-lower alkoxycarbonyl, all the mentioned amino acids being in the D-, D,L- or L-form,
R₂ is hydrogen,
R₃ is phenyl-lower alkyl, 4-fluorophenyl-lower alkyl or cyclohexyl-lower alkyl,
R₄ is hydrogen,
R₅ is lower alkanoyloxy, octanoyloxy, decanoyloxy, dodecanoyloxy, carboxy-lower alkanoyloxy, furyl-lower alkanoyloxy, imidazolyl-lower alkanoyloxy, pyridyl-lower alkanoyloxy, quinolyl-lower alkanoyloxy, aminoacetoxy (glycyloxy), N-lower alkylaminoacetoxy, N,N-di-lower alkylaminoacetoxy, N-lower alkyl-N-phenyl-lower alkoxycarbonylaminoacetoxy, phenyl-lower alkanoyloxy, 4-morpholinomethylbenzoyloxy, 4-halomethylbenzoyloxy, histidyloxy or prolyloxy,
R₆ is hydrogen,
R₇ is lower alkyl, cyclohexyl-lower alkyl, phenyl-lower alkyl, 4-cyanophenyl-lower alkyl or 4-fluorophenyl-lower alkyl,
R₈ is hydrogen and
R₉ is one of the radicals mentioned above for R₁, and the asymmetric carbon atoms carrying the radicals R₃ and R₅ are in the S-configuration,
or a pharmaceutically acceptable salt thereof.

5. A compound of formula 1 according to claim 1 wherein R₁ and R₉ are N-methoxycarbonylvalyl, R₂, R₄, R₆ and R₈ are hydrogen, R₃ is benzyl or cyclohexylmethyl, R₅ is lower alkanoyloxy or pyridylcarbonyloxy and R₇ is cyclohexylmethyl or benzyl, or a pharmaceutically acceptable salt thereof.

6. An isomer of a compound of formula I according to claim 5, wherein the carbon atom carrying R₃ and the carbon atom carrying R₅ are in the (S)-configuration and the radicals R₁, R₂, R₃, R₄, R₅, R₆, R₇, R₈ and R₉ are as defined in claim 6, or a pharmaceutically acceptable salt thereof.

7. 1-[2(S)-Acetoxy-3(S)-(N-(2-methoxyethoxycarbonyl)-(L)-valyl)amino-4-phenylbutyl]-1-[cyclohexylmethyl]-2-[N-(2-methoxyethoxycarbonyl)-(L)-valyl]hydrazine according to claim 1, or a pharmaceutically acceptable salt thereof.

8. 1-[2(S)-Acetoxy-3(S)-(N-(methoxycarbonyl)-(L)-valyl)amino-4-phenyl-butyl]-1-[cyclohexylmethyl]-2-[N-(methoxycarbonyl)-(L)-valyl]hydrazine according to claim 1, or a pharmaceutically acceptable salt thereof.

9. 1-[2(S)-(2-Pyridylcarbonyl)oxy-3(S)-(N-(methoxycarbonyl)-(L)-valyl)amino-4-phenylbutyl]-1-[cyclohexylmethyl]-2-[N-(methoxycarbonyl)-(L)-valyl]hydrazine according to claim 1, or a pharmaceutically acceptable salt thereof.

10. A compound according to claim 1 selected from
1-[2(S)-propionyloxy-3(S)-(N-(methoxycarbonyl)-(L)-valyl)amino-4-phenyl-butyl]-1-[cyclohexylmethyl]-2-[N-(methoxycarbonyl)-(L)-valyl]hydrazine;
1-[2(S)-butyryloxy-3(S)-(N-(methoxycarbonyl)-(L)-valyl)amino-4-phenyl-butyl]-1-[cyclohexylmethyl]-2-[N-(methoxycarbonyl)-(L)-valyl]hydrazine;
1-[2(S)-pentanoyloxy-3(S)-(N-(methoxycarbonyl)-(L)-valyl)amino-4-phenyl-butyl]-1-[cyclohexylmethyl]-2-[N-(methoxycarbonyl)-(L)-valyl]hydrazine;
1-[2(S)-octanoyloxy-3(S)-(N-(methoxycarbonyl)-(L)-valyl)amino-4-phenyl-butyl]-1-[cyclohexylmethyl]-2-[N-(methoxycarbonyl)-(L)-valyl]hydrazine;
1-[2(S)-decanoyloxy-3(S)-(N-(methoxycarbonyl)-(L)-valyl)amino-4-phenyl-butyl]-1-[cyclohexylmethyl]-2-[N-(methoxycarbonyl)-(L)-valyl]hydrazine;
1-[2(S)-dodecanoyloxy-3(S)-(N-(methoxycarbonyl)-(L)-valyl)amino-4-phenyl-butyl]-1-[cyclohexylmethyl]-2-[N-(methoxycarbonyl)-(L)-valyl]hydrazine;
1-[2(S)-pivaloyloxy-3(S)-(N-(methoxycarbonyl)-(L)-valyl)amino-4-phenyl-butyl]-1-[cyclohexylmethyl]-2-[N-(methoxycarbonyl)-(L)-valyl]hydrazine;
1-[2(S)-(2-furylcarbonyl)oxy-3(S)-(N-(methoxycarbonyl)-(L)-valyl)amino-4-phenyl-butyl]-1-[cyclohexylmethyl]-2-[N-(methoxycarbonyl)-(L)-valyl]hydrazine;
1-[2(S)-(4-imidazolylcarbonyl)oxy-3(S)-(N-(methoxycarbonyl)-(L)-valyl)amino-4-phenyl-butyl]-1-[cyclohexylmethyl]-2-[N-(methoxycarbonyl)-(L)-valyl]hydrazine;
1-[2(S)-(4-imidazolylacetyl)oxy-3(S)-(N-(methoxycarbonyl)-(L)-valyl)amino-4-phenylbutyl]-1-[cyclohexylmethyl]-2-[N-(methoxycarbonyl)-(L)-valyl]hydrazine;
1-[2(S)-(3-(4-imidazolyl)-propionyl)oxy-3(S)-(N-(methoxycarbonyl)-(L)-valyl)amino-4-phenyl-butyl]-1-[cyclohexylmethyl]-2-[N-(methoxycarbonyl)-(L)-valyl]hydrazine;
1-[2(S)-benzoyloxy-3(S)-(N-(methoxycarbonyl)-(L)-valyl)amino-4-phenyl-butyl]-1-[cyclohexylmethyl]-2-[N-(methoxycarbonyl)-(L)-valyl]hydrazine;
1-[2(S)-(2-pyridylacetyl)oxy-3(S)-(N-(methoxycarbonyl)-(L)-valyl)amino-4-phenylbutyl]-1-[cyclohexylmethyl]-2-[N-(methoxycarbonyl)-(L)-valyl]hydrazine;
1-[2(S)-(3-(pyridin-2-yl)-propionyl)oxy-3(S)-(N-(methoxycarbonyl)-(L)-valyl)amino-4-phenylbutyl)]-1-[cyclohexylmethyl]-2-[N-(methoxycarbonyl)-(L)-valyl]hydrazine;
1-[2(S)-(quinolin-2-ylcarbonyl)oxy-3(S)-(N-(methoxycarbonyl)-(L)-valyl)amino-4-phenyl-butyl]-1-[cyclohexylmethyl]-2-[N-(methoxycarbonyl)-(L)-valyl]hydrazine;
1-[2(S)-(aminoacetyl)oxy-3(S)-(N-(methoxycarbonyl)-(L)-valyl)amino-4-phenyl-butyl]-1-[cyclohexylmethyl]-2-[N-(methoxycarbonyl)-(L)-valyl]hydrazine;
1-[2(S)-(N-methylaminoacetyl)oxy-3(S)-(N-(methoxycarbonyl)-(L)-valyl)amino-4-phenyl-butyl]-1-[cyclohexylmethyl]-2-[N-(methoxycarbonyl)-(L)-valyl]hydrazine;
1-[2(S)-(N,N-dimethylaminoacetyl)oxy-3(S)-(N-(methoxycarbonyl)-(L)-valyl)amino-4-phenyl-butyl]-1-[cyclohexylmethyl]-2-[N-(methoxycarbonyl)-(L)-valyl]hydrazine;
1-[2(S)-(N-benzyloxycarbonyl-N-methyl-aminoacetyl)oxy-3(S)-(N-(methoxycarbonyl)-(L)-valyl)amino-4-phenyl-butyl]-1-[cyclohexylmethyl]-2-[N-(methoxycarbonyl)-(L)-valyl]hydrazine;
1-[2(S)-prolyloxy-3(S)-(N-(methoxycarbonyl)-(L)-valyl)amino-4-phenyl-butyl]-1-[cyclohexylmethyl]-2-[N-(methoxycarbonyl)-(L)-valyl]hydrazine;
1-[2(S)-(4-morpholinomethylbenzoyl)oxy-3(S)-(N-(methoxycarbonyl)-(L)-valyl)amino-4-phenyl-butyl]-1-[cyclohexylmethyl]-2-[N-(methoxycarbonyl)-(L)-valyl]hydrazine;
1-[2(S)-(4-chloromethylbenzoyl)oxy-3(S)-(N-(methoxycarbonyl)-(L)-valyl)amino-4-phenyl-butyl]-1-[cyclohexylmethyl]-2-[N-(methoxycarbonyl)-(L)-valyl]hydrazine; and
1-[2(S)-(3-carboxypropionyl)oxy-3(S)-(N-(methoxycarbonyl)-(L)-valyl)amino-4-phenylbutyl]-1-[cyclohexylmethyl]-2-[N-(methoxycarbonyl)-(L)-valyl]hydrazine, or a pharmaceutically acceptable salt thereof.

11. 1-[2(S)-Butyryloxy-3(S)-(N-(methoxycarbonyl)-(L)-valyl)amino-4-phenyl-butyl]-1-[cyclohexylmethyl]-2-[N-methoxycarbonyl-(L)-valyl]hydrazine of formula I according to claim 1, or a pharmaceutically acceptable salt thereof.

12. 1-[2(S)-(Methoxy-acetoxy)-3(S)-(N-(methoxycarbonyl)-(L)-valyl)amino-4-phenylbutyl]-1-[cyclohexylmethyl]-2-[N-methoxycarbonyl-(L)-valyl]hydrazine of formula I according to claim 1, or a pharmaceutically acceptable salt thereof.

13. A compound of formula I or a pharmaceutically acceptable salt thereof according to any one of claims 1 to 12 for use in a method for the diagnostic or therapeutic treatment of the human or animal body.

14. The use of a compound of formula I according to any one of claims 1 to 12, or of a pharmaceutically acceptable salt thereof, in the preparation of pharmaceutical compositions for the treatment of AIDS.

15. A pharmaceutical composition comprising a compound of formula I according to any one of claims 1 to 12 or a pharmaceutically acceptable salt of such a compound having at least one salt-forming group together with a pharmaceutically acceptable carrier.

16. A process for the preparation of a compound of formula I according to claim 1, wherein
a) a hydroxy compound of formula II 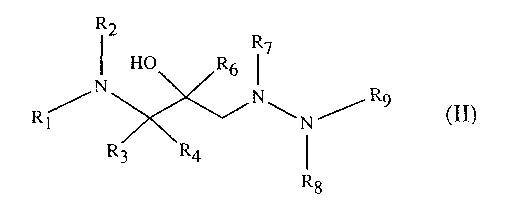 wherein the radicals R₁ to R₄ and R₆ to R₉ are as defined for compounds of formula I, is acylated with a carboxylic acid of formula III
R₅-H (III),
or with a reactive acid derivative thereof, wherein R₅ is as defined for compounds of formula I, free functional groups in the starting materials of formulae II and III that are not to participate in the reaction being if necessary in protected form, and any protecting groups present are removed, or
b) for the preparation of a compound of formula I wherein R₉ is acyl, sulfo, or sulfonyl substituted by unsubstituted or substituted alkyl, aryl or heterocyclyl, and the remaining radicals R₁ to R₈ are as defined for compounds of formula I, an amino compound of formula 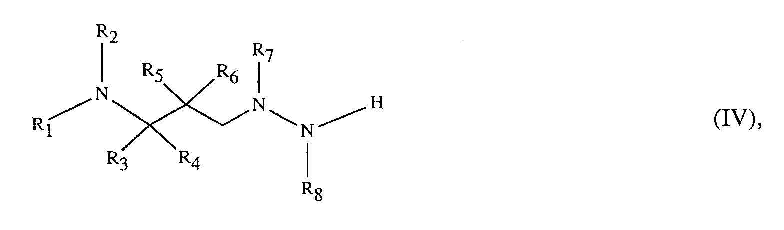 wherein the radicals R₁ to R₈ are as defined for compounds of formula I, is condensed with an acid of formula
R₉'-OH (V),
or with a reactive acid derivative thereof, wherein R₉' is as defined above under (b) for R₉ with the exception of hydrogen and unsubstituted or substituted alkyl, free functional groups, with the exception of those participating in the reaction, being if necessary in protected form, and any protecting groups present are removed, or
c) for the preparation of a compound of formula I wherein R₁ is acyl, sulfo, or sulfonyl substituted by unsubstituted or substituted alkyl, aryl or heterocyclyl, and the remaining radicals R₂ to R₉ are as defined for compounds of formula I, an amino compound of formula 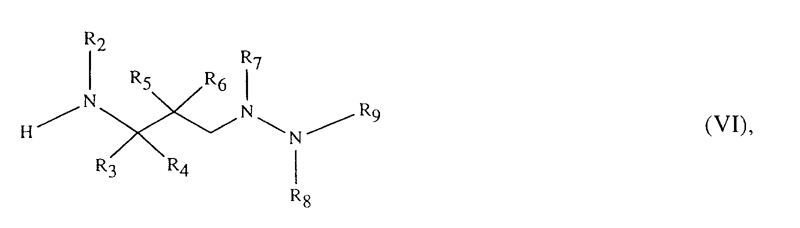 wherein the radicals R₂ to R₉ are as defined for compounds of formula I, is condensed with an acid of formula
R₁'-OH (VII),
or with a reactive acid derivative thereof, wherein R₁' is as defined above under (c) for R₁ with the exception of hydrogen and unsubstituted or substituted alkyl, free functional groups, with the exception of those participating in the reaction, being if necessary in protected form, and any protecting groups present are removed, or
d) for the preparation of a compound of formula I wherein R₁ and R₉ are two identical radicals selected from acyl, sulfo, and sulfonyl substituted by unsubstituted or substituted alkyl, aryl or heterocyclyl, and the remaining radicals R₂ to R₈ are as defined for compounds of formula I, a diamino compound of formula 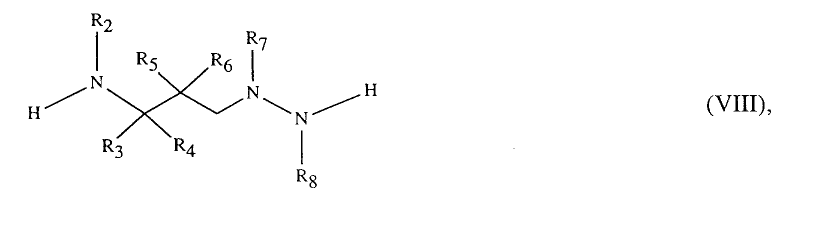 wherein the radicals R₂ to R₈ are as defined for compounds of formula I, is condensed with an acid suitable for introducing the identical radicals R₁ and R₉, or with a reactive acid derivative thereof, wherein R₁ and R₉ are as defined immediately above, free functional groups, with the exception of those participating in the reaction, being if necessary in protected form, and any protecting groups present are removed, or
e) for the preparation of a compound of formula I wherein R₇ is unsubstituted or substituted alkyl or cycloalkyl, and the remaining radicals R₁ to R₆, R₈ and R₉ are as defined for compounds of formula I, in a compound of formula I' 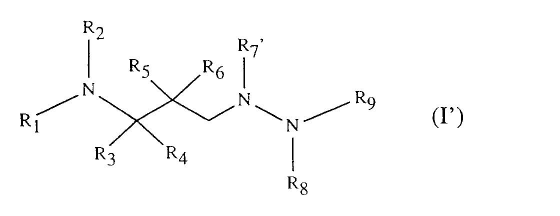 wherein R₇' is hydrogen and the remaining radicals R₁ to R₆, R₈ and R₉ are as defined for compounds of formula I, the radical R₇ is introduced by substitution with a compound of formula XII
R₇-X (XII),
wherein X is a leaving group and R₇ is unsubstituted or substituted alkyl or cycloalkyl, free functional groups, with the exception of those participating in the reaction, being if necessary in protected form, and any protecting groups present are removed,
and, if desired, as a further process step a compound of formula I obtainable in accordance with any one of processes a) to e) above having at least one salt-forming group is converted into its salt or an obtainable salt is converted into the free compound or into a different salt and/or any isomeric mixtures that are obtainable are separated and/or a compound of formula I according to the invention is converted into a different compound of formula I according to the invention.

17. A compound of formula II shown in claim 16, selected from the compounds having the names:
1-[2(S)-hydroxy-3(S)-(N-(methoxycarbonyl)-(L)-valyl)amino-4-phenyl-butyl]-1-[cyclohexylmethyl]-2-[N-(methoxycarbonyl)-(L)-valyl]hydrazine,
1-[2(S)-hydroxy-3(S)-(N-(methoxycarbonyl)-(L)-valyl)amino-4-phenyl-butyl]-1-[thien-2-ylmethyl]-2-[N-methoxycarbonyl)-L-valyl]hydrazine,
1-[2(S)-hydroxy-3(S)-(N-(quinoline-2-carbonyl)-(L)-asparaginyl)amino-4-phenyl-butyl]-1-[4-methoxyphenylmethyl]-2-[N-benzyloxy-carbonyl)-(L)-valyl]hydrazine,
1-[2(S)-hydroxy-3(S)-(N-methoxycarbonyl-(L)-valyl)amino-4-phenyl-butyl]-1-[4-methoxyphenylmethyl]-2-[N-methoxycarbonyl)-(L)-valyl]hydrazine,
1-[2(S)-hydroxy-3(S)-(N-(methoxycarbonyl)-(L)-valyl)amino-4-phenyl-butyl]-1-[4-biphenylylmethyl]-2-[N-(methoxycarbonyl)-(L)-valyl]hydrazine,
1-[2(S)-hydroxy-3(S)-(N-(ethoxycarbonyl)-(L)-valyl)amino-4-phenyl-butyl]-1-[4-biphenylylmethyl]-2-[N-(ethoxycarbonyl)-(L)-valyl]hydrazine,
1-[2(S)-hydroxy-3(S)-(N-(quinoline-2-carbonyl)-(L)-asparaginyl)amino-4-phenyl-butyl]-1-[benzyl]-2-[N-(benzyloxycarbonyl)-(L)-valyl]hydrazine, and
1-[2(S)-hydroxy-3(S)-(N-acetyl-(L)-valyl)amino-4-phenyl-butyl]-1-[4-biphenylylmethyl]-2-[N-acetyl-(L)-valyl]hydrazine,
or a pharmaceutically acceptable salt thereof.

18. The compound of formula II shown in claim 16 having the name 1-[2(S)-hydroxy-3(S)-(N-(methoxycarbonyl)-(L)-valyl)amino-4-phenyl-butyl]-1-[cyclohexylmethyl]-2-[N-(methoxycarbonyl)-(L)-valyl]hydrazine, or a pharmaceutically acceptable salt thereof.

19. The compound of formula II shown in claim 16 having the name 1-[2(S)-hydroxy-3(S)-(N-(methoxycarbonyl)-(L)-valyl)amino-4-phenyl-butyl]-1-[4-biphenylylmethyl]-2-[N-(methoxycarbonyl)-(L)-valyl]hydrazine, or a pharmaceutically acceptable salt thereof.

## Revendications

1. Composés de formule 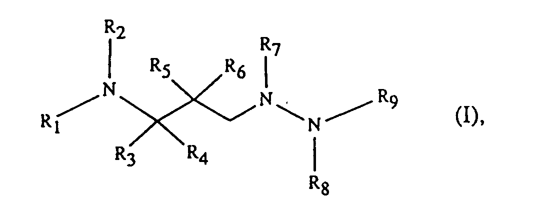 dans laquelle R₁ et R₉ représentent indépendamment l'un de l'autre un hydrogène, un acyle, un alkyle non substitué ou substitué ; un sulfo ; ou un sulfonyle substitué par un alkyle substitué, un aryle ou un hétérocyclyle non substitué ou substitué, sous réserve qu'au plus l'un des radicaux R₁ et R₉ représente un hydrogène ; et R₂ et R₈ représentent chacun indépendamment l'un de l'autre un hydrogène ou un alkyle non substitué ou substitué ;
R₃ et R₄ représentent indépendamment l'un de l'autre un hydrogène, un alkyle non substitué ou substitué :
R₅ représente un acyloxy ;
R₆ représente un hydrogène ;
et R₇ représente un alkyle non substitué ou substitué, un cycloalkyle ou un aryle non substitué ou substitué ;
ainsi que les sels des composés mentionnés, dans la mesure où des groupes salificateurs existent ; à l'exclusion du composé dans lequel R₁ et R₉ représentent chacun un acétyle, R₂, R₃, R₄, R₈, et R₈ représentent chacun un hydrogène, R₅ représente un acétoxy et R₇ représente un 2,2-[N-éthoxycarbonylméthyl)-N-méthyl]hydrazin-1-ylcarbonyl-méthyle.

2. Composé de formule I selon la revendication 1, dans lequel
R₁ et R₉ représentent Indépendamment l'un de l'autre
(a) un hydrogène ;
(b) un alcanoyle inférieur,
(c) un arylalcanoyle inférieur, où aryle comporte de 6 à 14 atomes de carbone, et est non substitué ou peut être mono- à trisubstitué par un alkyle inférieur, halogène-alkyle inférieur, phényle, 1- ou 2-naphtyle, hydroxy, alcoxy inférieur, carbamoyl-alcoxy inférieur, N-alkyle inférieur-carbamoyl-alcoxy inférieur ou N,N-di-alkyle inférieur-carbamoyl-alcoxy inférieur, amino, mono- ou di-alkyle inférieur-amino, alcanoyle inférieur-amino, halogène, carboxy, alcoxy inférieur-carbonyle, phényl-, naphtyl- ou fluorényl-alcoxy inférieur-carbonyle, alcanoyle inférieur, sulfo, alkyle inférieur-sulfonyle, phosphono, hydroxy-alcoxy inférieur-phosphoryle ou dialcoxy inférieur-phosphoryle, carbamoyle, mono- ou dialkyle inférieur-carbamoyle, sulfamoyle, mono- ou di-alkyle inférieur-aminosulfonyle, nitro et/ou cyano, et où un alcanoyle inférieur est non substitué ou substitué par un carbamoyle un carbamoyle substitué sur l'atome d'azote par 1 ou 2 radicaux choisis parmi alkyle inférieur, carboxyalkyle inférieur, alcoxy inférieur-carbonyle-alkyle inférieur, dialkyle inférieur-amino-alkyle inférieur, hydroxy alkyle inférieur, et dihydroxyalkyle inférieur-alkyle inférieur ;
(d) hétérocyclyl-alcanoyle inférieur, où hétérocyclyle représente un thiényle, un furyle, un pyrrolyle, un imidazolyle, un pyrazolyle, un oxazolyle, un thiazolyle, un tétrazolyle, un pyridyle, un pyrazinyle, un pyrimidinyle, un pyridazinyle, un indolyle, un benzimidazolyle, un quinoléyle, un isoquinoléyle, un 3,1-benzofuranyle, un cyclohexa[b]pyrrolyle, un cyclohexa[b]pyridyle, un cyclohexa[b]pyrazinyle, un cyclohexa[b]pyrimidinyle, un pyrrolidinyle, un pyrrolinyle, un imidazolidyle, un pipéridyle, un pipérazinyle, un morpholinyle, un htiomorpholinyle, un S,S-di-oxo-thiomorpholinyle, un indolinyle, un isoindolinyle, un 4,5,6,7-tétrahydroindolyle, un 1,2,3,4-tétrahydroquinoléyle ou un 1,2,3,4-tétrahydroisoquinoléyle, qui est lié par l'intermédiaire d'un atome de carbone du cycle ou d'un atome d'azote du cycle ;
(e) un (alcoxy inférieur-alcoxy inférieur)-alcanoyle inférieur ;
(f) un amino-alcanoyle inférieur substitué sur l'atome d'azote de l'amino par un hétérocyclyle-alcanoyle inférieur, où l'hétérocyclyl-alcanoyle inférieur est défini indépendamment comme ci-dessus en
d) en R₁ ou R₉ pour hétérocyclyle-alcanoyle inférieur ;
(g) un halogène-alcanoyle inférieur, qui contient jusqu'à trois atomes d'halogène ;
(h) un (N-hétérocyclyl-alkyle inférieur-carbamoyle)-alcanoyle inférieur, où hétérocyclyle est choisi de préférence parmi pyrrolyle, furyle, thiényle, imidazolyle, pyrazolyle, oxazolyle, thiazolyle, pyridyle, pyrazinyle, pyrimidinyle, indolyle, quinoléyle et isoquinoléyle, qui peuvent également être entièrement ou partiellement saturés, choisis parmi morpholinyle et thiomorpholinyle ;
(i) un alcoxy inférieur-carbonyle ;
(j) un aryl-alcoxy inférieur-carbonyle, où aryle représente un phényle, biphénylyle, 1- ou 2-naphtyle, fluorényle ou un phényle mono- ou polysubstitué par un alkyle inférieur, un hydroxy, un alcoxy inférieur, un halogène, et/ou un nitro ;
(k) un hétérocyclyl-alcoxy inférieur-carbonyle, où hétérocyclyle est choisi parmi un pyrrolyle, furyle, thiényle, imidazolyle, pyrazolyle, oxazolyle, thiazolyle, pyridyle, pyrazinyle, pyrimidinyle, indolyle, quinoléyle, et isoquinoléyle, qui peuvent également être entièrement ou partiellement saturés, choisis d'après morpholinyle et thiomorpholinyle, et non substitués ou substitués par un alkyle inférieur,
(l) un alcényloxy inférieur-carbonyle, où le radical alcényle inférieur est lié à l'atome d'oxygène par l'intermédiaire d'un atome de carbone saturé ;
(m) un alcoxy inférieur-alcoxy inférieur-carbonyle ;
(n) un (alcoxy inférieur-alcoxy inférieur)alcoxy inférieur-carbonyle ;
(o) un alcane inférieur-sulfonyle ;
(p) un hétérocyclylsulfonyle, où hétérocyclyle est choisi parmi pyrrolyle, furyle, thiényle, imidazolyle, pyrazolyle, oxazolyle, thiazolyle, pyridyle, pyrazinyle, pyrimidinyle, indolyle, quinoléyle, et isoquinoléyle, qui peuvent également être entièrement ou partiellement saturés, choisis d'après morpholinyle et thiomorpholinyle, non substitués ou substitués par un alkyle inférieur ;
(q) un carbamoyle ;
(r) un N-hétérocyclyl-alkyle inférieur-N-alkyle inférieur-carbamoyle, où hétérocyclyle représente indépendamment l'un des trois radicaux mentionnés ci-dessus en d) dans la définition d'un hétérocyclylalcanoyle inférieur R₁ ou R₉ pour hétérocyclyle ; ou
(s) un radical acyle, lié par l'intermédiaire du groupe carbonyle de la fonction 1-carboxy, d'un acide aminé, dont la fonction amino est libre ou est acylée par l'un des autres radicaux mentionnés jusqu'à présent pour R₁ et R₉, les radicaux acides aminés étant choisis parmi les radicaux, liés par l'intermédiaire du carbonyle de leur groupe 1-carboxy, des acides aminés glycine, alanine, valine, norvaline, leucine, isoleucine, norleucine, sérine, homosérine, thréonine, méthionine, cystéine, proline, trans-3- et trans-4-hydroxyproline, phénylalanine, tyrosine, 4-aminophénylalanine, 4-chlorophénylalanine, 4-carboxyphénylalanine, β-phénylsérine, phénylglycine, α-naphtylalanine, cyclohexylalanine, cyclohexylglycine, tryptophane, acide indoline-2-carboxylique, acide 1,2,3,4-tétrahydroisoquinoléine-3-carboxylique, acide aspartique, asparagine, acide aminomalonique, monoamide del'acide aminomalonique, acide glutamique, glutamine, histidine, arginine, lysine, δ-hydroxylysine, ornithine, acide 3-aminopropanoïque, acide α,γ-diaminobutyrique, et acide α,β-diaminopropionique,
où, en dehors de la glycine, chacun des acides aminés mentionnés peut se présenter sous forme D, L ou (D,L)
et où le groupe α-amino est non substitué ou N-acylé par l'un des radicaux mentionnés ci-dessus en (a) à (r), pour R₁ ou R₉ ,
sous réserve qu'au plus un des deux radicaux R₁ ou R₉ peut représenter un hydrogène ;
R₂, R₄, R₆ et R₈ représentent un hydrogène ;
R₃ représente
(i) un alkyle inférieur ;
(ii) un C₃₋₇-cycloalkyle-alkyle inférieur, où C₃₋₇-cycloalkyle est non substitué ou mono- à trisubstitué par un alkyle inférieur, un halogène-alkyle inférieur, un hydroxy, alcoxy inférieur, amino, mono- ou di-alkyle inférieur-amino, halogène, nitro et/ou cyano ; ou
(iii) un aryl-alkyle inférieur, où aryle est défini indépendamment comme R₁ ou R₉ dans arylalcanoyle inférieur ;
R₅ représente un alcanoyloxy inférieur, octanoyloxy, nonanoyloxy, décanoyloxy, undécanoyloxy, dodécanoyloxy, hydroxy-alcanoyloxy inférieur, alcoxy inférieur-alcanoyloxy inférieur, alcanoyloxy inférieur-alcanoyloxy inférieur, halogène-alcanoyloxy inférieur, carboxy-alcanoyloxy inférieur, alcoxy inférieur-carbonyl-alcanoyloxy inférieur, carbamoyl-alcanoyloxy inférieur, alkyle inférieur-carbamoyl-alcanoyloxy inférieur, dialkyle inférieur-carbamoyl-alcanoyloxy inférieur, hydroxy-carboxy-alcanoyloxy inférieur, hydroxy-alcoxy inférieur-carbonyle-alcanoyloxy inférieur, dihydroxy-carboxy-alcanoyloxy inférieur, dihydroxy-alcoxy inférieur-carbonyle-alcanoyloxy inférieur, pyrrolylcarbonyloxy, furyl-alcanoyloxy inférieur, thiénylcarbonyloxy, imidazolyl-alcanoyloxy inférieur, imidazolylacétoxy, imidazolylpropionyloxy, pyridyl-alcanoyloxy inférieur, indolylcarbonyloxy, quinoléyl-alcanoyloxy inférieur, pyrrolidinylcarboxy, pipéridinylcarbonyloxy, morpholinocarbonyloxy, thiomorpholinocarbonyloxy, morpholinoacétyloxy, thiomorpholinoacétyloxy ou 4-alkyle inférieur-1-pipérazinoacétyloxy, alcénoyloxy inférieur, alcynoyloxy inférieur, cycloalkyle en C₃₋₈-carbonyloxy, cycloalkyle en C₃₋₈-acétoxy, phényl-alcanoyloxy inférieur, non substitué, mono- ou polysubstitué par un radical phényle par un alkyle inférieur, haloalkyle inférieur, un halogène, un hydroxy, alcoxy inférieur, pipéridinométhyle, pipérazin-1-ylméthyle, 4-alkyle inférieur-pipérazin-1-ylméthyle, 4-alcanoyle inférieur-pipérazin-1-ylméthyle, morpholino-alkyle inférieur, thiomorpholinométhyle, cyano et/ou nitro, ou qui représente le radical d'un acide aminé lié par l'intermédiaire d'un groupe carbonyloxy qui contient le carbonyle du groupe carboxy de l'acide aminé en question, et qui est choisi parmi glycine, alanine, acide 2-aminobutyrique, acide 3-aminobutyrique, l'acide 4-aminobutyrique, l'acide 3-aminopentanoïque, l'acide 4-aminopentanoïque, l'acide 5-aminopentanoïque, l'acide 3-aminohexanoïque, l'acide 4-aminohexanoïque, l'acide 5-aminohexanoïque, la valine, la norvaline, la leucine, l'isoleucine, la norleucine, la sérine, l'homosérine, la thréonine, la méthionine, la cystéine, la proline, la phénylalanine, la tyrosine, la cyclohexylalanine, le tryptophane, l'acide aspartique, l'asparagine, l'acide glutamique, la glutamine, l'histidine, l'arginine, la lysine, l'ornithine, l'acide 3-aminopropanoïque, l'acide α,γ-diaminobutyrique, et l'acide α,β-diaminopropionique ; où (en dehors des cas où il n'y a pas d'atome de carbone asymétrique), chacun des acides aminés mentionnés peut se présenter sous forme D, L ou (D,L) ;
et où un groupe amino est mono- ou bi- N-alkylé par un alkyle inférieur, par un pyridyl-alkyle inférieur, et/ou par un phényl-dialkyle inférieur, et/ou N-acylé par un alcanoyle inférieur, par un phényl-alcanoyle inférieur, par un alcoxy inférieur-carbonyle, ou par un phényl-alcoxy inférieur-carbonyle, et
R₇ représente indépendamment de R₃ l'un des radicaux qui est défini pour R₃,
ou un de ses sels, dans la mesure où au moins un groupe salificateur est présent.

3. Composé de formule I selon la revendication 1, dans lequel :
R₁ et R₉ représentent indépendamment l'un de l'autre
(a) un hydrogène,
(b) un alcanoyle inférieur,
(c) un phényl-alcanoyle inférieur,
(d) un phényl-alcanoyle inférieur où le radical alcanoyle inférieur est substitué par un carbamoyle,
(e) un morpholino-alcanoyle inférieur,
(f) un thiomorpholino-alcanoyle inférieur,
(g) un pyridyl-alcanoyle inférieur,
(h) un quinoléyl-alcanoyle inférieur,
(i) un tétrazolyl-alcanoyle inférieur,
(j) un aminoalcanoyle inférieur, substitué sur l'atome d'azote de l'amino par un N-morpholino- ou N-thiomorpholino-carbonyle,
(k) un halogène-alcanoyle inférieur, qui contient jusqu'à trois atomes d'halogène,
(l) un 2-(N-morpholino-alkyle inférieur-carbamoyle)-alcanoyle inférieur,
(m) un 2-(N-pyridyl-alkyle inférieur-carbamoyl)-alcanoyle inférieur,
(n) un alcoxy inférieur-carbonyle,
(o) un phényl-alcoxy inférieur-carbonyle,
(p) un tétrahydrofuryl-alcoxy inférieur-carbonyle,
(q) un alcényloxy inférieur-carbonyle,
(r) un alcoxy inférieur-alcoxy inférieur-carbonyle,
(s) un (alcoxy inférieur-alcoxy inférieur)-alcoxy inférieur-carbonyle
(t) un alcane inférieur-sulfonyle,
(u) un morpholinosulfonyle,
(v) un thiomorpholinosulfonyle,
(w) un N-pyridyl-alkyle inférieur-N-alkyle inférieur-carbamoyle, ou
(x) un radical acyle, lié par l'intermédiaire du carbonyle de son groupe carboxy, d'un acide aminé choisi parmi la glycine, l'alanine, la valine, la leucine, l'isoleucine, l'acide glutamique, et l'asparagine sous forme (D), (L) ou (D,L) (en dehors de la glycine), où le groupe α-aminé est non substitué ou acylé par l'un des autres radicaux R₁ ou R₉ mentionnés jusqu'ici en (a) à (w) ;
sous réserve qu'au moins l'un des radicaux R₁ et R₉ représente un hydrogène,
R₂, R₄, R₆ et R₈ représentent un hydrogène ;
R₃ représente un alkyle inférieur, un cyclohexyl-alkyle inférieur ou un phényl-alkyle inférieur, qui est non substitué ou substitué par un halogène, un alcoxy inférieur ou un cyano,
R₅ représente un alcanoyloxy inférieur, octanoyloxy, nonanoyloxy, décanoyloxy, dodécanoyloxy, carboxy-alcanoyloxy inférieur, furyl-alcanoyloxy inférieur, imidazolyl-alcanoyloxy inférieur, pyridyl-alcanoyloxy inférieur, quinoléyl-alcanoyloxy inférieur, aminoacétyloxy, N-alkyle inférieur-amino-acétyloxy, N,N-di-alkyle inférieur-amino-acétyloxy, N-alkyle inférieur-N-phényl-alcoxy inférieur-carbonyle-aminoacétyloxy, phényl-alcanoyloxy inférieur, 4-morpholino-alkyle inférieur-benzoyloxy, 4-halogénométhyl-benzoyloxy, histidyloxy ou prolyloxy, et
R₇ a les mêmes significations que définies pour R₃,
ou un de ses sels pharmaceutiquement acceptables, dans la mesure où au moins un groupe salificateur est présent.

4. Composé de formule I selon la revendication 1, dans lequel
R₁ représente un alcoxy inférieur-carbonyle, halogénalcoxy inférieur-carbonyle, phényl-alcoxy inférieur-carbonyle, le radical monovalent, lié par l'intermédiaire du carbonyle, d'un acide aminé aliphatique choisi parmi la valine, l'alanine, la leucine et l'isoleucine ou le radical, lié par l'intermédiaire d'un carbonyle, d'un acide aminé aliphatique acylé par l'un des radicaux phényl-alcanoyle inférieur, morpholinyl-alcanoyle inférieur, thiomorpholinyl-alcanoyle inférieur, pyridyl-alcanoyle inférieur, alcoxy inférieur-carbonyle ou phényl-alcoxy inférieur-carbonyle, tel que défini ci-dessus, tous les acides aminés mentionnés se présentant sous forme (D), (D,L) ou (L),
R₂ représente un hydrogène,
R₃ représente un phényl-alkyle inférieur, 4-fluorophényl-alkyle inférieur ou cyclohexyl-alkyle inférieur,
R₄ représente un hydrogène,
R₅ représente un alcanoyloxy inférieur, octanoyloxy, nonanoyloxy, décanoyloxy, dodécanoyloxy, carboxy-alcanoyloxy inférieur, furyl-alcanoyloxy inférieur, imidazolyl-alcanoyloxy inférieur, pyridyl-alcanoyloxy inférieur, quinoléyl-alcanoyloxy inférieur, aminoacétyloxy (glycyloxy), N-alkyle inférieur-aminoacétyloxy, N,N-di-alkyle inférieur-amino-acétyloxy, N-alkyle inférieur-N-phényl-alcoxy inférieur-carbonyleaminoacétyloxy, phényl-alcanoyloxy inférieur, 4-morpholino-alkyle inférieur-benzoyloxy, 4-halogénométhyl-benzoyloxy, histidyloxy ou prolyloxy, R₈ représente un hydrogène,
R₇ représente un alkyle inférieur, un cyclohexyl-alkyle inférieur, un phényl-alkyle inférieur, 4-cyanophényl-alkyle inférieur, ou 4-fluorophényl-alkyle inférieur,
R₈ représente un hydrogène, et
R₉ représente l'un des radicaux mentionnés ci-dessus pour R₁ , et
les radicaux R₃ et R₅ portent des atomes de carbone asymétriques en configuration S,
ainsi que leurs sels pharmaceutiquement acceptables.

5. Composé de formule I, selon la revendication 1, dans lequel R₁ et R₉ représentent un N-méthoxycarbonylvalyle, R₂, R₄, R₈ et R₈ représentent un hydrogène, R₃ représente un benzyle ou cyclohexylméthyle, R₅ représente un alcanoyloxy inférieur ou un pyridyl-carbonyloxy et R₇ représente un cyclohexylméthyle ou un benzyle, ainsi que leurs sels pharmaceutiquement acceptables.

6. Isomère d'un composé de formule I selon la revendication 5, dans lequel le radical R₃ et l'atome de carbone portant R₅ se présentent sous la configuration (S) et les radicaux R₁ , R₂, R₃, R₄ , R₅ , R₈ ,R₇ , R₈ et R₉ ont les significations mentionnées dans la revendication 6, ou leurs sels pharmaceutiquement acceptables.

7. 1-[2(S)-acétoxy-3(S)-(N-(2-méthoxyéthoxycarbonyl)-(L)-valyl)amino-4-phénylbutyl]-1-[cyclohexylméthyl]-2-[N-(2-méthoxyéthoxycarbonyl)-(L)-valyl]hydrazine selon la revendication 1, ou ses sels pharmaceutiquement acceptables.

8. 1-[2(S)-acétoxy-3(S)-(N-(méthoxycarbonyl)-(L)-valyl)amino-4-phénylbutyl]-1-[cyclohexylméthyl]-2-[N-(méthoxycarbonyl)-(L)-valyl]hydrazine selon la revendication 1, ou ses sels pharmaceutiquement acceptables.

9. 1-[2(S)-(2(pyridylcarbonyl)oxy-3(S)-(N-(méthoxycarbonyl)-(L)-valyl)amino-4-phénylbutyl]-1-[cyclohexylméthyl]-2-[N-(méthoxycarbonyl)-(L)-valyl]-hydrazine selon la revendication 1, ou ses sels pharmaceutiquement acceptables.

10. Composé selon la revendication 1, choisi parmi
1-[2(S)-propionyloxy-3(S)-(N-(méthoxycarbonyl)-(L)-valyl)amino-4-phényl-butyl]-1-[cyclohexylméthyl]-2-[N-(méthoxycarbonyl)-(L)-valyl]hydrazine ;
1-[2(S)-butyryloxy-3(S)-(N-(méthoxycarbonyl)-(L)-valyl)amino-4-phényl-butyl]-1-[cyclohexylméthyl]-2-[N-(méthoxycarbonyl)-(L)-valyl]hydrazine ;
1-[2(S)-pentanoyloxy-3(S)-(N-(méthoxycarbonyl)-(L)-valyl)amino-4-phényl-butyl]-1-[cyclohexylméthyl]-2-[N-(méthoxycarbonyl)-(L)-valyl]hydrazine ;
1-[2(S)-octanoyloxy-3(S)-(N-(méthoxycarbonyl)-(L)-valyl)amino-4-phényl-butyl]-1-[cyclohexylméthyl]-2-(N-(méthoxycarbonyl)-(L)-valyl]hydrazine ;
1-[2(S)-décanoyloxy-3(S)-(N-(méthoxycarbonyl)-(L)-valyl)amino-4-phényl-butyl]-1-[cyclohexylméthyl]-2-[N-(méthoxycarbonyl)-(L)-valyl]hydrazine ;
1-[2(S)-dodécanoyloxy-3(S)-(N-(méthoxycarbonyl)-(L)-valyl)amino-4-phényl-butyl]-1-[cyclohexylméthyl]-2-[N-(méthoxycarbonyl)-(L)-valyl]hydrazine ;
1-[2(S)-pivaloyloxy-3(S)-(N-(méthoxycarbonyl)-(L)-valyl)amino-4-phényl-butyl]-1-[cyclohexylméthyl]-2-[N-(méthoxycarbonyl)-(L)-valyl]hydrazine ;
1-[2(S)-(2-furylcarbonyl)oxy-3(S)-(N-(méthoxycarbonyl)-(L)-valyl)amino-4-phényl-butyl]-1-[cyclohexylméthyl]-2-[N-(méthoxycarbonyl)-(L)-valyl]hydrazine ;
1-[2(S)-(4-imidazolylcarbonyl)oxy-3(S)-(N-(méthoxycarbonyl)-(L)-valyl)amino-4-phényl-butyl]-1-[cyclohexylméthyl]-2-[N-(méthoxycarbonyl)-(L)-valyl]hydrazine ;
1-[2(S)-(4-imidazolylacétyl)oxy-3(S)-(N-(méthoxycarbonyl)-(L)-valyl)amino-4-phényl-butyl]-1-[cyclohexylméthyl]-2-(N-(méthoxycarbonyl)-(L)-valyl]hydrazine ;
1-[2(S)-(3-(4-imidazolyl)-propionyl)-oxy-3(S)-(N-(méthoxycarbonyl)-(L)-valyl)amino-4-phényl-butyl]-1-[cyclohexylméthyl]-2-[N-(méthoxycarbonyl)-(L)-valyl]hydrazine ;
1-[2(S)-benzoyloxy-3(S)-(N-(méthoxycarbonyl)-(L)-valyl)amino-4-phényl-butyl]-1-[cyclohexylméthyl]-2-[N-(méthoxycarbonyl)-(L)-valyl]hydrazine ;
1-[2(S)-(2-pyridylacétyl)oxy-3(S)-(N-(méthoxycarbonyl)-(L)-valyl)amino-4-phényl-butyl]-1-[cyclohexylméthyl]-2-[N-(méthoxycarbonyl)-(L)-valyl]hydrazine ;
1-[2(S)-(3-(pyridin-2-yl)-propionyl)oxy-3(S)-(N-(méthoxycarbonyl)-(L)-valyl)amino-4-phényl-butyl]-1-[cyclohexylméthyl]-2-[N-(méthoxycarbonyl)-(L)-valyllhydrazine ;
1-[2(S)-(quinoléine-2-ylcarbonyl)oxy-3(S)-(N-(méthoxycarbonyl)-(L)-valyl)amino-4-phényl-butyl]-1-[cyclohexylméthyl]-2-[N-(méthoxycarbonyl)-(L)-valyl]hydrazine ;
1-[2(S)-(aminoacétyl)oxy-3(S)-(N-(méthoxycarbonyl)-(L)-valyl)amino-4-phényl-butyl]-1-[cyclohexylméthyl]-2-[N-(méthoxycarbonyl)-(L)-valyl]hydrazine ;
1-[2(S)-(N-méthylaminoacétyl)oxy-3(S)-(N-(méthoxycarbonyl)-(L)-valyl)amino-4-phényl-butyl]-1-[cyclohexylméthyl]-2-[N-(méthoxycarbonyl)-(L)-valyl]hydrazine ;
1-[2(S)-(N,N-diméthylaminoacétyl)oxy-3(S)-(N-(méthoxycarbonyl)-(L)-valyl)amino-4-phényl-butyl]-1-[cyclohexylméthyl]-2-[N-(méthoxycarbonyl)-(L)-valyl]hydrazine ;
1-[2(S)-(N-benzyloxycarbonyl-N-méthylaminoacétyl)oxy-3(S)-(N-(méthoxycarbonyl)-(L)-valyl)amino-4-phényl-butyl]-1-[cyclohexylméthyl]-2-[N-(méthoxycarbonyl)-(L)-valyl]hydrazine ;
1-[2(S)-prolyloxy-3(S)-(N-(méthoxycarbonyl)-(L)-valyl)amino-4-phényl-butyl]-1-[cyclohexylméthyl]-2-[N-(méthoxycarbonyl)-(L)-valyl]hydrazine ;
1-[2(S)-(4-morpholinométhylbenzoyl)oxy-3(S)-(N-(méthoxycarbonyl)-(L)-valyl)amino-4-phényl-butyl]-1-[cyclohexylméthyl]-2-[N-(méthoxycarbonyl)-(L)-valyl]hydrazine ;
1-[2(S)-(4-chlorométhylbenzoyl)oxy-3(S)-(N-(méthoxycarbonyl)-(L)-valyl)amino-4-phényl-butyl]-1-[cyclohexylméthyl]-2-[N-(méthoxycarbonyl)-(L)-valyl]hydrazine ; et
1-[2(S)-(3-carboxypropionyl)oxy-3(S)-(N-(méthoxycarbonyl)-(L)-valyl)amino-4-phényl-butyl]-1-[cyclohexylméthyl]-2-[N-(méthoxycarbonyl)-(L)-valyl]hydrazine ou leurs sels pharmaceutiquement acceptables.

11. 1-[2(S)-butyryloxy-3(S)-(N-(méthoxycarbonyl)-(L)-valyl)amino-4-phényl-butyl]-1-[cyclohexylméthyl]-2-[N-méthoxycarbonyl-(L)-valyl]hydrazine de formule I selon la revendication 1, ou un de ses sels pharmaceutiquement acceptables.

12. 1-[2(S)-(méthoxy-acétoxy)-3(S)-(N-(méthoxycarbonyl)-(L)-valyl)amino-4-phénylbutyl]-1-[cyclohexylméthyl]-2-[N-méthoxycarbonyl-(L)-valyl]-hydrazine de formule I selon la revendication 1 ou un de ses sels pharmaceutiquement acceptables.

13. Composé de formule I ou un de ses sels pharmaceutiquement acceptables selon l'une des revendications 1 à 12 aux fins d'application dans un procédé de traitement diagnostique ou thérapeutique du corps humain ou animal.

14. Application d'un composé de formule I selon l'une des revendications 1 à 12, ou d'un de ses sels pharmaceutiquement acceptables, à la préparation de préparations pharmaceutiques pour le traitement du SIDA.

15. Préparation pharmaceutique contenant des composés de formule I selon l'un des revendications 1 à 12 ou un sel pharmaceutiquement acceptable d'un tel composé ayant au moins un groupe salificateur, avec un support pharmaceutiquement acceptable.

16. Procédé de préparation de composés de formule I selon la revendication, caractérisé en ce que ou bien
a) on acyle un composé hydroxy de formule II, 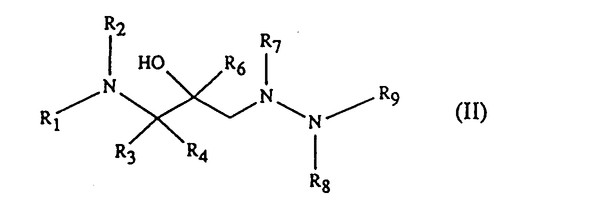 dans laquelle les radicaux R₁ à R₄ et R₈ à R₉ ont les significations données pour les composés de formule I, avec un acide carboxylique de formule III
R₅ -H (III)
ou un de ses dérivés acides réactifs, où R₅ a les significations données pour la formule I, les groupes fonctionnels dans les produits de départ de formules II et III qui ne doivent pas participer à la réaction se présentant si nécessaire sous forme protégée, et on sépare les groupes protecteurs présents, ou bien
b) pour préparer des composés de formule I dans laquelle R₉ représente un acyle, un sulfo ou un sulfonyle non substitué ou substitué par un alkyle substitué, un aryle ou un hétérocyclyle, et les autres radicaux R₁ à R₈ ont les significations données pour les composés de formule I, on condense un composé amino de formule 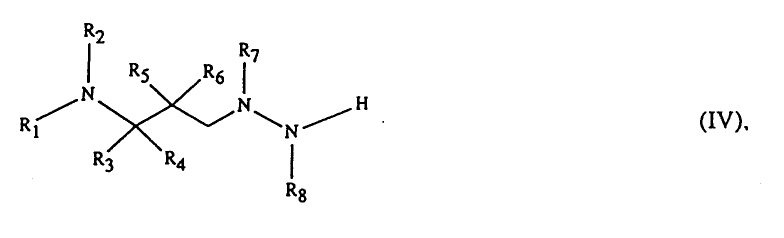 dans laquelle les radicau R₁ à R₈ ont les significations données pour les composés de formule I, avec un acide de formule
R₉ '-OH (V)
ou un de ses dérivés acides réactifs, où R₉' a les significations données sous b) pour R₉ en dehors de l'hydrogène et comporte un alkyle non substitué ou substitué, les groupes fonctionnels libres à l'exception de ceux qui participent à la réaction se présentant si nécessaire sous forme protégée, et on sépare les groupes protecteurs présents, ou bien
c) pour préparer des composés de formule I, dans laquelle R₁ représente un acyle, un sulfo ou un sulfonyle non substitué ou substitué par un alkyle substitué, un aryle ou un hétérocyclyle, et les autres radicaux R₂ à R₉ ont les significations données pour les composés de formule I, on condense un composé amino de formule 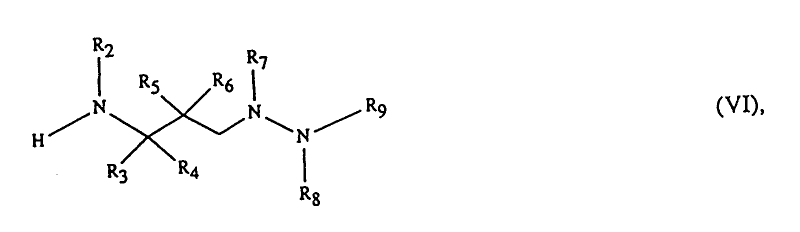 dans laquelle les radicaux R₂ à R₉ ont les significations données pour les composés de formule I, avec un acide de formule
R₁'-OH (VII)
ou un de ses dérivés acides réactifs, où R₁ a les significations données en c) ci-dessus pour R₁ en dehors de l'hydrogène et comporte un alkyle non substitué ou substitué, les groupes fonctionnels, à l'exception de ceux qui participent à la réaction, se présentant si nécessaire sous forme protégée, et on sépare les groupes protecteurs présents, ou bien
d) pour préparer des composés de formule I, dans laquelle R₁ et R₉ représentent deux radicaux identiques choisis parmi acyle, sulfo et sulfonyle non substitué ou substitué par un alkyle substitué, un aryle ou un hétérocyclyle, et les autres radicaux R₈ et R₉ ont les significations mentionnées pour les composés de formule I, on condense un composé diamino de formule 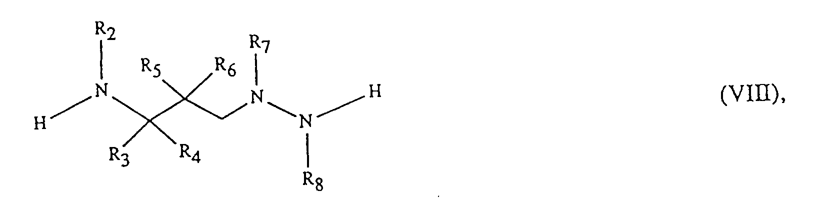 dans laquelle les radicaux R₂ à R₈ ont les significations données pour les composés de formule I, avec un acide approprié pour l'introduction des radicaux identiques R₁ et R₉ on un de ses dérivés acides réactifs, R₁ et R₉ ayant les significations déjà données, et les groupes fonctionnels libres, à l'exception de ceux qui participent à la réaction, se présentant si nécessaire sous forme protégée, et on sépare les groupes protecteurs présents, ou bien
e) pour préparer un composé de formule I, dans laquelle R₇ représente un alkyle ou cycloalkyle non substitué ou substitué et les autres radicaux R₁ à R₆ , R₈ et R₉ ont les significations données pour les composés de formule I, on introduit le radical R₇ dans un composé de formule I', 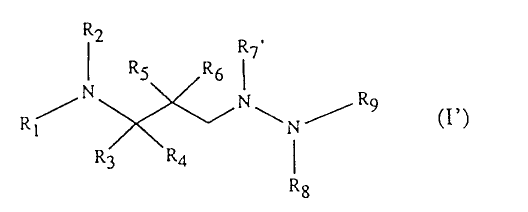 dans laquelle R₇' représente un hydrogène, et les autres radicaux R₁ à R₆ , R₈ et R₉ ont les significations données pour les composés de formule I, par substitution avec un composé de formule XII
R₇ - X (XII)
dans laquelle X est groupe sortant et R₇ représente un alkyle ou cycloalkyle non substitué ou substitué, les groupes fonctionnels libres, à l'exception de ceux qui participent à la réaction, se présentant si nécessaire sous forme protégée, et le cas échant, on sépare les groupes protecteurs présents, et
si on le désire, en tant que mesure supplémentaire à prendre pour la réalisation du procédé, on transforme un composé de formule I obtenu selon l'une des étapes a) à e) ci-dessus et ayant au moins un groupe salificateur en son sel, ou un sel obtenu en le composé libre ou en un autre sel, et/ou si on le désire, on sépare les mélanges d'isomères obtenus et/ou transforme un composé de formule I selon l'invention en un autre composé de formule I selon l'invention.

17. Composé de formule II présenté dans la revendication 16, choisi parmi les composés suivants :
1-[2(S)-hydroxy-3(S)-(N-(méthoxycarbonyl)-(L)-valyl)-amino-4-phényl-butyl]-1-[cyclohexylméthyl]-2-[N-(méthoxycarbonyl)-(L)-valyl]-hydrazine,
1-[2(S)-hydroxy-3(S)-(N-(méthoxycarbonyl)-(L)-valyl)-amino-4-phénylbutyl]-1-[thién-2-ylméthyl]-2-[N-(méthoxycarbonyl)-(L)-valyl]-hydrazine,
1-[2(S)-hydroxy-3(S)-(N-(quinoléine-2-carbonyl)-(L)-asparaginyl)amino-4-phénylbutyl]-1-[4-méthoxyphénylméthyl]-2-[N-benzyloxy-carbonyl)-(L)-valyl]-hydrazine,
1-[2(S)-hydroxy-3(S)-(N-méthoxycarbonyl)-(L)-valyl)amino-4-phénylbutyl]-1-[4-méthoxyphénylméthyl]-2-[N-méthoxycarbonyl -(L)-valyl]-hydrazine,
1-[2(S)-hydroxy-3(S)-(N-(méthoxycarbonyl)-(L)-valyl)-amino-4-phénylbutyl]-1-[4-biphénylylméthyl]-2-[N-(méthoxycarbonyl)-(L)(valyl]-hydrazine,
1-[2(S)-hydroxy-3(S)-(N-(éthoxycarbonyl)-(L)-valyl)-amino-4-phénylbutyl]-1-[4-biphénylylméthyl]-2-[N-(éthoxycarbonyl)-(L)-valyl]-hydrazine,
1-[2(S)-hydroxy-3(S)-(N-(quinoléine-2-carbonyl)-(L)-asparaginyl)-amino-4-phénylbutyl]-1-[benzyl]-2-[N-(benzyloxycarbonyl) -(L)-valyl]-hydrazine, et
1-[2(S)-hydroxy-3(S)-(N-acétyl-(L)-valyl)-amino-4-phénylbutyl]-1-[4-biphénylylméthyl]-2-[N-acétyl-L)-valyl]hydrazine,
ou un de ses sels pharmaceutiquement acceptables.

18. Le composé de formule II présenté dans la revendication 16, qui est la 1-[2(S)-hydroxy-3(S)-(N-(méthoxycarbonyl)-L)-valyl)-amino-4-phényl-butyl]-1-[cyclohexylméthyl]-2-[N-(méthoxycarbonyl)-(L)-valyl]-hydrazine, ou un de ses sels pharmaceutiquement acceptables.

19. Composé de formule II présenté dans la revendication 16, qui est la 1-[2(S)-hydroxy-3(S)-(N-(méthoxycarbonyl)-(L)-valyl)-amino-4-phényl-butyl]-1-[4-biphénylylméthyl]-2-[N-(méthoxycarbonyl)-(L)-valyl]-hydrazine ou un de ses sels pharmaceutiquement acceptables.
